(19) Europäisches Patentamt
European Patent Office
Office européen des brevets

(11) **EP 3 383 887 B1**

(12) **FASCICULE DE BREVET EUROPEEN**

(45) Date de publication et mention de la délivrance du brevet:
**16.03.2022 Bulletin 2022/11**

(21) Numéro de dépôt: **16815613.1**

(22) Date de dépôt: **29.11.2016**

(51) Classification Internationale des Brevets (IPC):
**C07K 14/08** (2006.01)  **G01N 33/576** (2006.01)
**C07K 14/005** (2006.01)

(52) Classification Coopérative des Brevets (CPC):
**C07K 14/005;** C12N 2770/28122

(86) Numéro de dépôt international:
**PCT/FR2016/053127**

(87) Numéro de publication internationale:
**WO 2017/093649 (08.06.2017 Gazette 2017/23)**

(54) **POLYPEPTIDES MUTÉS DE HEV ET LEUR UTILISATION POUR LE DOSAGE D'ANTICORPS ANTI-HEV**

MUTIERTE HEV-POLYPEPTIDE UND VERWENDUNG DAVON ZUM TESTEN VON ANTI-HEV-ANTIKÖRPERN

MUTATED HEV POLYPEPTIDES AND THE USE THEREOF FOR ASSAYING ANTI-HEV ANTIBODIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.11.2015 FR 1561596**

(43) Date de publication de la demande:
**10.10.2018 Bulletin 2018/41**

(73) Titulaire: **Biomérieux**
**69280 Marcy l'Étoile (FR)**

(72) Inventeurs:
• **ATAMAN-ONAL, Yasemin**
**01600 Reyrieux (FR)**
• **DANIEL, Soizic**
**01600 Trevoux (FR)**
• **GOUTAGNY, Nadège**
**69160 Tassin La Demi Lune (FR)**
• **LUCIANI, Françoise**
**69380 Saint-jean des Vignes (FR)**

(56) Documents cités:
**EP-A2- 2 298 793**

• **COURSAGET P ET AL: "Mapping of linear B cell epitopes on open reading frames 2- and 3-encoded proteins of hepatitis E virus using synthetic peptides", FEMS MICROBIOLOGY LETTERS, WILEY-BLACKWELL PUBLISHING LTD, GB, vol. 109, no. 2-3, 15 mai 1993 (1993-05-15), pages 251-255, XP023897873, ISSN: 0378-1097, DOI: 10.1111/J.1574-6968.1993.TB06176.X [extrait le 1993-05-15]**
• **YURY E. KHUDYAKOV ET AL: "Antigenic Domains of the Open Reading Frame 2-Encoded Protein of Hepatitis E Virus", JOURNAL OF CLINICAL MICROBIOLOGY, vol. 37, no. 9, septembre 1999 (1999-09), pages 2863-2871, XP055263906, US ISSN: 0095-1137**
• **RIDDELL M A ET AL: "IDENTIFICATION OF IMMUNODOMINANT AND CONFORMATIONAL EPITOPES IN THECAPSID PROTEIN OF HEPATITIS E VIRUS BY USING MONOCLONAL ANTIBODIES", JOURNAL OF VIROLOGY, THE AMERICAN SOCIETY FOR MICROBIOLOGY, US, vol. 74, no. 17, septembre 2000 (2000-09), pages 8011-8017, XP000942369, ISSN: 0022-538X, DOI: 10.1128/JVI.74.17.8011-8017.2000**

EP 3 383 887 B1

**(Cont. page suivante)**

• LI TIAN-CHENG ET AL: "Essential elements of the capsid protein for self-assembly into empty virus-like particles of hepatitis E virus", JOURNAL OF VIROLOGY, THE AMERICAN SOCIETY FOR MICROBIOLOGY, US, vol. 79, no. 20, octobre 2005 (2005-10), pages 12999-13006, XP007916926, ISSN: 0022-538X, DOI: 10.1128/JVI.79.20.12999?13006.2005

SEQUENCE LISTING

<110> bioMérieux

<120> Polypeptides mutés de HEV et leur utilisation pour le dosage d'anticorps anti-HEV

<130> Mutacys

<150> FR1561596
<151> 2015-11-30

<160> 50

<170> PatentIn version 3.5

<210> 1
<211> 674
<212> PRT
<213> Hepatitis E virus

<400> 1

```
Met Asn Asn Met Phe Phe Cys Ser Val His Gly Asp Ala Thr Met Arg
1               5               10              15

Ser Arg Ala Phe Leu Phe Leu Phe Leu Val Leu Leu Pro Met Leu Pro
            20              25              30

Ala Pro Pro Ala Gly Gln Pro Ser Gly Arg Arg Arg Gly Arg Arg Ser
        35              40              45

Gly Gly Ala Gly Gly Gly Phe Trp Gly Asp Arg Val Asp Ser Gln Pro
    50              55              60

Phe Ala Leu Pro Tyr Ile His Pro Thr Asn Pro Phe Ala Ser Asp Ile
65              70              75              80

Pro Ala Ala Ala Gly Ala Gly Ala Arg Pro Arg Gln Pro Ala Arg Pro
            85              90              95

Leu Gly Ser Ala Trp Arg Asp Gln Ser Gln Arg Pro Ala Thr Ser Ala
        100             105             110

Arg Arg Arg Ser Ala Pro Ala Gly Ala Ser Pro Leu Thr Ala Val Ala
        115             120             125

Pro Ala Pro Asp Thr Ala Pro Val Pro Asp Val Asp Ser Arg Gly Ala
    130             135             140

Ile Leu Arg Arg Gln Tyr Asn Leu Ser Thr Ser Pro Leu Thr Ser Thr
145             150             155             160
```

```
Ile Ala Thr Gly Thr Asn Leu Val Leu Tyr Ala Ala Pro Leu Ser Pro
             165             170             175

Leu Leu Pro Leu Gln Asp Gly Thr Asn Thr His Ile Met Ala Thr Glu
             180             185             190

Ala Ser Asn Tyr Ala Gln Tyr Arg Val Val Arg Ala Thr Ile Arg Tyr
             195             200             205

Arg Pro Leu Val Pro Asn Ala Val Gly Gly Tyr Ala Ile Ser Ile Ser
    210             215             220

Phe Trp Pro Gln Thr Thr Thr Thr Pro Thr Ser Val Asp Met Asn Ser
225             230             235             240

Ile Thr Ser Thr Asp Val Arg Ile Leu Val Gln Pro Gly Ile Ala Ser
             245             250             255

Glu Leu Val Ile Pro Ser Glu Arg Leu His Tyr Arg Asn Gln Gly Trp
             260             265             270

Arg Ser Val Glu Thr Ser Gly Val Ala Glu Glu Glu Ala Thr Ser Gly
             275             280             285

Leu Val Met Leu Cys Ile His Gly Ser Pro Val Asn Ser Tyr Thr Asn
             290             295             300

Thr Pro Tyr Thr Gly Ala Leu Gly Leu Leu Asp Phe Ala Leu Glu Leu
305             310             315             320

Glu Phe Arg Asn Leu Thr Pro Gly Asn Thr Asn Thr Arg Val Ser Arg
             325             330             335

Tyr Ser Ser Ser Ala Arg His Lys Leu Arg Arg Gly Pro Asp Gly Thr
             340             345             350

Ala Glu Leu Thr Thr Thr Ala Ala Thr Arg Phe Met Lys Asp Leu His
             355             360             365

Phe Thr Gly Thr Asn Gly Val Gly Glu Val Gly Arg Gly Ile Ala Leu
             370             375             380

Thr Leu Phe Asn Leu Ala Asp Thr Leu Leu Gly Gly Leu Pro Thr Glu
385             390             395             400

Leu Ile Ser Ser Ala Gly Gly Gln Leu Phe Tyr Ser Arg Pro Val Val
             405             410             415
```

```
Ser Ala Asn Gly Glu Pro Thr Val Lys Leu Tyr Thr Ser Val Glu Asn
            420         425             430

Ala Gln Gln Asp Lys Gly Ile Ala Ile Pro His Asp Ile Asp Leu Gly
            435         440             445

Glu Ser Arg Val Val Ile Gln Asp Tyr Asp Asn Gln His Glu Gln Asp
            450         455             460

Arg Pro Thr Pro Ser Pro Ala Pro Ser Arg Pro Phe Ser Val Leu Arg
465         470             475             480

Ala Asn Asp Val Leu Trp Leu Ser Leu Thr Ala Ala Glu Tyr Asp Gln
            485             490             495

Thr Thr Tyr Gly Ser Ser Thr Asn Pro Met Tyr Val Ser Asp Thr Val
            500             505             510

Thr Phe Val Asn Val Ala Thr Gly Ala Gln Gly Val Ser Arg Ser Leu
            515             520             525

Asp Trp Ser Lys Val Thr Leu Asp Gly Arg Pro Leu Met Thr Ile Gln
    530             535             540

Gln Tyr Ser Lys Thr Phe Phe Val Leu Pro Leu Arg Gly Lys Leu Ser
545             550             555             560

Phe Trp Glu Ala Gly Thr Thr Lys Ala Gly Tyr Pro Tyr Asn Tyr Asn
                565             570             575

Thr Thr Ala Ser Asp Gln Ile Leu Ile Glu Asn Ala Ala Gly His Arg
            580             585             590

Val Cys Ile Ser Thr Tyr Thr Thr Asn Leu Gly Ser Gly Pro Val Ser
            595             600             605

Ile Ser Ala Val Gly Val Leu Ala Pro His Ser Ala Leu Ala Ala Leu
    610             615             620

Glu Asp Thr Val Asp Tyr Pro Ala Arg Ala His Thr Phe Asp Asp Phe
625             630             635             640

Cys Pro Glu Cys Arg Ala Leu Gly Leu Gln Gly Cys Ala Phe Gln Ser
                645             650             655

Thr Val Ala Glu Leu Gln Arg Leu Lys Met Lys Val Gly Lys Thr Arg
            660             665             670
```

25

Glu Tyr

<210> 2
<211> 674
<212> PRT
<213> Hepatitis E virus

<400> 2

```
Met Asn Asn Met Phe Phe Cys Ser Val His Gly Asp Ala Thr Met Arg
1               5                   10                  15

Ser Arg Ala Leu Leu Phe Leu Leu Phe Val Leu Leu Pro Met Leu Pro
                20                  25                  30

Ala Pro Pro Ala Gly Gln Pro Ser Gly Arg Arg Arg Gly Arg Arg Ser
            35                  40                  45

Gly Gly Ala Gly Gly Gly Phe Trp Gly Asp Arg Val Asp Ser Gln Pro
    50                  55                  60

Phe Ala Leu Pro Tyr Ile His Pro Thr Asn Pro Phe Ala Ser Asp Ile
65                  70                  75                  80

Pro Thr Ala Ala Gly Ser Gly Ala Arg Pro Arg Gln Pro Ala Arg Pro
                85                  90                  95

Leu Gly Ser Ala Trp Arg Asp Gln Ser Gln Arg Pro Ala Ala Pro Ala
            100                 105                 110

Arg Arg Arg Ser Ala Pro Ala Gly Ala Ser Pro Leu Thr Ala Val Ala
            115                 120                 125

Pro Ala Pro Asp Thr Ala Pro Val Pro Asp Val Asp Ser Arg Gly Ala
    130                 135                 140

Ile Leu Arg Arg Gln Tyr Asn Leu Ser Thr Ser Pro Leu Thr Ser Thr
145                 150                 155                 160

Ile Ala Thr Gly Thr Asn Leu Val Leu Tyr Ala Ala Pro Leu Ser Pro
                165                 170                 175

Leu Leu Pro Leu Gln Asp Gly Thr Asn Thr His Ile Met Ala Thr Glu
            180                 185                 190

Ala Ser Asn Tyr Ala Gln Tyr Arg Val Val Arg Ala Thr Ile Arg Tyr
            195                 200                 205
```

Arg Pro Leu Val Pro Asn Ala Val Gly Gly Tyr Ala Ile Ser Ile Ser
    210             215             220

Phe Trp Pro Gln Thr Thr Thr Thr Pro Thr Ser Val Asp Met Asn Ser
225             230             235             240

Ile Thr Ser Thr Asp Val Arg Ile Leu Val Gln Pro Gly Ile Ala Ser
            245             250             255

Glu Leu Val Ile Pro Ser Glu Arg Leu His Tyr Arg Asn Gln Gly Trp
            260             265             270

Arg Ser Val Glu Thr Ser Gly Val Ala Glu Glu Glu Ala Thr Ser Gly
            275             280             285

Leu Val Met Leu Cys Ile His Gly Ser Pro Val Asn Ser Tyr Thr Asn
    290             295             300

Thr Pro Tyr Thr Gly Ala Leu Gly Leu Leu Asp Phe Ala Leu Glu Leu
305             310             315             320

Glu Phe Arg Asn Leu Thr Pro Gly Asn Thr Asn Thr Arg Val Ser Arg
            325             330             335

Tyr Ser Ser Ser Ala Arg His Lys Leu Arg Arg Gly Pro Asp Gly Thr
            340             345             350

Ala Glu Leu Thr Thr Thr Ala Ala Thr Arg Phe Met Lys Asp Leu His
    355             360             365

Phe Thr Gly Thr Asn Gly Val Gly Glu Val Gly Arg Gly Ile Ala Leu
    370             375             380

Thr Leu Phe Asn Leu Ala Asp Thr Leu Leu Gly Gly Leu Pro Thr Glu
385             390             395             400

Leu Ile Ser Ser Ala Gly Gly Gln Leu Phe Tyr Ser Arg Pro Val Val
            405             410             415

Ser Ala Asn Gly Glu Pro Thr Val Lys Leu Tyr Thr Ser Val Glu Asn
            420             425             430

Ala Gln Gln Asp Lys Gly Ile Ala Ile Pro His Asp Ile Asp Leu Gly
    435             440             445

Glu Ser Arg Val Val Ile Gln Asp Tyr Asp Asn Gln His Glu Gln Asp

27

450 455 460

Arg Pro Thr Pro Ser Pro Ala Pro Ser Arg Pro Phe Ser Val Leu Arg
465                 470                 475                 480

Ala Asn Asp Val Leu Trp Leu Ser Leu Thr Ala Ala Glu Tyr Asp Gln
                485                 490                 495

Thr Thr Tyr Gly Ser Ser Thr Asn Pro Met Tyr Val Ser Asp Thr Val
            500                 505                 510

Thr Phe Val Asn Val Ala Thr Gly Ala Gln Gly Val Ser Arg Ser Leu
            515                 520                 525

Asp Trp Ser Lys Val Thr Leu Asp Gly Arg Pro Leu Thr Thr Ile Gln
        530                 535                 540

Gln Tyr Ser Lys Thr Phe Phe Val Leu Pro Leu Arg Gly Lys Leu Ser
545                 550                 555                 560

Phe Trp Glu Ala Gly Thr Thr Lys Ala Gly Tyr Pro Tyr Asn Tyr Asn
                565                 570                 575

Thr Thr Ala Ser Asp Gln Ile Leu Ile Glu Asn Ala Ala Gly His Arg
            580                 585                 590

Val Cys Ile Ser Thr Tyr Thr Thr Asn Leu Gly Ser Gly Pro Val Ser
            595                 600                 605

Ile Ser Ser Val Gly Val Leu Ala Pro His Ser Ala Leu Ala Ala Leu
    610                 615                 620

Glu Asp Thr Val Asp Tyr Pro Ala Arg Ala His Thr Phe Asp Asp Phe
625                 630                 635                 640

Cys Pro Glu Cys Arg Thr Leu Gly Leu Gln Gly Cys Ala Phe Gln Ser
                645                 650                 655

Thr Val Ala Glu Leu Gln Arg Leu Lys Met Lys Val Gly Lys Thr Arg
            660                 665                 670

Glu Tyr

<210> 3
<211> 674
<212> PRT
<213> Hepatitis E virus

28

<400> 3

```
Met Asn Asn Met Phe Phe Cys Ser Val His Gly Asp Ala Thr Met Arg
1               5               10              15

Ser Arg Ala Leu Leu Phe Leu Leu Phe Val Leu Leu Pro Met Leu Pro
        20              25              30

Ala Pro Pro Ala Gly Gln Pro Ser Gly Arg Arg Arg Gly Arg Arg Ser
        35              40              45

Gly Gly Ala Gly Gly Gly Phe Trp Gly Asp Arg Val Asp Ser Gln Pro
    50              55              60

Phe Ala Leu Pro Tyr Ile His Pro Thr Asn Pro Phe Ala Ser Asp Ile
65              70              75              80

Pro Thr Ala Ala Gly Ser Gly Ala Arg Pro Arg Gln Pro Ala Arg Pro
            85              90              95

Leu Gly Ser Ala Trp Arg Asp Gln Ser Gln Arg Pro Ala Ala Ser Ala
            100             105             110

Arg Arg Arg Ser Ala Pro Ala Gly Ala Ser Pro Leu Thr Ala Val Ala
        115             120             125

Pro Ala Pro Asp Thr Ala Pro Val Pro Asp Val Asp Ser Arg Gly Ala
    130             135             140

Ile Leu Arg Arg Gln Tyr Asn Leu Ser Thr Ser Pro Leu Thr Ser Thr
145             150             155             160

Ile Ala Thr Gly Thr Asn Leu Val Leu Tyr Ala Ala Pro Leu Ser Pro
            165             170             175

Leu Leu Pro Leu Gln Asp Gly Thr Asn Thr His Ile Met Ala Thr Glu
            180             185             190

Ala Ser Asn Tyr Ala Gln Tyr Arg Val Val Arg Ala Thr Ile Arg Tyr
        195             200             205

Arg Pro Leu Val Pro Asn Ala Val Gly Gly Tyr Ala Ile Ser Ile Ser
    210             215             220

Phe Trp Pro Gln Thr Thr Thr Thr Pro Thr Ser Val Asp Met Asn Ser
225             230             235             240
```

```
Ile Thr Ser Thr Asp Val Arg Ile Leu Val Gln Pro Gly Ile Ala Ser
            245             250             255

Glu Leu Val Ile Pro Ser Glu Arg Leu His Tyr Arg Asn Gln Gly Trp
            260             265             270

Arg Ser Val Glu Thr Ser Gly Val Ala Glu Glu Glu Ala Thr Ser Gly
            275             280             285

Leu Val Met Leu Cys Ile His Gly Ser Pro Val Asn Ser Tyr Thr Asn
        290             295             300

Thr Pro Tyr Thr Gly Ala Leu Gly Leu Leu Asp Phe Ala Leu Glu Leu
305             310             315             320

Glu Phe Arg Asn Leu Thr Pro Gly Asn Thr Asn Thr Arg Val Ser Arg
            325             330             335

Tyr Ser Ser Ser Ala Arg His Lys Leu Arg Arg Gly Pro Asp Gly Thr
            340             345             350

Ala Glu Leu Thr Thr Thr Ala Ala Thr Arg Phe Met Lys Asp Leu His
        355             360             365

Phe Thr Gly Thr Asn Gly Val Gly Glu Val Gly Arg Gly Ile Ala Leu
        370             375             380

Thr Leu Phe Asn Leu Ala Asp Thr Leu Leu Gly Gly Leu Pro Thr Glu
385             390             395             400

Leu Ile Ser Ser Ala Gly Gly Gln Leu Phe Tyr Ser Arg Pro Val Val
            405             410             415

Ser Ala Asn Gly Glu Pro Thr Val Lys Leu Tyr Thr Ser Val Glu Asn
            420             425             430

Ala Gln Gln Asp Lys Gly Ile Ala Ile Pro His Asp Ile Asp Leu Gly
            435             440             445

Glu Ser Arg Val Val Ile Gln Asp Tyr Asp Asn Gln His Glu Gln Asp
        450             455             460

Arg Pro Thr Pro Ser Pro Ala Pro Ser Arg Pro Phe Ser Val Leu Arg
465             470             475             480

Ala Asn Asp Val Leu Trp Leu Ser Leu Thr Ala Ala Glu Tyr Asp Gln
            485             490             495
```

```
Thr Thr Tyr Gly Ser Ser Thr Asn Pro Met Tyr Val Ser Asp Thr Val
            500             505             510

Thr Phe Val Asn Val Ala Thr Gly Ala Gln Gly Val Ser Arg Ser Leu
            515             520             525

Asp Trp Ser Lys Val Thr Leu Asp Gly Arg Pro Leu Thr Thr Ile Gln
            530             535             540

Gln Tyr Ser Lys Thr Phe Phe Val Leu Pro Leu Arg Gly Lys Leu Ser
545             550             555             560

Phe Trp Glu Ala Gly Thr Thr Lys Ala Gly Tyr Pro Tyr Asn Tyr Asn
            565             570             575

Thr Thr Ala Ser Asp Gln Ile Leu Ile Glu Asn Ala Ala Gly His Arg
            580             585             590

Val Cys Ile Ser Thr Tyr Thr Thr Asn Leu Gly Ser Gly Pro Val Ser
            595             600             605

Ile Ser Ser Val Gly Val Leu Ala Pro His Ser Ala Leu Ala Ala Leu
            610             615             620

Glu Asp Thr Val Asp Tyr Pro Ala Arg Ala His Thr Phe Asp Asp Phe
625             630             635             640

Cys Pro Glu Cys Arg Thr Leu Gly Leu Gln Gly Cys Ala Phe Gln Ser
            645             650             655

Thr Val Ala Glu Leu Gln Arg Leu Lys Met Lys Val Gly Lys Thr Arg
            660             665             670

Glu Tyr
```

<210> 4
<211> 674
<212> PRT
<213> Hepatitis E virus

<400> 4

```
Met Asn Asn Met Phe Phe Cys Ser Val His Gly Asp Ala Thr Met Arg
1               5               10              15

Ser Arg Ala Phe Leu Phe Leu Phe Leu Val Leu Leu Pro Met Leu Pro
            20              25              30
```

31

```
Ala Pro Pro Ala Gly Gln Pro Ser Gly Arg Arg Arg Gly Arg Arg Ser
        35              40              45

Gly Gly Ala Gly Gly Gly Phe Trp Gly Asp Arg Val Asp Ser Gln Pro
        50              55              60

Phe Ala Leu Pro Tyr Ile His Pro Thr Asn Pro Phe Ala Ser Asp Ile
65              70              75              80

Pro Ala Ala Ala Gly Ala Gly Ala Arg Pro Arg Gln Pro Ala Arg Pro
                85              90              95

Leu Gly Ser Ala Trp Arg Asp Gln Ser Gln Arg Pro Ala Thr Ser Ala
        100             105             110

Arg Arg Arg Ser Ala Pro Ala Gly Ala Ser Pro Leu Thr Ala Val Ala
        115             120             125

Pro Ala Pro Asp Thr Ala Pro Val Pro Asp Val Asp Ser Arg Gly Ala
        130             135             140

Ile Leu Arg Arg Gln Tyr Asn Leu Ser Thr Ser Pro Leu Thr Ser Thr
145             150             155             160

Ile Ala Thr Gly Thr Asn Leu Val Leu Tyr Ala Ala Pro Leu Ser Pro
                165             170             175

Leu Leu Pro Leu Gln Asp Gly Thr Asn Thr His Ile Met Ala Thr Glu
        180             185             190

Ala Ser Asn Tyr Ala Gln Tyr Arg Val Val Arg Ala Thr Ile Arg Tyr
        195             200             205

Arg Pro Leu Val Pro Asn Ala Val Gly Gly Tyr Ala Ile Ser Ile Ser
210             215             220

Phe Trp Pro Gln Thr Thr Thr Thr Pro Thr Ser Val Asp Met Asn Ser
225             230             235             240

Ile Thr Ser Thr Asp Val Arg Ile Leu Val Gln Pro Gly Ile Ala Ser
                245             250             255

Glu Leu Val Ile Pro Ser Glu Arg Leu His Tyr Arg Asn Gln Gly Trp
        260             265             270

Arg Ser Val Glu Thr Ser Gly Val Ala Glu Glu Glu Ala Thr Ser Gly
        275             280             285
```

```
Leu Val Met Leu Cys Ile His Gly Ser Pro Val Asn Ser Tyr Thr Asn
    290             295             300

Thr Pro Tyr Thr Gly Ala Leu Gly Leu Leu Asp Phe Ala Leu Glu Leu
    305             310             315             320

Glu Phe Arg Asn Leu Thr Pro Gly Asn Thr Asn Thr Arg Val Ser Arg
            325             330             335

Tyr Ser Ser Ser Ala Arg His Lys Leu Arg Arg Gly Pro Asp Gly Thr
            340             345             350

Ala Glu Leu Thr Thr Thr Ala Ala Thr Arg Phe Met Lys Asp Leu His
            355             360             365

Phe Thr Gly Thr Asn Gly Val Gly Glu Val Gly Arg Gly Ile Ala Leu
    370             375             380

Thr Leu Phe Asn Leu Ala Asp Thr Leu Leu Gly Gly Leu Pro Thr Glu
385             390             395             400

Leu Ile Ser Ser Ala Gly Gly Gln Leu Phe Tyr Ser Arg Pro Val Val
            405             410             415

Ser Ala Asn Gly Glu Pro Thr Val Lys Leu Tyr Thr Ser Val Glu Asn
            420             425             430

Ala Gln Gln Asp Lys Gly Ile Ala Ile Pro His Asp Ile Asp Leu Gly
            435             440             445

Glu Ser Arg Val Val Ile Gln Asp Tyr Asp Asn Gln His Glu Gln Asp
            450             455             460

Arg Pro Thr Pro Ser Pro Ala Pro Ser Arg Pro Phe Ser Val Leu Arg
465             470             475             480

Ala Asn Asp Val Leu Trp Leu Ser Leu Thr Ala Ala Glu Tyr Asp Gln
            485             490             495

Thr Thr Tyr Gly Ser Ser Thr Asn Pro Met Tyr Val Ser Asp Thr Val
            500             505             510

Thr Phe Val Asn Val Ala Thr Gly Ala Gln Gly Val Ser Arg Ser Leu
    515             520             525

Asp Trp Ser Lys Val Thr Leu Asp Gly Arg Pro Leu Met Thr Ile Gln
```

530                     535                         540

Gln Tyr Ser Lys Thr Phe Phe Val Leu Pro Leu Arg Gly Lys Leu Ser
545                 550                 555                     560

Phe Trp Glu Ala Gly Thr Thr Lys Ala Gly Tyr Pro Tyr Asn Tyr Asn
                565                 570                 575

Thr Thr Ala Ser Asp Gln Ile Leu Ile Glu Asn Ala Ala Gly His Arg
                580                 585                 590

Val Cys Ile Ser Thr Tyr Thr Thr Asn Leu Gly Ser Gly Pro Val Ser
        595                 600                 605

Ile Ser Ala Val Gly Val Leu Ala Pro His Ser Ala Leu Ala Ala Leu
        610                 615                 620

Glu Asp Thr Val Asp Tyr Pro Ala Arg Ala His Thr Phe Asp Asp Phe
625                 630                 635                     640

Cys Pro Glu Cys Arg Thr Leu Gly Leu Gln Gly Cys Ala Phe Gln Ser
                645                 650                 655

Thr Val Ala Glu Leu Gln Arg Leu Lys Met Lys Val Gly Lys Thr Arg
                660                 665                 670

Glu Tyr

<210> 5
<211> 674
<212> PRT
<213> Hepatitis E virus

<400> 5

34

```
Met Asn Asn Met Phe Phe Cys Ser Val His Gly Asp Ala Thr Met Arg
1               5               10              15

Ser Arg Ala Phe Leu Phe Leu Phe Leu Val Leu Leu Pro Met Leu Pro
        20              25              30

Ala Pro Pro Ala Gly Gln Pro Ser Gly Arg Arg Arg Gly Arg Arg Ser
        35              40              45

Gly Gly Ala Gly Gly Gly Phe Trp Gly Asp Arg Val Asp Ser Gln Pro
    50              55              60

Phe Ala Leu Pro Tyr Ile His Pro Thr Asn Pro Phe Ala Ser Asp Ile
```

```
              65                    70                    75                    80

      Pro Ala Ala Ala Gly Ala Gly Ala Arg Pro Arg Gln Pro Ala Arg Pro
                      85                90                95

      Leu Gly Ser Ala Trp Arg Asp Gln Ser Gln Arg Pro Ala Thr Ser Ala
                  100               105               110

      Arg Arg Arg Ser Ala Pro Ala Gly Ala Ser Pro Leu Thr Ala Val Ala
              115               120               125

      Pro Ala Pro Asp Thr Ala Pro Val Pro Asp Val Asp Ser Arg Gly Ala
          130               135               140

      Ile Leu Arg Arg Gln Tyr Asn Leu Ser Thr Ser Pro Leu Thr Ser Thr
      145               150               155               160

      Ile Ala Thr Gly Thr Asn Leu Val Leu Tyr Ala Ala Pro Leu Ser Pro
                      165               170               175

      Leu Leu Pro Leu Gln Asp Gly Thr Asn Thr His Ile Met Ala Thr Glu
                  180               185               190

      Ala Ser Asn Tyr Ala Gln Tyr Arg Val Val Arg Ala Thr Ile Arg Tyr
              195               200               205

      Arg Pro Leu Val Pro Asn Ala Val Gly Gly Tyr Ala Ile Ser Ile Ser
          210               215               220

      Phe Trp Pro Gln Thr Thr Thr Thr Pro Thr Ser Val Asp Met Asn Ser
      225               230               235               240

      Ile Thr Ser Thr Asp Val Arg Ile Leu Val Gln Pro Gly Val Ala Ser
                      245               250               255

      Glu Leu Val Ile Pro Ser Glu Arg Leu His Tyr Arg Asn Gln Gly Trp
                  260               265               270

      Arg Ser Val Glu Thr Ser Gly Val Ala Glu Glu Glu Ala Thr Ser Gly
              275               280               285

      Leu Val Met Leu Cys Ile His Gly Ser Pro Val Asn Ser Tyr Thr Asn
          290               295               300

      Thr Pro Tyr Thr Gly Ala Leu Gly Leu Leu Asp Phe Ala Leu Glu Leu
      305               310               315               320
```

```
Glu Phe Arg Asn Leu Thr Pro Gly Asn Thr Asn Thr Arg Val Ser Arg
                325                 330                 335

Tyr Ser Ser Ser Ala Arg His Lys Leu Arg Arg Gly Pro Asp Gly Thr
                340                 345                 350

Ala Glu Leu Thr Thr Thr Ala Ala Thr Arg Phe Met Lys Asp Leu His
                355                 360                 365

Phe Thr Gly Thr Asn Gly Val Gly Glu Val Gly Arg Gly Ile Ala Leu
    370                 375                 380

Thr Leu Phe Asn Leu Ala Asp Thr Leu Leu Gly Gly Leu Pro Thr Glu
385                 390                 395                 400

Leu Ile Ser Ser Ala Gly Gly Gln Leu Phe Tyr Ser Arg Pro Val Val
                405                 410                 415

Ser Ala Asn Gly Glu Pro Thr Val Lys Leu Tyr Thr Ser Val Glu Asn
                420                 425                 430

Ala Gln Gln Asp Lys Gly Ile Ala Ile Pro His Asp Ile Asp Leu Gly
                435                 440                 445

Glu Ser Arg Val Val Ile Gln Asp Tyr Asp Asn Gln His Glu Gln Asp
    450                 455                 460

Arg Pro Thr Pro Ser Pro Ala Pro Ser Arg Pro Phe Ser Val Leu Arg
465                 470                 475                 480

Ala Asn Asp Val Leu Trp Leu Ser Leu Thr Ala Ala Glu Tyr Asp Gln
                485                 490                 495

Thr Thr Tyr Gly Ser Ser Thr Asn Pro Met Tyr Val Ser Asp Thr Val
                500                 505                 510

Thr Phe Val Asn Val Ala Thr Gly Ala Gln Gly Val Ser Arg Ser Leu
                515                 520                 525

Asp Trp Ser Lys Val Thr Leu Asp Gly Arg Pro Leu Met Thr Ile Gln
    530                 535                 540

Gln Tyr Ser Lys Thr Phe Phe Val Leu Pro Leu Arg Gly Lys Leu Ser
545                 550                 555                 560

Phe Trp Glu Ala Gly Thr Thr Lys Ala Gly Tyr Pro Tyr Asn Tyr Asn
                565                 570                 575
```

37

```
Thr Thr Ala Ser Asp Gln Ile Leu Ile Glu Asn Ala Ala Gly His Arg
        580             585             590

Val Cys Ile Ser Thr Tyr Thr Thr Asn Leu Gly Ser Gly Pro Val Ser
        595             600             605

Ile Ser Ala Val Gly Val Leu Ala Pro His Ser Ala Leu Ala Ala Leu
        610             615             620

Glu Asp Thr Val Asp Tyr Pro Ala Arg Ala His Thr Phe Asp Asp Phe
625             630             635             640

Cys Pro Glu Cys Arg Ala Leu Gly Leu Gln Gly Cys Ala Phe Gln Ser
            645             650             655

Thr Val Ala Glu Leu Gln Arg Leu Lys Met Lys Val Gly Lys Thr Arg
        660             665             670

Glu Tyr
```

<210> 6
<211> 674
<212> PRT
<213> Hepatitis E virus

<400> 6

```
Met Asn Asn Met Phe Phe Cys Ser Leu His Gly Asp Ala Thr Met Arg
1               5               10              15

Ser Arg Ala Leu Leu Phe Leu Leu Leu Leu Leu Leu Pro Met Leu Pro
        20              25              30

Ala Pro Pro Ala Gly Gln Pro Ser Gly Arg Arg Arg Gly Arg Arg Ser
        35              40              45

Gly Gly Ala Gly Ser Gly Phe Trp Gly Asp Arg Val Asp Ser Gln Pro
    50              55              60

Phe Ala Leu Pro Tyr Ile His Pro Thr Asn Pro Phe Ala Ser Asp Ile
65              70              75              80

Pro Ala Ala Ala Gly Ala Gly Ala Arg Pro Arg Gln Pro Ala Arg Pro
            85              90              95

Leu Gly Ser Ala Trp Arg Asp Gln Ser Gln Arg Pro Ala Ala Pro Ala
        100             105             110
```

38

```
Arg Arg Arg Ser Ala Pro Ala Gly Ala Ser Pro Leu Thr Ala Val Ala
        115             120             125

Pro Ala Pro Asp Thr Ala Pro Val Pro Asp Val Asp Ser Arg Gly Ala
        130             135             140

Ile Leu Arg Arg Gln Tyr Asn Leu Ser Thr Ser Pro Leu Thr Ser Thr
145             150             155             160

Ile Ala Thr Gly Thr Asn Leu Val Leu Tyr Ala Ala Pro Leu Ser Pro
                165             170             175

Leu Leu Pro Leu Gln Asp Gly Thr Asn Thr His Ile Met Ala Thr Glu
            180             185             190

Ala Ser Asn Tyr Ala Gln Tyr Arg Val Val Arg Ala Thr Ile Arg Tyr
            195             200             205

Arg Pro Leu Val Pro Asn Ala Val Gly Gly Tyr Ala Ile Ser Ile Ser
    210             215             220

Phe Trp Pro Gln Thr Thr Thr Thr Pro Thr Ser Val Asp Met Asn Ser
225             230             235             240

Ile Thr Ser Thr Asp Val Arg Ile Leu Val Gln Pro Gly Ile Ala Ser
                245             250             255

Glu Leu Val Ile Pro Ser Glu Arg Leu His Tyr Arg Asn Gln Gly Trp
                260             265             270

Arg Ser Val Glu Thr Ser Gly Val Ala Glu Glu Glu Ala Thr Ser Gly
            275             280             285

Leu Val Met Leu Cys Ile His Gly Ser Pro Val Asn Ser Tyr Thr Asn
            290             295             300

Thr Pro Tyr Thr Gly Ala Leu Gly Leu Leu Asp Phe Ala Leu Glu Leu
305             310             315             320

Glu Phe Arg Asn Leu Thr Pro Gly Asn Thr Asn Thr Arg Val Ser Arg
            325             330             335

Tyr Ser Ser Ser Ala Arg His Lys Leu Arg Arg Gly Ala Asp Gly Thr
            340             345             350

Ala Glu Leu Thr Thr Thr Ala Ala Thr Arg Phe Met Lys Asp Leu His
        355             360             365
```

```
Phe Thr Gly Thr Asn Gly Val Gly Glu Val Gly Arg Gly Ile Ala Leu
    370             375             380

Thr Leu Phe Asn Leu Ala Asp Thr Leu Leu Gly Gly Leu Pro Thr Glu
385             390             395             400

Leu Ile Ser Ser Ala Gly Gly Gln Leu Phe Tyr Ser Arg Pro Val Val
            405             410             415

Ser Ala Asn Gly Glu Pro Thr Val Lys Leu Tyr Thr Ser Val Glu Asn
            420             425             430

Ala Gln Gln Asp Lys Gly Ile Ala Ile Pro His Asp Ile Asp Leu Gly
            435             440             445

Glu Ser Arg Val Val Ile Gln Asp Tyr Asp Asn Gln His Glu Gln Asp
    450             455             460

Arg Pro Thr Pro Ser Pro Ala Pro Ser Arg Pro Phe Ser Val Leu Arg
465             470             475             480

Ala Asn Asp Val Leu Trp Leu Ser Leu Thr Ala Ala Glu Tyr Asp Gln
            485             490             495

Thr Thr Tyr Gly Ser Ser Thr Asn Pro Met Tyr Val Ser Asp Thr Val
            500             505             510

Thr Phe Val Asn Val Ala Thr Gly Ala Gln Gly Val Ser Arg Ser Leu
            515             520             525

Asp Trp Ser Lys Val Thr Leu Asp Gly Arg Pro Leu Thr Thr Ile Gln
    530             535             540

Gln Tyr Ser Lys Thr Phe Tyr Val Leu Pro Leu Arg Gly Lys Leu Ser
545             550             555             560

Phe Trp Glu Ala Gly Thr Thr Lys Ala Gly Tyr Pro Tyr Asn Tyr Asn
            565             570             575

Thr Thr Ala Ser Asp Gln Ile Leu Ile Glu Asn Ala Ala Gly His Arg
            580             585             590

Val Cys Ile Ser Thr Tyr Thr Thr Asn Leu Gly Ser Gly Pro Val Ser
    595             600             605

Ile Ser Ala Val Gly Val Leu Ala Pro His Ser Ala Leu Ala Val Leu
```

```
                610                      615                      620

        Glu Asp Thr Val Asp Tyr Pro Ala Arg Ala His Thr Phe Asp Asp Phe
        625                 630             635                 640


        Cys Pro Glu Cys Arg Ala Leu Gly Leu Gln Gly Cys Ala Phe Gln Ser
                        645             650                 655


        Thr Val Ala Glu Leu Gln Arg Leu Lys Met Lys Val Gly Lys Thr Arg
                    660             665             670


        Glu Tyr
```

<210> 7
<211> 674
<212> PRT
<213> Hepatitis E virus

<400> 7

```
Met Asn Asn Met Phe Phe Cys Ser Ala His Gly Asp Ala Thr Met Arg
1               5               10              15

Ser Arg Ala Leu Leu Phe Leu Leu Leu Val Phe Leu Pro Met Leu Pro
            20              25              30

Ala Pro Pro Ala Gly Gln Pro Ser Gly Arg Arg Arg Gly Arg Arg Ser
        35              40              45

Gly Gly Ala Gly Ser Gly Phe Trp Gly Asp Arg Val Asp Ser Gln Pro
    50              55              60

Phe Ala Leu Pro Tyr Ile His Pro Thr Asn Pro Phe Ala Ser Asp Ile
65              70              75              80

Pro Ala Ala Ala Gly Ala Gly Ala Arg Pro Arg Gln Pro Ala Arg Pro
            85              90              95

Leu Gly Ser Ala Trp Arg Asp Gln Ser Gln Arg Pro Ala Ala Ser Thr
            100             105             110

Arg Arg Arg Pro Ala Pro Ala Gly Ala Ser Pro Leu Thr Ala Val Ala
        115             120             125

Pro Ala Pro Asp Thr Ala Pro Val Pro Asp Val Asp Ser Arg Gly Ala
        130             135             140

Ile Leu Arg Arg Gln Tyr Asn Leu Ser Thr Ser Pro Leu Thr Ser Thr
```

```
         145              150              155              160

         Ile Ala Thr Gly Thr Asn Leu Val Leu Tyr Ala Ala Pro Leu Ser Pro
                         165              170              175

         Leu Leu Pro Leu Gln Asp Gly Thr Asn Thr His Ile Met Ala Thr Glu
                     180              185              190

         Ala Ser Asn Tyr Ala Gln Tyr Arg Val Val Arg Ala Thr Ile Arg Tyr
                     195              200              205

         Arg Pro Leu Val Pro Asn Ala Val Gly Gly Tyr Ala Ile Ser Ile Ser
             210              215              220

         Phe Trp Pro Gln Thr Thr Thr Thr Pro Thr Ser Val Asp Met Asn Ser
         225              230              235              240

         Ile Thr Ser Thr Asp Val Arg Ile Leu Val Gln Pro Gly Ile Ala Ser
                         245              250              255

         Glu Leu Val Ile Pro Ser Glu Arg Leu His Tyr Arg Asn Gln Gly Trp
                     260              265              270

         Arg Ser Val Glu Thr Ser Gly Val Ala Glu Glu Glu Ala Thr Ser Gly
                     275              280              285

         Leu Val Met Leu Cys Ile His Gly Ser Pro Val Asn Ser Tyr Thr Asn
             290              295              300

         Thr Pro Tyr Thr Gly Ala Leu Gly Leu Leu Asp Phe Ala Leu Glu Leu
         305              310              315              320

         Glu Phe Arg Asn Leu Thr Pro Gly Asn Thr Asn Thr Arg Val Ser Arg
                         325              330              335

         Tyr Ser Ser Ser Ala Arg His Lys Leu Arg Arg Gly Pro Asp Gly Thr
                     340              345              350

         Val Glu Leu Thr Thr Thr Ala Ala Thr Arg Phe Met Lys Asp Leu His
                     355              360              365

         Phe Thr Gly Thr Asn Gly Val Gly Glu Val Gly Arg Gly Ile Ala Leu
             370              375              380

         Thr Leu Phe Asn Leu Ala Asp Thr Leu Leu Gly Gly Leu Pro Thr Glu
         385              390              395              400
```

```
Leu Ile Ser Ser Ala Gly Gly Gln Leu Phe Tyr Ser Arg Pro Val Val
                405                 410                 415

Ser Ala Asn Gly Glu Pro Thr Val Lys Leu Tyr Thr Ser Val Glu Asn
                420                 425                 430

Ala Gln Gln Asp Lys Gly Ile Ala Ile Pro His Asp Ile Asp Leu Gly
                435                 440                 445

Glu Ser Arg Val Val Ile Gln Asp Tyr Asp Asn Gln His Glu Gln Asp
            450                 455                 460

Arg Pro Thr Pro Ser Pro Ala Pro Ser Arg Pro Phe Ser Val Leu Arg
465                 470                 475                 480

Ala Asn Asp Val Leu Trp Leu Ser Leu Thr Ala Ala Glu Tyr Asp Gln
                485                 490                 495

Thr Thr Tyr Gly Ser Ser Thr Asn Pro Met Tyr Val Ser Asp Thr Val
                500                 505                 510

Thr Phe Val Asn Val Ala Thr Gly Ala Gln Gly Val Ser Arg Ser Leu
                515                 520                 525

Asp Trp Ser Lys Val Thr Leu Asp Gly Arg Pro Leu Thr Thr Ile Gln
            530                 535                 540

Gln Tyr Ser Lys Thr Phe Tyr Val Leu Pro Leu Arg Gly Lys Leu Ser
545                 550                 555                 560

Phe Trp Glu Ala Gly Thr Thr Lys Ala Gly Tyr Pro Tyr Asn Tyr Asn
                565                 570                 575

Thr Thr Ala Ser Asp Gln Ile Leu Ile Glu Asn Ala Ala Gly His Arg
                580                 585                 590

Val Cys Ile Ser Thr Tyr Thr Thr Asn Leu Gly Ser Gly Pro Val Ser
                595                 600                 605

Ile Ser Ala Val Gly Val Leu Ala Pro His Ser Ala Leu Ala Ile Leu
            610                 615                 620

Glu Asp Thr Ala Asp Tyr Pro Ala Arg Ala His Thr Phe Asp Asp Phe
625                 630                 635                 640

Cys Pro Glu Cys Arg Ser Leu Gly Leu Gln Gly Cys Ala Phe Gln Ser
                645                 650                 655
```

44

```
Thr Val Ala Glu Leu Gln Arg Leu Lys Met Lys Val Gly Lys Thr Arg
            660                 665             670
```

```
Glu Tyr
```

<210> 8
<211> 672
<212> PRT
<213> Hepatitis E virus

<400> 8

```
Met Asn Asn Met Phe Phe Cys Ser Val His Gly Asp Ala Thr Met Arg
1               5               10              15
```

```
Ser Arg Ala Leu Leu Phe Leu Leu Phe Val Leu Leu Pro Met Leu Pro
            20              25              30
```

```
Ala Pro Pro Ala Gly Gln Pro Ser Gly Arg Arg Arg Gly Gln Ala Gly
            35              40              45
```

```
Cys Gly Gly Gly Phe Trp Gly Asp Arg Val Asp Ser Gln Pro Phe Ala
        50              55              60
```

```
Leu Pro Tyr Ile His Pro Thr Asn Pro Phe Ala Ser Asp Ile Pro Ala
65              70              75              80
```

```
Ala Ala Gly Thr Gly Ala Arg Pro Arg Gln Pro Ile Arg Pro Leu Gly
                85              90              95
```

```
Ser Ala Trp Arg Asp Gln Ser Gln Arg Pro Ala Ala Ser Thr Arg Arg
            100             105             110
```

```
Arg Pro Ala Pro Ala Gly Ala Ser Pro Leu Thr Ala Val Ala Pro Ala
            115             120             125
```

```
Pro Asp Thr Ala Pro Val Pro Asp Ala Asp Ser Arg Gly Ala Ile Leu
    130             135             140
```

```
Arg Arg Gln Tyr Asn Leu Ser Thr Ser Pro Leu Thr Ser Thr Ile Ala
145             150             155             160
```

```
Thr Gly Thr Asn Phe Val Leu Tyr Ala Ala Pro Leu Ser Pro Leu Leu
                165             170             175
```

```
Pro Leu Gln Asp Gly Thr Asn Thr His Ile Met Ala Thr Glu Ala Ser
            180             185             190
```

```
Asn Tyr Ala Gln Tyr Arg Val Val Arg Ala Thr Ile Arg Tyr Arg Pro
        195             200             205

Leu Val Pro Asn Ala Val Gly Gly Tyr Ala Ile Ser Ile Ser Phe Trp
        210             215             220

Pro Gln Thr Thr Thr Thr Pro Thr Ser Val Asp Met Asn Ser Ile Thr
225             230             235             240

Ser Thr Asp Val Arg Ile Leu Val Gln Pro Gly Ile Ala Ser Glu Leu
            245             250             255

Val Thr Pro Ser Glu Arg Leu His Tyr Arg Asn Gln Gly Trp Arg Ser
        260             265             270

Val Glu Thr Ser Gly Val Ala Glu Glu Glu Ala Thr Ser Gly Leu Val
        275             280             285

Met Leu Cys Ile His Gly Ser Pro Val Asn Ser Tyr Thr Asn Thr Pro
        290             295             300

Tyr Thr Gly Ala Leu Gly Leu Leu Asp Phe Ala Leu Glu Leu Glu Phe
305             310             315             320

Arg Asn Leu Thr Pro Gly Asn Thr Asn Thr Arg Val Ser Arg Tyr Ser
            325             330             335

Ser Ser Ala Arg His Lys Leu Arg Arg Gly Pro Asp Gly Thr Ala Glu
        340             345             350

Leu Thr Thr Thr Ala Ala Thr Arg Phe Met Lys Asp Leu His Phe Thr
        355             360             365

Gly Thr Asn Gly Val Gly Glu Val Gly Arg Gly Ile Ala Leu Thr Leu
        370             375             380

Phe Asn Leu Ala Asp Thr Leu Leu Gly Gly Leu Pro Thr Glu Leu Ile
385             390             395             400

Ser Ser Ala Gly Gly Gln Leu Phe Tyr Ser Arg Pro Val Val Ser Ala
            405             410             415

Asn Gly Glu Leu Thr Val Lys Leu Tyr Thr Ser Val Glu Asn Ala Gln
        420             425             430

Gln Asp Lys Gly Val Ala Ile Pro His Asp Ile Asp Leu Gly Glu Ser
        435             440             445
```

46

```
Arg Val Val Ile Gln Asp Tyr Asp Asn Gln His Glu Gln Asp Arg Pro
    450             455             460

Thr Pro Ser Pro Ala Pro Ser Arg Pro Phe Ser Val Leu Arg Ala Asn
465             470             475             480

Asp Val Leu Trp Leu Ser Leu Thr Ala Ala Glu Tyr Asp Gln Thr Thr
            485             490             495

Tyr Gly Ser Ser Thr Asn Pro Met Tyr Val Ser Asp Thr Val Thr Phe
            500             505             510

Val Asn Val Ala Thr Gly Ala Gln Gly Val Ser Arg Ser Leu Asp Trp
        515             520             525

Ser Lys Val Thr Leu Asp Gly Arg Pro Leu Thr Thr Ile Gln Gln Tyr
    530             535             540

Ser Lys Thr Phe Tyr Val Leu Pro Leu Arg Gly Lys Leu Ser Phe Trp
545             550             555             560

Glu Ala Gly Thr Thr Lys Ala Gly Tyr Pro Tyr Asn Tyr Asn Thr Thr
            565             570             575

Ala Ser Asp Gln Ile Leu Ile Glu Asn Ala Ala Gly His Arg Val Cys
            580             585             590

Ile Ser Thr Tyr Thr Thr Asn Leu Gly Ser Gly Pro Val Ser Val Ser
        595             600             605

Ala Val Gly Val Leu Ala Pro His Ser Ala Leu Ala Ala Leu Glu Asp
    610             615             620

Thr Ala Asp Tyr Pro Ala Arg Ala His Thr Phe Asp Asp Phe Cys Pro
625             630             635             640

Glu Cys Arg Ala Leu Gly Leu Gln Gly Cys Ala Phe Gln Ser Thr Val
            645             650             655

Gly Glu Leu Gln Arg Leu Lys Met Lys Val Gly Lys Thr Arg Glu Tyr
            660             665             670
```

<210> 9
<211> 671
<212> PRT
<213> Hepatitis E virus

<400> 9

```
Met Phe Phe Cys Ser Val His Gly Asp Ala Thr Met Arg Ser Arg Ala
1               5                   10              15

Leu Leu Phe Leu Leu Phe Val Leu Leu Pro Met Leu Pro Ala Pro Pro
            20              25              30

Ala Gly Gln Pro Ser Gly Arg Arg Arg Gly Arg Arg Ser Gly Gly Ala
            35              40              45

Gly Gly Gly Phe Trp Gly Asp Arg Val Asp Ser Gln Pro Phe Ala Leu
        50              55              60

Pro Tyr Ile His Pro Thr Asn Pro Phe Ala Ser Asp Ile Pro Thr Ala
65              70              75              80

Ala Gly Ser Gly Ala Arg Pro Arg Gln Pro Val Arg Pro Leu Gly Ser
                85              90              95

Ala Trp Arg Asp Gln Ser Gln Arg Pro Ala Ala Ser Ala Arg Arg Arg
            100             105             110

Pro Ala Pro Ala Gly Ala Ser Pro Leu Thr Ala Val Ala Pro Ala Pro
        115             120             125

Asp Thr Ala Pro Val Pro Asp Val Asp Ser Arg Gly Ala Ile Leu Arg
    130             135             140

Arg Gln Tyr Asn Leu Ser Thr Ser Pro Leu Thr Ser Thr Ile Ala Thr
145             150             155             160

Gly Thr Asn Leu Val Leu Tyr Ala Ala Pro Leu Ser Pro Leu Leu Pro
            165             170             175

Leu Gln Asp Gly Thr Asn Thr His Ile Met Ala Thr Glu Ala Ser Asn
            180             185             190

Tyr Ala Gln Tyr Arg Val Val Arg Ala Thr Ile Arg Tyr Arg Pro Leu
        195             200             205

Val Pro Asn Ala Val Gly Gly Tyr Ala Ile Ser Ile Ser Phe Trp Pro
    210             215             220

Gln Thr Thr Thr Thr Pro Thr Ser Val Asp Met Asn Ser Ile Thr Ser
225             230             235             240

Thr Asp Val Arg Ile Leu Val Gln Pro Gly Ile Ala Ser Glu Leu Val
```

|  | 245 | | | | | 250 | | | | | 255 | | |
|---|---|---|---|---|---|---|---|---|---|---|---|---|---|
| Ile | Pro | Ser | Glu | Arg | Leu | His | Tyr | Arg | Asn | Gln | Gly | Trp | Arg | Ser | Val |
| | | | 260 | | | | | 265 | | | | | 270 | | |

Ile Pro Ser Glu Arg Leu His Tyr Arg Asn Gln Gly Trp Arg Ser Val
          260                 265                 270

Glu Thr Ser Gly Val Ala Glu Glu Glu Ala Thr Ser Gly Leu Val Met
          275                 280                 285

Leu Cys Ile His Gly Ser Pro Val Asn Ser Tyr Thr Asn Thr Pro Tyr
290                 295                 300

Thr Gly Ala Leu Gly Leu Leu Asp Phe Ala Leu Glu Leu Glu Phe Arg
305                 310                 315                 320

Asn Leu Thr Pro Gly Asn Thr Asn Thr Arg Val Ser Arg Tyr Ser Ser
              325                 330                 335

Ser Ala Arg His Lys Leu Arg Arg Gly Pro Asp Gly Thr Ala Glu Leu
          340                 345                 350

Thr Thr Thr Ala Ala Thr Arg Phe Met Lys Asp Leu His Phe Thr Gly
          355                 360                 365

Thr Asn Gly Val Gly Glu Val Gly Arg Gly Ile Ala Leu Thr Leu Phe
      370                 375                 380

Asn Leu Ala Asp Thr Leu Leu Gly Gly Leu Pro Thr Glu Leu Ile Ser
385                 390                 395                 400

Ser Ala Gly Gly Gln Leu Phe Tyr Ser Arg Pro Val Val Ser Ala Asn
              405                 410                 415

Gly Glu Pro Thr Val Lys Leu Tyr Thr Ser Val Glu Asn Ala Gln Gln
          420                 425                 430

Asp Lys Gly Ile Ala Ile Pro His Asp Ile Asp Leu Gly Glu Ser Arg
          435                 440                 445

Val Val Ile Gln Asp Tyr Asp Asn Gln His Glu Gln Asp Arg Pro Thr
      450                 455                 460

Pro Ser Pro Ala Pro Ser Arg Pro Phe Ser Val Leu Arg Ala Asn Asp
465                 470                 475                 480

Val Leu Trp Leu Ser Leu Thr Ala Ala Glu Tyr Asp Gln Thr Thr Tyr
              485                 490                 495

```
Gly Ser Ser Thr Asn Pro Met Tyr Val Ser Asp Thr Val Thr Phe Val
        500             505             510

Asn Val Ala Thr Gly Ala Gln Gly Val Ser Arg Ser Leu Asp Trp Ser
        515             520             525

Lys Val Thr Leu Asp Gly Arg Pro Leu Thr Thr Ile Gln Gln Tyr Ser
        530             535             540

Lys Thr Phe Phe Val Leu Pro Leu Arg Gly Lys Leu Ser Phe Trp Glu
545             550             555             560

Ala Gly Thr Thr Lys Ala Gly Tyr Pro Tyr Asn Tyr Asn Thr Thr Ala
        565             570             575

Ser Asp Gln Ile Leu Ile Glu Asn Ala Ala Gly His Arg Val Cys Ile
        580             585             590

Ser Thr Tyr Thr Thr Asn Leu Gly Ser Gly Pro Val Ser Ile Ser Ala
        595             600             605

Val Gly Val Leu Ala Pro His Ser Ala Leu Ala Ala Leu Glu Asp Thr
    610             615             620

Val Asp Tyr Pro Ala Arg Ala His Thr Phe Asp Asp Phe Cys Pro Glu
625             630             635             640

Cys Arg Thr Leu Gly Leu Gln Gly Cys Ala Phe Gln Ser Thr Val Ala
            645             650             655

Glu Leu Gln Arg Leu Lys Met Lys Val Gly Lys Thr Arg Glu Tyr
            660             665             670
```

<210> 10
<211> 668
<212> PRT
<213> Hepatitis E virus

<400> 10

```
Met Phe Phe Cys Ser Val His Gly Asp Ala Thr Met Arg Ser Arg Ala
1               5               10              15

Leu Leu Phe Leu Leu Leu Val Phe Leu Pro Met Leu Pro Ala Leu Pro
        20              25              30

Ala Gly Gln Pro Ser Gly Arg Arg Arg Gly Arg Arg Ser Gly Ser Ala
        35              40              45
```

```
Gly Gly Gly Phe Trp Gly Asp Arg Val Asp Ser Gln Pro Phe Ala Leu
    50              55              60

Pro Tyr Ile His Pro Thr Asn Pro Phe Ala Ser Asp Ile Pro Thr Ala
65              70              75              80

Ala Gly Ala Gly Ala Arg Pro Arg Gln Pro Ala Arg Pro Leu Gly Ser
                85              90              95

Ala Trp Arg Asp Gln Ser Gln Arg Pro Ala Thr Ser Thr Arg Arg Arg
            100             105             110

Ser Ala Pro Val Gly Ala Ser Pro Leu Thr Ala Val Ala Pro Ala Pro
        115             120             125

Asp Thr Ala Pro Val Pro Asp Val Asp Ser Arg Gly Ala Ile Leu Arg
    130             135             140

Arg Gln Tyr Asn Leu Ser Thr Ser Pro Leu Thr Ser Thr Ile Ala Thr
145             150             155             160

Gly Thr Asn Leu Val Leu Tyr Ala Ala Pro Leu Ser Pro Leu Leu Pro
            165             170             175

Leu Gln Asp Gly Thr Asn Thr His Ile Met Ala Thr Glu Ala Ser Asn
            180             185             190

Tyr Ala Gln Tyr Arg Val Val Arg Ala Thr Ile Arg Tyr Arg Pro Leu
        195             200             205

Val Pro Asn Ala Val Gly Gly Tyr Ala Ile Ser Ile Ser Phe Trp Pro
    210             215             220

Gln Thr Thr Thr Thr Pro Thr Ser Val Asp Met Asn Ser Ile Thr Ser
225             230             235             240

Thr Asp Val Arg Ile Leu Val Gln Ser Gly Ile Ala Ser Glu Leu Val
            245             250             255

Ile Pro Ser Glu Arg Leu His Tyr Arg Asn Gln Gly Trp Arg Ser Val
        260             265             270

Glu Thr Ser Gly Val Ala Glu Glu Ala Thr Ser Gly Leu Val Met
        275             280             285

Leu Cys Ile His Gly Ser Pro Val Asn Ser Tyr Thr Asn Thr Pro Tyr
    290             295             300
```

Thr Gly Ala Leu Gly Leu Leu Asp Phe Ala Leu Glu Leu Glu Phe Arg
305           310         315             320

Asn Leu Thr Pro Gly Asn Thr Asn Met Arg Val Ser Arg His Ser Ser
              325             330             335

Ser Ala Arg His Lys Leu Arg Arg Gly Pro Asp Gly Thr Ala Glu Leu
              340             345             350

Thr Thr Thr Ala Ala Thr Arg Phe Met Lys Asp Leu His Phe Thr Gly
              355             360             365

Thr Asn Gly Val Gly Glu Val Gly Arg Gly Ile Ala Leu Thr Leu Phe
              370             375             380

Asn Leu Ala Asp Thr Leu Leu Gly Gly Leu Pro Thr Glu Leu Ile Ser
385           390             395             400

Ser Ala Gly Gly Gln Leu Phe Tyr Ser Arg Pro Val Val Ser Ala Asn
              405             410             415

Gly Glu Pro Thr Val Lys Leu Tyr Thr Ser Val Glu Asn Ala Gln Gln
              420             425             430

Asp Lys Gly Ile Ala Ile Pro His Asp Ile Asp Leu Gly Glu Ser Arg
              435             440             445

Val Gly Ile Gln Asp Tyr Asp Asn Gln His Glu Gln Asp Arg Pro Thr
              450             455             460

Pro Ser Pro Ala Pro Ser Arg Pro Phe Ser Val Leu Arg Ala Asn Asp
465           470             475             480

Val Leu Trp Leu Ser Leu Thr Ala Ala Glu Tyr Asp Gln Thr Thr Tyr
              485             490             495

Gly Ser Ser Thr Asn Pro Met Tyr Val Ser Asp Thr Val Thr Phe Val
              500             505             510

Asn Val Ala Thr Gly Ala Gln Gly Val Ser Arg Ser Leu Asp Trp Ser
              515             520             525

Lys Val Thr Leu Asp Gly Arg Ser Leu Thr Thr Ile Gln Gln Tyr Ser
              530             535             540

Lys Thr Phe Phe Val Leu Pro Leu Arg Gly Lys Leu Ser Phe Trp Glu
545           550             555             560

52

```
Ala Gly Thr Thr Lys Ala Gly Tyr Pro Tyr Asn Tyr Asn Thr Thr Ala
            565             570             575

Ser Asp Gln Ile Leu Ile Glu Asn Ala Ala Gly His Arg Val Cys Ile
            580             585             590

Ser Thr Tyr Thr Thr Asn Leu Gly Ser Gly Pro Val Ser Ile Ser Ala
            595             600             605

Val Gly Val Leu Ala Pro His Ser Ala Leu Ala Val Leu Glu Asp Thr
    610             615             620

Val Asp Tyr Pro Ala Arg Ala His Thr Phe Asp Asp Phe Cys Pro Glu
625             630             635             640

Cys Arg Ala Leu Gly Leu Gln Gly Cys Ala Phe Gln Ser Thr Val Ala
                645             650             655

Glu Leu Gln Arg Leu Lys Met Lys Val Gly Asn His
            660             665
```

<210> 11
<211> 660
<212> PRT
<213> Hepatitis E virus

<400> 11

```
Met Arg Pro Arg Pro Ile Leu Leu Leu Leu Leu Met Phe Leu Pro Met
1               5               10              15

Leu Pro Ala Pro Pro Pro Gly Gln Pro Ser Gly Arg Arg Arg Gly Arg
            20              25              30

Arg Ser Gly Gly Ser Gly Gly Gly Phe Trp Gly Asp Arg Ala Asp Ser
            35              40              45

Gln Pro Phe Ala Ile Pro Tyr Ile His Pro Thr Asn Pro Phe Ala Pro
    50              55              60

Asp Val Thr Ala Ala Ala Gly Ala Gly Pro Arg Val Arg Gln Pro Ala
65              70              75              80

Arg Pro Leu Gly Ser Ala Trp Arg Asp Gln Ala Gln Arg Pro Ala Ala
                85              90              95

Ala Ser Arg Arg Arg Pro Thr Thr Ala Gly Ala Ala Pro Leu Thr Ala
            100             105             110
```

53

```
Val Ala Pro Ala His Asp Thr Pro Pro Val Pro Asp Val Asp Ser Arg
        115                 120             125

Gly Ala Ile Leu Arg Arg Gln Tyr Asn Leu Ser Thr Ser Pro Leu Thr
        130                 135             140

Ser Ser Val Ala Thr Gly Thr Asn Leu Val Leu Tyr Ala Ala Pro Leu
145                 150                 155                 160

Ser Pro Leu Leu Pro Leu Gln Asp Gly Thr Asn Thr His Ile Met Ala
                165                 170             175

Thr Glu Ala Ser Asn Tyr Ala Gln Tyr Arg Val Val Arg Ala Thr Ile
            180                 185             190

Arg Tyr Arg Pro Leu Val Pro Asn Ala Val Gly Gly Tyr Ala Ile Ser
        195                 200             205

Ile Ser Phe Trp Pro Gln Thr Thr Thr Pro Thr Ser Val Asp Met
        210                 215             220

Asn Ser Ile Thr Ser Thr Asp Val Arg Ile Leu Val Gln Pro Gly Ile
225                 230                 235                 240

Ala Ser Glu His Val Ile Pro Ser Glu Arg Leu His Tyr Arg Asn Gln
                245                 250                 255

Gly Trp Arg Ser Val Glu Thr Ser Gly Val Ala Glu Glu Glu Ala Thr
            260                 265                 270

Ser Gly Leu Val Met Leu Cys Ile His Gly Ser Leu Val Asn Ser Tyr
            275                 280                 285

Thr Asn Thr Pro Tyr Thr Gly Ala Leu Gly Leu Leu Asp Phe Ala Leu
            290                 295                 300

Glu Leu Glu Phe Arg Asn Leu Thr Pro Gly Asn Thr Asn Thr Arg Val
305                 310                 315                 320

Ser Arg Tyr Ser Ser Thr Ala Arg His Arg Leu Arg Arg Gly Ala Asp
                325                 330                 335

Gly Thr Ala Glu Leu Thr Thr Thr Ala Ala Thr Arg Phe Met Lys Asp
            340                 345                 350

Leu Tyr Phe Thr Ser Thr Asn Gly Val Gly Glu Ile Gly Arg Gly Ile
```

54

```
                355                     360                     365


        Ala Leu Thr Leu Phe Asn Leu Ala Asp Thr Leu Leu Gly Gly Leu Pro
            370                 375                 380


        Thr Glu Leu Ile Ser Ser Ala Gly Gly Gln Leu Phe Tyr Ser Arg Pro
        385                 390                 395                 400


        Val Val Ser Ala Asn Gly Glu Pro Thr Val Lys Leu Tyr Thr Ser Val
                        405                 410                 415


        Glu Asn Ala Gln Gln Asp Lys Gly Ile Ala Ile Pro His Asp Ile Asp
                    420                 425                 430


        Leu Gly Glu Ser Arg Val Val Ile Gln Asp Tyr Asp Asn Gln His Glu
                    435                 440                 445


        Gln Asp Arg Pro Thr Pro Ser Pro Ala Pro Ser Arg Pro Phe Ser Val
                    450                 455                 460


        Leu Arg Ala Asn Asp Val Leu Trp Leu Ser Leu Thr Ala Ala Glu Tyr
        465                 470                 475                 480


        Asp Gln Ser Thr Tyr Gly Ser Ser Thr Gly Pro Val Tyr Val Ser Asp
                        485                 490                 495


        Ser Val Thr Leu Val Asn Val Ala Thr Gly Ala Gln Ala Val Ala Arg
                    500                 505                 510


        Ser Leu Asp Trp Thr Lys Val Thr Leu Asp Gly Arg Pro Leu Ser Thr
                    515                 520                 525


        Thr Gln Gln Tyr Ser Lys Thr Phe Phe Val Leu Pro Leu Arg Gly Lys
            530                 535                 540


        Leu Ser Phe Trp Glu Ala Gly Thr Thr Lys Ala Gly Tyr Pro Tyr Asn
        545                 550                 555                 560


        Tyr Asn Thr Thr Ala Ser Asp Gln Leu Leu Val Glu Asn Ala Ala Gly
                        565                 570                 575


        His Arg Val Ala Ile Ser Thr Tyr Thr Thr Ser Leu Gly Ala Gly Pro
                    580                 585                 590


        Val Ser Ile Ser Ala Val Ala Val Leu Ala Pro His Ser Ala Leu Ala
                    595                 600                 605
```

```
Leu Leu Glu Asp Thr Met Asp Tyr Pro Ala Arg Ala His Thr Phe Asp
    610             615             620

Asp Phe Cys Pro Glu Cys Arg Pro Leu Gly Leu Gln Gly Cys Ala Phe
625             630             635             640

Gln Ser Thr Val Ala Glu Leu Gln Arg Leu Lys Met Lys Val Gly Lys
                645             650             655

Thr Arg Glu Leu
            660
```

<210> 12
<211> 660
<212> PRT
<213> Hepatitis E virus

<400> 12

```
Met Arg Pro Arg Pro Ile Leu Leu Leu Leu Leu Met Phe Leu Pro Met
1               5               10              15

Leu Pro Ala Pro Pro Pro Gly Gln Pro Ser Gly Arg Arg Arg Gly Arg
            20              25              30

Arg Ser Gly Gly Ser Gly Gly Gly Phe Trp Gly Asp Arg Val Asp Ser
            35              40              45

Gln Pro Phe Ala Ile Pro Tyr Ile His Pro Thr Asn Pro Phe Ala Pro
    50              55              60

Asp Val Thr Ala Ala Ala Gly Ala Gly Pro Arg Val Arg Gln Pro Ala
65              70              75              80

Arg Pro Leu Gly Ser Ala Trp Arg Asp Gln Ala Gln Arg Pro Ala Val
            85              90              95

Ala Ser Arg Arg Arg Pro Thr Thr Ala Gly Ala Ala Pro Leu Thr Ala
            100             105             110

Val Ala Pro Ala His Asp Thr Pro Pro Val Pro Asp Val Asp Ser Arg
            115             120             125

Gly Ala Ile Leu Arg Arg Gln Tyr Asn Leu Ser Thr Ser Pro Leu Thr
            130             135             140

Ser Ser Val Ala Thr Gly Thr Asn Leu Val Leu Tyr Ala Ala Pro Leu
145             150             155             160
```

```
Ser Pro Leu Leu Pro Leu Gln Asp Gly Thr Asn Thr His Ile Met Ala
            165             170             175

Thr Glu Ala Ser Asn Tyr Ala Gln Tyr Arg Val Ala Arg Ala Thr Ile
            180             185             190

Arg Tyr Arg Pro Leu Val Pro Asn Ala Val Gly Gly Tyr Ala Ile Ser
            195             200             205

Ile Ser Phe Trp Pro Gln Thr Thr Thr Pro Thr Ser Val Asp Met
210             215             220

Asn Ser Ile Thr Ser Thr Asp Val Arg Ile Leu Val Gln Pro Gly Ile
225             230             235             240

Ala Ser Glu Leu Val Ile Pro Ser Glu Arg Leu His Tyr Arg Asn Gln
            245             250             255

Gly Trp Arg Ser Val Glu Thr Ser Gly Val Ala Glu Glu Glu Ala Thr
            260             265             270

Ser Gly Leu Val Met Leu Cys Ile His Gly Ser Leu Val Asn Ser Tyr
            275             280             285

Thr Asn Thr Pro Tyr Thr Gly Ala Leu Gly Leu Leu Asp Phe Ala Leu
            290             295             300

Glu Leu Glu Phe Arg Asn Leu Thr Pro Gly Asn Thr Asn Thr Arg Val
305             310             315             320

Ser Arg Tyr Ser Ser Thr Ala Arg His Arg Leu Arg Arg Gly Ala Asp
            325             330             335

Gly Thr Ala Glu Leu Thr Thr Thr Ala Ala Thr Arg Phe Met Lys Asp
            340             345             350

Leu Tyr Phe Thr Ser Thr Asn Gly Val Gly Glu Ile Gly Arg Gly Ile
            355             360             365

Ala Leu Thr Leu Phe Asn Leu Ala Asp Thr Leu Leu Gly Gly Leu Pro
            370             375             380

Thr Glu Leu Ile Ser Ser Ala Gly Gly Gln Leu Phe Tyr Ser Arg Pro
385             390             395             400

Val Val Ser Ala Asn Gly Glu Pro Thr Val Lys Leu Tyr Thr Ser Val
            405             410             415
```

```
Glu Asn Ala Gln Gln Asp Lys Gly Ile Ala Ile Pro His Asp Ile Asp
            420             425             430

Leu Gly Glu Ser Arg Val Val Ile Gln Asp Tyr Asp Asn Gln His Glu
            435             440             445

Gln Asp Arg Pro Thr Pro Ser Pro Ala Pro Ser Arg Pro Phe Ser Val
            450             455             460

Leu Arg Ala Asn Asp Val Leu Trp Leu Ser Leu Thr Ala Ala Glu Tyr
465             470             475             480

Asp Gln Ser Thr Tyr Gly Ser Ser Thr Gly Pro Val Tyr Val Ser Asp
                485             490             495

Ser Val Thr Leu Val Asn Val Ala Thr Gly Ala Gln Ala Val Ala Arg
            500             505             510

Ser Leu Asp Trp Thr Lys Val Thr Leu Asp Gly Arg Pro Leu Ser Thr
            515             520             525

Ile Gln Gln Tyr Ser Lys Thr Phe Phe Val Leu Pro Leu Arg Gly Lys
            530             535             540

Leu Ser Phe Trp Glu Ala Gly Thr Thr Lys Ala Gly Tyr Pro Tyr Asn
545             550             555             560

Tyr Asn Thr Thr Ala Ser Asp Gln Leu Leu Val Glu Asn Ala Ala Gly
                565             570             575

His Arg Val Ala Ile Ser Thr Tyr Thr Thr Ser Leu Gly Ala Gly Pro
            580             585             590

Val Ser Ile Ser Ala Val Ala Val Leu Ala Pro His Ser Ala Leu Ala
            595             600             605

Leu Leu Glu Asp Thr Leu Asp Tyr Pro Ala Arg Ala His Thr Phe Asp
            610             615             620

Asp Phe Cys Pro Glu Cys Arg Pro Leu Gly Leu Gln Gly Cys Ala Phe
625             630             635             640

Gln Ser Thr Val Ala Glu Leu Gln Arg Leu Lys Met Lys Val Gly Lys
                645             650             655

Thr Arg Glu Leu
            660
```

<210> 13
<211> 660
<212> PRT
<213> Hepatitis E virus

<400> 13

```
Met Arg Pro Arg Ala Val Leu Leu Leu Leu Phe Val Leu Leu Pro Met
1               5                   10                  15

Leu Pro Ala Pro Pro Ala Gly Gln Pro Ser Gly Arg Arg Arg Gly Arg
            20              25              30

Arg Asn Gly Gly Ala Gly Gly Gly Phe Trp Gly Asp Arg Val Asp Ser
        35              40              45

Gln Pro Phe Ala Leu Pro Tyr Ile His Pro Thr Asn Pro Phe Ala Ala
    50              55              60

Asp Val Val Ser Gln Pro Gly Ala Gly Ala Arg Pro Arg Gln Pro Pro
65              70              75              80

Arg Pro Leu Gly Ser Ala Trp Arg Asp Gln Ser Gln Arg Pro Ser Thr
            85              90              95

Ala Pro Arg Arg Arg Ser Ala Pro Ala Gly Ala Ala Pro Leu Thr Ala
            100             105             110

Val Ser Pro Ala Pro Asp Thr Ala Pro Val Pro Asp Val Asp Ser Arg
            115             120             125

Gly Ala Ile Leu Arg Arg Gln Tyr Asn Leu Ser Thr Ser Pro Leu Thr
        130             135             140

Ser Ser Val Ala Ala Gly Thr Asn Leu Val Leu Tyr Ala Ala Pro Leu
145             150             155             160

Asn Pro Leu Leu Pro Leu Gln Asp Gly Thr Asn Thr His Ile Met Ala
            165             170             175

Thr Glu Ala Ser Asn Tyr Ala Gln Tyr Arg Val Val Arg Ala Thr Ile
            180             185             190

Arg Tyr Arg Pro Leu Val Pro Asn Ala Val Gly Gly Tyr Ala Ile Ser
        195             200             205

Ile Ser Phe Trp Pro Gln Thr Thr Thr Pro Thr Ser Val Asp Met
        210             215             220
```

```
Asn Ser Ile Thr Ser Thr Asp Val Arg Ile Leu Val Gln Pro Gly Ile
225             230             235             240

Ala Ser Glu Leu Val Ile Pro Ser Glu Arg Leu His Tyr Arg Asn Gln
            245             250             255

Gly Trp Arg Ser Val Glu Thr Thr Gly Val Ala Glu Glu Glu Ala Thr
            260             265             270

Ser Gly Leu Val Met Leu Cys Ile His Gly Ser Pro Val Asn Ser Tyr
            275             280             285

Thr Asn Thr Pro Tyr Thr Gly Ala Leu Gly Leu Leu Asp Phe Ala Leu
            290             295             300

Glu Leu Glu Phe Arg Asn Leu Thr Pro Gly Asn Thr Asn Thr Arg Val
305             310             315             320

Ser Arg Tyr Thr Ser Thr Ala Arg His Arg Leu Arg Arg Gly Ala Asp
            325             330             335

Gly Thr Ala Glu Leu Thr Thr Thr Ala Ala Thr Arg Phe Met Lys Asp
            340             345             350

Leu His Phe Thr Gly Thr Asn Gly Val Gly Glu Val Gly Arg Gly Ile
            355             360             365

Ala Leu Thr Leu Phe Asn Leu Ala Asp Thr Leu Leu Gly Gly Leu Pro
    370             375             380

Thr Glu Leu Ile Ser Ser Ala Gly Gly Gln Leu Phe Tyr Ser Arg Pro
385             390             395             400

Val Val Ser Ala Asn Gly Glu Pro Thr Val Lys Leu Tyr Thr Ser Val
            405             410             415

Glu Asn Ala Gln Gln Asp Lys Gly Ile Thr Ile Pro His Asp Ile Asp
            420             425             430

Leu Gly Asp Ser Arg Val Val Ile Gln Asp Tyr Asp Asn Gln His Glu
            435             440             445

Gln Asp Arg Pro Thr Pro Ser Pro Ala Pro Ser Arg Pro Phe Ser Val
            450             455             460

Leu Arg Ala Asn Asp Val Leu Trp Leu Ser Leu Thr Ala Ala Glu Tyr
```

| | | | 465 | | | | 470 | | | | 475 | | | | 480 |

Asp Gln Thr Thr Tyr Gly Ser Ser Thr Asn Pro Met Tyr Val Ser Asp
              485                    490                  495

Thr Val Thr Leu Val Asn Val Ala Thr Gly Ala Gln Ala Val Ala Arg
          500                505              510

Ser Leu Asp Trp Ser Lys Val Thr Leu Asp Gly Arg Pro Leu Thr Thr
          515                520              525

Ile Gln Gln Tyr Ser Lys Thr Phe Tyr Val Leu Pro Leu Arg Gly Lys
          530                535              540

Leu Ser Phe Trp Glu Ala Gly Thr Thr Lys Ala Gly Tyr Pro Tyr Asn
545                550              555              560

Tyr Asn Thr Thr Ala Ser Asp Gln Ile Leu Ile Glu Asn Ala Ala Gly
          565                570              575

His Arg Val Ala Ile Ser Thr Tyr Thr Thr Ser Leu Gly Ala Gly Pro
          580                585              590

Thr Ser Ile Ser Ala Val Gly Val Leu Ala Pro His Ser Ala Leu Ala
          595                600              605

Val Leu Glu Asp Thr Val Asp Tyr Pro Ala Arg Ala His Thr Phe Asp
          610                615              620

Asp Phe Cys Pro Glu Cys Arg Thr Leu Gly Leu Gln Gly Cys Ala Phe
625                630              635              640

Gln Ser Thr Ile Ala Glu Leu Gln Arg Leu Lys Met Lys Val Gly Lys
          645                650              655

Thr Arg Glu Ser
          660

<210> 14
<211> 660
<212> PRT
<213> Hepatitis E virus

<400> 14

```
Met Arg Pro Arg Pro Ile Leu Leu Leu Leu Leu Met Phe Leu Pro Met
1               5                   10                      15

Leu Pro Ala Pro Pro Pro Gly Gln Pro Ser Gly Arg Arg Arg Gly Arg
```

```
                    20                      25                        30

        Arg Ser Gly Gly Ser Gly Gly Gly Phe Trp Gly Asp Arg Val Asp Ser
                35              40                  45

        Gln Pro Phe Ala Ile Pro Tyr Ile His Pro Thr Asn Pro Phe Ala Pro
                50              55                  60

        Asp Val Thr Ala Ala Ala Gly Ala Gly Pro Arg Val Arg Gln Pro Ala
        65              70                  75                      80

        Arg Pro Leu Gly Ser Ala Trp Arg Asp Gln Ala Gln Arg Pro Ala Val
                        85                  90                  95

        Ala Ser Arg Arg Arg Pro Thr Thr Ala Gly Ala Ala Pro Leu Thr Ala
                100                 105                 110

        Val Ala Pro Ala His Asp Thr Pro Pro Val Pro Asp Val Asp Ser Arg
                115                 120                 125

        Ala Ala Ile Leu Arg Arg Gln Tyr Asn Leu Ser Thr Ser Pro Leu Thr
                130                 135                 140

        Ser Ser Val Ala Thr Gly Thr Asn Leu Val Leu Tyr Ala Ala Pro Leu
        145                 150                 155                 160

        Ser Pro Leu Leu Pro Leu Gln Asp Gly Thr Asn Thr His Ile Met Ala
                        165                 170                 175

        Thr Glu Ala Ser Asn Tyr Ala Gln Tyr Arg Val Val Arg Ala Thr Ile
                180                 185                 190

        Arg Tyr Arg Pro Leu Val Pro Asn Ala Val Gly Gly Tyr Ala Ile Ser
                195                 200                 205

        Ile Ser Phe Trp Pro Gln Thr Thr Thr Pro Thr Ser Val Asp Met
                210                 215                 220

        Asn Ser Ile Thr Ser Thr Asp Val Arg Ile Leu Val Gln Pro Gly Ile
        225                 230                 235                 240

        Ala Ser Glu Leu Val Ile Pro Ser Glu Arg Leu His Tyr Arg Asn Gln
                        245                 250                 255

        Gly Trp Arg Ser Val Glu Thr Ser Gly Val Ala Glu Glu Glu Ala Thr
                260                 265                 270
```

63

```
Ser Gly Leu Val Met Leu Cys Ile His Gly Ser Pro Val Asn Ser Tyr
        275             280             285

Thr Asn Thr Pro Tyr Thr Gly Ala Leu Gly Leu Leu Asp Phe Ala Leu
        290             295             300

Glu Leu Glu Phe Arg Asn Leu Thr Pro Gly Asn Thr Asn Thr Arg Val
305             310             315             320

Ser Arg Tyr Ser Ser Thr Ala Arg His Arg Leu Arg Arg Gly Ala Asp
        325             330             335

Gly Thr Ala Glu Leu Thr Thr Thr Ala Ala Thr Arg Phe Met Lys Asp
        340             345             350

Leu Tyr Phe Thr Ser Thr Asn Gly Val Gly Glu Ile Gly Arg Gly Ile
        355             360             365

Ala Leu Thr Leu Phe Asn Leu Ala Asp Thr Leu Leu Gly Gly Leu Pro
        370             375             380

Thr Glu Leu Ile Ser Ser Ala Gly Gly Gln Leu Phe Tyr Ser Arg Pro
385             390             395             400

Val Val Ser Ala His Gly Glu Pro Thr Val Lys Leu Tyr Thr Ser Val
            405             410             415

Glu Asn Ala Gln Gln Asp Lys Gly Ile Ala Ile Pro His Asp Ile Asp
        420             425             430

Leu Gly Glu Ser Arg Val Val Ile Gln Asp Tyr Asp Asn Gln His Glu
        435             440             445

Gln Asp Arg Pro Thr Pro Ser Pro Ala Pro Ser Arg Pro Phe Ser Val
        450             455             460

Leu Arg Ala Asn Asp Val Leu Trp Leu Ser Leu Thr Ala Ala Glu Tyr
465             470             475             480

Asp Gln Ser Thr Tyr Gly Ser Ser Thr Ala Pro Val Tyr Val Ser Asp
            485             490             495

Ser Val Thr Leu Val Asn Val Ala Thr Gly Ala Gln Ala Val Ala Arg
        500             505             510

Ser Leu Asp Trp Thr Lys Val Thr Leu Asp Gly Arg Pro Leu Ser Thr
        515             520             525
```

64

```
Ile Gln Gln Tyr Pro Lys Thr Phe Phe Val Leu Pro Leu Arg Gly Lys
    530              535              540

Leu Ser Phe Trp Glu Ala Gly Thr Thr Lys Ala Gly Tyr Pro Tyr Asn
545              550              555              560

Tyr Asn Thr Thr Ala Ser Asp Gln Leu Leu Val Glu Asn Ala Ala Gly
                565              570              575

His Arg Val Ala Ile Ser Thr Tyr Thr Thr Ser Leu Gly Ala Gly Pro
            580              585              590

Val Ser Ile Ser Ala Val Ala Val Leu Ala Pro His Ser Ala Leu Ala
        595              600              605

Leu Leu Glu Asp Thr Leu Asp Tyr Pro Ala Cys Ala His Thr Phe Asp
    610              615              620

Asp Phe Cys Pro Glu Cys Arg Pro Leu Gly Leu Gln Gly Cys Ala Phe
625              630              635              640

Gln Ser Thr Val Ala Glu Leu Gln Arg Leu Lys Met Lys Val Gly Lys
            645              650              655

Thr Arg Glu Leu
            660
```

<210> 15
<211> 660
<212> PRT
<213> Hepatitis E virus

<400> 15

```
Met Gly Pro Arg Pro Ile Leu Leu Leu Phe Leu Met Phe Leu Pro Met
1               5               10              15

Leu Leu Ala Pro Pro Pro Gly Gln Pro Ser Gly Arg Arg Arg Gly Arg
            20              25              30

Arg Ser Gly Gly Ser Gly Gly Gly Phe Trp Gly Asp Arg Val Asp Ser
        35              40              45

Gln Pro Phe Ala Ile Pro Tyr Ile His Pro Thr Asn Pro Phe Ala Pro
    50              55              60

Asn Val Thr Ala Ala Ala Gly Ala Gly Pro Arg Val Arg Gln Pro Val
65              70              75              80
```

Arg Pro Leu Gly Ser Ala Trp Arg Asp Gln Ala Gln Arg Pro Ala Ala
85 90 95

Ala Ser Arg Arg Arg Pro Thr Thr Ala Gly Ala Ala Pro Leu Thr Ala
100 105 110

Val Ala Pro Ala His Asp Thr Pro Pro Val Pro Asp Val Asp Ser Arg
115 120 125

Gly Ala Ile Leu Arg Arg Gln Tyr Asn Leu Ser Thr Ser Pro Leu Thr
130 135 140

Ser Ser Val Ala Thr Gly Thr Asn Leu Val Leu Tyr Ala Ala Pro Leu
145 150 155 160

Ser Pro Leu Leu Pro Leu Gln Asp Gly Thr Asn Thr His Ile Met Ala
165 170 175

Thr Glu Ala Ser Asn Tyr Ala Gln Tyr Arg Val Ala Arg Ala Thr Ile
180 185 190

Arg Tyr Arg Pro Leu Val Pro Asn Ala Val Gly Gly Tyr Ala Ile Ser
195 200 205

Ile Ser Phe Trp Pro Gln Thr Thr Pro Thr Pro Thr Ser Val Asp Met
210 215 220

Asn Ser Ile Thr Ser Thr Asp Val Arg Ile Leu Val Gln Pro Gly Ile
225 230 235 240

Ala Ser Glu Leu Val Ile Pro Ser Glu Arg Leu His Tyr Arg Asn Gln
245 250 255

Gly Trp Arg Ser Val Glu Thr Ser Gly Val Ala Glu Glu Glu Ala Thr
260 265 270

Ser Gly Leu Val Met Leu Cys Ile His Gly Ser Pro Val Asn Ser Tyr
275 280 285

Thr Asn Thr Pro Tyr Thr Gly Ala Leu Gly Leu Leu Asp Phe Ala Leu
290 295 300

Glu Leu Glu Phe Arg Asn Leu Thr Pro Gly Asn Thr Asn Thr Arg Val
305 310 315 320

Ser Arg Tyr Ser Ser Thr Ala Arg His Arg Leu Arg Arg Gly Ala Asp
325 330 335

```
Gly Thr Ala Glu Leu Thr Thr Thr Ala Ala Thr Arg Phe Met Lys Asp
        340             345             350

Leu Tyr Phe Thr Ser Thr Asn Gly Val Gly Glu Ile Gly Arg Gly Ile
        355             360             365

Ala Leu Thr Leu Phe Asn Leu Ala Asp Thr Leu Leu Gly Gly Leu Pro
        370             375             380

Thr Glu Leu Ile Ser Ser Ala Gly Gly Gln Leu Phe Tyr Ser Arg Pro
385             390             395             400

Val Val Ser Ala Asn Gly Glu Pro Thr Val Lys Leu Tyr Thr Ser Val
            405             410             415

Glu Asn Ala Gln Gln Asp Lys Gly Ile Ala Ile Pro Asn Asp Ile Asp
        420             425             430

Leu Gly Glu Ser Arg Val Val Ile Gln Asp Tyr Asp Asn Gln His Glu
        435             440             445

Gln Asp Arg Pro Thr Pro Ser Pro Ala Pro Ser Arg Pro Phe Ser Val
        450             455             460

Leu Arg Ala Asn Asp Val Leu Trp Leu Ser Leu Thr Ala Ala Glu Tyr
465             470             475             480

Asp Gln Ser Thr Tyr Gly Ser Ser Thr Gly Pro Val Tyr Val Ser Asp
            485             490             495

Ser Val Thr Leu Val Asn Val Ala Thr Gly Ala Gln Ala Val Ala Arg
        500             505             510

Ser Leu Asp Trp Thr Lys Val Thr Leu Asp Gly Arg Pro Leu Ser Thr
        515             520             525

Ile Gln Gln Tyr Ser Lys Ile Phe Phe Val Leu Pro Leu Arg Gly Lys
        530             535             540

Leu Ser Phe Trp Glu Ala Gly Thr Thr Arg Pro Gly Tyr Pro Tyr Asn
545             550             555             560

Tyr Asn Thr Thr Ala Ser Asp Gln Leu Leu Val Glu Asn Ala Ala Gly
            565             570             575

His Arg Val Ala Ile Ser Thr Tyr Thr Thr Ser Leu Gly Ala Gly Pro
```

580                    585                    590

Val Ser Ile Ser Ala Val Ala Val Leu Gly Pro His Ser Ala Leu Ala
        595                600                605

Leu Leu Glu Asp Thr Leu Asp Tyr Pro Ala Arg Ala His Thr Phe Asp
    610                615                620

Asp Phe Cys Pro Glu Cys Arg Pro Leu Gly Leu Gln Gly Cys Ala Phe
625                630                635                640

Gln Ser Thr Val Ala Glu Leu Gln Arg Leu Lys Met Lys Val Gly Lys
            645                650                655

Thr Arg Glu Leu
            660

<210> 16
<211> 660
<212> PRT
<213> Hepatitis E virus

<400> 16

68

```
Met Arg Pro Arg Ala Val Leu Leu Leu Leu Phe Val Leu Leu Pro Met
1                5                10                15

Leu Pro Ala Pro Pro Ala Gly Gln Pro Ser Gly Arg Arg Arg Gly Arg
        20                25                30

Arg Ser Gly Gly Ala Gly Gly Gly Phe Trp Gly Asp Arg Val Asp Ser
        35                40                45

Gln Pro Phe Ala Leu Pro Tyr Ile His Pro Thr Asn Pro Phe Ala Ala
    50                55                60

Asp Val Val Ser Gln Pro Gly Ala Gly Thr Arg Pro Arg Gln Pro Pro
65                70                75                80

Arg Pro Leu Gly Ser Ala Trp Arg Asp Gln Ser Gln Arg Pro Ser Ala
            85                90                95

Ala Pro Arg Arg Arg Ser Ala Pro Ala Gly Ala Ala Pro Leu Thr Ala
        100                105                110

Val Ser Pro Ala Pro Asp Thr Ala Pro Val Pro Asp Val Asp Ser Arg
        115                120                125

Gly Ala Ile Leu Arg Arg Gln Tyr Asn Leu Ser Thr Ser Pro Leu Thr
```

                    130                        135                              140


        Ser Ser Val Ala Ser Gly Thr Asn Leu Val Leu Tyr Ala Ala Pro Leu
        145                 150                 155                 160


        Asn Pro Leu Leu Pro Leu Gln Asp Gly Thr Asn Thr His Ile Met Ala
                        165                 170                 175


        Thr Glu Ala Ser Asn Tyr Ala Gln Tyr Arg Val Val Arg Ala Thr Ile
                    180                 185                 190


        Arg Tyr Arg Pro Leu Val Pro Asn Ala Val Gly Gly Tyr Ala Ile Ser
                    195                 200                 205


        Ile Ser Phe Trp Pro Gln Thr Thr Thr Pro Thr Ser Val Asp Met
                    210                 215                 220


        Asn Ser Ile Thr Ser Thr Asp Val Arg Ile Leu Val Gln Pro Gly Ile
        225                 230                 235                 240


        Ala Ser Glu Leu Val Ile Pro Ser Glu Arg Leu His Tyr Arg Asn Gln
                        245                 250                 255


        Gly Trp Arg Ser Val Glu Thr Thr Gly Val Ala Glu Glu Glu Ala Thr
                    260                 265                 270


        Ser Gly Leu Val Met Leu Cys Ile His Gly Ser Pro Val Asn Ser Tyr
                    275                 280                 285


        Thr Asn Thr Pro Tyr Thr Gly Ala Leu Gly Leu Leu Asp Phe Ala Leu
                    290                 295                 300


        Glu Leu Glu Phe Arg Asn Leu Thr Pro Gly Asn Thr Asn Thr Arg Val
        305                 310                 315                 320


        Ser Arg Tyr Thr Ser Thr Ala Arg His Arg Leu Arg Arg Gly Ala Asp
                        325                 330                 335


        Gly Thr Ala Glu Leu Thr Thr Thr Ala Ala Thr Arg Phe Met Lys Asp
                    340                 345                 350


        Leu His Phe Ala Gly Thr Asn Gly Val Gly Glu Val Gly Arg Gly Ile
                    355                 360                 365


        Ala Leu Thr Leu Phe Asn Leu Ala Asp Thr Leu Leu Gly Gly Leu Pro
                    370                 375                 380


                                        70

```
Thr Glu Leu Ile Ser Ser Ala Gly Gly Gln Leu Phe Tyr Ser Arg Pro
385             390         395             400

Val Val Ser Ala Asn Gly Glu Pro Thr Val Lys Leu Tyr Thr Ser Val
            405             410             415

Glu Asn Ala Gln Gln Asp Lys Gly Ile Thr Ile Pro His Asp Ile Asp
        420             425             430

Leu Gly Asp Ser Arg Val Val Ile Gln Asp Tyr Asp Asn Gln His Glu
        435             440             445

Gln Asp Arg Pro Thr Pro Ser Pro Ala Pro Ser Arg Pro Phe Ser Val
        450             455             460

Leu Arg Ala Asn Asp Val Leu Trp Leu Ser Leu Thr Ala Ala Glu Tyr
465             470             475             480

Asp Gln Thr Thr Tyr Gly Ser Ser Thr Asn Pro Met Tyr Val Ser Asp
            485             490             495

Thr Val Thr Leu Val Asn Val Ala Thr Gly Ala Gln Ala Val Ala Arg
            500             505             510

Ser Leu Asp Trp Ser Lys Val Thr Leu Asp Gly Arg Pro Leu Thr Thr
        515             520             525

Ile Gln Gln Tyr Ser Lys Thr Phe Tyr Val Leu Pro Leu Arg Gly Lys
        530             535             540

Leu Ser Phe Trp Glu Ala Gly Thr Thr Lys Ala Gly Tyr Pro Tyr Asn
545             550             555             560

Tyr Asn Thr Thr Ala Ser Asp Gln Ile Leu Ile Glu Asn Ala Ala Gly
            565             570             575

His Arg Val Ala Ile Ser Thr Tyr Thr Thr Ser Leu Gly Ala Gly Pro
            580             585             590

Thr Ser Ile Ser Ala Val Gly Val Leu Ala Pro His Ser Ala Leu Ala
            595             600             605

Val Leu Glu Asp Thr Ile Asp Tyr Pro Ala Arg Ala His Thr Phe Asp
        610             615             620

Asp Phe Cys Pro Glu Cys Arg Thr Leu Gly Leu Gln Gly Cys Ala Phe
625             630             635             640
```

71

```
      Gln Ser Thr Ile Ala Glu Leu Gln Arg Leu Lys Met Lys Val Gly Lys
                      645             650             655

      Thr Arg Glu Ser
                  660
```

<210> 17
<211> 660
<212> PRT
<213> Hepatitis E virus

<400> 17

```
      Met Arg Pro Arg Pro Ile Leu Leu Leu Leu Met Phe Leu Pro Met
      1               5               10              15

      Leu Pro Ala Pro Pro Pro Gly Gln Pro Ser Gly Arg Arg Arg Gly Arg
                  20              25              30

      Arg Ser Gly Gly Ser Gly Gly Gly Phe Trp Gly Asp Arg Val Asp Ser
                  35              40              45

      Gln Pro Phe Ala Ile Pro Tyr Ile His Pro Thr Asn Pro Phe Ala Pro
          50              55              60

      Asp Val Thr Ala Ala Ala Gly Ala Gly Pro Arg Val Arg Gln Pro Ala
      65              70              75              80

      Arg Pro Leu Gly Ser Ala Trp Arg Asp Gln Ala Gln Arg Pro Ala Ala
                      85              90              95

      Ala Ser Arg Arg Arg Pro Thr Thr Ala Gly Ala Ala Pro Leu Thr Ala
                  100             105             110

      Val Ala Pro Ala His Asp Thr Pro Pro Val Pro Asp Val Asp Ser Arg
                  115             120             125

      Gly Ala Ile Leu Arg Arg Gln Tyr Asn Leu Ser Thr Ser Pro Leu Thr
          130             135             140

      Ser Ser Val Ala Thr Gly Thr Asn Leu Val Leu Tyr Ala Ala Pro Leu
      145             150             155             160

      Ser Pro Leu Leu Pro Leu Gln Asp Gly Thr Asn Thr His Ile Met Ala
                      165             170             175

      Thr Glu Ala Ser Asn Tyr Ala Gln Tyr Arg Val Ala Arg Ala Thr Ile
                  180             185             190
```

Arg Tyr Arg Pro Leu Val Pro Asn Ala Val Gly Gly Tyr Ala Ile Ser
        195                 200                 205

Ile Ser Phe Trp Pro Gln Thr Thr Thr Thr Pro Thr Ser Val Asp Met
        210                 215                 220

Asn Ser Ile Thr Ser Thr Asp Val Arg Ile Leu Val Gln Pro Gly Ile
225                 230                 235                 240

Ala Ser Glu Leu Val Ile Pro Ser Glu Arg Leu His Tyr Arg Asn Gln
                245                 250                 255

Gly Trp Arg Ser Val Glu Thr Ser Gly Val Ala Glu Glu Glu Ala Thr
        260                 265                 270

Ser Gly Leu Val Met Leu Cys Ile His Gly Ser Pro Val Asn Ser Tyr
        275                 280                 285

Thr Asn Thr Pro Tyr Thr Gly Ala Leu Gly Leu Leu Asp Phe Ala Leu
        290                 295                 300

Glu Leu Glu Phe Arg Asn Leu Thr Pro Gly Asn Thr Asn Thr Arg Val
305                 310                 315                 320

Ser Arg Tyr Ser Ser Thr Ala Arg His Arg Leu Arg Arg Gly Ala Asp
                325                 330                 335

Gly Thr Ala Glu Leu Thr Thr Thr Ala Ala Thr Arg Phe Met Lys Asp
                340                 345                 350

Leu Tyr Phe Thr Ser Thr Asn Gly Val Gly Glu Ile Gly Arg Gly Ile
        355                 360                 365

Ala Leu Thr Leu Phe Asn Leu Ala Asp Thr Leu Leu Gly Gly Leu Pro
        370                 375                 380

Thr Glu Leu Ile Ser Ser Ala Gly Gly Gln Leu Phe Tyr Ser Arg Pro
385                 390                 395                 400

Val Val Ser Ala Asn Gly Glu Pro Thr Val Lys Leu Tyr Thr Ser Val
                405                 410                 415

Glu Asn Ala Gln Gln Asp Lys Gly Ile Ala Ile Pro His Asp Ile Asp
                420                 425                 430

Leu Gly Glu Ser Arg Val Val Ile Gln Asp Tyr Asp Asn Gln His Glu
        435                 440                 445

73

```
Gln Asp Arg Pro Thr Pro Ser Pro Ala Pro Ser Arg Pro Phe Ser Val
    450             455             460

Leu Arg Ala Asn Asp Val Leu Trp Leu Ser Leu Thr Ala Ala Glu Tyr
465             470             475             480

Asp Gln Ser Thr Tyr Gly Ser Ser Thr Gly Pro Val Tyr Val Ser Asp
            485             490             495

Ser Val Thr Leu Val Asn Val Ala Thr Gly Ala Gln Ala Val Ala Arg
        500             505             510

Ser Leu Asp Trp Thr Lys Val Thr Leu Asp Gly Arg Pro Leu Ser Thr
        515             520             525

Ile Gln Gln Tyr Ser Lys Thr Phe Phe Val Leu Pro Leu Arg Gly Lys
    530             535             540

Leu Ser Phe Trp Glu Ala Gly Thr Thr Lys Ala Gly Tyr Pro Tyr Asn
545             550             555             560

Tyr Asn Thr Thr Ala Ser Asp Gln Leu Leu Val Glu Asn Ala Ala Gly
            565             570             575

His Arg Val Ala Ile Ser Thr Tyr Thr Thr Ser Leu Gly Ala Gly Pro
        580             585             590

Val Ser Ile Ser Ala Val Ala Val Leu Ala Pro His Ser Val Leu Ala
    595             600             605

Leu Leu Glu Asp Thr Met Asp Tyr Pro Ala Arg Ala His Thr Phe Asp
    610             615             620

Asp Phe Cys Pro Glu Cys Arg Pro Leu Gly Leu Gln Gly Cys Ala Phe
625             630             635             640

Gln Ser Thr Val Ala Glu Leu Gln Arg Leu Lys Met Lys Val Gly Lys
            645             650             655

Thr Arg Glu Leu
            660
```

<210> 18
<211> 660
<212> PRT
<213> Hepatitis E virus

<220>

<221> misc_feature
<222> (481)..(481)
<223> Xaa can be any naturally occurring amino acid

<400> 18

```
Met Arg Pro Arg Ala Val Leu Leu Leu Phe Leu Met Phe Leu Pro Met
1               5                   10                  15

Leu Pro Ala Pro Pro Ala Gly Gln Pro Ser Gly Arg Arg Arg Gly Arg
            20              25                  30

Arg Ser Gly Gly Ala Gly Gly Gly Phe Trp Ser Asp Arg Val Asp Ser
            35              40                  45

Gln Pro Phe Ala Leu Pro Tyr Ile His Pro Thr Asn Pro Phe Ala Ala
        50              55                  60

Asp Val Val Ser Gln Pro Gly Ala Gly Thr Arg Pro Arg Gln Pro Pro
65              70                  75                  80

Arg Pro Leu Gly Ser Ala Trp Arg Asp Gln Ser Lys Arg Pro Ser Val
            85              90                  95

Ala Pro Arg Arg Arg Ser Thr Pro Ala Gly Ala Ala Pro Leu Thr Ala
            100             105                 110

Ile Ser Pro Ala Pro Asp Thr Ala Pro Val Pro Asp Val Asp Ser Arg
            115             120                 125

Gly Ala Ile Leu Arg Arg Gln Tyr Asn Leu Ser Thr Ser Pro Leu Thr
            130             135                 140

Ser Ser Val Ala Ser Gly Thr Asn Leu Val Leu Tyr Ala Ala Pro Leu
145             150                 155                 160

Asn Pro Leu Leu Pro Leu Gln Asp Gly Thr Asn Thr His Ile Met Ala
            165             170                 175

Thr Glu Ala Ser Asn Tyr Ala Gln Tyr Arg Val Val Arg Ala Thr Ile
            180             185                 190

Arg Tyr Arg Pro Leu Val Pro Asn Ala Val Gly Gly Tyr Ala Ile Ser
            195             200                 205

Ile Ser Phe Trp Pro Gln Thr Thr Thr Pro Thr Ser Val Asp Met
            210             215                 220
```

```
Asn Ser Ile Thr Ser Thr Asp Val Arg Ile Leu Val Gln Pro Gly Ile
225             230         235             240

Ala Ser Glu Leu Val Ile Pro Ser Glu Arg Leu His Tyr Arg Asn Gln
            245             250             255

Gly Trp Arg Ser Val Glu Thr Thr Gly Val Ala Glu Glu Glu Ala Thr
            260             265             270

Ser Gly Leu Val Met Leu Cys Ile His Gly Ser Pro Val Asn Ser Tyr
            275             280             285

Thr Asn Thr Pro Tyr Thr Gly Ala Leu Gly Leu Leu Asp Phe Ala Leu
    290             295             300

Glu Leu Glu Phe Arg Asn Leu Thr Pro Gly Asn Thr Asn Thr Arg Val
305             310             315             320

Ser Arg Tyr Thr Ser Thr Ala Arg His Arg Leu Arg Arg Gly Ala Asp
            325             330             335

Gly Thr Ala Glu Leu Thr Thr Thr Ala Ala Thr Arg Phe Met Lys Asp
            340             345             350

Leu His Phe Thr Gly Thr Asn Gly Val Gly Glu Val Gly Arg Gly Ile
    355             360             365

Ala Leu Thr Leu Phe Asn Leu Ala Asp Thr Leu Leu Gly Gly Leu Pro
    370             375             380

Thr Glu Leu Ile Ser Ser Ala Gly Gly Gln Leu Phe Tyr Ser Arg Pro
385             390             395             400

Val Val Ser Ala Asn Gly Glu Pro Thr Val Lys Leu Tyr Thr Ser Val
            405             410             415

Glu Asn Ala Gln Gln Asp Lys Gly Ile Thr Ile Pro His Asp Ile Asp
            420             425             430

Leu Gly Asp Ser Arg Val Val Ile Gln Asp Tyr Asp Asn Gln His Glu
            435             440             445

Gln Asp Arg Pro Thr Pro Ser Pro Ala Pro Ser Arg Pro Phe Ser Val
    450             455             460

Leu Arg Ala Asn Asp Val Leu Trp Leu Ser Leu Thr Ala Ala Glu Tyr
465             470             475             480
```

```
Xaa Gln Thr Thr Tyr Gly Ser Ser Thr Asn Pro Met Tyr Val Ser Asp
            485                 490                 495

Thr Val Thr Leu Val Asn Val Ala Thr Gly Ala Gln Ala Val Ala Arg
            500                 505                 510

Ser Leu Asp Trp Ser Lys Val Thr Leu Asp Gly Arg Pro Leu Thr Thr
            515                 520                 525

Ile Gln Gln Tyr Ser Lys Lys Phe Tyr Val Leu Pro Leu Arg Gly Lys
    530                 535                 540

Leu Ser Phe Trp Glu Ala Gly Thr Thr Lys Ala Gly Tyr Pro Tyr Asn
545                 550                 555                 560

Tyr Asn Thr Thr Ala Ser Asp Gln Ile Leu Ile Glu Asn Ala Ala Gly
            565                 570                 575

His Arg Val Ala Ile Ser Thr Tyr Thr Thr Ser Leu Gly Ala Gly Pro
            580                 585                 590

Thr Ser Ile Ser Ala Val Gly Val Leu Ala Pro His Ser Ala Leu Ala
            595                 600                 605

Val Leu Glu Asp Thr Val Asp Tyr Pro Ala Arg Ala His Thr Phe Asp
    610                 615                 620

Asp Phe Cys Pro Glu Cys Arg Thr Leu Gly Leu Gln Gly Cys Ala Phe
625                 630                 635                 640

Gln Ser Thr Ile Ala Glu Leu Gln Arg Leu Lys Met Lys Val Gly Lys
            645                 650                 655

Thr Arg Glu Ser
            660
```

<210> 19
<211> 660
<212> PRT
<213> Hepatitis E virus

<400> 19

```
Met Arg Pro Arg Ala Val Leu Leu Leu Phe Phe Val Leu Leu Pro Met
1               5                   10                  15

Leu Pro Ala Pro Pro Ala Gly Gln Pro Ser Gly Arg Arg Arg Gly Arg
            20                  25                  30
```

```
Arg Ser Gly Gly Thr Gly Gly Gly Phe Trp Gly Asp Arg Val Asp Ser
        35              40              45

Gln Pro Phe Ala Leu Pro Tyr Ile His Pro Thr Asn Pro Phe Ala Ser
        50              55              60

Asp Ile Pro Thr Ala Thr Gly Ala Gly Ala Arg Pro Arg Gln Pro Ala
65              70              75              80

Arg Pro Leu Gly Ser Ala Trp Arg Asp Gln Ser Gln Arg Pro Ala Ala
            85              90              95

Pro Ala Arg Arg Arg Ser Ala Pro Ala Gly Ala Ser Pro Leu Thr Ala
            100             105             110

Val Ala Pro Ala Pro Asp Thr Ala Pro Val Pro Asp Val Asp Ser Arg
            115             120             125

Gly Ala Ile Leu Arg Arg Gln Tyr Asn Leu Ser Thr Ser Pro Leu Thr
    130             135             140

Ser Thr Ile Ala Thr Gly Thr Asn Leu Val Leu Tyr Ala Ala Pro Leu
145             150             155             160

Ser Pro Leu Leu Pro Leu Gln Asp Gly Thr Asn Thr His Ile Ile Ala
            165             170             175

Thr Glu Ala Ser Asn Tyr Ala Gln Tyr Arg Val Val Arg Ala Thr Ile
            180             185             190

Arg Tyr Arg Pro Leu Val Pro Asn Ala Val Gly Gly Tyr Ala Ile Ser
            195             200             205

Ile Ser Phe Trp Pro Gln Thr Thr Thr Pro Thr Ser Val Asp Met
    210             215             220

Asn Ser Ile Thr Ser Thr Asp Val Arg Ile Leu Val Gln Pro Gly Ile
225             230             235             240

Ala Ser Glu Leu Val Ile Pro Ser Glu Arg Leu His Tyr Arg Asn Gln
            245             250             255

Gly Trp Arg Ser Val Glu Thr Ser Gly Val Ala Glu Glu Glu Ala Thr
            260             265             270

Ser Gly Leu Val Met Leu Cys Ile His Gly Ser Pro Val Asn Ser Tyr
```

|     | 275 |     |     |     | 280 |     |     |     | 285 |     |     |     |     |     |
| --- | --- | --- | --- | --- | --- | --- | --- | --- | --- | --- | --- | --- | --- | --- |

Thr Asn Thr Pro Tyr Thr Gly Ala Leu Gly Leu Leu Asp Phe Ala Leu
    290         295         300

Glu Leu Glu Phe Arg Asn Leu Thr Pro Gly Asn Thr Asn Thr Arg Val
305         310         315         320

Ser Arg Tyr Ser Ser Ser Ala Arg His Lys Leu Cys Arg Gly Pro Asp
         325         330         335

Gly Thr Ala Glu Leu Thr Thr Thr Ala Ala Thr Arg Phe Met Lys Asp
        340         345         350

Leu His Phe Thr Gly Thr Asn Gly Val Gly Glu Val Gly Arg Gly Ile
        355         360         365

Ala Leu Thr Leu Leu Asn Leu Ala Asp Thr Leu Leu Gly Gly Leu Pro
        370         375         380

Thr Glu Leu Ile Ser Ser Ala Gly Gly Gln Leu Phe Tyr Ser Arg Pro
385         390         395         400

Val Val Ser Ala Asn Gly Glu Pro Thr Val Lys Leu Tyr Thr Ser Val
        405         410         415

Glu Asn Ala Gln Gln Asp Lys Gly Ile Ala Ile Pro His Asp Ile Asp
        420         425         430

Leu Gly Glu Ser Arg Val Val Ile Gln Asp Tyr Asp Asn Gln His Glu
        435         440         445

Gln Asp Arg Pro Thr Pro Ser Pro Ala Pro Ser Arg Pro Phe Ser Val
        450         455         460

Leu Arg Ala Asn Asp Val Leu Trp Leu Ser Leu Thr Ala Ala Glu Tyr
465         470         475         480

Asp Gln Thr Thr Tyr Gly Ser Ser Thr Asn Pro Met Tyr Val Ser Asp
        485         490         495

Thr Val Thr Phe Val Asn Val Ala Thr Gly Thr Gln Gly Val Ser Arg
        500         505         510

Ser Leu Asp Trp Ser Lys Val Thr Leu Asp Gly Arg Pro Leu Thr Thr
        515         520         525

```
        Ile Gln Gln Tyr Ser Lys Thr Phe Phe Val Leu Pro Leu Arg Gly Lys
            530                 535                 540

        Leu Ser Phe Trp Glu Ala Gly Thr Thr Lys Ala Gly Tyr Pro Tyr Asn
        545                 550                 555                 560

        Tyr Asn Thr Thr Ala Ser Asp Gln Ile Leu Ile Glu Asn Ala Pro Gly
                        565                 570                 575

        His Arg Val Cys Ile Ser Thr Tyr Thr Thr Asn Leu Gly Ser Gly Pro
                    580                 585                 590

        Val Ser Ile Ser Ala Val Gly Val Leu Ala Pro His Ser Ala Leu Ala
                    595                 600                 605

        Ala Leu Glu Asp Thr Val Asp Tyr Pro Ala Arg Ala His Thr Phe Asp
            610                 615                 620

        Asp Phe Cys Pro Glu Cys Arg Ala Leu Gly Leu Gln Gly Cys Ala Phe
        625                 630                 635                 640

        Gln Ser Thr Val Ala Glu Leu Gln Arg Leu Lys Met Lys Val Gly Lys
                        645                 650                 655

        Thr Gln Glu Tyr
                    660
```

<210> 20
<211> 660
<212> PRT
<213> Hepatitis E virus

<400> 20

```
        Met Arg Pro Arg Pro Ile Leu Leu Leu Leu Leu Met Phe Leu Pro Met
        1               5                   10                  15

        Leu Pro Ala Pro Pro Pro Gly Gln Pro Ser Gly Arg Arg Arg Gly Arg
                    20                  25                  30

        Arg Ser Gly Gly Ser Gly Gly Gly Phe Trp Gly Asp Arg Val Asp Ser
                    35                  40                  45

        Gln Pro Phe Ala Ile Pro His Ile His Pro Thr Asn Pro Phe Ala Pro
            50                  55                  60

        Asp Val Thr Ala Ala Ala Gly Ala Gly Pro Arg Val Arg Gln Pro Ala
        65                  70                  75                  80
```

80

Arg Pro Leu Gly Ser Ala Trp Arg Asp Gln Ala Gln Arg Pro Ala Ala
                    85                  90                  95

Thr Ser Arg Arg Arg Pro Thr Thr Ala Gly Ala Ala Pro Leu Thr Ala
                    100                 105                 110

Val Ala Pro Ala His Asp Thr Pro Pro Val Pro Asp Val Asp Ser Arg
                    115                 120                 125

Gly Ala Ile Leu Arg Arg Gln Tyr Asn Leu Ser Thr Ser Pro Leu Thr
            130                 135                 140

Ser Pro Val Ala Thr Gly Thr Asn Leu Val Leu Tyr Ala Ala Pro Leu
145                 150                 155                 160

Ser Pro Leu Leu Pro Leu Gln Asp Gly Thr Asn Thr His Ile Met Ala
                    165                 170                 175

Thr Glu Ala Ser Asn Tyr Ala Gln Tyr Arg Val Ala Arg Ala Thr Ile
                    180                 185                 190

Arg Tyr Arg Pro Leu Val Pro Asn Ala Val Gly Gly Tyr Ala Ile Ser
                    195                 200                 205

Ile Ser Phe Trp Pro Gln Thr Thr Thr Pro Thr Ser Val Asp Met
            210                 215                 220

Asn Ser Ile Thr Ser Thr Asp Val Arg Ile Leu Val Gln Pro Gly Ile
225                 230                 235                 240

Ala Ser Glu Leu Val Ile Pro Ser Glu Arg Leu His Tyr Arg Asn Gln
                    245                 250                 255

Gly Trp Arg Ser Val Glu Thr Ser Gly Val Ala Glu Glu Glu Ala Thr
                    260                 265                 270

Ser Gly Leu Val Met Leu Cys Ile His Gly Leu Pro Val Asn Ser Tyr
            275                 280                 285

Thr Asn Thr Pro Tyr Thr Gly Ala Leu Gly Leu Leu Asp Phe Ala Leu
            290                 295                 300

Glu Phe Glu Phe Arg Asn Leu Thr Pro Gly Asn Thr Asn Thr Arg Val
305                 310                 315                 320

Ser Arg Tyr Ser Ser Thr Ala Arg His Arg Leu Arg Arg Gly Ala Asp
                    325                 330                 335

Gly Thr Ala Glu Leu Thr Thr Thr Ala Ala Thr Arg Phe Met Lys Asp
340                     345             350

Leu Tyr Phe Thr Ser Thr Asn Gly Val Gly Glu Ile Gly Arg Gly Ile
355                 360             365

Ala Leu Thr Leu Phe Asn Leu Ala Asp Thr Leu Leu Gly Gly Leu Pro
370                 375             380

Thr Glu Leu Ile Ser Ser Ala Gly Gly Gln Leu Phe Tyr Ser Arg Pro
385                 390             395             400

Val Val Ser Ala Asn Gly Glu Pro Thr Val Lys Leu Tyr Thr Ser Val
405                 410             415

Glu Asn Ala Gln Gln Asp Lys Gly Ile Ala Ile Pro His Asp Ile Asp
420                 425             430

Leu Gly Glu Ser Arg Val Val Ile Gln Asp Tyr Asp Asn Gln His Glu
435                 440             445

Gln Asp Arg Pro Thr Pro Ser Pro Ala Pro Ser Arg Pro Phe Ser Val
450                 455             460

Leu Arg Ala Asn Asp Val Leu Trp Leu Ser Leu Thr Ala Ala Glu Tyr
465                 470             475             480

Asp Gln Ser Thr Tyr Gly Ser Ser Thr Gly Pro Val Tyr Val Ser Asp
485                 490             495

Ser Val Thr Leu Val Asn Val Ala Thr Gly Ala Gln Ala Val Ala Arg
500                 505             510

Ser Leu Asp Trp Thr Lys Val Thr Leu Asp Gly Arg Pro Leu Ser Thr
515                 520             525

Ile Gln Gln Tyr Ser Lys Thr Phe Phe Val Leu Pro Leu Arg Gly Lys
530                 535             540

Leu Ser Phe Trp Glu Ala Gly Thr Thr Lys Ala Gly Tyr Pro Tyr Asn
545                 550             555             560

Tyr Asn Thr Thr Ala Ser Asp Gln Leu Leu Ile Glu Asn Ala Ala Gly
565                 570             575

His Arg Val Ala Ile Ser Thr Tyr Thr Thr Ser Leu Gly Ala Gly Pro
580                 585             590

82

```
         Val Ala Ile Ser Ala Val Ala Val Leu Ala Pro His Ser Ala Leu Ala
                 595                 600                 605

         Leu Leu Glu Asp Thr Met Asp Tyr Pro Ala Arg Ala His Thr Phe Asp
             610                 615                 620

         Asp Phe Cys Pro Glu Cys Arg Pro Leu Gly Leu Gln Gly Cys Ala Phe
         625                 630                 635                 640

         Gln Ser Thr Val Ala Glu Leu Gln Arg Leu Lys Met Lys Val Gly Lys
                         645                 650                 655

         Thr Arg Glu Leu
                     660
```

<210> 21
<211> 660
<212> PRT
<213> Hepatitis E virus

<400> 21

```
         Met Arg Pro Arg Ala Val Leu Leu Leu Phe Phe Val Leu Leu Pro Met
         1               5                   10                  15

         Leu Pro Ala Pro Pro Ala Gly Gln Pro Ser Gly Arg Arg Arg Gly Arg
                     20                  25                  30

         Arg Ser Gly Gly Ala Gly Gly Gly Phe Trp Gly Asp Arg Val Asp Ser
                 35                  40                  45

         Gln Pro Phe Ala Leu Pro Tyr Ile His Pro Thr Asn Pro Phe Ala Ala
             50                  55                  60

         Asp Val Ala Ser Gln Ser Gly Ala Gly Ala Arg Pro Arg Gln Pro Pro
         65                  70                  75                  80

         Arg Pro Leu Gly Ser Ala Trp Arg Asp Gln Ser Gln Arg Pro Pro Ala
                         85                  90                  95

         Val Pro Arg Arg Arg Ser Ala Pro Ala Gly Ala Ala Pro Leu Thr Ala
                     100                 105                 110

         Ile Ser Pro Ala Pro Asp Thr Ala Pro Val Pro Asp Val Asp Ser Arg
                 115                 120                 125

         Gly Ala Ile Leu Arg Arg Gln Tyr Asn Leu Ser Thr Ser Pro Leu Thr
             130                 135                 140
```

Ser Ser Val Ala Ser Gly Thr Asn Leu Val Leu Tyr Ala Ala Pro Leu
145             150             155             160

Asn Pro Leu Leu Pro Leu Gln Asp Gly Thr Asn Thr His Ile Met Ala
                165             170             175

Thr Glu Ala Ser Asn Tyr Ala Gln Tyr Arg Val Val Arg Ala Thr Ile
            180             185             190

Arg Tyr Arg Pro Leu Val Pro Asn Ala Val Gly Gly Tyr Ala Ile Ser
        195             200             205

Ile Ser Phe Trp Pro Gln Thr Thr Thr Pro Thr Ser Val Asp Met
    210             215             220

Asn Ser Ile Thr Ser Thr Asp Val Arg Ile Leu Val Gln Pro Gly Ile
225             230             235             240

Ala Ser Glu Leu Val Ile Pro Ser Glu Arg Leu His Tyr Arg Asn Gln
            245             250             255

Gly Trp Arg Ser Val Glu Thr Thr Gly Val Ala Glu Glu Glu Ala Thr
        260             265             270

Ser Gly Leu Val Met Leu Cys Ile His Gly Ser Pro Val Asn Ser Tyr
        275             280             285

Thr Asn Thr Pro Tyr Thr Gly Ala Leu Gly Leu Leu Asp Phe Ala Leu
    290             295             300

Glu Leu Glu Phe Arg Asn Leu Thr Pro Gly Asn Thr Asn Thr Arg Val
305             310             315             320

Ser Arg Tyr Thr Ser Thr Ala Arg His Arg Leu Arg Arg Gly Ala Asp
        325             330             335

Gly Thr Ala Glu Leu Thr Thr Thr Ala Ala Thr Arg Phe Met Lys Asp
        340             345             350

Leu His Phe Thr Gly Thr Asn Gly Val Gly Glu Val Gly Arg Gly Ile
    355             360             365

Ala Leu Thr Leu Phe Asn Leu Ala Asp Thr Leu Leu Gly Gly Leu Pro
    370             375             380

Thr Glu Leu Ile Ser Ser Ala Gly Gly Gln Leu Phe Tyr Ser Arg Pro

84

385               390               395               400

Val Ala Ser Ala Asn Gly Glu Pro Thr Val Lys Leu Tyr Thr Ser Val
              405           410            415

Glu Asn Ala Gln Gln Asp Lys Gly Ile Thr Ile Pro His Asp Ile Asp
         420           425          430

Leu Gly Asp Ser Arg Val Val Ile Gln Asp Tyr Asp Asn Gln His Glu
         435           440          445

Gln Asp Arg Pro Thr Pro Ser Pro Ala Pro Ser Arg Pro Phe Ser Val
         450           455          460

Leu Arg Ala Asn Asp Val Leu Trp Leu Ser Leu Thr Val Ala Glu Tyr
465              470          475          480

Asp Gln Thr Thr Tyr Gly Ser Ser Thr Asn Pro Met Tyr Val Ser Asp
             485           490          495

Thr Ala Thr Phe Val Asn Val Ala Thr Gly Ala Gln Ala Val Ala Arg
         500           505          510

Ser Leu Asp Trp Ser Lys Val Thr Leu Asp Gly Arg Pro Leu Thr Thr
         515           520          525

Ile Gln Gln Tyr Ser Lys Thr Phe Tyr Val Leu Pro Leu Arg Gly Lys
         530           535          540

Leu Ser Phe Trp Glu Ala Gly Thr Thr Lys Ala Gly Tyr Pro Tyr Asn
545              550          555          560

Tyr Asn Thr Ala Ala Ser Asp Gln Ile Leu Ile Glu Asn Ala Ala Gly
         565           570          575

His Arg Val Ala Ile Ser Thr Tyr Thr Thr Ser Leu Gly Ala Ser Pro
         580           585          590

Thr Ser Ile Ser Ala Val Gly Val Leu Ala Pro His Ser Ala Leu Ala
         595           600          605

Val Leu Glu Asp Thr Val Asp Tyr Pro Ala Arg Ala His Thr Phe Asp
         610           615          620

Asp Phe Cys Pro Glu Cys Arg Thr Leu Gly Leu Gln Gly Cys Ala Phe
625              630          635          640

```
Gln Ser Thr Ile Ala Glu Leu Gln Arg Leu Lys Met Lys Val Gly Lys
                645                 650                 655

Thr Arg Glu Ser
            660
```

<210> 22
<211> 660
<212> PRT
<213> Hepatitis E virus

<400> 22

```
Met Arg Pro Arg Ala Val Leu Leu Leu Phe Val Leu Leu Pro Met
1                5                   10                  15

Leu Pro Ala Pro Pro Ala Gly Gln Pro Ser Gly Arg Arg Arg Gly Arg
            20              25                  30

Arg Ser Gly Gly Ala Gly Gly Gly Phe Trp Gly Asp Arg Val Asp Ser
            35              40                  45

Gln Pro Phe Ala Leu Pro Tyr Ile His Pro Thr Asn Pro Phe Ala Ala
            50              55                  60

Asp Val Val Ser Gln Pro Gly Ala Gly Thr Arg Pro Arg Gln Pro Pro
65              70                  75                  80

Arg Pro Leu Gly Ser Ala Trp Arg Asp Gln Ser Gln Arg Pro Ser Ala
                85                  90                  95

Ala Pro Arg Arg Arg Pro Ala Pro Ala Gly Ala Thr Pro Leu Thr Ala
            100                 105                 110

Val Ser Pro Ala Pro Asp Ala Ala Pro Val Pro Asp Val Asp Ser Arg
            115                 120                 125

Gly Ala Ile Leu Arg Arg Gln Tyr Asn Leu Ser Thr Ser Pro Leu Thr
            130                 135                 140

Ser Ser Val Ala Ser Gly Thr Asn Leu Val Leu Tyr Ala Ala Pro Leu
145                 150                 155                 160

Asn Pro Leu Leu Pro Leu Gln Asp Gly Thr Asn Thr His Ile Met Ala
                165                 170                 175

Thr Glu Ala Ser Asn Tyr Ala Gln Tyr Arg Val Val Arg Ala Thr Ile
            180                 185                 190
```

```
Arg Tyr Arg Pro Leu Val Pro Asn Ala Val Gly Gly Tyr Ala Val Ser
        195                 200                 205

Ile Ser Phe Trp Pro Gln Thr Thr Thr Thr Pro Thr Ser Val Asp Met
        210                 215                 220

Asn Ser Ile Thr Ser Thr Asp Val Arg Ile Leu Val Gln Pro Gly Val
225                 230                 235                 240

Ala Ser Glu Leu Val Ile Pro Ser Glu Arg Leu His Tyr Arg Asn Gln
                245                 250                 255

Gly Trp Arg Ser Val Glu Thr Thr Gly Val Ala Glu Glu Glu Ala Thr
                260                 265                 270

Ser Gly Leu Val Met Leu Cys Ile His Gly Ser Pro Val Asn Ser Tyr
                275                 280                 285

Thr Asn Thr Pro Tyr Thr Gly Ala Leu Gly Leu Leu Asp Phe Ala Leu
        290                 295                 300

Glu Leu Glu Phe Arg Asn Leu Thr Pro Gly Asn Thr Asn Thr Arg Val
305                 310                 315                 320

Ser Arg Tyr Thr Ser Thr Ala Arg His Arg Leu Arg Arg Gly Ala Asp
                325                 330                 335

Gly Thr Ala Glu Leu Thr Thr Thr Ala Ala Thr Arg Phe Met Lys Asp
                340                 345                 350

Leu His Phe Thr Gly Thr Asn Gly Val Gly Glu Val Gly Arg Gly Ile
        355                 360                 365

Ala Leu Thr Leu Phe Asn Leu Ala Asp Thr Leu Leu Gly Gly Leu Pro
        370                 375                 380

Thr Glu Leu Ile Ser Ser Ala Gly Gly Gln Leu Phe Tyr Ser Arg Pro
385                 390                 395                 400

Val Val Ser Ala Asn Gly Glu Pro Thr Val Lys Leu Tyr Thr Ser Val
                405                 410                 415

Glu Asn Ala Gln Gln Asp Lys Gly Ile Thr Ile Pro His Asp Ile Asp
                420                 425                 430

Leu Gly Asp Ser Arg Val Val Ile Gln Asp Tyr Asp Asn Gln His Glu
        435                 440                 445
```

87

```
Gln Asp Arg Pro Thr Pro Ser Pro Ala Pro Ser Arg Pro Phe Ser Val
    450             455             460

Leu Arg Ala Asn Asp Val Leu Trp Leu Ser Leu Thr Ala Ala Glu Tyr
465             470             475             480

Asp Gln Thr Thr Tyr Gly Ser Ser Thr Asn Pro Met Tyr Val Ser Asp
            485             490             495

Thr Val Thr Leu Val Asn Val Ala Thr Gly Ala Gln Ala Val Ala Arg
            500             505             510

Ser Leu Asp Trp Ser Lys Val Thr Leu Asp Gly Arg Pro Leu Thr Thr
            515             520             525

Ile Gln Gln Tyr Ser Lys Thr Phe Tyr Val Leu Pro Leu Arg Gly Lys
    530             535             540

Leu Ser Phe Trp Glu Ala Gly Thr Thr Lys Ala Gly Tyr Pro Tyr Asn
545             550             555             560

Tyr Asn Thr Thr Ala Ser Asp Gln Ile Leu Ile Glu Asn Ala Ser Gly
            565             570             575

His Arg Val Ala Ile Ser Thr Tyr Thr Thr Ser Leu Gly Ala Gly Pro
            580             585             590

Thr Ser Ile Ser Ala Val Gly Val Leu Ala Pro His Ser Ala Leu Ala
            595             600             605

Val Leu Glu Asp Thr Ile Asp Tyr Pro Ala Arg Ala His Thr Phe Asp
    610             615             620

Asp Phe Cys Pro Glu Cys Arg Ala Leu Gly Phe Gln Gly Cys Ala Phe
625             630             635             640

Gln Ser Thr Ile Ala Glu Leu Gln Arg Leu Lys Met Lys Val Gly Lys
            645             650             655

Thr Arg Glu Ser
            660
```

<210> 23
<211> 660
<212> PRT
<213> Hepatitis E virus

<400> 23

```
Met Cys Pro Arg Ala Val Leu Leu Leu Leu Phe Val Leu Leu Pro Met
1               5                   10                  15

Leu Pro Ala Pro Pro Ala Gly Gln Pro Ser Gly Arg Arg Arg Gly Arg
            20              25                  30

Arg Ser Gly Gly Ala Gly Gly Gly Phe Trp Gly Asp Arg Val Asp Ser
        35              40                  45

Gln Pro Phe Ala Leu Pro Tyr Ile His Pro Thr Asn Pro Phe Ala Ala
        50                  55                  60

Asp Val Phe Ser Gln Ser Gly Ala Gly Ala Arg Pro Arg Gln Pro Pro
65                  70                  75                  80

Arg Pro Leu Gly Ser Ala Trp Arg Asp Gln Ser Gln Arg Pro Ser Ala
                85                  90                  95

Ala Pro Arg Arg Arg Ser Thr Pro Ala Gly Ala Ala Pro Leu Thr Ala
            100                 105                 110

Thr Ser Pro Ala Pro Asp Thr Ala Pro Val Pro Asp Val Asp Ser Arg
            115                 120                 125

Gly Ala Ile Leu Arg Arg Gln Tyr Asn Leu Ser Thr Ser Pro Leu Thr
            130                 135                 140

Ser Ser Val Ala Ser Gly Thr Asn Leu Val Leu Tyr Ala Ala Pro Leu
145                 150                 155                 160

Asn Pro Leu Leu Pro Leu Gln Asp Gly Thr Asn Thr His Ile Met Ala
                165                 170                 175

Thr Glu Ala Ser Asn Tyr Ala Gln Tyr Arg Val Val Arg Ala Thr Ile
            180                 185                 190

Arg Tyr Arg Pro Leu Val Pro Asn Ala Val Gly Gly Tyr Ala Ile Ser
            195                 200                 205

Ile Ser Phe Trp Pro Gln Thr Thr Thr Pro Thr Ser Val Asp Met
        210                 215                 220

Asn Ser Ile Thr Ser Thr Asp Val Arg Ile Leu Val Gln Pro Gly Ile
225                 230                 235                 240

Ala Ser Glu Leu Val Ile Pro Ser Glu Arg Leu His Tyr Arg Asn Gln
                245                 250                 255
```

```
Gly Trp Arg Ser Val Glu Thr Thr Gly Val Ala Glu Glu Glu Ala Thr
        260                 265                 270

Ser Gly Leu Val Met Leu Cys Ile His Gly Ser Pro Val Asn Ser Tyr
        275                 280                 285

Thr Asn Thr Pro Tyr Thr Gly Ala Leu Gly Leu Leu Asp Phe Ala Leu
        290                 295                 300

Glu Leu Glu Phe Arg Asn Leu Thr Pro Gly Asn Thr Asn Thr Arg Val
305                 310                 315                 320

Ser Arg Tyr Thr Ser Thr Ala Arg His Arg Leu Arg Arg Gly Ala Asp
        325                 330                 335

Gly Thr Ala Glu Leu Thr Thr Thr Ala Ala Thr Arg Phe Met Lys Asp
        340                 345                 350

Leu His Phe Thr Gly Thr Asn Gly Val Gly Glu Val Gly Arg Gly Ile
        355                 360                 365

Ala Leu Thr Leu Phe Asn Leu Ala Asp Thr Leu Leu Gly Gly Leu Pro
        370                 375                 380

Thr Glu Leu Ile Ser Ser Ala Gly Gly Gln Leu Phe Tyr Ser Arg Pro
385                 390                 395                 400

Val Val Ser Ala Asn Gly Glu Pro Thr Val Lys Leu Tyr Thr Ser Val
        405                 410                 415

Glu Asn Ala Gln Gln Asp Lys Gly Ile Thr Ile Pro His Asp Ile Asp
        420                 425                 430

Leu Gly Asp Ser Arg Val Val Ile Gln Asp Tyr Asp Asn Gln His Glu
        435                 440                 445

Gln Asp Arg Pro Thr Pro Ser Pro Ala Pro Ser Arg Pro Phe Ser Val
        450                 455                 460

Leu Arg Ala Asn Asp Val Leu Trp Leu Ser Leu Thr Ala Ala Glu Tyr
465                 470                 475                 480

Asp Gln Thr Thr Tyr Gly Ser Ser Thr Asn Pro Met Tyr Val Ser Asp
        485                 490                 495

Thr Val Thr Phe Val Asn Val Ala Thr Gly Ala Gln Ala Val Ala Arg
```

90

500                      505                      510


Ser Leu Asp Trp Ser Lys Val Thr Leu Asp Gly Arg Pro Leu Thr Thr
        515                  520                  525

Ile Gln Gln Tyr Ser Lys Thr Phe Tyr Val Leu Pro Leu Arg Gly Lys
    530                  535                  540

Leu Ser Phe Trp Glu Ala Gly Thr Thr Lys Ala Gly Tyr Pro Tyr Asn
545                  550                  555                  560

Tyr Asn Thr Thr Ala Ser Asp Gln Ile Leu Ile Glu Asn Ala Ala Gly
                565                  570                  575

His Arg Val Ala Ile Ser Thr Tyr Thr Thr Ser Leu Gly Ala Gly Pro
            580                  585                  590

Thr Ser Ile Ser Ala Val Gly Val Leu Ala Pro His Ser Ala Leu Ala
        595                  600                  605

Val Leu Glu Asp Thr Val Asp Tyr Pro Ala Arg Ala His Thr Phe Asp
    610                  615                  620

Asp Phe Cys Pro Glu Cys Arg Ala Leu Gly Leu Gln Gly Cys Ala Phe
625                  630                  635                  640

Gln Ser Thr Val Ala Glu Leu Gln Arg Leu Lys Met Lys Val Gly Lys
                645                  650                  655

Thr Arg Glu Ser
            660

<210> 24
<211> 659
<212> PRT
<213> Hepatitis E virus

<400> 24

```
Met Arg Pro Arg Pro Leu Leu Leu Leu Phe Leu Leu Phe Leu Pro Met
1               5               10                  15

Leu Pro Ala Pro Pro Thr Gly Gln Pro Ser Gly Arg Arg Arg Gly Arg
            20              25                  30

Arg Ser Gly Gly Thr Gly Gly Gly Phe Trp Gly Asp Arg Val Asp Ser
        35              40                  45

Gln Pro Phe Ala Ile Pro Tyr Ile His Pro Thr Asn Pro Phe Ala Pro
```

50 55 60

Asp Val Ala Ala Ala Ser Gly Ser Gly Pro Arg Leu Arg Gln Pro Ala
65 70 75 80

Arg Pro Leu Gly Ser Thr Trp Arg Asp Gln Ala Gln Arg Pro Ser Ala
85 90 95

Ala Ser Arg Arg Arg Pro Ala Thr Ala Gly Ala Ala Ala Leu Thr Ala
100 105 110

Val Ala Pro Ala His Asp Thr Ser Pro Val Pro Asp Val Asp Ser Arg
115 120 125

Gly Ala Ile Leu Arg Arg Gln Tyr Asn Leu Ser Thr Ser Pro Leu Thr
130 135 140

Ser Ser Val Ala Ser Gly Thr Asn Leu Val Leu Tyr Ala Ala Pro Leu
145 150 155 160

Asn Pro Pro Leu Pro Leu Gln Asp Gly Thr Asn Thr His Ile Met Ala
165 170 175

Thr Glu Ala Ser Asn Tyr Ala Gln Tyr Arg Val Ala Arg Ala Thr Ile
180 185 190

Arg Tyr Arg Pro Leu Val Pro Asn Ala Val Gly Gly Tyr Ala Ile Ser
195 200 205

Ile Ser Phe Trp Pro Gln Thr Thr Thr Pro Thr Ser Val Asp Met
210 215 220

Asn Ser Ile Thr Ser Thr Asp Val Arg Ile Leu Val Gln Pro Gly Ile
225 230 235 240

Ala Ser Glu Leu Val Ile Pro Ser Glu Arg Leu His Tyr Arg Asn Gln
245 250 255

Gly Trp Arg Ser Val Glu Thr Ser Gly Val Ala Glu Glu Glu Ala Thr
260 265 270

Ser Gly Leu Val Met Leu Cys Ile His Gly Ser Pro Val Asn Ser Tyr
275 280 285

Thr Asn Thr Pro Tyr Thr Gly Ala Leu Gly Leu Leu Asp Phe Ala Leu
290 295 300

```
Glu Leu Glu Phe Arg Asn Leu Thr Thr Cys Asn Thr Asn Thr Arg Val
305             310             315             320

Ser Arg Tyr Ser Ser Thr Ala Arg His Ser Ala Arg Gly Ala Asp Gly
                325             330             335

Thr Ala Glu Leu Thr Thr Thr Ala Ala Thr Arg Phe Met Lys Asp Leu
            340             345             350

His Phe Thr Gly Leu Asn Gly Val Gly Glu Val Gly Arg Gly Ile Ala
            355             360             365

Leu Thr Leu Leu Asn Leu Ala Asp Thr Leu Leu Gly Gly Leu Pro Thr
            370             375             380

Glu Leu Ile Ser Ser Ala Gly Gly Gln Leu Phe Tyr Ser Arg Pro Val
385             390             395             400

Val Ser Ala Asn Gly Glu Pro Thr Val Lys Leu Tyr Thr Ser Val Glu
                405             410             415

Asn Ala Gln Gln Asp Lys Gly Val Ala Ile Pro His Asp Ile Asp Leu
            420             425             430

Gly Asp Ser Arg Val Val Ile Gln Asp Tyr Asp Asn Gln His Glu Gln
            435             440             445

Asp Arg Pro Thr Pro Ser Pro Ala Pro Ser Arg Pro Phe Ser Val Leu
            450             455             460

Arg Ala Asn Asp Val Leu Trp Leu Ser Leu Thr Ala Ala Glu Tyr Asp
465             470             475             480

Gln Ser Thr Tyr Gly Ser Ser Thr Gly Pro Val Tyr Ile Ser Asp Ser
                485             490             495

Val Thr Leu Val Asn Val Ala Thr Gly Ala Gln Ala Val Ala Arg Ser
            500             505             510

Leu Asp Trp Ser Lys Val Thr Leu Asp Gly Arg Pro Leu Pro Thr Val
            515             520             525

Glu Gln Tyr Ser Lys Thr Phe Phe Val Leu Pro Leu Arg Gly Lys Leu
            530             535             540

Ser Phe Trp Glu Ala Gly Thr Thr Lys Ala Gly Tyr Pro Tyr Asn Tyr
545             550             555             560
```

Asn Thr Thr Ala Ser Asp Gln Ile Leu Ile Glu Asn Ala Ala Gly His
                565                 570                 575

Arg Val Ala Ile Ser Thr Tyr Thr Thr Arg Leu Gly Ala Gly Pro Val
                580                 585                 590

Ala Ile Ser Ala Ala Ala Val Leu Ala Pro Arg Ser Ala Leu Ala Leu
                595                 600                 605

Leu Glu Asp Thr Phe Asp Tyr Pro Gly Arg Ala His Thr Phe Asp Asp
                610                 615                 620

Phe Cys Pro Glu Cys Arg Ala Leu Gly Leu Gln Gly Cys Ala Phe Gln
625                 630                 635                 640

Ser Thr Val Ala Glu Leu Gln Arg Leu Lys Val Lys Val Gly Lys Thr
                645                 650                 655

Arg Glu Leu

<210> 25
<211> 12
<212> PRT
<213> artificial sequence

<220>
<223> HEV peptide

<220>
<221> MISC_FEATURE
<222> (6)..(6)
<223> X représente P, T, S ou A

<220>
<221> MISC_FEATURE
<222> (9)..(9)
<223> X représente L ou F

<400> 25
Cys Pro Glu Cys Arg Xaa Leu Gly Xaa Gln Gly Cys

1 5 10

<210> 26
<211> 267
<212> PRT
<213> Hepatitis E virus

<400> 26

Gln Leu Phe Tyr Ser Arg Pro Val Val Ser Ala Asn Gly Glu Pro Thr
1                 5                 10                 15

```
Val Lys Leu Tyr Thr Ser Val Glu Asn Ala Gln Gln Asp Lys Gly Ile
        20                  25                  30

Ala Ile Pro His Asp Ile Asp Leu Gly Glu Ser Arg Val Val Ile Gln
        35                  40                  45

Asp Tyr Asp Asn Gln His Glu Gln Asp Arg Pro Thr Pro Ser Pro Ala
        50                  55                  60

Pro Ser Arg Pro Phe Ser Val Leu Arg Ala Asn Asp Val Leu Trp Leu
65              70                  75                  80

Ser Leu Thr Ala Ala Glu Tyr Asp Gln Ser Thr Tyr Gly Ser Ser Thr
                85                  90                  95

Gly Pro Val Tyr Val Ser Asp Ser Val Thr Leu Val Asn Val Ala Thr
            100                 105                 110

Gly Ala Gln Ala Val Ala Arg Ser Leu Asp Trp Thr Lys Val Thr Leu
        115                 120                 125

Asp Gly Arg Pro Leu Ser Thr Thr Gln Gln Tyr Ser Lys Thr Phe Phe
        130                 135                 140

Val Leu Pro Leu Arg Gly Lys Leu Ser Phe Trp Glu Ala Gly Thr Thr
145                 150                 155                 160

Lys Ala Gly Tyr Pro Tyr Asn Tyr Asn Thr Thr Ala Ser Asp Gln Leu
                165                 170                 175

Leu Val Glu Asn Ala Ala Gly His Arg Val Ala Ile Ser Thr Tyr Thr
            180                 185                 190

Thr Ser Leu Gly Ala Gly Pro Val Ser Ile Ser Ala Val Ala Val Leu
        195                 200                 205

Ala Pro His Ser Ala Leu Ala Leu Leu Glu Asp Thr Met Asp Tyr Pro
        210                 215                 220

Ala Arg Ala His Thr Phe Asp Asp Phe Cys Pro Glu Cys Arg Pro Leu
225                 230                 235                 240

Gly Leu Gln Gly Cys Ala Phe Gln Ser Thr Val Ala Glu Leu Gln Arg
                245                 250                 255

Leu Lys Met Lys Val Gly Lys Thr Arg Glu Leu
```

260                           265

<210> 27
<211> 267
<212> PRT
<213> artificial sequence

<220>
<223> HEV Peptide muté

<400> 27

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
        35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Ser Thr Tyr Gly Ser Ser Thr
            85              90              95

Gly Pro Val Tyr Val Ser Asp Ser Val Thr Leu Val Asn Val Ala Thr
            100             105             110

Gly Ala Gln Ala Val Ala Arg Ser Leu Asp Trp Thr Lys Val Thr Leu
            115             120             125

Asp Gly Arg Pro Leu Ser Thr Thr Gln Gln Tyr Ser Lys Thr Phe Phe
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Leu
            165             170             175

Leu Val Glu Asn Ala Ala Gly His Arg Val Ala Ile Ser Thr Tyr Thr
            180             185             190

Thr Ser Leu Gly Ala Gly Pro Val Ser Ile Ser Ala Val Ala Val Leu
            195             200             205
```

```
Ala Pro His Ser Ala Leu Ala Leu Leu Glu Asp Thr Met Asp Tyr Pro
    210             215             220
```

```
Ala Arg Ala His Thr Phe Asp Asp Phe Ser Pro Glu Ser Arg Pro Leu
225             230             235             240
```

```
Gly Leu Gln Gly Ser Ala Phe Gln Ser Thr Val Ala Glu Leu Gln Arg
            245             250             255
```

```
Leu Lys Met Lys Val Gly Lys Thr Arg Glu Leu
            260             265
```

<210> 28
<211> 267
<212> PRT
<213> artificial sequence

<220>
<223> Hepatitis E virus

<400> 28

```
Gln Leu Phe Tyr Ser Arg Pro Val Val Ser Ala Asn Gly Glu Pro Thr
1               5               10              15
```

```
Val Lys Leu Tyr Thr Ser Val Glu Asn Ala Gln Gln Asp Lys Gly Ile
            20              25              30
```

```
Ala Ile Pro His Asp Ile Asp Leu Gly Glu Ser Arg Val Val Ile Gln
            35              40              45
```

```
Asp Tyr Asp Asn Gln His Glu Gln Asp Arg Pro Thr Pro Ser Pro Ala
    50              55              60
```

```
Pro Ser Arg Pro Phe Ser Val Leu Arg Ala Asn Asp Val Leu Trp Leu
65              70              75              80
```

```
Ser Leu Thr Ala Ala Glu Tyr Asp Gln Thr Thr Tyr Gly Ser Ser Thr
            85              90              95
```

```
Asn Pro Met Tyr Val Ser Asp Thr Val Thr Phe Val Asn Val Ala Thr
            100             105             110
```

```
Gly Ala Gln Gly Val Ser Arg Ser Leu Asp Trp Ser Lys Val Thr Leu
            115             120             125
```

```
Asp Gly Arg Pro Leu Met Thr Ile Gln Gln Tyr Ser Lys Thr Phe Phe
    130             135             140
```

99

```
Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
            165             170             175

Leu Ile Glu Asn Ala Ala Gly His Arg Val Cys Ile Ser Thr Tyr Thr
            180             185             190

Thr Asn Leu Gly Ser Gly Pro Val Ser Ile Ser Ala Val Gly Val Leu
            195             200             205

Ala Pro His Ser Ala Leu Ala Ala Leu Glu Asp Thr Val Asp Tyr Pro
    210             215             220

Ala Arg Ala His Thr Phe Asp Asp Phe Cys Pro Glu Cys Arg Ala Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Arg Glu Tyr
            260             265
```

<210> 29
<211> 267
<212> PRT
<213> artificial sequence

<220>
<223> Hepatitis E virus

<400> 29

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
            35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80
```

```
Ser Leu Thr Ala Ala Glu Tyr Asp Gln Thr Thr Tyr Gly Ser Ser Thr
            85                  90                  95

Asn Pro Met Tyr Val Ser Asp Thr Val Thr Phe Val Asn Val Ala Thr
            100                 105                 110

Gly Ala Gln Gly Val Ser Arg Ser Leu Asp Trp Ser Lys Val Thr Leu
            115                 120                 125

Asp Gly Arg Pro Leu Thr Thr Ile Gln Gln Tyr Ser Lys Thr Phe Phe
        130                 135                 140

Val Leu Pro Leu Arg Gly Lys Leu Ser Phe Trp Glu Ala Gly Thr Thr
145                 150                 155                 160

Lys Ala Gly Tyr Pro Tyr Asn Tyr Asn Thr Thr Ala Ser Asp Gln Ile
                165                 170                 175

Leu Ile Glu Asn Ala Ala Gly His Arg Val Cys Ile Ser Thr Tyr Thr
            180                 185                 190

Thr Asn Leu Gly Ser Gly Pro Val Ser Ile Ser Ser Val Gly Val Leu
            195                 200                 205

Ala Pro His Ser Ala Leu Ala Ala Leu Glu Asp Thr Val Asp Tyr Pro
        210                 215                 220

Ala Arg Ala His Thr Phe Asp Asp Phe Cys Pro Glu Cys Arg Thr Leu
225                 230                 235                 240

Gly Leu Gln Gly Cys Ala Phe Gln Ser Thr Val Ala Glu Leu Gln Arg
                245                 250                 255

Leu Lys Met Lys Val Gly Lys Thr Arg Glu Tyr
            260                 265
```

<210> 30
<211> 267
<212> PRT
<213> artificial sequence

<220>
<223> Hepatitis E virus

<400> 30

```
Gln Leu Phe Tyr Ser Arg Pro Val Val Ser Ala Asn Gly Glu Pro Thr
1               5                   10                  15

Val Lys Leu Tyr Thr Ser Val Glu Asn Ala Gln Gln Asp Lys Gly Ile
```

```
                    20                      25                      30

        Ala Ile Pro His Asp Ile Asp Leu Gly Glu Ser Arg Val Val Ile Gln
                35                  40                  45

        Asp Tyr Asp Asn Gln His Glu Gln Asp Arg Pro Thr Pro Ser Pro Ala
                50                  55                  60

        Pro Ser Arg Pro Phe Ser Val Leu Arg Ala Asn Asp Val Leu Trp Leu
        65                  70                  75                  80

        Ser Leu Thr Ala Ala Glu Tyr Asp Gln Thr Thr Tyr Gly Ser Ser Thr
                        85                  90                  95

        Asn Pro Met Tyr Val Ser Asp Thr Val Thr Phe Val Asn Val Ala Thr
                    100                 105                 110

        Gly Ala Gln Gly Val Ser Arg Ser Leu Asp Trp Ser Lys Val Thr Leu
                    115                 120                 125

        Asp Gly Arg Pro Leu Thr Thr Ile Gln Gln Tyr Ser Lys Thr Phe Phe
                    130                 135                 140

        Val Leu Pro Leu Arg Gly Lys Leu Ser Phe Trp Glu Ala Gly Thr Thr
        145                 150                 155                 160

        Lys Ala Gly Tyr Pro Tyr Asn Tyr Asn Thr Thr Ala Ser Asp Gln Ile
                        165                 170                 175

        Leu Ile Glu Asn Ala Ala Gly His Arg Val Cys Ile Ser Thr Tyr Thr
                    180                 185                 190

        Thr Asn Leu Gly Ser Gly Pro Val Ser Ile Ser Ser Val Gly Val Leu
                    195                 200                 205

        Ala Pro His Ser Ala Leu Ala Ala Leu Glu Asp Thr Val Asp Tyr Pro
                    210                 215                 220

        Ala Arg Ala His Thr Phe Asp Asp Phe Cys Pro Glu Cys Arg Thr Leu
        225                 230                 235                 240

        Gly Leu Gln Gly Cys Ala Phe Gln Ser Thr Val Ala Glu Leu Gln Arg
                        245                 250                 255

        Leu Lys Met Lys Val Gly Lys Thr Arg Glu Tyr
                    260                 265
```

<210> 31
<211> 267
<212> PRT
<213> artificial sequence

<220>
<223> Hepatitis E virus

<400> 31

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
        20              25              30

Ala Ile Pro His Asp Ile Asp Leu Gly Glu Ser Arg Val Val Ile Gln
            35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Thr Thr Tyr Gly Ser Ser Thr
            85              90              95

Asn Pro Met Tyr Val Ser Asp Thr Val Thr Phe Val Asn Val Ala Thr
            100             105             110

Gly Ala Gln Gly Val Ser Arg Ser Leu Asp Trp Ser Lys Val Thr Leu
            115             120             125

Asp Gly Arg Pro Leu Met Thr Ile Gln Gln Tyr Ser Lys Thr Phe Phe
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
            165             170             175

Leu Ile Glu Asn Ala Ala Gly His Arg Val Cys Ile Ser Thr Tyr Thr
            180             185             190

Thr Asn Leu Gly Ser Gly Pro Val Ser Ile Ser Ala Val Gly Val Leu
            195             200             205

Ala Pro His Ser Ala Leu Ala Ala Leu Glu Asp Thr Val Asp Tyr Pro
```

```
                210                   215                    220


       Ala Arg Ala His Thr Phe Asp Asp Phe Cys Pro Glu Cys Arg Thr Leu
       225                 230                 235                 240


       Gly Leu Gln Gly Cys Ala Phe Gln Ser Thr Val Ala Glu Leu Gln Arg
                       245                 250                 255


       Leu Lys Met Lys Val Gly Lys Thr Arg Glu Tyr
                   260                 265
```

<210> 32
<211> 267
<212> PRT
<213> artificial sequence

<220>
<223> Hepatitis E virus

<400> 32

Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
        35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Thr Thr Tyr Gly Ser Ser Thr
            85              90              95

Asn Pro Met Tyr Val Ser Asp Thr Val Thr Phe Val Asn Val Ala Thr
            100             105             110

Gly Ala Gln Gly Val Ser Arg Ser Leu Asp Trp Ser Lys Val Thr Leu
        115             120             125

Asp Gly Arg Pro Leu Met Thr Ile Gln Gln Tyr Ser Lys Thr Phe Phe
    130             135             140

Val Leu Pro Leu Arg Gly Lys Leu Ser Phe Trp Glu Ala Gly Thr Thr
145             150             155             160

```
Lys Ala Gly Tyr Pro Tyr Asn Tyr Asn Thr Thr Ala Ser Asp Gln Ile
                165             170             175

Leu Ile Glu Asn Ala Ala Gly His Arg Val Cys Ile Ser Thr Tyr Thr
            180             185             190

Thr Asn Leu Gly Ser Gly Pro Val Ser Ile Ser Ala Val Gly Val Leu
        195             200             205

Ala Pro His Ser Ala Leu Ala Leu Glu Asp Thr Val Asp Tyr Pro
    210             215             220

Ala Arg Ala His Thr Phe Asp Asp Phe Cys Pro Glu Cys Arg Ala Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Arg Glu Tyr
            260             265
```

<210> 33
<211> 267
<212> PRT
<213> artificial sequence

<220>
<223> Hepatitis E virus

<400> 33

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
            35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Thr Thr Tyr Gly Ser Ser Thr
            85              90              95
```

```
Asn Pro Met Tyr Val Ser Asp Thr Val Thr Phe Val Asn Val Ala Thr
            100             105             110

Gly Ala Gln Gly Val Ser Arg Ser Leu Asp Trp Ser Lys Val Thr Leu
        115             120             125

Asp Gly Arg Pro Leu Thr Thr Ile Gln Gln Tyr Ser Lys Thr Phe Tyr
        130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
                165             170             175

Leu Ile Glu Asn Ala Ala Gly His Arg Val Cys Ile Ser Thr Tyr Thr
            180             185             190

Thr Asn Leu Gly Ser Gly Pro Val Ser Ile Ser Ala Val Gly Val Leu
        195             200             205

Ala Pro His Ser Ala Leu Ala Val Leu Glu Asp Thr Val Asp Tyr Pro
    210             215             220

Ala Arg Ala His Thr Phe Asp Asp Phe Cys Pro Glu Cys Arg Ala Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Arg Glu Tyr
        260             265
```

<210> 34
<211> 267
<212> PRT
<213> artificial sequence

<220>
<223> Hepatitis E virus

<400> 34

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30
```

```
    Ala Ile Pro His Asp Ile Asp Leu Gly Glu Ser Arg Val Val Ile Gln
            35                  40                  45

    Asp Tyr Asp Asn Gln His Glu Gln Asp Arg Pro Thr Pro Ser Pro Ala
            50                  55                  60

    Pro Ser Arg Pro Phe Ser Val Leu Arg Ala Asn Asp Val Leu Trp Leu
    65                  70                  75                  80

    Ser Leu Thr Ala Ala Glu Tyr Asp Gln Thr Thr Tyr Gly Ser Ser Thr
                    85                  90                  95

    Asn Pro Met Tyr Val Ser Asp Thr Val Thr Phe Val Asn Val Ala Thr
                    100                 105                 110

    Gly Ala Gln Gly Val Ser Arg Ser Leu Asp Trp Ser Lys Val Thr Leu
                    115                 120                 125

    Asp Gly Arg Pro Leu Thr Thr Ile Gln Gln Tyr Ser Lys Thr Phe Tyr
            130                 135                 140

    Val Leu Pro Leu Arg Gly Lys Leu Ser Phe Trp Glu Ala Gly Thr Thr
    145                 150                 155                 160

    Lys Ala Gly Tyr Pro Tyr Asn Tyr Asn Thr Thr Ala Ser Asp Gln Ile
                    165                 170                 175

    Leu Ile Glu Asn Ala Ala Gly His Arg Val Cys Ile Ser Thr Tyr Thr
                    180                 185                 190

    Thr Asn Leu Gly Ser Gly Pro Val Ser Ile Ser Ala Val Gly Val Leu
                    195                 200                 205

    Ala Pro His Ser Ala Leu Ala Ile Leu Glu Asp Thr Ala Asp Tyr Pro
            210                 215                 220

    Ala Arg Ala His Thr Phe Asp Asp Phe Cys Pro Glu Cys Arg Ser Leu
    225                 230                 235                 240

    Gly Leu Gln Gly Cys Ala Phe Gln Ser Thr Val Ala Glu Leu Gln Arg
                    245                 250                 255

    Leu Lys Met Lys Val Gly Lys Thr Arg Glu Tyr
                    260                 265
```

<210> 35
<211> 267

<212> PRT
<213> artificial sequence

<220>
<223> Hepatitis E virus

<400> 35

```
Gln Leu Phe Tyr Ser Arg Pro Val Val Ser Ala Asn Gly Glu Leu Thr
1               5               10                  15

Val Lys Leu Tyr Thr Ser Val Glu Asn Ala Gln Gln Asp Lys Gly Val
            20              25              30

Ala Ile Pro His Asp Ile Asp Leu Gly Glu Ser Arg Val Val Ile Gln
            35              40              45

Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Thr Thr Tyr Gly Ser Ser Thr
            85              90              95

Asn Pro Met Tyr Val Ser Asp Thr Val Thr Phe Val Asn Val Ala Thr
            100             105             110

Gly Ala Gln Gly Val Ser Arg Ser Leu Asp Trp Ser Lys Val Thr Leu
            115             120             125

Asp Gly Arg Pro Leu Thr Thr Ile Gln Gln Tyr Ser Lys Thr Phe Tyr
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
            165             170             175

Leu Ile Glu Asn Ala Ala Gly His Arg Val Cys Ile Ser Thr Tyr Thr
            180             185             190

Thr Asn Leu Gly Ser Gly Pro Val Ser Val Ser Ala Val Gly Val Leu
            195             200             205

Ala Pro His Ser Ala Leu Ala Ala Leu Glu Asp Thr Ala Asp Tyr Pro
    210             215             220
```

```
Ala Arg Ala His Thr Phe Asp Asp Phe Cys Pro Glu Cys Arg Ala Leu
225                 230                 235                 240


Gly Leu Gln Gly Cys Ala Phe Gln Ser Thr Val Gly Glu Leu Gln Arg
                245                 250                 255


Leu Lys Met Lys Val Gly Lys Thr Arg Glu Tyr
                260                 265
```

<210> 36
<211> 267
<212> PRT
<213> artificial sequence

<220>
<223> Hepatitis E virus

<400> 36

```
Gln Leu Phe Tyr Ser Arg Pro Val Val Ser Ala Asn Gly Glu Pro Thr
1               5               10                  15

Val Lys Leu Tyr Thr Ser Val Glu Asn Ala Gln Gln Asp Lys Gly Ile
            20              25                  30

Ala Ile Pro His Asp Ile Asp Leu Gly Glu Ser Arg Val Val Ile Gln
            35              40                  45

Asp Tyr Asp Asn Gln His Glu Gln Asp Arg Pro Thr Pro Ser Pro Ala
    50              55                  60

Pro Ser Arg Pro Phe Ser Val Leu Arg Ala Asn Asp Val Leu Trp Leu
65              70                  75                      80

Ser Leu Thr Ala Ala Glu Tyr Asp Gln Thr Thr Tyr Gly Ser Ser Thr
                85                  90                  95

Asn Pro Met Tyr Val Ser Asp Thr Val Thr Phe Val Asn Val Ala Thr
            100                 105                 110

Gly Ala Gln Gly Val Ser Arg Ser Leu Asp Trp Ser Lys Val Thr Leu
            115                 120                 125

Asp Gly Arg Pro Leu Thr Thr Ile Gln Gln Tyr Ser Lys Thr Phe Phe
    130                 135                 140

Val Leu Pro Leu Arg Gly Lys Leu Ser Phe Trp Glu Ala Gly Thr Thr
145                 150                 155                 160

Lys Ala Gly Tyr Pro Tyr Asn Tyr Asn Thr Thr Ala Ser Asp Gln Ile
```

```
                          165                     170                     175


        Leu Ile Glu Asn Ala Ala Gly His Arg Val Cys Ile Ser Thr Tyr Thr
                    180                     185                 190


        Thr Asn Leu Gly Ser Gly Pro Val Ser Ile Ser Ala Val Gly Val Leu
                    195                     200                 205


        Ala Pro His Ser Ala Leu Ala Ala Leu Glu Asp Thr Val Asp Tyr Pro
                210                     215                 220


        Ala Arg Ala His Thr Phe Asp Asp Phe Cys Pro Glu Cys Arg Thr Leu
            225                     230                 235                 240


        Gly Leu Gln Gly Cys Ala Phe Gln Ser Thr Val Ala Glu Leu Gln Arg
                        245                     250                 255


        Leu Lys Met Lys Val Gly Lys Thr Arg Glu Tyr
                        260                     265
```

<210> 37
<211> 264
<212> PRT
<213> artificial sequence

<220>
<223> Hepatitis E virus

<400> 37

```
Gln Leu Phe Tyr Ser Arg Pro Val Val Ser Ala Asn Gly Glu Pro Thr
1               5               10                  15

Val Lys Leu Tyr Thr Ser Val Glu Asn Ala Gln Gln Asp Lys Gly Ile
            20              25                  30

Ala Ile Pro His Asp Ile Asp Leu Gly Glu Ser Arg Val Gly Ile Gln
            35                  40                  45

Asp Tyr Asp Asn Gln His Glu Gln Asp Arg Pro Thr Pro Ser Pro Ala
        50                  55                  60

Pro Ser Arg Pro Phe Ser Val Leu Arg Ala Asn Asp Val Leu Trp Leu
65                  70                  75                  80

Ser Leu Thr Ala Ala Glu Tyr Asp Gln Thr Thr Tyr Gly Ser Ser Thr
                85                  90                  95

Asn Pro Met Tyr Val Ser Asp Thr Val Thr Phe Val Asn Val Ala Thr
                100                 105                 110
```

```
Gly Ala Gln Gly Val Ser Arg Ser Leu Asp Trp Ser Lys Val Thr Leu
        115                 120             125

Asp Gly Arg Ser Leu Thr Thr Ile Gln Gln Tyr Ser Lys Thr Phe Phe
        130                 135             140

Val Leu Pro Leu Arg Gly Lys Leu Ser Phe Trp Glu Ala Gly Thr Thr
145                 150                 155                 160

Lys Ala Gly Tyr Pro Tyr Asn Tyr Asn Thr Thr Ala Ser Asp Gln Ile
                165                 170                 175

Leu Ile Glu Asn Ala Ala Gly His Arg Val Cys Ile Ser Thr Tyr Thr
            180                 185                 190

Thr Asn Leu Gly Ser Gly Pro Val Ser Ile Ser Ala Val Gly Val Leu
        195                 200                 205

Ala Pro His Ser Ala Leu Ala Val Leu Glu Asp Thr Val Asp Tyr Pro
    210                 215                 220

Ala Arg Ala His Thr Phe Asp Asp Phe Cys Pro Glu Cys Arg Ala Leu
225                 230                 235                 240

Gly Leu Gln Gly Cys Ala Phe Gln Ser Thr Val Ala Glu Leu Gln Arg
                245                 250                 255

Leu Lys Met Lys Val Gly Asn His
            260
```

<210> 38
<211> 267
<212> PRT
<213> artificial sequence

<220>
<223> Hepatitis E virus

<400> 38

```
Gln Leu Phe Tyr Ser Arg Pro Val Val Ser Ala Asn Gly Glu Pro Thr
1               5                   10                  15

Val Lys Leu Tyr Thr Ser Val Glu Asn Ala Gln Gln Asp Lys Gly Ile
            20                  25                  30

Ala Ile Pro His Asp Ile Asp Leu Gly Glu Ser Arg Val Val Ile Gln
            35                  40                  45
```

```
Asp Tyr Asp Asn Gln His Glu Gln Asp Arg Pro Thr Pro Ser Pro Ala
    50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Ser Thr Tyr Gly Ser Ser Thr
            85              90              95

Gly Pro Val Tyr Val Ser Asp Ser Val Thr Leu Val Asn Val Ala Thr
            100             105             110

Gly Ala Gln Ala Val Ala Arg Ser Leu Asp Trp Thr Lys Val Thr Leu
        115             120             125

Asp Gly Arg Pro Leu Ser Thr Ile Gln Gln Tyr Ser Lys Thr Phe Phe
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Leu
            165             170             175

Leu Val Glu Asn Ala Ala Gly His Arg Val Ala Ile Ser Thr Tyr Thr
            180             185             190

Thr Ser Leu Gly Ala Gly Pro Val Ser Ile Ser Ala Val Ala Val Leu
        195             200             205

Ala Pro His Ser Ala Leu Ala Leu Leu Glu Asp Thr Leu Asp Tyr Pro
    210             215             220

Ala Arg Ala His Thr Phe Asp Asp Phe Cys Pro Glu Cys Arg Pro Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Arg Glu Leu
            260             265
```

<210> 39
<211> 267
<212> PRT
<213> artificial sequence

<220>

118

<223> Hepatitis E virus

<400> 39

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
          20              25              30

Thr Ile Pro His Asp Ile Asp Leu Gly Asp Ser Arg Val Val Ile Gln
          35              40              45

Asp Tyr Asp Asn Gln His Glu Gln Asp Arg Pro Thr Pro Ser Pro Ala
      50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Thr Thr Tyr Gly Ser Ser Thr
              85              90              95

Asn Pro Met Tyr Val Ser Asp Thr Val Thr Leu Val Asn Val Ala Thr
          100             105             110

Gly Ala Gln Ala Val Ala Arg Ser Leu Asp Trp Ser Lys Val Thr Leu
          115             120             125

Asp Gly Arg Pro Leu Thr Thr Ile Gln Gln Tyr Ser Lys Thr Phe Tyr
      130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
              165             170             175

Leu Ile Glu Asn Ala Ala Gly His Arg Val Ala Ile Ser Thr Tyr Thr
          180             185             190

Thr Ser Leu Gly Ala Gly Pro Thr Ser Ile Ser Ala Val Gly Val Leu
          195             200             205

Ala Pro His Ser Ala Leu Ala Val Leu Glu Asp Thr Val Asp Tyr Pro
      210             215             220

Ala Arg Ala His Thr Phe Asp Asp Phe Cys Pro Glu Cys Arg Thr Leu
225             230             235             240
```

```
        Gly Leu Gln Gly Cys Ala Phe Gln Ser Thr Ile Ala Glu Leu Gln Arg
                        245                 250                 255

        Leu Lys Met Lys Val Gly Lys Thr Arg Glu Ser
                        260                 265
```

\<210\> 40
\<211\> 267
\<212\> PRT
\<213\> artificial sequence

\<220\>
\<223\> Hepatitis E virus

\<400\> 40

```
        Gln Leu Phe Tyr Ser Arg Pro Val Val Ser Ala His Gly Glu Pro Thr
        1                   5                   10                  15

        Val Lys Leu Tyr Thr Ser Val Glu Asn Ala Gln Gln Asp Lys Gly Ile
                        20                  25                  30

        Ala Ile Pro His Asp Ile Asp Leu Gly Glu Ser Arg Val Val Ile Gln
                        35                  40                  45

        Asp Tyr Asp Asn Gln His Glu Gln Asp Arg Pro Thr Pro Ser Pro Ala
                50                  55                  60

        Pro Ser Arg Pro Phe Ser Val Leu Arg Ala Asn Asp Val Leu Trp Leu
        65                  70                  75                  80

        Ser Leu Thr Ala Ala Glu Tyr Asp Gln Ser Thr Tyr Gly Ser Ser Thr
                        85                  90                  95

        Ala Pro Val Tyr Val Ser Asp Ser Val Thr Leu Val Asn Val Ala Thr
                        100                 105                 110

        Gly Ala Gln Ala Val Ala Arg Ser Leu Asp Trp Thr Lys Val Thr Leu
                        115                 120                 125

        Asp Gly Arg Pro Leu Ser Thr Ile Gln Gln Tyr Pro Lys Thr Phe Phe
                130                 135                 140

        Val Leu Pro Leu Arg Gly Lys Leu Ser Phe Trp Glu Ala Gly Thr Thr
        145                 150                 155                 160

        Lys Ala Gly Tyr Pro Tyr Asn Tyr Asn Thr Thr Ala Ser Asp Gln Leu
                        165                 170                 175
```

```
        Leu Val Glu Asn Ala Ala Gly His Arg Val Ala Ile Ser Thr Tyr Thr
                    180                 185                 190

        Thr Ser Leu Gly Ala Gly Pro Val Ser Ile Ser Ala Val Ala Val Leu
                    195                 200                 205

        Ala Pro His Ser Ala Leu Ala Leu Leu Glu Asp Thr Leu Asp Tyr Pro
                    210                 215                 220

        Ala Cys Ala His Thr Phe Asp Asp Phe Cys Pro Glu Cys Arg Pro Leu
        225                 230                 235                 240

        Gly Leu Gln Gly Cys Ala Phe Gln Ser Thr Val Ala Glu Leu Gln Arg
                        245                 250                 255

        Leu Lys Met Lys Val Gly Lys Thr Arg Glu Leu
                    260                 265
```

<210> 41
<211> 267
<212> PRT
<213> artificial sequence

<220>
<223> Hepatitis E virus

<400> 41

```
Gln Leu Phe Tyr Ser Arg Pro Val Val Ser Ala Asn Gly Glu Pro Thr
1               5                   10                  15

Val Lys Leu Tyr Thr Ser Val Glu Asn Ala Gln Gln Asp Lys Gly Ile
            20                  25                  30

Ala Ile Pro Asn Asp Ile Asp Leu Gly Glu Ser Arg Val Val Ile Gln
            35                  40                  45

Asp Tyr Asp Asn Gln His Glu Gln Asp Arg Pro Thr Pro Ser Pro Ala
    50                  55                  60

Pro Ser Arg Pro Phe Ser Val Leu Arg Ala Asn Asp Val Leu Trp Leu
65                  70                  75                  80

Ser Leu Thr Ala Ala Glu Tyr Asp Gln Ser Thr Tyr Gly Ser Ser Thr
                85                  90                  95

Gly Pro Val Tyr Val Ser Asp Ser Val Thr Leu Val Asn Val Ala Thr
            100                 105                 110

Gly Ala Gln Ala Val Ala Arg Ser Leu Asp Trp Thr Lys Val Thr Leu
```

```
                    115                  120                      125


        Asp Gly Arg Pro Leu Ser Thr Ile Gln Gln Tyr Ser Lys Ile Phe Phe
            130                 135                 140


        Val Leu Pro Leu Arg Gly Lys Leu Ser Phe Trp Glu Ala Gly Thr Thr
        145                 150                 155                 160


        Arg Pro Gly Tyr Pro Tyr Asn Tyr Asn Thr Thr Ala Ser Asp Gln Leu
                        165                 170                 175


        Leu Val Glu Asn Ala Ala Gly His Arg Val Ala Ile Ser Thr Tyr Thr
                    180                 185                 190


        Thr Ser Leu Gly Ala Gly Pro Val Ser Ile Ser Ala Val Ala Val Leu
                    195                 200                 205


        Gly Pro His Ser Ala Leu Ala Leu Leu Glu Asp Thr Leu Asp Tyr Pro
            210                 215                 220


        Ala Arg Ala His Thr Phe Asp Asp Phe Cys Pro Glu Cys Arg Pro Leu
        225                 230                 235                 240


        Gly Leu Gln Gly Cys Ala Phe Gln Ser Thr Val Ala Glu Leu Gln Arg
                        245                 250                 255


        Leu Lys Met Lys Val Gly Lys Thr Arg Glu Leu
                        260                 265
```

<210> 42
<211> 267
<212> PRT
<213> artificial sequence

<220>
<223> Hepatitis E virus

<400> 42

EP 3 383 887 B1

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Thr Ile Pro His Asp Ile Asp Leu Gly Asp Ser Arg Val Val Ile Gln
            35              40              45

Asp Tyr Asp Asn Gln His Glu Gln Asp Arg Pro Thr Pro Ser Pro Ala
            50              55              60
```

```
Pro Ser Arg Pro Phe Ser Val Leu Arg Ala Asn Asp Val Leu Trp Leu
65              70              75                          80

Ser Leu Thr Ala Ala Glu Tyr Asp Gln Thr Thr Tyr Gly Ser Ser Thr
            85              90                          95

Asn Pro Met Tyr Val Ser Asp Thr Val Thr Leu Val Asn Val Ala Thr
            100             105             110

Gly Ala Gln Ala Val Ala Arg Ser Leu Asp Trp Ser Lys Val Thr Leu
            115             120             125

Asp Gly Arg Pro Leu Thr Thr Ile Gln Gln Tyr Ser Lys Thr Phe Tyr
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
            165             170             175

Leu Ile Glu Asn Ala Ala Gly His Arg Val Ala Ile Ser Thr Tyr Thr
            180             185             190

Thr Ser Leu Gly Ala Gly Pro Thr Ser Ile Ser Ala Val Gly Val Leu
            195             200             205

Ala Pro His Ser Ala Leu Ala Val Leu Glu Asp Thr Ile Asp Tyr Pro
    210             215             220

Ala Arg Ala His Thr Phe Asp Asp Phe Cys Pro Glu Cys Arg Thr Leu
225             230             235             240

Gly Leu Gln Gly Cys Ala Phe Gln Ser Thr Ile Ala Glu Leu Gln Arg
            245             250             255

Leu Lys Met Lys Val Gly Lys Thr Arg Glu Ser
            260             265
```

<210> 43
<211> 267
<212> PRT
<213> artificial sequence

<220>
<223> Hepatitis E virus

<400> 43

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
            35              40              45

Asp Tyr Asp Asn Gln His Glu Gln Asp Arg Pro Thr Pro Ser Pro Ala
        50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Ser Thr Tyr Gly Ser Ser Thr
            85              90              95

Gly Pro Val Tyr Val Ser Asp Ser Val Thr Leu Val Asn Val Ala Thr
            100             105             110

Gly Ala Gln Ala Val Ala Arg Ser Leu Asp Trp Thr Lys Val Thr Leu
        115             120             125

Asp Gly Arg Pro Leu Ser Thr Ile Gln Gln Tyr Ser Lys Thr Phe Phe
        130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Leu
            165             170             175

Leu Val Glu Asn Ala Ala Gly His Arg Val Ala Ile Ser Thr Tyr Thr
            180             185             190

Thr Ser Leu Gly Ala Gly Pro Val Ser Ile Ser Ala Val Ala Val Leu
        195             200             205

Ala Pro His Ser Val Leu Ala Leu Leu Glu Asp Thr Met Asp Tyr Pro
        210             215             220

Ala Arg Ala His Thr Phe Asp Asp Phe Cys Pro Glu Cys Arg Pro Leu
225             230             235             240

Gly Leu Gln Gly Cys Ala Phe Gln Ser Thr Val Ala Glu Leu Gln Arg
            245             250             255
```

127

```
Leu Lys Met Lys Val Gly Lys Thr Arg Glu Leu
          260                 265
```

<210> 44
<211> 267
<212> PRT
<213> artificial sequence

<220>
<223> Hepatitis E virus

<220>
<221> misc_feature
<222> (88)..(88)
<223> Xaa can be any naturally occurring amino acid

<400> 44

```
Gln Leu Phe Tyr Ser Arg Pro Val Val Ser Ala Asn Gly Glu Pro Thr
1               5               10                  15

Val Lys Leu Tyr Thr Ser Val Glu Asn Ala Gln Gln Asp Lys Gly Ile
            20              25                  30

Thr Ile Pro His Asp Ile Asp Leu Gly Asp Ser Arg Val Val Ile Gln
            35              40                  45

Asp Tyr Asp Asn Gln His Glu Gln Asp Arg Pro Thr Pro Ser Pro Ala
    50              55                  60

Pro Ser Arg Pro Phe Ser Val Leu Arg Ala Asn Asp Val Leu Trp Leu
65              70                  75                  80

Ser Leu Thr Ala Ala Glu Tyr Xaa Gln Thr Thr Tyr Gly Ser Ser Thr
                85              90                  95

Asn Pro Met Tyr Val Ser Asp Thr Val Thr Leu Val Asn Val Ala Thr
            100             105                 110

Gly Ala Gln Ala Val Ala Arg Ser Leu Asp Trp Ser Lys Val Thr Leu
            115             120                 125

Asp Gly Arg Pro Leu Thr Thr Ile Gln Gln Tyr Ser Lys Lys Phe Tyr
    130             135                 140

Val Leu Pro Leu Arg Gly Lys Leu Ser Phe Trp Glu Ala Gly Thr Thr
145             150                 155                 160

Lys Ala Gly Tyr Pro Tyr Asn Tyr Asn Thr Thr Ala Ser Asp Gln Ile
```

                        165                     170                     175


        Leu Ile Glu Asn Ala Ala Gly His Arg Val Ala Ile Ser Thr Tyr Thr
                        180                     185                 190


        Thr Ser Leu Gly Ala Gly Pro Thr Ser Ile Ser Ala Val Gly Val Leu
                    195                 200                 205


        Ala Pro His Ser Ala Leu Ala Val Leu Glu Asp Thr Val Asp Tyr Pro
            210                 215                 220


        Ala Arg Ala His Thr Phe Asp Asp Phe Cys Pro Glu Cys Arg Thr Leu
        225                 230                 235                 240


        Gly Leu Gln Gly Cys Ala Phe Gln Ser Thr Ile Ala Glu Leu Gln Arg
                        245                 250                 255


        Leu Lys Met Lys Val Gly Lys Thr Arg Glu Ser
                    260                 265

<210> 45
<211> 267
<212> PRT
<213> artificial sequence

<220>
<223> Hepatitis E virus

<400> 45

```
Gln Leu Phe Tyr Ser Arg Pro Val Val Ser Ala Asn Gly Glu Pro Thr
1               5                   10                  15

Val Lys Leu Tyr Thr Ser Val Glu Asn Ala Gln Gln Asp Lys Gly Ile
                20                  25                  30

Ala Ile Pro His Asp Ile Asp Leu Gly Glu Ser Arg Val Val Ile Gln
            35                  40                  45

Asp Tyr Asp Asn Gln His Glu Gln Asp Arg Pro Thr Pro Ser Pro Ala
        50                  55                  60

Pro Ser Arg Pro Phe Ser Val Leu Arg Ala Asn Asp Val Leu Trp Leu
65                  70                  75                  80

Ser Leu Thr Ala Ala Glu Tyr Asp Gln Thr Thr Tyr Gly Ser Ser Thr
                85                  90                  95

Asn Pro Met Tyr Val Ser Asp Thr Val Thr Phe Val Asn Val Ala Thr
                100                 105                 110
```

```
Gly Thr Gln Gly Val Ser Arg Ser Leu Asp Trp Ser Lys Val Thr Leu
        115             120             125

Asp Gly Arg Pro Leu Thr Thr Ile Gln Gln Tyr Ser Lys Thr Phe Phe
        130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Ile
                165             170             175

Leu Ile Glu Asn Ala Pro Gly His Arg Val Cys Ile Ser Thr Tyr Thr
            180             185             190

Thr Asn Leu Gly Ser Gly Pro Val Ser Ile Ser Ala Val Gly Val Leu
        195             200             205

Ala Pro His Ser Ala Leu Ala Ala Leu Glu Asp Thr Val Asp Tyr Pro
    210             215             220

Ala Arg Ala His Thr Phe Asp Asp Phe Cys Pro Glu Cys Arg Ala Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Gln Glu Tyr
            260             265
```

<210> 46
<211> 267
<212> PRT
<213> artificial sequence

<220>
<223> Hepatitis E virus

<400> 46

```
Gln Leu Phe Tyr Ser Arg Pro Val Val Ser Ala Asn Gly Glu Pro Thr
1               5               10              15

Val Lys Leu Tyr Thr Ser Val Glu Asn Ala Gln Gln Asp Lys Gly Ile
            20              25              30

Ala Ile Pro His Asp Ile Asp Leu Gly Glu Ser Arg Val Val Ile Gln
            35              40              45
```

131

```
Asp Tyr Asp Asn Gln His Glu Gln Asp Arg Pro Thr Pro Ser Pro Ala
        50              55              60

Pro Ser Arg Pro Phe Ser Val Leu Arg Ala Asn Asp Val Leu Trp Leu
65              70              75              80

Ser Leu Thr Ala Ala Glu Tyr Asp Gln Ser Thr Tyr Gly Ser Ser Thr
            85              90              95

Gly Pro Val Tyr Val Ser Asp Ser Val Thr Leu Val Asn Val Ala Thr
            100             105             110

Gly Ala Gln Ala Val Ala Arg Ser Leu Asp Trp Thr Lys Val Thr Leu
        115             120             125

Asp Gly Arg Pro Leu Ser Thr Ile Gln Gln Tyr Ser Lys Thr Phe Phe
    130             135             140

Val Leu Pro Leu Arg Gly Lys Leu Ser Phe Trp Glu Ala Gly Thr Thr
145             150             155             160

Lys Ala Gly Tyr Pro Tyr Asn Tyr Asn Thr Thr Ala Ser Asp Gln Leu
            165             170             175

Leu Ile Glu Asn Ala Ala Gly His Arg Val Ala Ile Ser Thr Tyr Thr
            180             185             190

Thr Ser Leu Gly Ala Gly Pro Val Ala Ile Ser Ala Val Ala Val Leu
        195             200             205

Ala Pro His Ser Ala Leu Ala Leu Leu Glu Asp Thr Met Asp Tyr Pro
    210             215             220

Ala Arg Ala His Thr Phe Asp Asp Phe Cys Pro Glu Cys Arg Pro Leu
225             230             235             240

Gly Leu Gln Gly Cys Ala Phe Gln Ser Thr Val Ala Glu Leu Gln Arg
            245             250             255

Leu Lys Met Lys Val Gly Lys Thr Arg Glu Leu
            260             265
```

<210> 47
<211> 267
<212> PRT
<213> artificial sequence

<220>

<223> Hepatitis E virus

<400> 47

```
Gln Leu Phe Tyr Ser Arg Pro Val Ala Ser Ala Asn Gly Glu Pro Thr
1               5               10                  15

Val Lys Leu Tyr Thr Ser Val Glu Asn Ala Gln Gln Asp Lys Gly Ile
            20              25                  30

Thr Ile Pro His Asp Ile Asp Leu Gly Asp Ser Arg Val Val Ile Gln
        35              40                  45

Asp Tyr Asp Asn Gln His Glu Gln Asp Arg Pro Thr Pro Ser Pro Ala
    50              55                  60

Pro Ser Arg Pro Phe Ser Val Leu Arg Ala Asn Asp Val Leu Trp Leu
65              70              75                  80

Ser Leu Thr Val Ala Glu Tyr Asp Gln Thr Thr Tyr Gly Ser Ser Thr
            85              90                  95

Asn Pro Met Tyr Val Ser Asp Thr Ala Thr Phe Val Asn Val Ala Thr
        100             105                 110

Gly Ala Gln Ala Val Ala Arg Ser Leu Asp Trp Ser Lys Val Thr Leu
        115             120                 125

Asp Gly Arg Pro Leu Thr Thr Ile Gln Gln Tyr Ser Lys Thr Phe Tyr
    130             135                 140

Val Leu Pro Leu Arg Gly Lys Leu Ser Phe Trp Glu Ala Gly Thr Thr
145             150             155                 160

Lys Ala Gly Tyr Pro Tyr Asn Tyr Asn Thr Ala Ala Ser Asp Gln Ile
            165             170                 175

Leu Ile Glu Asn Ala Ala Gly His Arg Val Ala Ile Ser Thr Tyr Thr
        180             185                 190

Thr Ser Leu Gly Ala Ser Pro Thr Ser Ile Ser Ala Val Gly Val Leu
        195             200                 205

Ala Pro His Ser Ala Leu Ala Val Leu Glu Asp Thr Val Asp Tyr Pro
    210             215                 220

Ala Arg Ala His Thr Phe Asp Asp Phe Cys Pro Glu Cys Arg Thr Leu
225             230             235                 240
```

134

Gly Leu Gln Gly Cys Ala Phe Gln Ser Thr Ile Ala Glu Leu Gln Arg
                    245                 250                 255

Leu Lys Met Lys Val Gly Lys Thr Arg Glu Ser
                260                 265

<210> 48
<211> 267
<212> PRT
<213> artificial sequence

<220>
<223> Hepatitis E virus

<400> 48

Gln Leu Phe Tyr Ser Arg Pro Val Val Ser Ala Asn Gly Glu Pro Thr
1                   5                   10                  15

Val Lys Leu Tyr Thr Ser Val Glu Asn Ala Gln Gln Asp Lys Gly Ile
                20                  25                  30

Thr Ile Pro His Asp Ile Asp Leu Gly Asp Ser Arg Val Val Ile Gln
                35                  40                  45

Asp Tyr Asp Asn Gln His Glu Gln Asp Arg Pro Thr Pro Ser Pro Ala
        50                  55                  60

Pro Ser Arg Pro Phe Ser Val Leu Arg Ala Asn Asp Val Leu Trp Leu
65                  70                  75                  80

Ser Leu Thr Ala Ala Glu Tyr Asp Gln Thr Thr Tyr Gly Ser Ser Thr
                85                  90                  95

Asn Pro Met Tyr Val Ser Asp Thr Val Thr Leu Val Asn Val Ala Thr
                100                 105                 110

Gly Ala Gln Ala Val Ala Arg Ser Leu Asp Trp Ser Lys Val Thr Leu
                115                 120                 125

Asp Gly Arg Pro Leu Thr Thr Ile Gln Gln Tyr Ser Lys Thr Phe Tyr
        130                 135                 140

Val Leu Pro Leu Arg Gly Lys Leu Ser Phe Trp Glu Ala Gly Thr Thr
145                 150                 155                 160

Lys Ala Gly Tyr Pro Tyr Asn Tyr Asn Thr Thr Ala Ser Asp Gln Ile
                165                 170                 175

```
Leu Ile Glu Asn Ala Ser Gly His Arg Val Ala Ile Ser Thr Tyr Thr
            180                 185                 190

Thr Ser Leu Gly Ala Gly Pro Thr Ser Ile Ser Ala Val Gly Val Leu
            195                 200                 205

Ala Pro His Ser Ala Leu Ala Val Leu Glu Asp Thr Ile Asp Tyr Pro
    210                 215                 220

Ala Arg Ala His Thr Phe Asp Asp Phe Cys Pro Glu Cys Arg Ala Leu
225                 230                 235                 240

Gly Phe Gln Gly Cys Ala Phe Gln Ser Thr Ile Ala Glu Leu Gln Arg
                245                 250                 255

Leu Lys Met Lys Val Gly Lys Thr Arg Glu Ser
            260                 265
```

<210> 49
<211> 267
<212> PRT
<213> artificial sequence

<220>
<223> Hepatitis E virus

<400> 49

Gln Leu Phe Tyr Ser Arg Pro Val Val Ser Ala Asn Gly Glu Pro Thr
1               5                   10                  15

Val Lys Leu Tyr Thr Ser Val Glu Asn Ala Gln Gln Asp Lys Gly Ile
                20                  25                  30

Thr Ile Pro His Asp Ile Asp Leu Gly Asp Ser Arg Val Val Ile Gln
            35                  40                  45

Asp Tyr Asp Asn Gln His Glu Gln Asp Arg Pro Thr Pro Ser Pro Ala
        50                  55                  60

Pro Ser Arg Pro Phe Ser Val Leu Arg Ala Asn Asp Val Leu Trp Leu
65                  70                  75                  80

Ser Leu Thr Ala Ala Glu Tyr Asp Gln Thr Thr Tyr Gly Ser Ser Thr
                85                  90                  95

Asn Pro Met Tyr Val Ser Asp Thr Val Thr Phe Val Asn Val Ala Thr
            100                 105                 110

Gly Ala Gln Ala Val Ala Arg Ser Leu Asp Trp Ser Lys Val Thr Leu

                    115                    120                    125

Asp Gly Arg Pro Leu Thr Thr Ile Gln Gln Tyr Ser Lys Thr Phe Tyr
    130             135                 140

Val Leu Pro Leu Arg Gly Lys Leu Ser Phe Trp Glu Ala Gly Thr Thr
145             150                 155                 160

Lys Ala Gly Tyr Pro Tyr Asn Tyr Asn Thr Thr Ala Ser Asp Gln Ile
            165             170                 175

Leu Ile Glu Asn Ala Ala Gly His Arg Val Ala Ile Ser Thr Tyr Thr
            180             185                 190

Thr Ser Leu Gly Ala Gly Pro Thr Ser Ile Ser Ala Val Gly Val Leu
        195             200                 205

Ala Pro His Ser Ala Leu Ala Val Leu Glu Asp Thr Val Asp Tyr Pro
    210             215                 220

Ala Arg Ala His Thr Phe Asp Asp Phe Cys Pro Glu Cys Arg Ala Leu
225             230                 235                 240

Gly Leu Gln Gly Cys Ala Phe Gln Ser Thr Val Ala Glu Leu Gln Arg
            245             250                 255

Leu Lys Met Lys Val Gly Lys Thr Arg Glu Ser
            260             265

<210> 50
<211> 267
<212> PRT
<213> artificial sequence

<220>
<223> Hepatitis E virus

<400> 50

Gln Leu Phe Tyr Ser Arg Pro Val Val Ser Ala Asn Gly Glu Pro Thr
1                   5                   10                  15

Val Lys Leu Tyr Thr Ser Val Glu Asn Ala Gln Gln Asp Lys Gly Val
                20                  25                  30

Ala Ile Pro His Asp Ile Asp Leu Gly Asp Ser Arg Val Val Ile Gln
                35                  40                  45

Asp Tyr Asp Asn Gln His Glu Gln Asp Arg Pro Thr Pro Ser Pro Ala
        50                  55                  60

```
Pro Ser Arg Pro Phe Ser Val Leu Arg Ala Asn Asp Val Leu Trp Leu
65              70          75              80

Ser Leu Thr Ala Ala Glu Tyr Asp Gln Ser Thr Tyr Gly Ser Ser Thr
            85          90              95

Gly Pro Val Tyr Ile Ser Asp Ser Val Thr Leu Val Asn Val Ala Thr
            100         105             110

Gly Ala Gln Ala Val Ala Arg Ser Leu Asp Trp Ser Lys Val Thr Leu
            115         120             125

Asp Gly Arg Pro Leu Pro Thr Val Glu Gln Tyr Ser Lys Thr Phe Phe
    130         135             140

Val Leu Pro Leu Arg Gly Lys Leu Ser Phe Trp Glu Ala Gly Thr Thr
145         150             155             160

Lys Ala Gly Tyr Pro Tyr Asn Tyr Asn Thr Thr Ala Ser Asp Gln Ile
            165         170             175

Leu Ile Glu Asn Ala Ala Gly His Arg Val Ala Ile Ser Thr Tyr Thr
            180         185             190

Thr Arg Leu Gly Ala Gly Pro Val Ala Ile Ser Ala Ala Ala Val Leu
    195             200             205

Ala Pro Arg Ser Ala Leu Ala Leu Leu Glu Asp Thr Phe Asp Tyr Pro
    210             215             220

Gly Arg Ala His Thr Phe Asp Asp Phe Cys Pro Glu Cys Arg Ala Leu
225             230             235             240

Gly Leu Gln Gly Cys Ala Phe Gln Ser Thr Val Ala Glu Leu Gln Arg
            245             250             255

Leu Lys Val Lys Val Gly Lys Thr Arg Glu Leu
    260             265
```

**Description**

La présente invention concerne le domaine des infections par le virus de l'hépatite E (HEV). En particulier, l'invention concerne la détection des hépatites dues au virus de l'hépatite E.

Les hépatites sont des lésions inflammatoires du foie dont les causes peuvent être multiples : infectieuses, médicamenteuses, auto-immunes, etc. Les atteintes hépatiques aiguës d'origine virale sont fréquentes, souvent asymptomatiques. Elles sont dues soit à une action cytopathique directe du virus, soit, le plus souvent, à la réaction immunitaire dirigée contre les cellules hépatiques infectées. Les symptômes, quand ils existent, associent un ictère fébrile, prurigineux, une décoloration des selles, un brunissement des urines et une augmentation plus ou moins importante des transaminases, témoignant de la cytolyse et du dysfonctionnement hépatique.

De nombreux virus sont capables d'entraîner des lésions hépatiques, par exemple le virus d'Epstein-Barr (EBV) ou le Cytomégalovirus (CMV), mais seuls six virus sont reconnus comme responsables de ce que l'on appelle communément les "hépatites virales". Ces virus sont les virus des hépatites A, B, C, Delta, E et G, lesquels sont des virus appartenant à des familles bien différentes.

Le virus de l'hépatite G est très peu décrit.

Le virus de l'hépatite A, ou VHA ou HAV, appartient à la famille des Picornaviridae et est le seul représentant du genre Hepatovirus. Il s'agit d'un virus nu à ARN. Le réservoir de virus est le sujet infecté, malade ou non. Les modes de transmission sont déterminés par l'exceptionnelle résistance du virus et sa concentration élevée dans les selles. Le principal mode de transmission est essentiellement fécal-oral. Un risque particulier est lié à la consommation de coquillages et de crudités souillées.

Le virus de l'hépatite B, ou VHB ou HBV, appartient à la famille des hepadnaviridæ. Il s'agit d'un virus à ADN circulaire, bicaténaire sur les 3/4 de sa circonférence. Ce virus expose au risque d'hépatite fulminante, d'hépatite chronique active, de cirrhose et d'hépatocarcinome. Le principal vecteur du virus est le sang, mais il peut être transmis par voie sexuelle. Au niveau mondial, on estime à 350 millions le nombre de personnes infectées chroniquement par ce virus et qu'il est à l'origine de plus d'un million de décès annuellement.

Le virus de l'hépatite C, ou VHC ou HCV ou NANBH pour « Non-A, Non-B Hepatitis », virus à génome à ARN de polarité positive, a une organisation proche de celle des flavivirus avec 9500 nucléotides (9,5 kb), des extrémités 5' et 3' non codantes, et en partant de l'extrémité 5' des gènes de capside (C), d'enveloppe (El et E2) et de protéines non structurales (NS1 à NS5). L'HCV est un virus strictement humain. Le mode de contamination se fait principalement par voie veineuse, par exemple par utilisation d'aiguilles non stérilisées, la contamination par transfusion de sang étant encore présente dans les pays en développement où il n'y a pas de dépistage des donneurs. L'élément le plus inquiétant de l'hépatite C est, qu'au-delà d'une primo-infection généralement asymptomatique (90 % des cas), l'évolution se fait dans 70 à 80 % des cas vers la chronicité, avec chez 20 % des infectés chroniques un risque de cirrhose et de cancer primitif du foie après une incubation de 20 ans en moyenne pour la cirrhose et de 30 ans pour le cancer.

Le virus de l'hépatite DELTA, ou VHD ou HDV, est un très petit virus à ARN, incapable de se répliquer sans l'HBV qui lui prête son antigène de surface HBs. L'infection à virus DELTA ne survient qu'en même temps qu'une infection à HBV dont le pronostic s'en trouve aggravé : risque accru d'hépatite fulminante et de passage à l'hépatite chronique active.

Le virus de l'hépatite E, ou VHE ou HEV ou ET-NANBH pour « Enterically Transmitted Non-A, Non-B Hepatitis », est un petit virus nu non enveloppé dont le génome est un ARN simple brin de polarité positive. Initialement classé dans la famille des Caliciviridae dont il est proche, la connaissance de son génome entier conduit aujourd'hui à le classer à part, comme seul membre du genre *Hepevirus,* de la famille des Hepeviridæ (Emerson, S. U., & Purcell, R. H., 2007). La transmission inter-humaine de ce virus se fait principalement par voie fécale-orale (eau souillée, aliments). Les infections sont endémiques dans certaines régions d'Asie, d'Afrique et d'Amérique centrale et du Sud. Le virus de l'hépatite E est identifié comme l'agent principal d'épidémies d'hépatites aiguës dans les pays à faible niveau d'hygiène. Plus récemment, il a été clairement défini comme responsable de véritables cas sporadiques d'hépatites aiguës dans les pays industrialisés chez des patients n'ayant jamais séjourné en zone d'endémie. Il est actuellement clairement démontré que l'hépatite E est une zoonose et que de nombreuses espèces animales domestiquées et sauvages sont infectées par HEV, constituant le réservoir de virus. L'hépatite E, comme l'hépatite A, ne passe généralement pas à la chronicité, sauf pour certains groupes de patients comme ceux ayant reçu un transplant d'organe solide. Il a toutefois une particularité mal expliquée : bien que généralement spontanément résolutive, il a été observé que, en Inde, la mortalité pouvait atteindre 20% chez les femmes enceintes, à mesure que l'âge gestationnel augmente, ce qui pourrait faire de l'infection à HEV l'hépatite la plus grave de toutes les hépatites virales pendant la grossesse. Il est donc fondamental de disposer d'outils de détection de l'infection par HEV performants et fiables.

Le génome du virus de l'hépatite E a une longueur approximative de 7,5 kb et présente 3 cadres de lectures (ORF1, ORF2 et ORF3) partiellement chevauchants encadrés à l'extrémité 5' d'une séquence non codante de 27 à 32 nucléotides et à l'extrémité 3' d'une séquence de 65 à 74 bases suivie par une extrémité polyadénylée de longueur variable selon les virus. L'ORF1 code pour une polyprotéine de 186 kDa environ, appelée protéine p-ORF1, ultérieurement clivée en protéines non structurales dont une méthyl-transférase, démontrant que le virus est coiffé à son extrémité 5', et l'ARN polymérase ARN dépendante. L'ORF2 code pour la protéine de capside glycosylée, appelée protéine p-ORF2, ayant de 659 à 674 acides aminés selon les variants décrits à ce jour, la majorité des protéines p-ORF2 des variants ayant 660 acides aminés. Cette protéine p-ORF2 présente plusieurs sites immunogènes dont un épitope immunodominant conformationnel entre les acides aminés 394 et 457, numérotés par rapport à la protéine de 660 acides aminés, et un épitope cible des anticorps neutralisants, également conformationnel, situé entre les acides aminés 452 et 617, avec la même numérotation (Meng J, et al., 2001). Elle comporte également un autre épitope immunodominant, appelé épitope 406.3-2, lequel correspond aux acides aminés 613-654 d'un variant ORF2 de 660 acides aminés (WO93/14116). La phosphoprotéine d'un poids moléculaire de 13 kDa, codée par l'ORF3, appelée protéine p-ORF3, est très variable selon les virus. Cette protéine dont le rôle reste à préciser, serait impliquée dans les fonctions de régulation de la réplication virale ou dans l'assemblage de la nucléocapside.

Le diagnostic actuel repose soit sur la détection du virus par amplification génique à partir d'échantillons de selles et de sérum, voire de bile ou de biopsie hépatique, soit sur la détection de la réponse anticorps sérique anti-HEV.

L'amplification génique est mise en œuvre par RT-PCR, PCR nichée, ou PCR en temps réel en utilisant plusieurs couples d'amorces suivant les génotypes, à partir des régions les plus conservées du génome. Avec un seuil de détection de 10 à $10^3$ molécules d'ADNc/réaction, suivant les techniques, l'excrétion virale dans les selles peut atteindre $10^6$ molécules d'ADNc. La caractérisation du génotype peut être réalisée dans un second temps. Ces techniques sont essentiellement utiles pour la détection de la virémie dans le sang de façon précoce par rapport à l'infection, avant apparition des symptômes et des anticorps. Toutefois, ces techniques visant la détection des acides nucléiques viraux ont pour inconvénients que la période de virémie est courte (1 à 2 semaines dans le sang, 3 à 4 semaines dans les selles) et qu'elles nécessitent un appareillage coûteux et non utilisable au plus près du patient.

Le diagnostic sérologique de l'infection par HEV repose sur la détection d'anticorps spécifiques anti-HEV de type IgM et/ou IgG dont la cible principale est p-ORF2. Plusieurs kits sont commercialisés. Ainsi, la société MP Diagnostic™ propose la trousse ASSURE® HEV IgM qui est un dispositif de test immuno-chromatographique destiné à la détection rapide des anticorps IgM dirigés contre la protéine p-ORF2 du virus de l'hépatite E. Pour ce faire, le kit met en œuvre un polypeptide recombinant, le polypeptide 394-660, numéroté par rapport à la séquence 1-660 de p-ORF2, autrement appelé polypeptide p-ORF2.1, correspondant aux 267 derniers acides aminés de la protéine. Des anticorps de souris dirigés contre des IgM humains sont immobilisés sur la membrane d'immunochromatographie, ce qui permet de capturer les différents IgM humains présents dans l'échantillon. La présence d'IgM dirigés spécifiquement contre HEV est révélée en utilisant, comme partenaire de détection, le polypeptide recombinant 394-660 complexé à un anticorps monoclonal anti-HEV marqué à l'or. Les raisons de l'utilisation du polypeptide recombinant 394-660 plutôt que de la protéine entière sont divulguées dans la demande WO95/08632. Selon les enseignements de cette demande de brevet, la réactivité immunologique de la protéine complète p-ORF2 exprimée dans E. coli n'est pas optimale, une partie de la molécule pouvant réduire voire inhiber l'immunoréactivité d'une autre partie de la molécule. Pour pallier à cet effet inhibiteur, la demande de brevet WO95/08632 a proposé d'utiliser des protéines p-ORF2 délétées ou tronquées. Parmi les différentes constructions testées, le polypeptide recombinant 394-660, délété des 393 premiers acides aminés, présentait la meilleure immunoréactivité.

La caractérisation détaillée de la structure antigénique du polypeptide 394-660 et sa comparaison avec celui des « virus-like particles » ou VLP, formés par auto-assemblage *in vitro* et proches antigéniquement de la particule virale HEV, sont décrites dans Riddell M.A., et al., 2000.

L'inconvénient d'utiliser le polypeptide 394-660 est qu'il contient dans sa partie C-terminale un domaine qui inhibe, au moins partiellement, l'auto-assemblage du polypeptide en oligomères et VLP. Ceci peut interférer avec la bonne présentation des épitopes conformationnels.

Pour pallier ces inconvénients, la Société Wantai a modifié le polypeptide 394-660 en supprimant les acides aminés 607-660 (numérotés par rapport à une séquence 1-660 de p-ORF2) qui interfèrent avec l'oligomérisation et la capacité d'auto-assemblage. Le polypeptide ainsi obtenu a été appelé polypeptide pE2, comme décrit dans la demande de brevet WO01/22916. L'avantage de ce polypeptide pE2, de séquence 394-606, est qu'il se dimérise naturellement et que l'immunoréactivité du pE2 dimérique est bien supérieure à celle du pE2 monomérique en favorisant la bonne présentation des épitopes conformationnels. L'inconvénient est qu'un tel polypeptide tronqué ne comprend pas un épitope important, l'épitope 406.3-2 correspondant aux acides aminés 613-654 d'un variant ORF2 de 660 acides aminés, comme indiqué dans la demande de brevet WO93/14116. Une telle délétion peut alors conduire à une diminution de la sensibilité d'un test de diagnostic mettant en œuvre un tel polypeptide tronqué.

La Demanderesse a découvert contre toute attente qu'il était possible de pallier aux inconvénients des polypeptides de l'art antérieur en effectuant, dans le peptide 394-660 de ORF2 de HEV, numéroté par rapport à une protéine p-ORF2 de 660 aminés, 3 mutations aux positions 627, 630 et 638, et ce en améliorant son antigénicité et son immunoréactivité. Ainsi, le peptide muté, qu'on peut appeler p-ORF2-MUT, possède tous les épitopes importants, se dimérise naturellement de façon non covalente, est capable de s'oligomériser sans aucune agrégation et présente une immunoréactivité supérieure à celle du polypeptide recombinant 394-660 non muté.

Aussi, comme cela est défini dans la revendication 1, l'invention concerne un polypeptide apte à se lier à des anticorps anti-HEV, choisi parmi ;

- un polypeptide comprenant au moins la séquence 394-660 d'une protéine p-ORF2 du virus de l'hépatite E ayant une longueur de 660 acides aminés de long et dans lequel les trois acides aminés en positions 627, 630 et 638 sont différentes de la cystéine ; dans lequel, ladite protéine p-ORF2 de 660 acides aminés de long ayant une identité de séquence d'au moins 89 % avec SEQ ID N°11, et ladite séquence 394-660 ayant une identité de séquence d'au moins 90 % avec SEQ ID N°26 ; et
- un polypeptide comprenant une séquence analogue à SEQ ID N°26, ayant une identité de séquence d'au moins 90 % avec SEQ ID N°26 et provenant d'une protéine p-ORF2 du virus de l'hépatite E de longueur différente à 660 acides aminés et ayant une identité de séquence d'au moins 89 % avec SEQ ID N°11, et dans lequel les trois acides aminés correspondant aux trois cystéines des positions 627, 630 et 638 de SEQ ID N°11, sont différentes de la cystéine.

Un autre objet de l'invention concerne les acides nucléiques isolés comprenant une séquence de nucléotides codant pour les polypeptides de l'invention (c'est-à-dire codant pour les polypeptides définis par la revendication 1) ou une séquence complémentaire à ladite séquence codante, ainsi que les vecteurs d'expression comprenant ces séquences.

Encore un autre objet concerne les cellules hôtes comprenant ces mêmes séquences nucléiques, directement insérées ou par l'intermédiaire des vecteurs d'expression.

Elle concerne de plus l'utilisation des polypeptides de l'invention pour la détermination de la présence d'une réponse anticorps dirigée contre la protéine p-ORF-2 du virus de l'hépatite E ou bien pour la détermination du taux de ces anticorps.

Ainsi, un autre objet de l'invention concerne un procédé de détermination par immunoessai de la présence d'une réponse anticorps dirigée contre la protéine p-ORF-2 du virus de l'hépatite E dans un échantillon biologique issu d'un sujet, susceptible de contenir les anticorps de ladite réponse, lequel comprend les étapes suivantes :

- mettre en contact ledit échantillon biologique avec un polypeptide de l'invention,
- détecter un signal émis par la liaison entre ledit polypeptide et lesdits anticorps, s'ils sont présents, en utilisant un marqueur capable d'émettre un signal détectable,
- comparer le signal ainsi obtenu avec un signal de référence S préalablement déterminé avec deux populations de témoins, l'une ayant développé lesdits anticorps et l'autre n'ayant pas développé lesdits anticorps,
- un signal inférieur audit signal de référence S signifiant que l'échantillon ne contient pas lesdits anticorps, et
- un signal supérieur audit signal de référence S signifiant que l'échantillon contient lesdits anticorps.

Un autre objet concerne également un procédé de détermination par immunoessai du taux d'anticorps dirigés contre la protéine p-ORF-2 du virus de l'hépatite E dans un échantillon biologique issu d'un sujet, susceptible de contenir lesdits anticorps, lequel comprend les étapes suivantes :

- mettre en contact ledit échantillon biologique avec un polypeptide de l'invention,
- détecter un signal émis par la liaison entre ledit polypeptide et lesdits anticorps, s'ils sont présents, en utilisant un marqueur capable d'émettre un signal détectable,
- transformer le signal détecté en un taux d'anticorps.

Encore un autre objet concerne l'utilisation de ces procédés pour l'aide au diagnostic *in vitro,* pour le diagnostic *in vitro* d'une infection au virus de l'hépatite E chez un sujet susceptible d'être infecté, pour le suivi thérapeutique d'un sujet infecté par le virus de l'hépatite E, pour faire des études épidémiologiques de la séroprévalence des anticorps anti-HEV dans une population ou dans un territoire géographique donné ou pour déterminer si un sujet a besoin d'être vacciné ou revacciné contre le virus de l'hépatite E.

Enfin, un dernier objet concerne les trousses pour la détermination par immunoessai de la présence de la réponse humorale ou du taux d'anticorps dirigés contre la protéine p-ORF2 du virus de l'hépatite E chez un sujet susceptible d'avoir produit ces anticorps, comprenant un polypeptide de l'invention.

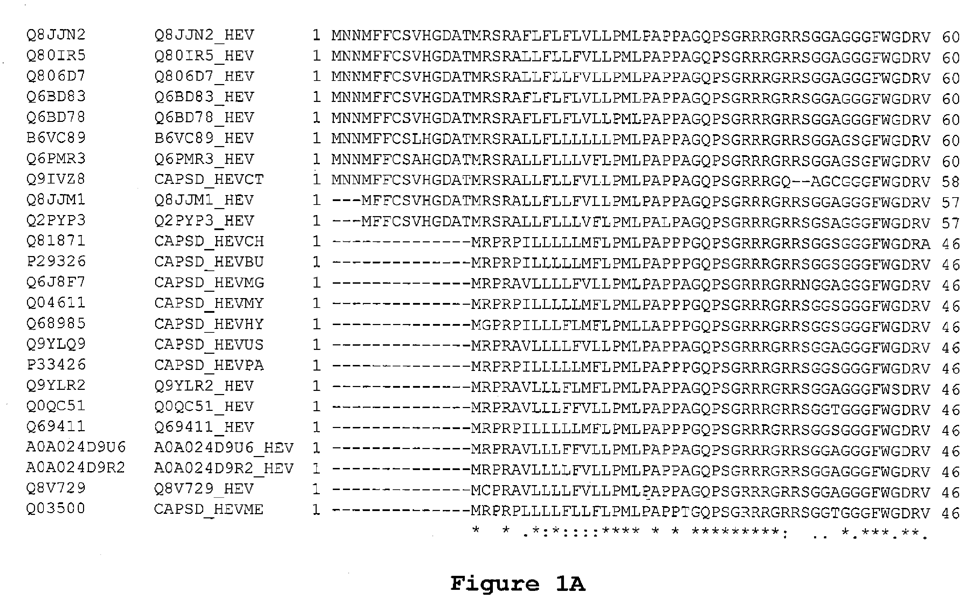

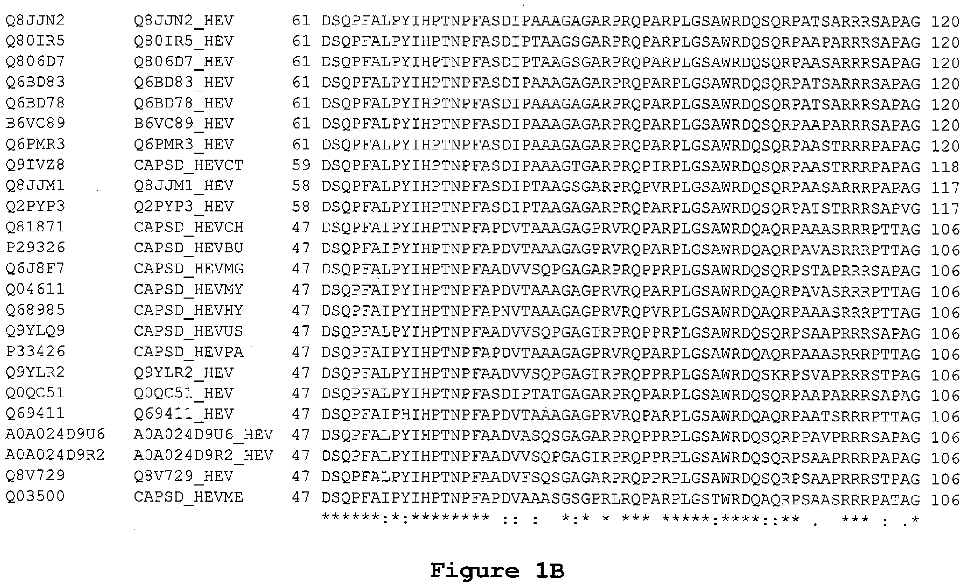

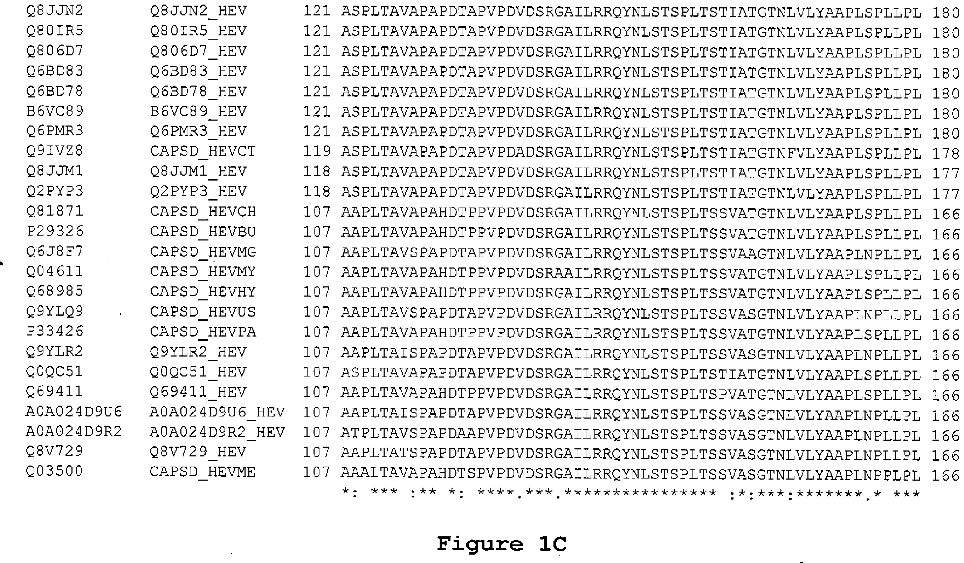

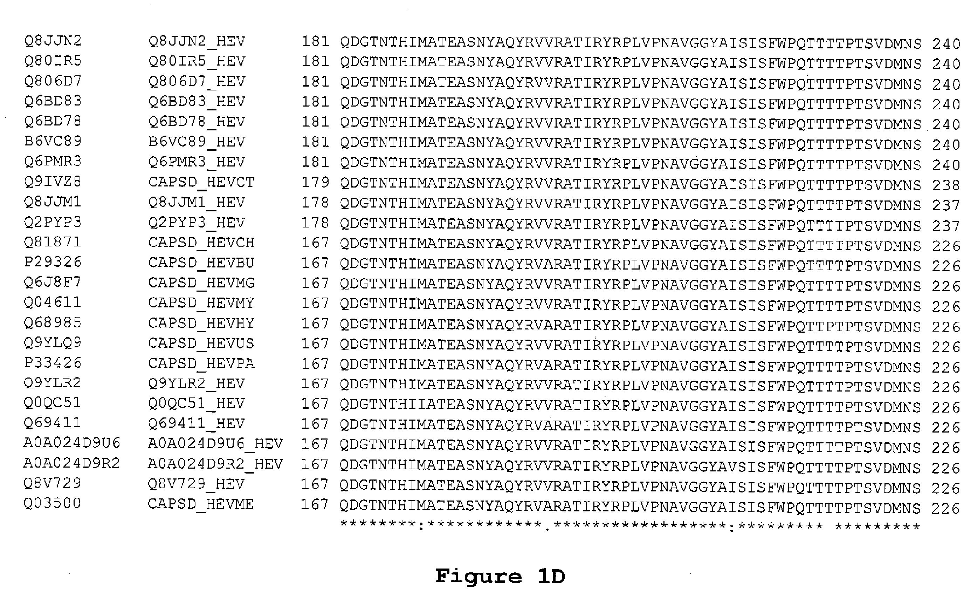

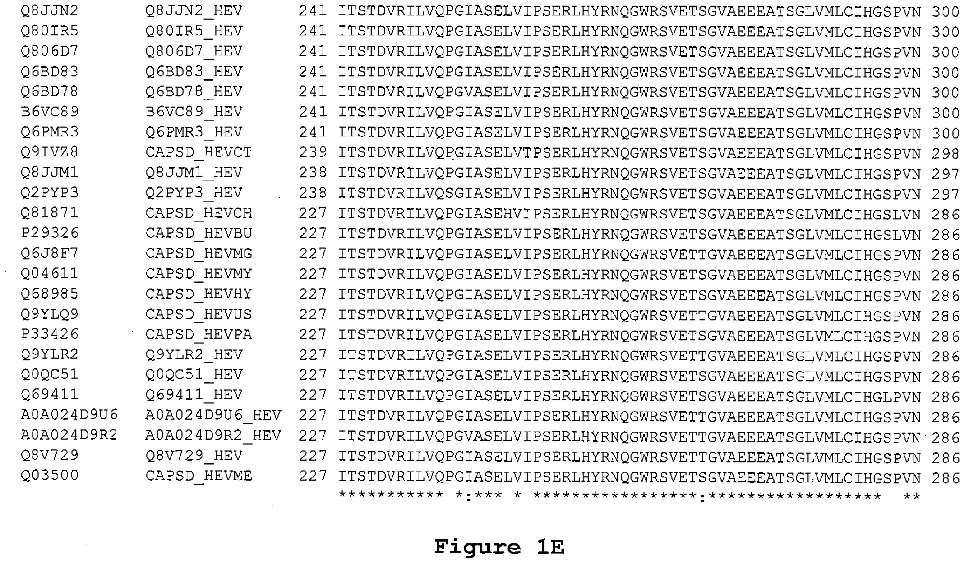

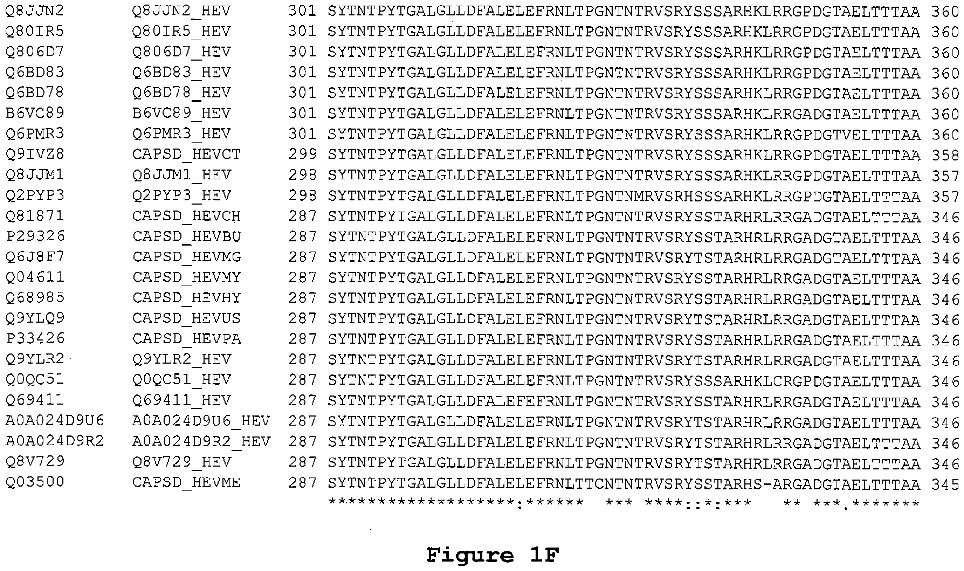

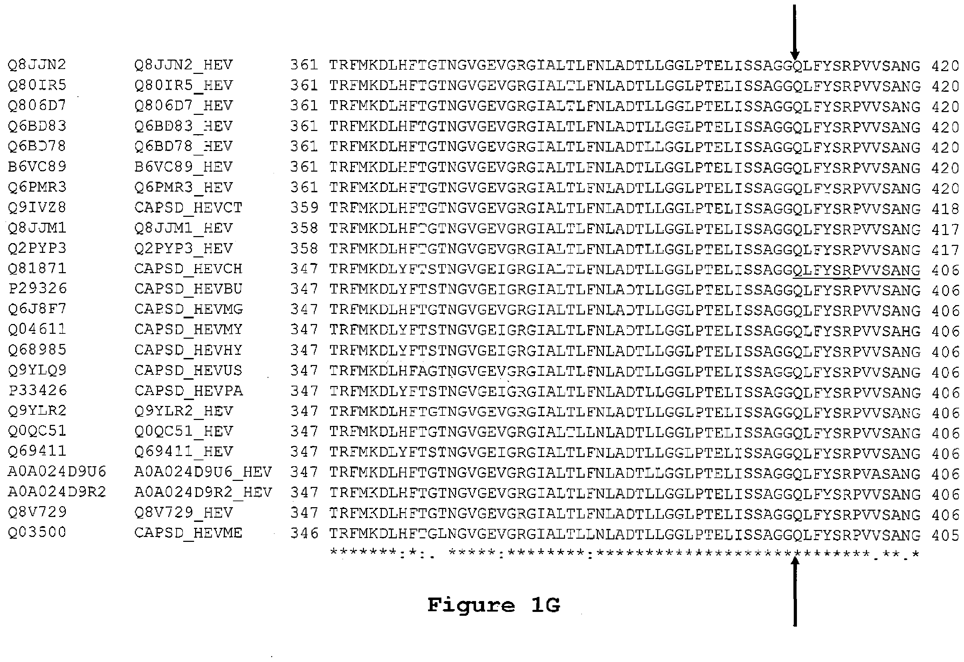

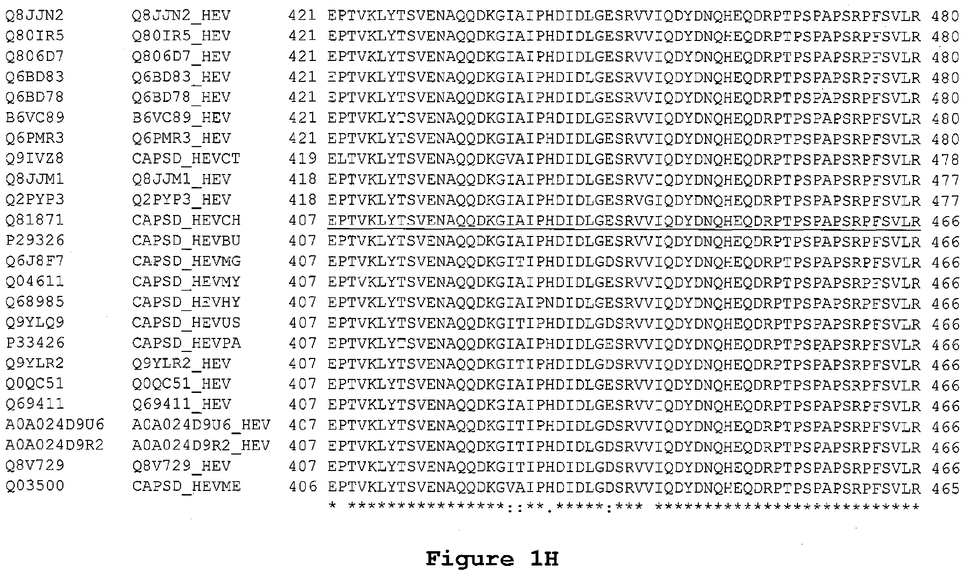

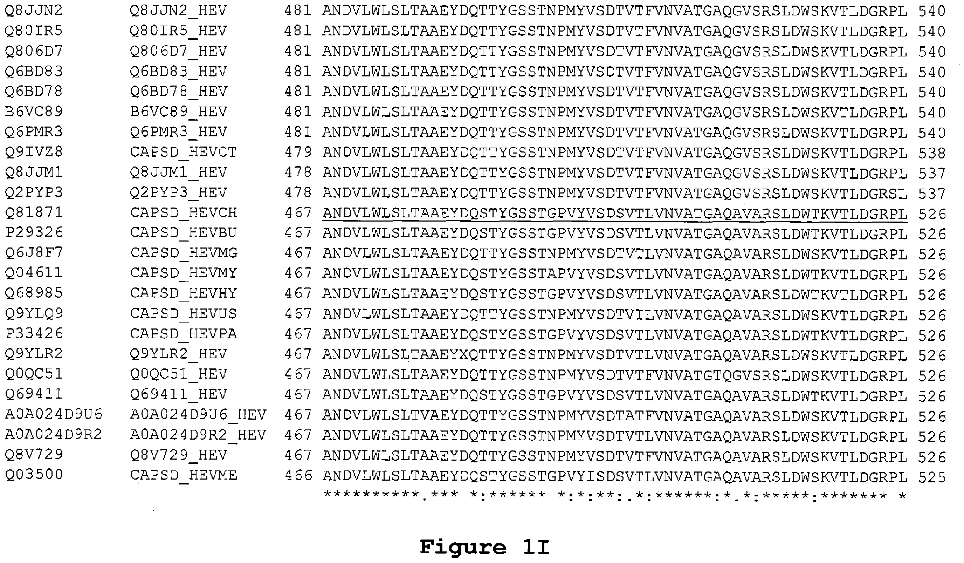

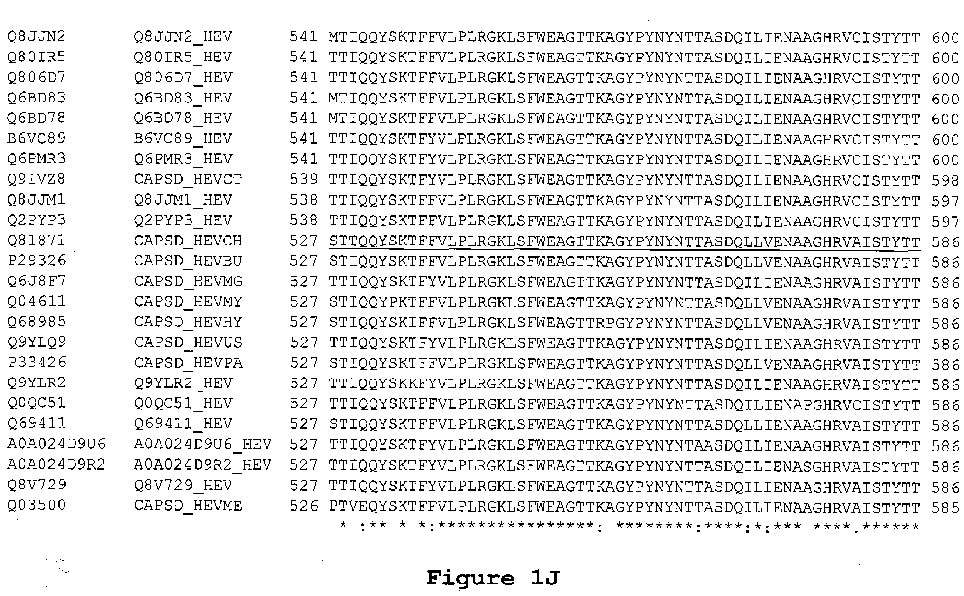

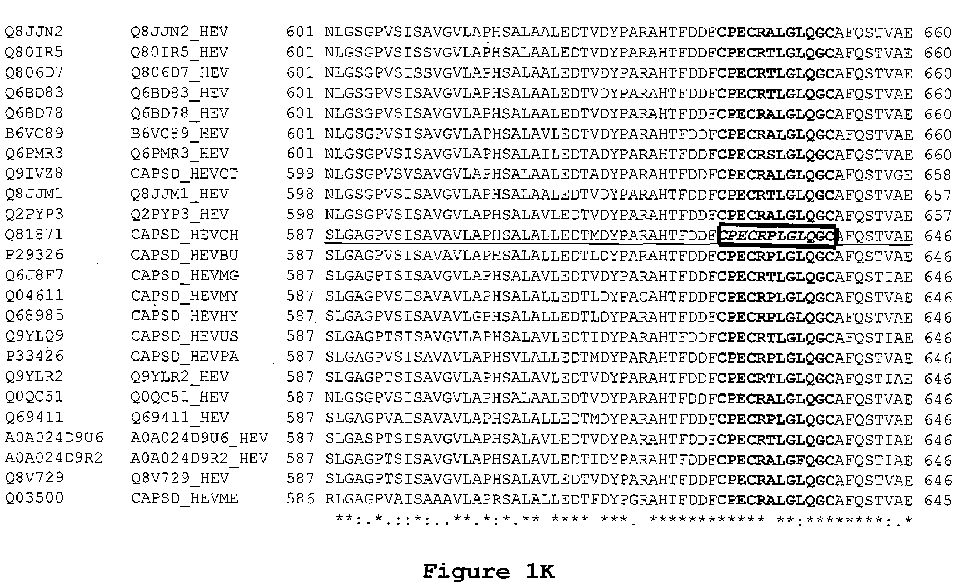

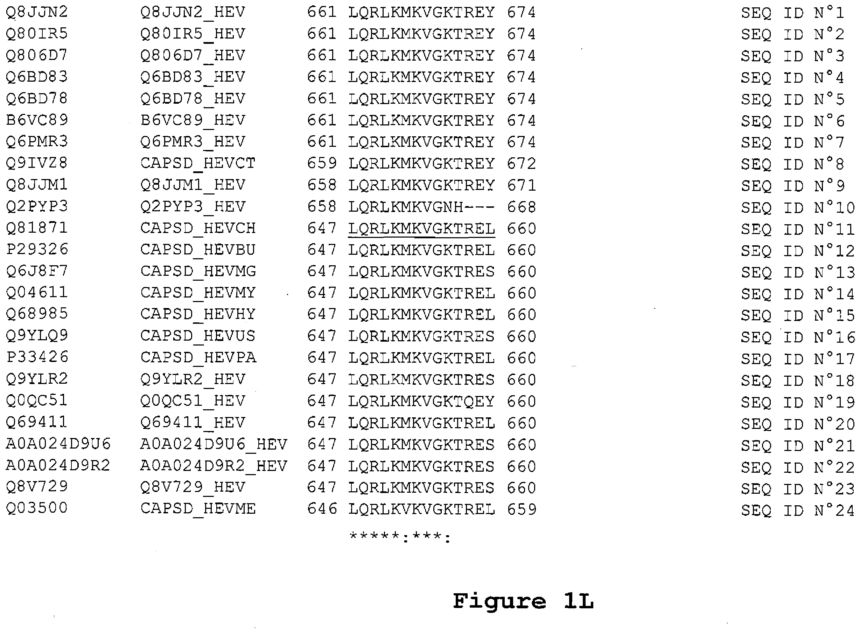

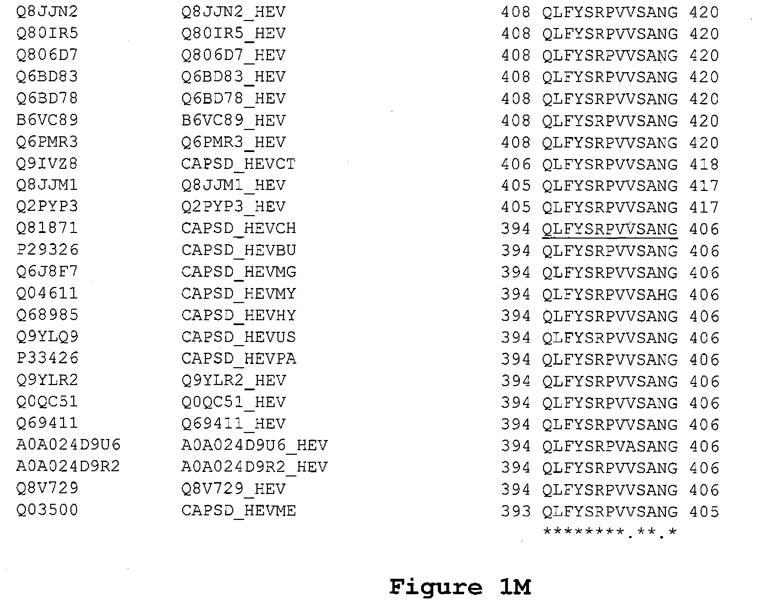

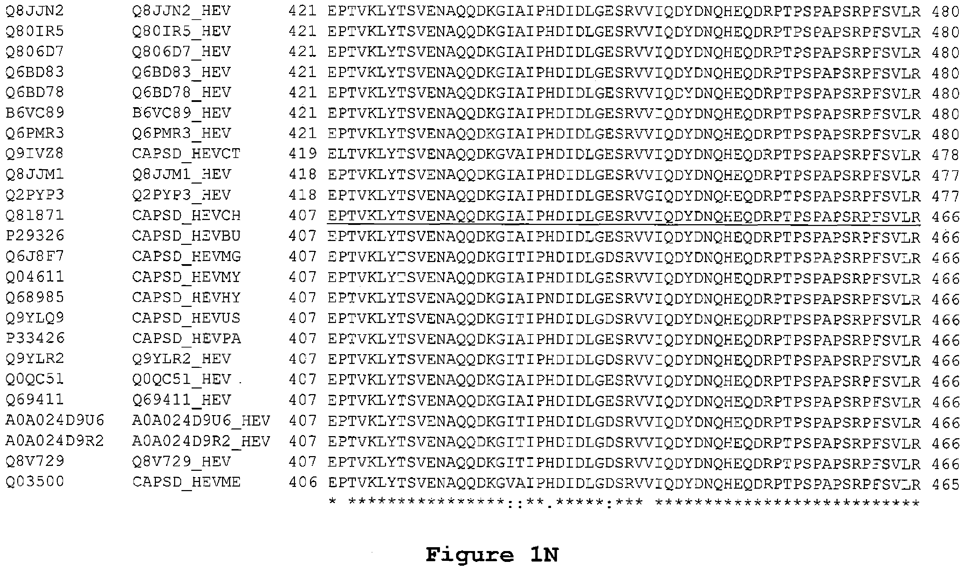

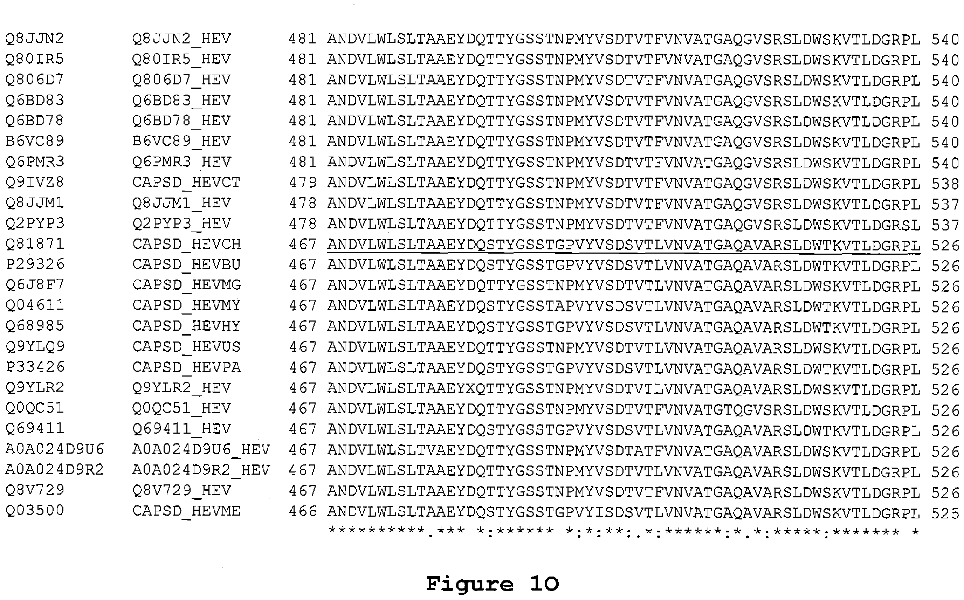

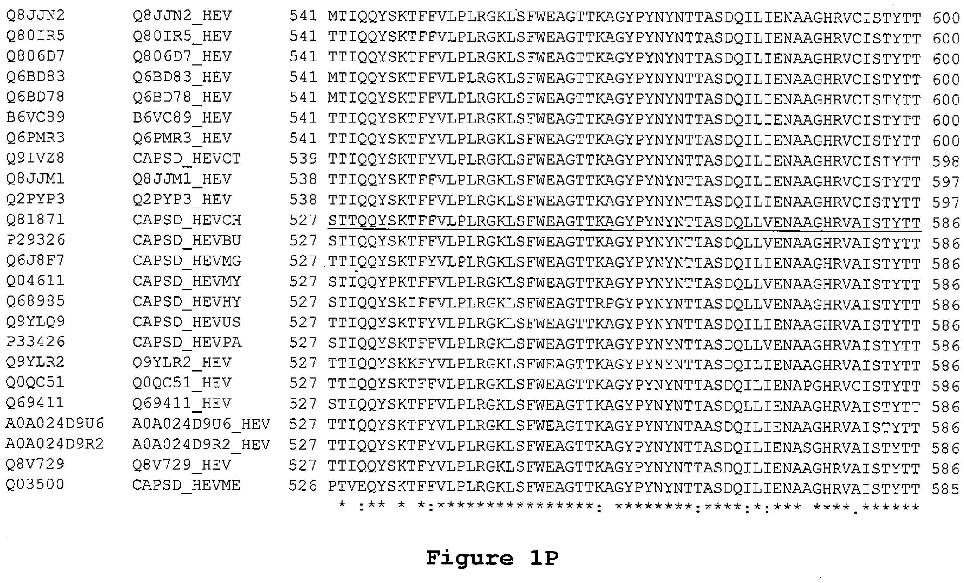

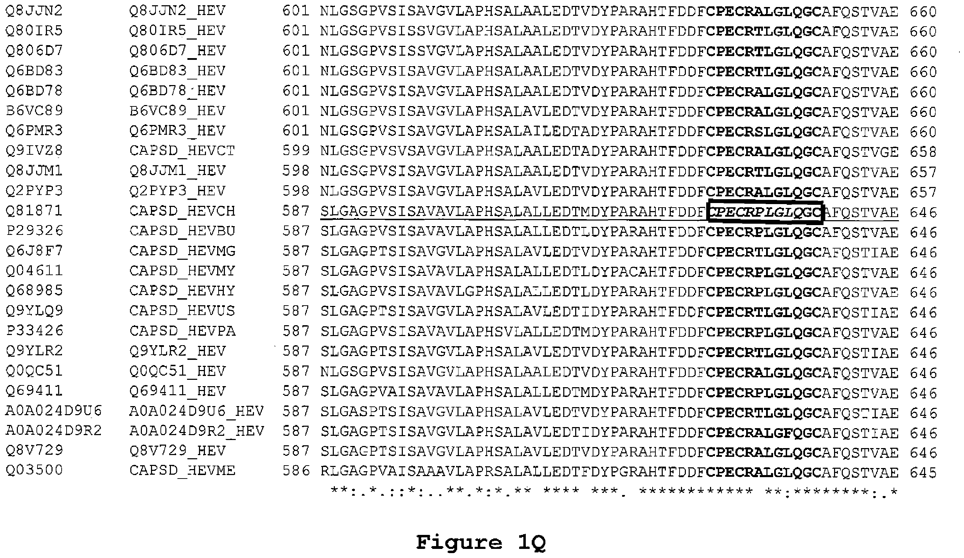

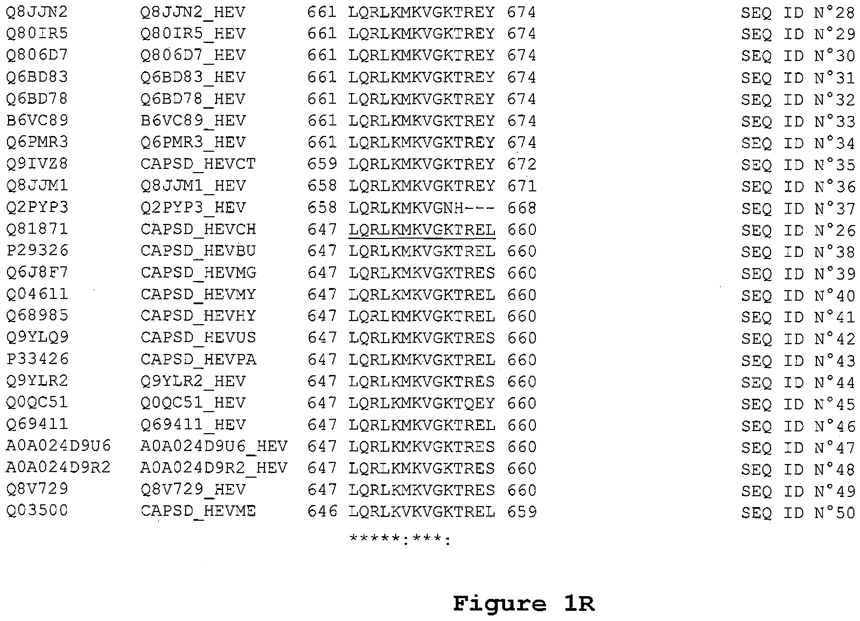

L'invention sera mieux comprise à la lecture de la description non limitative qui suit et des figures 1 à 6 annexées, dans lesquelles :

- La Figure 1 donne un alignement de séquences en acides aminés de différentes protéines p-ORF2 des principaux variants de virus HEV obtenus à partir de la base de données Uniprot, la première colonne correspondant à la référence UNIPROT, la deuxième colonne correspondant au nom de la souche de HEV et la dernière colonne correspondant à l'alignement des séquences. L'alignement de séquences a été réalisé par le programme Clustal Oméga accessible sur le site web d'UNIPROT. La dernière ligne sous chaque alignement de séquences montre l'identité d'acides aminés ou non entre chaque variant, « * » indiquant une position totalement conservée, avec les acides aminés identiques chez tous les variants, « : » indiquant une position bien conservée, avec des acides aminés ayant des propriétés fortement similaires et un score >0,5 dans la matrice Gonnet PAM 250, « . » indiquant une position assez conservée, avec des acides aminés ayant des propriétés faiblement similaires et un score =<0,5 dans la matrice Gonnet PAM 250. Les autres positions sont marquées par « ° ». Les différentes parties de l'alignement sont réparties des Figures 1A à 1R. Les Figures 1A à IL donnent l'alignement des protéines entières des différents variants. La séquence 394-660 du variant Q81871, de 660 acides aminés (SEQ ID N°11), y est soulignée comme référence. Les flèches sur la Figure 1G indiquent le premier acide aminé de la séquence minimale des polypeptides de l'invention et le rectangle sur la Figure 1K montre la séquence de 12 acides aminés du variant Q81871 dans laquelle se trouvent les 3 Cystéines à muter. Les Figures 1M à 1R donnent l'alignement des séquences minimales des polypeptides de l'invention des différents variants extraites des Figures 1A à IL à partir des flèches. La séquence 394-660 du polypeptide de référence du variant Q81871 (SEQ ID N°26) est soulignée.

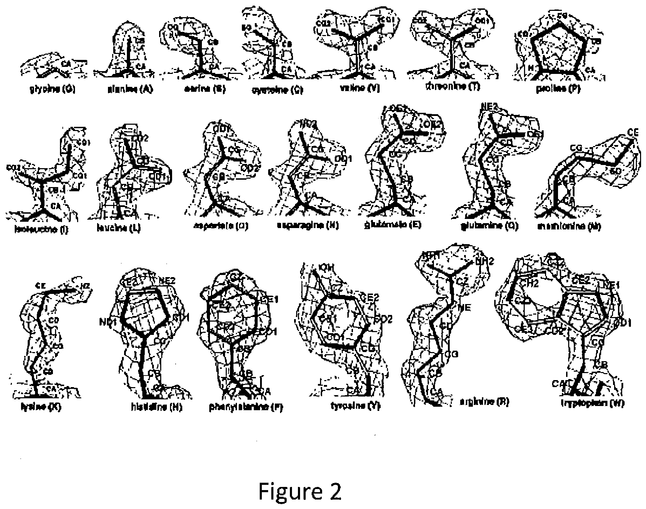

- La Figure 2 montre les représentations des cartes de densité électronique des chaînes latérales d'acides aminés naturels, obtenues par diffraction aux rayons X, calculées à une résolution de 1,5 Angstrom, imprimées du site (référence en date du 13 novembre 2015) :
http://people.mbi.ucla.edu/sawaya/m230d/Modelbuilding/modelbuilding.html.

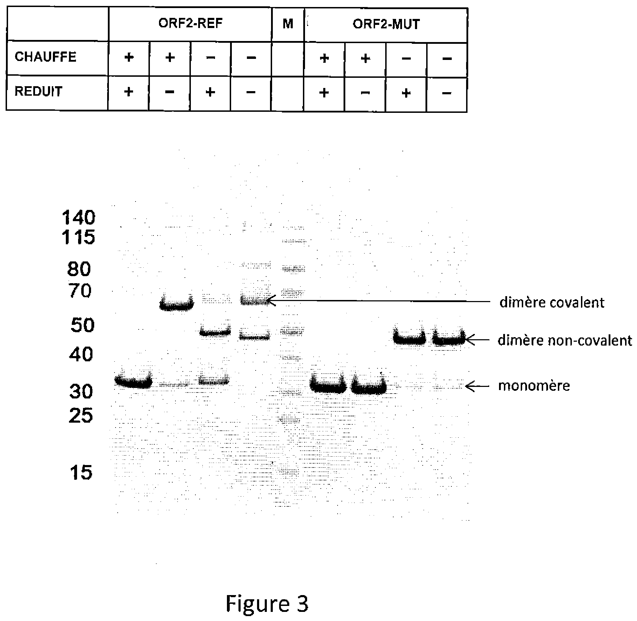

- La Figure 3 est la photographie d'un gel d'analyse SDS-PAGE (4-12%) coloré en bleu de Coomassie afin de visualiser un polypeptide de l'invention, ORF2-MUT, et un polypeptide non muté, ORF2-REF qui correspond au polypeptide p-ORF2.1 (acides aminés 394-660) divulgué dans la demande WO95/08632. Avant l'analyse sur gel, les polypeptides ORF2-REF et ORF2-MUT purifiés et dialysés ont subi soit une réduction par ajout de dithiothreitol (DTT), soit une dénaturation par chauffage (10 min à 75°C), soit les deux traitements à la fois, soit aucun traitement, comme montré sur le tableau au-dessus du gel. La ligne M correspond au marqueur de poids moléculaire Page Ruler (Pierce), les poids moléculaires apparents des bandes sont indiqués à gauche en kilo Daltons (kDa).

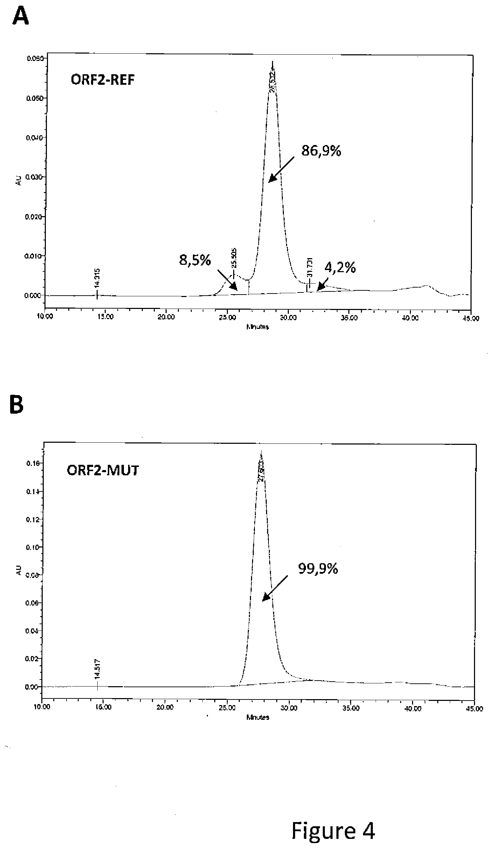

- La Figure 4 représente les chromatogrammes d'exclusion stérique obtenus en suivant l'absorbance UV à 280 nm pour le polypeptide de l'art antérieur ORF2-REF (Figure 4A) et le polypeptide de l'invention ORF2-MUT (Figure 4B). Pour permettre une bonne visualisation des différents pics de la figure 4A les deux chromatogrammes ne sont pas présentés à la même échelle pour l'axe des ordonnées.

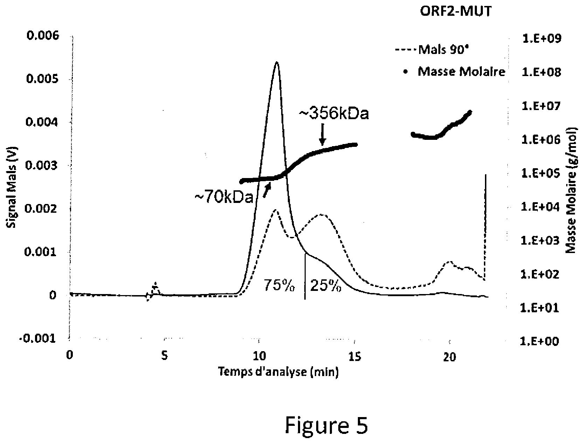

- La Figure 5 représente le graphe donnant les résultats obtenus par la technique AsFIFFF-MALS (« asymetric flow field flow fractionation-multi angle light scattering ») pour le polypeptide de l'invention ORF2-MUT. L'absorbance UV à 280 nm (trait plein fin), le signal de diffusion de lumière multi-angles (MALS, trait hachuré) et l'estimation de la masse molaire (trait plein épais) sont représentés de façon superposée en ordonnée en fonction du temps d'analyse (min).

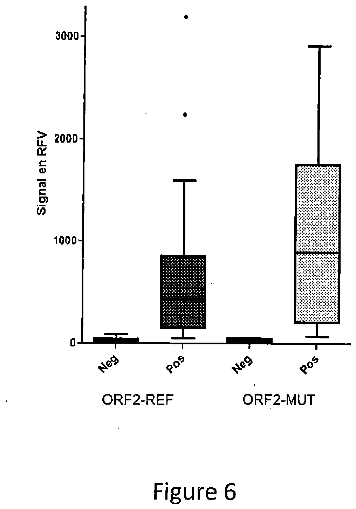

- La Figure 6 est une représentation en boîte à moustache des distributions des signaux RFV obtenus avec un immunoessai (automate VIDAS®, bioMérieux) utilisant comme antigène de capture le polypeptide de l'art antérieur ORF2-REF ou le polypeptide de l'invention ORF2-MUT,, sur des échantillons ne contenant pas d'anticorps anti-ORF2 (Neg) et des échantillons HEV positifs, contenant des d'anticorps anti-ORF2 (Pos). Les boîtes à moustaches ont été tracées selon la méthode de Tukey : les limites haute et basse de la boîte correspondent au 25e et au 75e percentile des distributions, respectivement. La valeur tracée vers la moitié de la boîte est la médiane. La moustache haute correspond au 75e percentile + 1,5 × l'écart interquartile et la moustache basse au 25e percentile - 1,5 × l'écart interquartile. Les valeurs au-delà et en-deçà des moustaches sont représentées sous forme de points individuels car il s'agit de valeurs extrêmes, peu fréquentes.

La Demanderesse a donc montré, contre toute attente, qu'il était possible, en vue de la prise en charge des sujets concernés par une infection à l'hépatite E, d'utiliser des polypeptides dérivés de la protéine p-ORF2 du virus de l'hépatite E comprenant au moins la séquence d'acides aminés 394-660, numérotés par rapport à une protéine p-ORF2 de 660 acides aminés, tout en évitant les inconvénients de l'art antérieur lorsque les acides aminés 607-660 sont inclus dans les polypeptides, à savoir qu'ils possèdent tous les épitopes importants, se dimérisent naturellement de façon non covalente, sont capables de s'oligomériser sans aucune agrégation. Par ailleurs, les polypeptides de l'invention sont produits de façon homogène, contrairement aux polypeptides de l'art antérieur. En effet, lors de leur production, le produit final présente de façon reproductible plus de 75% de dimères non covalents, le restant étant constitué de dodécamères, alors que la proportion de dimères non covalents, dimères covalents et agrégats des polypeptides de l'art antérieur varie d'une production à l'autre. De plus, les polypeptides de l'invention présentent une immunoréactivité supérieure à celle du polypeptide recombinant 394-660 non muté. Enfin, les polypeptides de l'invention permettent, lorsqu'ils sont utilisés dans un immunoessai, d'augmenter la spécificité diagnostique du test, sans en modifier la sensibilité diagnostique, ce qui est fondamental pour un test de détection du virus de l'hépatite E.

Comme indiqué précédemment et comme bien illustré sur la Figure 1, la protéine p-ORF2 du virus HEV possède différentes longueurs, de 659 à 674 acides aminés (voir Figure IL donnant les derniers acides aminés de la protéine p-ORF2). La majorité des protéines ayant 660 acides aminés, c'est une protéine de 660 acides aminés qui est souvent prise comme référence. Dans la présente demande la séquence de référence de 660 acides aminés est celle du variant Q81871 (SEQ ID N°11). Toutefois les protéines des autres variants, bien qu'ayant une séquence d'acides aminés différentes, par exemple de 674 acides aminés (SEQ ID N°1 à 7), de 672 acides aminés (SEQ ID N°8), de 671 acides aminés (SEQ ID N°9), de 668 aminés (SEQ ID N°10) ou de 659 acides aminés (SEQ ID N°24), ainsi que celles des variants dont la protéine a la même longueur (SEQ ID N°12 à 23) sont bien incluses dans la portée de l'invention.

Ainsi pour trouver tous les polypeptides de l'invention, étant définis par la revendication 1, il suffit à l'homme du métier d'effectuer un alignement par rapport à une protéine de 660 acides aminés. Ainsi, par exemple, si on se réfère à la Figure 1, qu'on prenne comme protéine de référence de 660 acides aminés celle appartenant au variant Q81871 (dans laquelle la séquence 394-660 est soulignée sur la Figure 1) et qu'on considère par exemple les protéines des variants, de 672, 671, 659, 668 et 674 acides aminés, les polypeptides de l'invention comprennent au moins :

- la séquence d'acides aminés 394-660 (SEQ ID N°26, et SEQ ID N°38 à 49) dont les cystéines en positions 627, 630 et 638 sont mutées (issue des variants Q81871, P29326, Q6J8F7, Q04611, Q68965, Q9YLQ9, P33426, Q9YLR2, Q0QC51, Q69411, A0A024D9U6, A0A024D9R2, Q8V729), ou
- la séquence d'acides aminés 408-674 (SEQ ID N°28 à 34) dont les cystéines en positions 641, 644 et 652 sont mutées (issue des variants Q8JJN2, Q80IR5, Q806D7, Q6BD83, Q6BD78, B6VC89, Q6PMR3).
- la séquence d'acides aminés 406-672 (SEQ ID N°35) dont les cystéines en positions 639, 642 et 650 sont mutées (issue du variant Q9IVZ8), ou
- la séquence d'acides aminés 405-671 (SEQ ID N°36) dont les cystéines en positions 638, 641 et 649 sont mutées (issue du variant Q8JJM1), ou
- la séquence d'acides aminés 405-668 (SEQ ID N°37) dont les cystéines en positions 638, 641 et 649 sont mutées (issue du variant Q2PYP3) ou
- la séquence d'acides aminés 393-659 (SEQ ID N°50) dont les cystéines en positions 626, 629 et 637 sont mutées (issue du variant Q03500).

Ainsi les différents fragments 394-660, 408-674, 406-672, 405-671, 405-668 et 393-659 des variants décrits sur les Figure 1M à 1R et extraits de les Figures 1G à IL à partir de la flèche sur la Figure 1G, correspondent aux séquences suivantes :

Fragments 394-660

| Q81871 | P29326 | Q6J8F7 | Q04611 | Q68985 | Q9YLQ9 |
|---|---|---|---|---|---|
| SEQ ID N°26 | SEQ ID N°38 | SEQ ID N°39 | SEQ ID N°40 | SEQ ID N°41 | SEQ ID N°42 |

| P33426 | Q9YLR2 | Q0QC51 | Q69411 | A0A024D9U6 | A0A024D9R2 |
|---|---|---|---|---|---|
| SEQ ID N°43 | SEQ ID N°44 | SEQ ID N°45 | SEQ ID N°46 | SEQ ID N°47 | SEQ ID N°48 |

| Q8V729 |
|---|
| SEQ ID N°49 |

Fragments 408-674

| Q8JJN2 | Q80IR5 | Q806D7 | Q6BD83 | Q6BD78 | B6VC89 |
|---|---|---|---|---|---|
| SEQ ID N°28 | SEQ ID N°29 | SEQ ID N°30 | SEQ ID N°31 | SEQ ID N°32 | SEQ ID N°33 |

| Q6PMR3 |
|---|
| SEQ ID N°34 |

Fragment 406-672
Q9IVZ8
SEQ ID N°35
Fragment 405-671
Q8JJM1
SEQ ID N°36
Fragment 405-668
Q2PYP3

SEQ ID N°37
Fragment 393-659
**Q03500**
SEQ ID N°50

La Figure 1 montre également que si on prend la séquence d'acides aminés 394-660 du variant Q81871 comme séquence de référence (SEQ ID N°26) pour toutes les protéines de 660 acides aminés, les séquences d'acides aminés 394-660 des autres variants (SEQ ID N°38 à 49) possèdent entre 90,64% et 99,25% d'identité avec la séquence SEQ ID N°26, tandis que si on prend la séquence totale de la protéine de 660 acides de ce même variant (SEQ ID N°11), les séquences de 660 acides aminés des autres variants (SEQ ID N°12 à 23) présentent entre 90,45% et 99,09% d'identité avec cette séquence SEQ ID N°11.

De même, si on prend la séquence d'acides aminés 408-674 du variant Q8JJN2 comme séquence de référence (SEQ ID N°28) pour toutes les protéines de 674 acides aminés, les séquences d'acides aminés 408-674 des autres variants (SEQ ID N°29 à 34) possèdent entre 98,50% et 98,88% d'identité avec la séquence SEQ ID N°28, tandis que si on prend la séquence totale de la protéine de 674 acides de ce même variant (SEQ ID N°1), les séquences de 674 acides aminés des autres variants (SEQ ID N°2 à 7) présentent entre 98,22% et 98,37% d'identité avec cette séquence SEQ ID N°1.

Plus globalement, si on prend la séquence d'acides aminés 394-660 du variant Q81871 comme séquence de référence (SEQ ID N°26), les séquences d'acides aminés correspondantes des autres variants (SEQ ID N°28 à 50) possèdent entre 90,64% et 99,25% d'identité avec la séquence SEQ ID N°26, donc au moins 90% d'identité, tandis que si on prend la séquence totale de la protéine de 660 acides de ce même variant (SEQ ID N°11), les séquences totales des protéines des autres variants (SEQ ID N°1 à 10 et 12 à 24) présentent entre 89,97% et 99,09% d'identité avec cette séquence SEQ ID N°11, donc au moins 89% d'identité.

Le pourcentage d'identité entre 2 séquences est calculé à partir de l'alignement des séquences multiples. Le programme Clustal Omega accessible dans une version plus paramétrable sur le site web EMBL-EMI (http://www.ebi.ac.uk/Tools/msa/clustalo/) génère en même temps que l'alignement multiple, un score d'alignement. Ce score est relié aux taux de similarité entre 2 séquences comparées et ce pour toutes les séquences, comme illustré sur la figure 1.

Comme montré sur la Figure 1K, les 3 cystéines à muter se trouvent dans une séquence de 12 acides aminés définie comme suit: $CPECRX_1LGX_2QGC$ (SEQ ID N°25), dans laquelle $X_1$ représente P, T, S ou A et $X_2$ représente L ou F.

Les mutations au niveau des trois cystéines ci-dessus se font par substitution desdites cystéines par tout acide aminé différent de la cystéine bien connu de l'homme du métier, tel que par exemple les acides aminés protéinogènes Histidine, Isoleucine, Leucine, Lysine, Méthionine, Phénylalanine, Thréonine, Tryptophane, Valine, Alanine, Arginine, Acide aspartique, Asparagine, Acide glutamique, Glutamine, Glycine, Proline, Sérine, et Tyrosine.

Toutefois, il est préférable de choisir l'acide aminé de substitution selon les deux critères suivants :

1) la « taille ou volume » de la chaine latérale des acides aminés en s'appuyant sur les représentations des cartes de densité électronique obtenue par diffraction aux rayons X, comme par exemple montré sur la figure 2 donnant une telle représentation, calculée à une résolution de 1,5 Angstrom, et issue du site (impression en date du 13 novembre 2013) :

http://people.mbi.ucla.edu/sawaya/m230d/Modelbuilding/modelbuilding.html.

En effet, à partir de ces cartes, on choisit les acides aminés dont la densité électronique est le plus similaire à celle de la cystéine (par exemple Sérine, Valine, et Thréonine) ou des acides aminés plus « petits » que la cystéine (par exemple Glycine, Alanine). On écarte de préférence les acides aminés trop « gros » (par exemple Lysine, Histidine, Phénylalanine, Tyrosine, Arginine et Tryptophane)

2) Les réactivités possibles. On ne veut pas que l'acide aminé substitué réagisse facilement avec d'autres acides aminés environnants. On écarte de préférence les acides aminés chargés tels que les acides aminés basiques (déjà exclus avec le 1er critère) et les acides aminés acides.

Selon un mode de réalisation, les mutations dans les polypeptides de l'invention sont mises en œuvre en remplaçant les trois cystéines par tout acide aminé à l'exception de la proline, des acides aminés dont les chaînes latérales sont chargées tels que lysine, arginine, histidine, acide aspartique, acide glutamique et des acides aminés dont les chaînes latérales comportent un cycle aromatique benzénique, tels que tyrosine, phénylalanine, tryptophane.

De préférence, les mutations dans les polypeptides de l'invention sont mises en œuvre en remplaçant les trois cystéines par un acide aminé choisi parmi l'alanine, la glycine, la thréonine, la valine et la sérine.

Les 3 cystéines peuvent être substituées par le même acide aminé ou par des acides aminés différents, de préférence selon les critères ci-dessus.

Selon un autre mode de réalisation, les mutations mises en œuvre consistent à substituer les 3 cystéines par le même acide aminé et de préférence par la sérine.

Les polypeptides de l'invention comprennent au moins la séquence d'acides aminés 394-660, numérotés par rapport à une protéine p-ORF2 de 660 acides aminés, et, pour une protéine p-ORF2 de longueur différente, ils comprennent au moins la séquence d'acides aminés correspondant aux acides aminés 394-660 de la protéine p-ORF2 de 660 acides aminés, lesdites séquences étant mutées comme indiqué précédemment.

Par polypeptide dérivé de la protéine p-ORF2 du virus de l'hépatite E, on entend un enchaînement continu des acides aminés en positions 394-660 ou positions équivalentes, issus de la protéine p-ORF2 du virus de l'hépatite E. On peut parler aussi indifféremment de polypeptide issu de la protéine p-ORF2, de polypeptide de la protéine p-ORF2, de polypeptide p-ORF2, de polypeptide muté p-ORF2, de protéine dérivée de la protéine p-ORF2, de protéine issue de la protéine p-ORF2 ou de protéine p-ORF2 mutée.

Par l'expression « comprend au moins la séquence », on entend que le polypeptide possède ledit enchainement continu des acides aminés issus de la protéine p-ORF2, ou bien il possède cet enchaînement d'acides aminés auxquels peuvent être ajouté(s) :

(i) un ou plusieurs acides aminés appartenant à la protéine p-ORF2, situés avant ladite séquence, et/ou
(ii) un ou plusieurs acides aminés n'appartenant pas à la protéine p-ORF2, tel qu'une queue polyhistidine, une queue polysine, ou une protéine de fusion, par exemple la GST (Glutathion S Transferase), la MBP (Maltose Binding Protein), le CBP (Calmodulin Binding Peptide), le CBD (Chitine Binding Domain), la Protéine A, la Thiorédoxine, et/ou
(iii) un marquage, par exemple (a) par couplage à une molécule marqueur connue de l'homme du métier telle que la biotine, une enzyme, un marqueur fluorescent, une molécule radioactive ou tout autre marqueur tel que défini plus loin, ou (b) par phosphorylation.

Ainsi, selon un mode de réalisation, les polypeptides de l'invention, comme définis par la revendication 1, comprennent une ou plusieurs des caractéristiques suivantes :

- ils consistent en les polypeptides de séquence d'acides aminés 394-660, numérotés par rapport à une protéine p-ORF2 de 660 acides aminés, dans laquelle les trois cystéines en positions 627, 630 et 638 ont été mutées ou, pour une protéine p-ORF2 de longueur différente, de séquence d'acides aminés correspondant aux acides aminés 394-660 de la protéine p-ORF2 de 660 acides aminés, dans laquelle les trois cystéines situées aux trois positions correspondant aux positions 627, 630 et 638 de la protéine p-ORF2 de 660 acides aminés ont été mutées ;
- ils comportent un ou plusieurs acides aminés n'appartenant pas à la protéine p-ORF2 ;
- ils sont marqués, par exemple comme illustré ci-dessus.

Les polypeptides de l'invention peuvent être produits par des techniques bien connues de l'homme du métier. Par exemple, les polypeptides de l'invention peuvent être obtenus par génie génétique en utilisant des étapes, classiquement connues de l'homme du métier, consistant à :

- disposer de l'ADN codant pour les polypeptides de l'invention,
- insérer cet ADN par clonage dans un vecteur d'expression tel qu'un plasmide, un cosmide, un phage λ ou un vecteur viral (baculovirus (Autographa californica Nuclear Polyhedrosis Virus), virus de la vaccine, virus de la fôret de Semliki, adénovirus, lentivirus, ...), lequel vecteur comprend également une origine de réplication (pour plasmides ou cosmides) ou système de réplication permettant son amplification dans la cellule hôte et un ou des promoteurs permettant la transcription d'ARN messagers qui seront traduits en protéines,
- introduire le vecteur pour expression dans une cellule hôte, tel qu'une cellule procaryote (par exemple bactérie comme *Escherichia coli, Bacillus subtilis*) par transformation ou infection ou une cellule eucaryote (par exemple levures (*Saccharomyces cerevisiae*, *Pichia pastoris*), cellules d'insectes (cellules Sf9, Sf21, High5), cellules de mammifères (CHO, 293, Per.C6, BHK-21, Vero, ...) par transfection transitoire ou permanente, ou encore infection virale,
- culture et éventuellement multiplication de la cellule hôte contenant le vecteur d'expression, avec éventuellement amplification du vecteur dans la cellule hôte,
- au besoin, induction de la transcription et de la synthèse protéique pour la production des polypeptides recombinants de l'invention, et
- purification pour extraire lesdits polypeptides, par exemple grâce à une queue polyHistidine. Les polypeptides sont alors dits recombinants.

Aussi, l'invention a également pour objet :

- les acides nucléiques isolés comprenant des séquences de nucléotides codant pour les polypeptides de l'invention tels que définis précédemment ou des séquences complémentaires auxdites séquences codantes,
- les vecteurs d'expression comprenant une séquence d'acide nucléique telle que définie ci-dessus.
- les cellules hôte, procaryotes ou eucaryotes, comprenant une séquence de nucléotides codant pour les polypeptides de l'invention tels que définis ci-dessus ou une séquence complémentaire à ladite séquence codante ou un vecteur d'expression tel que défini ci-dessus.

Lorsque les polypeptides de l'invention comprennent d'autres composants tels que des marqueurs de nature polypeptidique ou des protéines de fusion, comme décrit ci-dessus, la séquence en acides nucléiques codant pour ces composants peut également être insérée dans le même cadre de lecture dans le vecteur afin de permettre une production en fusion.

L'ajout de marqueurs non protéiques aux polypeptides de l'invention peut être mis en œuvre par des techniques connues de l'homme du métier en utilisant des liaisons -NH-OC- formées à partir des groupements -NH$_2$ et -COOR (R étant par exemple un groupe ester activé) des marqueurs et des polypeptides de l'invention. Ainsi, par exemple, lorsque le marqueur est la biotine, l'homme du métier pourra utiliser des réactifs commerciaux, tels que les réactifs EZ-Link® NHS-Biotin (ThermoScientific N°20217, 21336 et 21343), lesquels comprennent un groupement -COO-ester activé à mettre à réagir avec le groupement -NH$_2$ des polypeptides de l'invention selon les recommandations du fournisseur.

Comme indiqué précédemment, les polypeptides de l'invention sont particulièrement utiles pour la détermination de la présence d'une réponse anticorps dirigée contre la protéine p-ORF-2 du virus de l'hépatite E.

La détermination de la présence d'une réponse anticorps dirigée contre la protéine p-ORF-2 du virus de l'hépatite E dans un échantillon biologique issu d'un sujet, susceptible de contenir les anticorps de ladite réponse, peut être mise en œuvre par immunoessai et comprend ou consiste en les étapes suivantes :

- mettre en contact ledit échantillon biologique avec un polypeptide tel que défini précédemment,
- détecter un signal émis par la liaison entre ledit polypeptide et lesdits anticorps, s'ils sont présents, en utilisant un marqueur capable d'émettre un signal détectable,
- comparer le signal ainsi obtenu avec un signal de référence S préalablement déterminé avec deux populations de témoins, l'une ayant développé lesdits anticorps et l'autre n'ayant pas développé lesdits anticorps,
- un signal inférieur audit signal de référence S signifiant que l'échantillon ne contient pas lesdits anticorps, et
- un signal supérieur audit signal de référence S signifiant que l'échantillon contient lesdits anticorps.

Les sujets susceptibles d'être infectés par le virus HEV, chez qui la détermination de la présence de la réponse anticorps ou du taux d'anticorps est mise en œuvre, peuvent être tout sujet et en particulier :

◦ les sujets ayant des symptômes d'hépatite aiguë, comme par exemple jaunissement de la peau et des yeux (jaunisse ou ictère), urines foncées, selles décolorées, fatigue extrême, nausées, vomissements, fièvre, douleurs abdominales ou syndrome « pseudo-grippal ». Ces symptômes peuvent s'accompagner d'une élévation des enzymes hépatiques (ALAT/ASAT) ou non. Ces sujets ont déjà pu être testés positifs pour les virus HAV, HBV ou HCV ou non ;
◦ les sujets asymptomatiques ayant une élévation des enzymes hépatiques (ALAT/ASAT). Ces sujets ont déjà pu être testés positifs pour les virus HAV, HBV ou HCV ou non ;
◦ les individus appartenant à une population dite à risque soit de chronicisation, soit de forme sévère fulminante tels que :

• les immunodéprimés pour une cause quelconque, incluant les sujets transplantés, les sujets recevant une ou des thérapies immunomodulatrices ou immunosuppressives comme une chimiothérapie, un traitement anti-TNF alpha ou encore une corticothérapie, les sujets ayant une co-infection par le HIV, les personnes âgées (immuno sénescence),
• les femmes enceintes,
• les sujets ayant au préalable une hépatopathie chronique.

Les sujets peuvent être des mammifères tels que les hommes, les animaux domestiques (chiens, chats, chevaux, etc.) et les animaux d'élevage (ovins, bovins, caprins), de préférence les hommes.

A titre d'échantillons biologiques des sujets susceptibles de contenir les anticorps anti-p-ORF2 du virus de l'hépatite E, on peut citer les fluides biologiques tels que le sang total ou ses dérivés, par exemple sérum ou plasma, les urines, la salive et les épanchements, ainsi que les selles. On préfère le sang ou ses dérivés, ainsi que les selles. Ces échantillons peuvent être utilisés tels quels dans le procédé de l'invention ou avoir subi un prétraitement selon des méthodes connues de l'homme du métier.

Par détermination de la réponse anticorps dirigée contre la protéine p-ORF-2 du virus de l'hépatite E dans l'échantillon biologique issu d'un sujet, on entend la détermination de la présence ou non d'anticorps produits par le sujet dans le cas d'une infection par le virus HEV, ces anticorps étant dirigés contre la protéine p-ORF2.

Cette détermination est mise en œuvre par immunoessai qui est un essai largement connu de l'homme du métier. En quelques mots, il consiste à déterminer un analyte, dans le cas présent les anticorps anti-p-ORF2 de la réponse anticorps (également appelée humorale), en mettant en œuvre au moins un partenaire de liaison à l'analyte.

Bien entendu, le préfixe « immuno » dans le terme « immunoessai », par exemple, n'est pas à considérer dans la présente demande comme indiquant strictement que le partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner aussi des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition essentielle est que le partenaire de liaison concerné soit capable de se lier à l'analyte recherché, dans le cas présent de nature anticorps, de préférence de manière spécifique. Ainsi, il est connu de parler de l'essai ELISA pour des essais qui utilisent des partenaires de liaison non immunologiques *stricto sensu,* appelés plus largement en anglais « ligand binding assay », que l'on pourrait traduire en langue française par « essai utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

Par partenaire de liaison aux anticorps anti-p-ORF2, on entend toute molécule capable de se lier à ces anticorps. A titre d'exemple de tels partenaires de liaison, on peut citer les antigènes tels que la protéine p-ORF2 native ou recombinante, des fragments de cette protéine, et en particulier les polypeptides tels que décrits précédemment, les anticorps tels que les anticorps anti-Ig, par exemple anti-Ig totales pour une espèce donnée, ou bien anti-IgG ou anti-IgM selon qu'on recherche des IgG ou des IgM (en utilisant une anti-IgG ou anti-IgM espèce pour la détection d'IgG ou IgM chez cette espèce), les analogues d'anticorps (molécules capables de mimer les anticorps) tels que les nanofitines, les aptamères ou encore les « DARPins », ou toute autre molécule qui est connue pour avoir une interaction avec les anticorps. La condition essentielle pour la mise en œuvre du procédé de détermination de la présence de la réponse anticorps selon l'invention est l'utilisation, comme partenaire de liaison, aux moins des polypeptides de l'invention tels que décrits précédemment.

Les partenaires de liaison anticorps sont par exemple soit des anticorps polyclonaux, soit des anticorps monoclonaux, dont l'obtention est largement connue de l'homme du métier.

A titre d'exemple de fragments d'anticorps, on peut citer les fragments Fab, Fab', F(ab')2 ainsi que les scFv (Single chain variable fragment), dsFv (Double-stranded variable fragment). Ces fragments fonctionnels peuvent notamment être obtenus par génie génétique.

Les analogues d'anticorps nanofitines sont de petites protéines qui, comme les anticorps, sont capables de se lier à une cible biologique permettant ainsi de la détecter, de la capturer ou tout simplement de la cibler au sein d'un organisme.

Les analogues d'anticorps aptamères sont des oligonucléotides, généralement ARN ou ADN, identifiés dans des banques contenant jusqu'à $10^{15}$ séquences différentes, par une méthode combinatoire de sélection in vitro appelée SELEX pour « Systematic Evolution of Ligands by Exponentiel Enrichment » (Ellington AD et Szostak JW., 1990). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celle des anticorps.

Les analogues d'anticorps « DARPins » pour Designed Ankyrin Repeat ProteINS (Boersma YL et Plütckthun A, 2011) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par screening de banques combinatoires.

L'immunoessai consistant à déterminer la réponse anticorps est un essai qualitatif, semi-quantitatif ou quantitatif largement connu de l'homme du métier mettant en œuvre de préférence deux partenaires de liaison aux anticorps. L'un des deux partenaires peut être couplé à un marqueur pour former un conjugué ou un traceur. L'autre partenaire de liaison peut être capturé sur un support solide. On parle alors de partenaire de capture pour ce dernier et partenaire de détection pour le premier.

Les formats utilisant deux partenaires de liaison sont des formats sandwich bien connus de l'homme du métier, à savoir :

- un format communément appelé sandwich double antigène, utilisant en capture et en détection deux antigènes, de nature identique ou différente, capables d'être reconnus par l'anticorps recherché, étant entendu qu'au moins un des antigènes est un polypeptide de l'invention
- Un format communément appelé immunocapture, utilisant en capture un anticorps, un fragment d'anticorps ou un analogue d'anticorps, comme décrit précédemment, et en détection un polypeptide de l'invention et
- Un format communément appelé sandwich indirect, utilisant en capture un polypeptide de l'invention et en détection un anticorps, un fragment d'anticorps ou analogue d'anticorps.

De préférence, le partenaire de capture est un polypeptide de l'invention et le partenaire de détection est un anticorps anti-IgG ou IgM humain (format sandwich indirect).

Le signal mesuré émis lors de l'immunoessai est alors proportionnel à la quantité d'anticorps de l'échantillon biologique.

Par marqueur, on entend, notamment, toute molécule contenant un groupement réactif avec un groupement du partenaire de liaison, directement sans modification chimique, ou après modification chimique pour inclure un tel groupement, laquelle molécule est capable de générer directement ou indirectement un signal détectable. Une liste non limitative de ces marqueurs de détection directe consiste en :

- les enzymes qui produisent un signal détectable par exemple par colorimétrie, fluorescence, luminescence, comme la peroxydase de raifort, la phosphatase alcaline, la $\beta$-galactosidase, la glucose-6-phosphate déshydrogénase,
- les chromophores comme les composés fluorescents, luminescents, colorants,
- les molécules radioactives comme le $^{32}$P, le $^{35}$S ou le $^{125}$I,
- les molécules fluorescentes telles que les Alexa ou les phycocyanines, et
- les sels électrochimiluminescents tels que des dérivés organo-métalliques à base d'acridinium ou de ruthénium.

Des systèmes indirects de détection peuvent aussi être utilisés, comme par exemple des ligands capables de réagir avec un anti-ligand. Le ligand correspond alors au marqueur pour constituer, avec le partenaire de liaison, le conjugué.

Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Ces différents marqueurs peuvent être couplés aux polypeptides de l'invention comme indiqué précédemment.

Selon le type de marquage utilisé, l'homme du métier ajoutera des réactifs permettant la visualisation du marquage ou l'émission d'un signal détectable par tout type d'appareil de mesure approprié, comme par exemple un spectrophotomètre, un spectrofluorimètre, un densitomètre, un luminomètre ou encore une caméra haute définition.

L'immunoessai peut également comprendre d'autres étapes connues de l'homme du métier, telles que des étapes de lavage et des étapes d'incubation.

L'immunoessai peut être un essai en une étape ou en deux étapes, comme cela est largement connu de l'homme du métier. En quelques mots, un immunoessai en une étape comprend la mise en présence de l'échantillon à tester simultanément avec les deux partenaires de liaison, dont les polypeptides de l'invention tels que définis précédemment, alors qu'un immunoessai en deux étapes comprend la mise en présence de l'échantillon à tester d'une part avec le premier partenaire de liaison, puis le complexe analyte-premier partenaire de liaison ainsi formé est mis en présence du deuxième partenaire de liaison, l'un des deux partenaires de liaison étant un polypeptide de l'invention tel que défini précédemment.

Le signal de référence S utilisé dans le procédé selon l'invention est un signal obtenu au préalable avec deux populations de témoins, l'une ayant développé une réponse anticorps dirigée contre la protéine p-ORF2 suite à une infection par le virus HEV et l'autre n'ayant pas développé une telle réponse anticorps. Une telle détermination est largement connue de l'homme du métier. Elle consiste notamment à mettre en œuvre un immunoessai identique à celui mis en œuvre dans le procédé de l'invention, dans des échantillons biologiques de ces deux populations (de nature identique aux échantillons qui seront mis en œuvre dans le procédé de détermination de la présence de la réponse anticorps chez les sujets testés), et à déterminer la valeur du test (signal) permettant de faire une discrimination entre ces deux populations.

Le signal détecté, comparé au signal de référence, utilisé pour savoir si l'échantillon contient les anticorps recherchés ou non, peut correspondre au signal en tant que tel émis par le marqueur, ou bien il peut être transformé en un index qui est ratio signal détecté/signal de référence. Selon un exemple simple, pour lequel aucune zone grise n'existe, si l'index de référence est fixé à « 1 », un index pour l'échantillon testé supérieur à « 1 » signifie que l'échantillon contient lesdits anticorps et un index inférieur à « 1 » signifie que l'échantillon ne contient pas lesdits anticorps.

Bien entendu, toutes les définitions données précédemment au sujet des polypeptides d'appliquent au procédé de détermination de la présence d'une réponse anticorps dirigée contre la protéine p-ORF-2 du virus de l'hépatite E décrit ci-dessus.

Les polypeptides de l'invention peuvent également être utiles pour la détermination du taux d'anticorps dirigés contre la protéine p-ORF-2 du virus de l'hépatite E dans un échantillon biologique issu d'un sujet, susceptible de contenir lesdits anticorps. Cette détermination peut être mise en œuvre par immunoessai et comprend ou consiste en les étapes suivantes ::

- mettre en contact ledit échantillon biologique avec un polypeptide tel que défini précédemment,
- détecter un signal émis par la liaison entre ledit polypeptide et lesdits anticorps, s'ils sont présents, en utilisant un marqueur capable d'émettre un signal détectable,
- transformer le signal détecté en un taux d'anticorps.

Bien entendu, là encore, toutes les définitions données précédemment au sujet des polypeptides, ainsi que celles liées au procédé de détermination de présence de la réponse anticorps s'appliquent au procédé de détermination du taux d'anticorps. La seule différence consiste en le résultat donné, qui n'est pas un résultat de type « oui »/« non » suite à la comparaison du signal détecté à un signal de référence, mais un résultat de type concentration, ou titre, ou quantité, suite à la dernière étape consistant à transformer le signal détecté en un taux d'anticorps.

Cette étape de transformation du signal détecté en taux d'anticorps est largement connue de l'homme du métier. Elle consiste à utiliser un modèle mathématique préétabli à partir d'une gamme étalon. Cette gamme étalon sera obtenue préalablement de façon connue. En quelques mots, l'obtention d'une gamme étalon consiste à mesurer le signal généré par des quantités ou concentrations croissantes et connues de l'anticorps cible, à tracer la courbe donnant le signal en fonction du taux d'anticorps et à trouver un modèle mathématique qui représente de la manière la plus fidèle possible cette relation. Le modèle mathématique sera utilisé pour déterminer les quantités, titres ou concentrations d'anticorps anti-p-ORF2 inconnues, contenues dans l'échantillon biologique à tester.

Les anticorps recherchés dans l'échantillon biologique des sujets sont de diverses natures : IgM, IgG, IgA, IgE, les anticorps de type IgG et IgM étant préférés. On peut rechercher des anticorps de même nature, par exemple des IgG seules ou des IgM seules, ou bien on peut rechercher des anticorps de nature différente de manière combinée, par exemple des IgG et IgM simultanément ou tous les types d'immunoglobulines anti-ORF2 en même temps (Ig totales).

Quelle que soit la nature des anticorps recherchés, et de préférence lorsque ce sont des IgG ou des IgM, les procédés de détermination de la présence de la réponse anticorps ou du taux d'anticorps tels que décrits précédemment sont particulièrement utiles pour la prise en charge des sujets en lien avec une infection par le virus de l'hépatite E.

Par infection par le virus de l'hépatite E, on entend tant une infection présente, c'est-à-dire que le sujet chez qui on fait le test d'immunoessai est en train de faire l'infection, qu'une infection passée, c'est-à-dire que le sujet chez qui on fait le test d'immunoessai n'a plus de symptômes, mais a été en contact préalablement soit avec le virus, soit avec un vaccin contre le virus.

Aussi, un autre objet de l'invention concerne l'utilisation d'un procédé tel que défini précédemment pour l'aide au diagnostic *in vitro,* pour le diagnostic *in vitro* d'une infection au virus de l'hépatite E chez un sujet susceptible d'être infecté, pour le suivi thérapeutique d'un sujet infecté par le virus de l'hépatite E ou pour faire des études épidémiologiques de la séroprévalence des anticorps anti-HEV dans une population ou dans un territoire géographique donné.

Toutes ces utilisations sont bien connues de l'homme du métier, la seule condition étant qu'elles soient mises en œuvre avec les procédés décrits précédemment et donc les polypeptides décrits précédemment.

Lorsque les anticorps recherchés sont des IgG, les procédés tels que définis précédemment sont également particulièrement utiles pour déterminer si un sujet a besoin d'être vacciné ou revacciné contre le virus de l'hépatite E, ce qui constitue un autre objet de l'invention.

En effet, pour déterminer si le sujet a besoin d'être vacciné ou revacciné contre le HEV ou non, les étapes suivantes peuvent être mises en œuvre :

1. déterminer le taux d'anticorps IgG anti-HEV dans un échantillon biologique, notamment dans un échantillon de sang ou dérivé de sang, selon un procédé tel que défini précédemment, chez un sujet sain ou de préférence chez des patients à risque, tels que ceux décrits précédemment
2. comparer la réponse obtenue à un seuil, un tel seuil étant déterminé au préalable selon les exigences en vigueur,

3. la réponse obtenue étant inférieure au seuil signifiant qu'il convient de vacciner ou de revacciner le sujet

4. la réponse obtenue étant supérieure au seuil signifiant qu'il n'est pas nécessaire de vacciner ou de revacciner le sujet.

Bien entendu, les caractéristiques décrites précédemment dans le cadre des procédés de détermination de la présence de la réponse anticorps ou du taux d'anticorps s'appliquent aux utilisations faites de ces procédés, comme par exemple les polypeptides et leurs diverses longueurs et mutations, les échantillons biologiques et les sujets concernés.

Pour mettre en œuvre les procédés de l'invention, utilisés notamment selon les utilisations décrites ci-dessus, les polypeptides de l'invention peuvent être contenus dans des trousses.

Aussi, un autre objet de l'invention concerne les trousses pour la détermination par immunoessai de la présence de la réponse anticorps ou du taux d'anticorps dirigés contre la protéine p-ORF-2 du virus de l'hépatite E chez un sujet susceptible d'avoir produit ces anticorps, comprenant un polypeptide tel que défini précédemment.

Là encore, les caractéristiques décrites précédemment dans le cadre des polypeptides et procédés de l'invention s'appliquent aux trousses de l'invention.

Selon un mode de réalisation particulier, les trousses comprennent ou contiennent également au moins un contrôle positif. Ce contrôle positif comprend un composé capable de se lier aux partenaires de liaison mis en œuvre lors de l'utilisation de la trousse, le composé étant présent en un taux prédéterminé.

A titre d'exemples non limitatifs de tels composés, on peut citer les immunoglobulines naturelles anti-ORF2 (dans ce cas, le contrôle positif peut être un échantillon biologique séropositif ORF2), les immunoglobulines anti-ORF2 non naturelles, par exemple humanisées, les anticorps monoclonaux anti-ORF2, par exemple de souris.

Les trousses peuvent également contenir tous les composés nécessaires pour la mise en évidence de la réaction entre le ou les partenaires de liaison et les anticorps cibles, tels que des tampons de lavage ou des réactifs permettant la visualisation d'un marquage ou l'émission d'un signal détectable.

L'invention sera mieux comprise à l'aide des exemples suivants qui sont donnés à titre illustratif et non limitatif

## EXEMPLES

### Exemple 1 : Construction, expression et purification de fragments mutés et non-mutés 394-660 de la protéine de capside ORF2 du virus de l'hépatite E

La séquence ORF2 exprimée est celle de l'isolat Human/China/HeBei/1987 du virus de l'hépatite E qui est de génotype 1 (N° accession Uniprot Q81871 - Voir aussi figure 1 - SEQ ID N°11). Pour la construction de référence (ORF2-REF), la séquence correspondant aux acides aminés 394-660 de ORF2 (SEQ ID N°26) a été fusionnée du côté N-terminale avec un tag polyhistidines (8-his). Pour la construction selon l'invention (ORF2-MUT), 3 mutations non conservatives (cystéine vers sérine) ont été réalisées dans le fragment 394-660 de ORF2 au niveau des 3 cystéines en positions 627, 630 et 638 (SEQ ID N°27). Comme ORF2-REF, ORF2-MUT comporte un tag 8-his du côté N-terminale.

SEQ ID N°26 :

```
QLFYSRP     VVSANGEPTV   KLYTSVENAQ   QDKGIAIPHD   IDLGESRVVI
QDYDNQHEQD  RPTPSPAPSR   PFSVLRANDV   LWLSLTAAEY   DQSTYGSSTG
PVYVSDSVTL  VNVATGAQAV   ARSLDWTKVT   LDGRPLSTTQ   QYSKTFFVLP
LRGKLSFWEA  GTTKAGYPYN   YNTTASDQLL   VENAAGHRVA   ISTYTTSLGA
GPVSISAVAV  LAPHSALALL   EDTMDYPARA   HTFDDFCPEC   RPLGLQGCAF
QSTVAELQRL  KMKVGKTREL
```

SEQ ID N°27 :

```
QLFYSRP     VVSANGEPTV   KLYTSVENAQ   QDKGIAIPHD   IDLGESRVVI

QDYDNQHEQD  RPTPSPAPSR   PFSVLRANDV   LWLSLTAAEY   DQSTYGSSTG

PVYVSDSVTL  VNVATGAQAV   ARSLDWTKVT   LDGRPLSTTQ   QYSKTFFVLP

LRGKLSFWEA  GTTKAGYPYN   YNTTASDQLL   VENAAGHRVA   ISTYTTSLGA

GPVSISAVAV  LAPHSALALL   EDTMDYPARA   HTFDDFSPES   RPLGLQGSAF

QSTVAELQRL  KMKVGKTREL
```

Les fragments d'ADN correspondant aux constructions ORF2-REF et ORF2-MUT ont été obtenus sous forme de gènes synthétiques auprès de la société GeneArt® (Life Technologies). Ils ont été clonés entre les sites Nco I (5') et Bam HI (3') dans le vecteur pET3d (Novagen, EMD Millipore) sous le contrôle du promoteur T7 inductible à l'IPTG (isopropyl beta-D-1-thiogalactopyranoside). Les plasmides obtenus ont été vérifiés par séquençage au niveau des inserts afin de s'assurer qu'ils ne comportaient pas d'erreurs.

Les plasmides d'expression sont introduits dans des bactéries E. coli BL21 DE3 (Stratagene, Agilent Technologies) par transformation au choc thermique. Après isolement des colonies sur boîte de Pétri LB-agar contenant de l'ampicilline, une colonie correspondant à ORF2-REF et une correspondant à ORF2-MUT sont prélevées et ensemencées dans 200 mL de milieu de culture 2x YT, glucose 0,5%, en présence d'ampicilline 100 µg/mL, 1 nuit à 37°C, avec une agitation de 250 rpm. Un volume de 16 mL de chaque pré-culture est utilisé pour ensemencer 400 mL de milieu 2× YT-glucose 0,5%-ampicilline 100 µg/mL. Ces cultures sont incubées à 37°C sous agitation 250 rpm. Lorsque la densité optique (DO) mesurée à 600 nm atteint environ 1 unité de DO, l'induction de l'expression des protéines se fait par addition de 1 mM d'IPTG. La croissance des cultures est suivie par mesure de la densité optique à intervalles régulières. Au bout d'environ 3h d'induction, lorsque les cultures arrivent en phase stationnaire, les cultures sont arrêtées et les bactéries sont collectées par centrifugation (5000 g, 20 min, +2/8°C). Les culots bactériens sont pesés puis congelés à -80°C jusqu'à purification.

Pour la purification, les culots (2 à 2,2 g) sont repris par 30 mL de tampon de lyse (Tris HCl 20 mM, NaCl 100 mM, glycérol 5%, Benzonase® Nuclease 5U/mL (Novagen), MgCl2 0,48 g/L, inhibiteurs de proteases complete EDTA free (Roche, Ref 045-66462) 1 pastille/50 mL, pH 7,4). Les bactéries sont lysées par désintégration, en utilisant un Cell Disruption System (Constant Systems Ltd, Northants, Royaume-Uni), à 1600 bars en maintenant une réfrigération du système à +2/8°C. Le désintégrateur est rincé par 30 mL additionnels de tampon de lyse pour récupérer la totalité du lysat. Les lysats sont ensuite centrifugés à 10 000 g, 40 min, +2/8°C et les culots sont récupérés.

Afin de solubiliser les corps d'inclusions, chaque culot est repris par 30 mL d'un tampon Tris HCl 20 mM, NaCl 100 mM, glycérol 5%, urée 5M, pH 7,4 et agité 1h30 à +18/25°C. Les surnageants sont récupérés par centrifugation à 10 000 g, 20 min, température ambiante, puis filtrés successivement sur des filtres en nitrocellulose de 1,2 µm et 0,8 µm.

La purification des protéines ORF2-REF et ORF2-MUT se fait par chromatographie d'affinité métal chélate en une étape, grâce à leurs tags poly-histidines. La purification est réalisée sur un système automatisé de type ÄKTA (GE Healthcare Lifesciences). Le surnageant obtenu après centrifugation est chargé sur une colonne de résine Ni-NTA (Roche, Ref 058-93682001) équilibrée en tampon Tris HCl 20 mM, NaCl 100 mM, glycérol 5%, urée 5M, pH 7,4 (tampon d'équilibration, identique au tampon de solubilisation). Le tampon d'élution est du tampon d'équilibration contenant 300 mM d'imidazole et dont le pH a été réajusté à 7,4. Un cycle de lavage est effectué avec le tampon d'équilibration contenant 40 mM d'imidazole. Ensuite la protéine est éluée par un plateau à 100% de tampon d'élution, soit 300 mM d'imidazole. Les fractions de purification sont analysées sur gel SDS-PAGE coloré au bleu de Coomassie. Cette analyse permet de vérifier le déroulement du procédé de purification et la sélection des fractions contenant la protéine d'intérêt.

Les fractions sélectionnées sont poolées et dialysées en tampon Tris HCl 40 mM, NaCl 250 mM, mannitol 10%, arginine 0,4 M, urée 2M, pH 7,4. Deux dialyses successives sont effectuées à +18/25°C contre un volume de tampon 100 fois supérieur à celui de l'échantillon. Les protéines dialysées sont dosées en protéine totale par mesure de la densité optique à 280 nm, puis conservées à -80°C.

**Exemple 2 : Caractérisation par analyse SDS-PAGE des protéines ORF2-REF et ORF2-MUT**

Une première caractérisation des protéines purifiées ORF2-REF et ORF2-MUT a été réalisée par analyse SDS-PAGE sur un gel NuPAGE® Bis-Tris 4-12% en tampon NuPAGE® MES SDS (Life Technologies). Avant chargement sur le gel (10 µL/puits), les protéines ont été diluées dans le tampon NuPAGE® LDS Sample Buffer 4X (Life Technologies) (3/1, volume/volume) et ont subi différents traitements. La réduction se fait par ajout de 50 mM final de dithiothreitol (DTT). Le chauffage est de 10 min à 75°C. Les combinaisons testées sont les suivantes :

CHAUFFEE et REDUITE (avec DTT)
CHAUFFEE et NON REDUITE (sans DTT)
NON CHAUFFEE et REDUITE (avec DTT)
NON CHAUFFEE et NON REDUITE (sans DTT)

Une photographie du gel de SDS-PAGE coloré au bleu de Coomassie afin de visualiser des protéines totales est présentée en Figure 3. Réduites et chauffées (bandes sous les colonnes + et + dans le tableau), les protéines ORF2-REF et ORF2-MUT ont le même poids moléculaire qui est légèrement supérieur à 30 kDa. Cette condition d'analyse permet de visualiser la forme monomérique des deux protéines.

En condition non réduite et chauffée (bandes sous les colonnes + pour chauffe et

- pour réduit dans le tableau), l'ORF2-REF présente 4 bandes dont une majoritaire de poids moléculaire apparent inférieur à 70 kDa. Cette bande correspond à une forme dimérique de la protéine ORF2-REF : les deux monomères sont reliés par au moins une liaison covalente (pont disulfure) qui n'est pas détruite par une dénaturation à la chaleur et qui nécessite l'ajout d'un réducteur. Dans les mêmes conditions d'analyse, l'ORF2-MUT présente une bande unique, elle est donc monomérique.

En condition non chauffée, avec ou sans présence de réducteur (bande sous les colonnes - pour chauffe et respectivement + ou - pour réduit dans le tableau), l'ORF2-REF présente un profil de migration complexe avec de nombreuses bandes, soulignant la diversité des interactions ayant lieu entre les monomères. L'hétérogénéité formes oligomériques en présence dans l'ORF2-REF est bien mise en évidence dans la ligne analysée les conditions non-dénaturantes, c'est-à-dire non chauffées et non réduites. On observe la présence d'au moins de 5 bandes de haut poids moléculaire en plus des bandes qui correspondent au dimère covalent et non-covalent. A l'inverse l'ORF2-MUT non chauffée, réduite ou pas (bande sous les colonnes - pour chauffe et respectivement + ou - pour réduit dans le tableau), présente un profil de migration très simple, avec une bande largement majoritaire qui correspond au dimère non-covalent. On note également des traces de monomère et une bande qui migre à environ 80 kDa qui est très probablement la forme tetramérique non-covalente.

Ainsi, la protéine ORF2-MUT est bien plus homogène que la protéine ORF2-REF et se trouve essentiellement sous la forme d'un dimère non-covalent. L'ORF2-REF, très hétérogène, contient à la fois des dimères covalents (forme majoritaire), des dimères non-covalents et diverses formes de haut poids moléculaire.

## Exemple 3: Caractérisation des protéines ORF2-REF et ORF2-MUT par marquage fluorescent des cystéines libres

Afin d'affiner les résultats précédents, nous avons voulu déterminer, pour chaque préparation protéique, la proportion de cystéines libres et de cystéines engagées dans des ponts disulfures. L'échantillon protéique est divisé en deux: la première moitié subit une alkylation directe des thiols libres des cystéines accessibles ; la seconde moitié subit une alkylation après réduction et chauffage, traitement qui rend accessible toutes les cystéines.

L'alkylation se fait grâce au réactif fluorescent BODIPY® FL iodoacétamide (Life Technologies, Ref. D-6003) qui présente des caractéristiques spectrales très similaires à la fluorescéine. Le marquage se fait suivant les instructions du fabricant. Très brièvement, il faut préparer de manière extemporanée une solution mère de BODIPY® FL iodoacétamide à 1 ou 10 mM et diluer les protéines à 100 $\mu$M. A l'abri de la lumière, on ajoute goutte à goutte le BODIPY® FL iodoacétamide dans la solution de protéine à marquer (10 à 20 moles de BODIPY® FL iodoacétamide pour 1 mole de protéine) et on incube 30 à 60 min à l'obscurité. La protéine ainsi marquée est migrée sur un gel de SDS-PAGE afin de la séparer de l'excédent de fluorophore. Le gel est ensuite visualisé sur un système d'imagerie en fluorescence (ChemiDocTM XRS+, Bio-Rad) et l'intensité de fluorescence au niveau de la bande de protéine est mesurée. Cette fluorescence est spécifique et proportionnelle au nombre de cystéines marquées.

L'analyse est réalisée en quantité relative en prenant pour référence l'intensité de fluorescence du monomère ORF2-REF obtenu après chauffage et réduction. Dans cette molécule, il y a 3 cystéines et en théorie dans ces conditions toutes les cystéines sont marquées (100% de fluorescence). La protéine ORF2-MUT n'est pas marquée par le BODIPY® FL iodoacétamide. Environ 1% de fluorescence est détectée pour la protéine ORF2-MUT, il s'agit du bruit de fond non spécifique. Concernant la protéine ORF2-REF, il n'y a pas de fluorescence détectée dans l'échantillon non chauffé non réduit. Ceci indique qu'aucune cystéine n'est accessible par l'agent alkylant, ce qui est en accord avec le profil observé en SDS-PAGE (Figure 3). Pour l'échantillon d'ORF2-REF chauffé, la bande monomère correspond à une intensité de fluorescence de 5%, ce qui indique que 5% des cystéines d'ORF2-REF ne sont pas engagées dans des ponts disulfures mais enfouies dans le cœur de la protéine et donc non accessibles lorsque l'échantillon n'est pas chauffé.

Cette analyse permet bien de confirmer que la protéine ORF2-REF est majoritairement non-monomérique. Formant à la fois des dimères covalents et des dimères non-covalents, la protéine ORF2-REF est bien plus hétérogène que la protéine ORF2-MUT.

**Exemple 4: Caractérisation des protéines ORF2-REF et ORF2-MUT par chromatographie d'exclusion stérique (SEC - « size exclusion chromatography »)**

La chromatographie d'exclusion stérique permet de séparer les molécules selon leur taille. Chaque résine de chromatographie d'exclusion est caractérisée par un domaine de fractionnement spécifique, exprimé en masse moléculaire, à l'intérieur duquel la séparation des molécules est possible. Les molécules dont la taille est inférieure à la limite inférieure du domaine de fractionnement ou supérieure à sa limite supérieure ne sont pas fractionnées de manière efficace. Les molécules dont la taille dépasse la limite d'exclusion, exprimé aussi en masse moléculaire, ne sont pas fractionnées et sont élués ensemble dans le volume mort de la colonne.

Les analyses par chromatographie d'exclusion stérique ont été effectuées sur une chaine HPLC (« high performance liquid chromatography ») Waters Alliance avec une colonne Superdex 200 10/300 GL (GE Healthcare) en tampon PBS (« phosphate buffered saline »). Le domaine de fractionnement efficace de la résine Superdex 200 est de 10 à 600 kDa et sa limite d'exclusion est de 1300 kDa. Pour chaque protéine ORF2, 100 $\mu$L d'échantillon (175 $\mu$g environ) ont été injectés à 0,5 mL/min. La détection se fait par mesure de l'absorbance à 280 nm. Les chromatogrammes obtenus pour chaque protéine sont présentés en Figure 4. Le chromatogramme d'ORF2-REF (Figure 4A) montre 3 populations, une population majoritaire représentant 86,9% des formes observées, et deux populations supplémentaires, correspondant à 8,5% et 4,2% des formes observées, éluant un peu avant et un peu après le pic majoritaire, respectivement. En revanche, sur le chromatogramme d'ORF2-MUT (Figure 4B), on observe la présence d'un pic unique, représentant 99,9% des formes observées.

Pour chacun des chromatogrammes, l'intégration du signal d'absorbance à 280 nm au niveau des pics permet de déterminer la quantité totale de protéine qui a été fractionnée lors de l'analyse. Pour la protéine ORF2-REF, la somme des aires sous chacun des 3 pics est de 6800 mU*sec. Pour la protéine ORF2-MUT, l'aire sous le pic unique est de 16100 mU*sec. La quantité d'ORF2-REF fractionnée lors de l'analyse représente seulement 42% de la quantité d'ORF2-MUT fractionnée (ratio des aires), alors qu'initialement, la même quantité de chacune des protéines avait été injectée. On peut en déduire qu'une importante fraction d'ORF2-REF n'est pas rentrée dans la résine et se trouve donc sous forme de précipité retenu au niveau du préfiltre de la colonne. La précipitation par agrégation est favorisée du fait qu'à la différence de l'électrophorèse SDS-PAGE, aucun réactif de l'analyse par chromatographie SEC ne contient du SDS ou un autre détergent qui pourrait contribuer à la solubilisation des protéines.

En conclusion, l'analyse en chromatographie d'exclusion stérique a permis de confirmer par une technique indépendante que la protéine ORF2-MUT (1 forme observée) est bien plus homogène que la protéine ORF2-REF (3 formes observées). Dans les conditions de l'analyse, une grande partie de la protéine ORF2-REF se trouve sous forme de précipité et ne peut donc pas être étudiée. D'ailleurs, il ne peut pas être exclu qu'un phénomène de précipitation similaire ou encore d'auto-assemblage ait aussi lieu pour la protéine ORF2-MUT et qu'au moins une partie de celle-ci n'ait pas pu être analysé. Afin de compléter l'analyse SEC et de pouvoir démontrer de façon plus certaine que la protéine ORF2-MUT ne contient pas d'agrégats, il est nécessaire d'utiliser une technique de caractérisation biophysique alternative, permettant d'effectuer des analyses sur une très large gamme de tailles moléculaires.

**Exemple 5: Caractérisation des protéines ORF2-REF et ORF2-MUT par la technique AsFlFFF-MALS (« asymetric flow field flow fractionation-multi angle light scattering »)**

Pour pouvoir étudier l'état d'agrégation des protéines ORF2-REF et ORF2-MUT en conditions natives, nous avons mis en œuvre une technique qui permet la séparation d'une large gamme de molécules allant 5 kDa à 10 $\mu$m. Il s'agit du fractionnement par couplage flux/force avec flux asymétrique (« asymetric flow field flow fractionation », AsFlFFF ou AF4) couplé à une détection en diffusion de lumière multi-angle (« multi-angle light scattering ou MALS »). Les macromolécules sont séparées selon leur coefficient de diffusion, sous l'effet de flux croisés, sans aucune phase stationnaire et en conditions natives. L'absence de phase stationnaire est un avantage considérable car celle-ci peut interagir avec une ou partie des espèces moléculaires que l'on cherche à séparer et biaiser ainsi l'analyse.

L'analyse AsFlFFF-MALS a été réalisée par l'équipe Qualités Biologiques et Technologique des Matières Premières Végétales de l'INP de Toulouse (Ecole d'ingénieurs Purpan, Toulouse). Les conditions expérimentales de l'analyse telle que réalisée sont les suivantes :

| | |
|---|---|
| HPLC | Ultimate 3000 Dionex |
| AsFlFFF | Eclipse 2 Wyatt |
| MALS | Heleos II Wyatt (633nm) |
| Eluant | PBS 1x + 500 mM de NaCl |

(suite)

| Volume d'échantillon injecté | 30 $\mu$L et 60 $\mu$L |
|---|---|
| Cellule AsFIFFF | Small |
| Membrane | RC 5 kDa |
| Spacer | 350 $\mu$m W |
| Flux linéaire | 1 mL/min |
| Flux croisé | 3 à 0,1 |
| Débit d'injection | 0,2 mL/min |
| Détecteurs | UV 280 nm_1A |
| Paramètres traitements données | MALS: Model Zimm |
| | dn/dc : 0,185 mL/g |
| | UV extinction : 1246 mL/(g cm) |

Le profil du fractogramme obtenu pour la protéine ORF2-REF n'est pas présenté car l'analyse est difficilement interprétable. En effet un premier pic écrase tout le signal MALS de l'analyse, rendant peu précises et peu fiables les estimations de masse moléculaire. Toutefois on peut conclure à la présence de très grands agrégats dont la taille est estimée à $10^5$ - $10^6$ kDa.

Les profils de fractogrammes obtenus pour les signaux UV (trait plein fin) et MALS (trait hachuré) de la protéine ORF2-MUT sont donnés sur la Figure 5. On observe en UV (trait plain fin) un pic majoritaire avec un épaulement qui élue de 9 à 15 minutes. Le caractère bimodal de ce pic, suggéré en UV, apparaît très clairement en MALS (trait hachuré). Dans celui-ci, il y a présence d'une première population de masse molaire estimée environ à 70 kDa (75% de l'échantillon, élution entre 9,2 et 11,7 min) et d'une seconde population de masse molaire estimée environ à 356 kDa (25% de l'échantillon, élution entre 11,7 et 15,0 min). Pour un monomère de la protéine ORF2-MUT, la masse molaire théorique calculée à partir de sa séquence est de 31 kDa. Ce calcul théorique a été confirmé expérimentalement lors de l'analyse SDS-PAGE présentée dans l'Exemple 2. Ainsi, la masse molaire observée d'environ 70 kDa correspond à un dimère et celle de 356 kDa correspond à un dodécamère (12-mer) d'ORF2-MUT. Enfin, et contrairement à ORF2-REF, ORF2-MUT ne contient pas de gros agrégats détectables qui éluent en mode stérique en début de fractogramme.

En conclusion, l'analyse AsFIFFF-MALS, méthode sophistiquée permettant une caractérisation des espèces moléculaires en conditions natives, sans interactions éventuelles avec d'une phase stationnaire, a permis de monter que la protéine ORF2-MUT i) est un mélange de 75% de dimères non-covalents et 25% de dodécamères non-covalents, ii) ne contient aucun d'agrégats à l'état natif, et iii) est bien plus homogène que l'ORF2-REF. L'hétérogénéité des espèces moléculaires dans la protéine ORF2-REF est tellement importante que même une technique aussi sophistiquée et résolutive que l'AsFIFFF-MALS ne permet de caractériser de manière fiable la répartition des différentes formes.

### Exemple 6 : Comparaison des réactivités immunologiques des antigènes ORF2-REF et ORF2-MUT et des performances diagnostiques des immunoessais utilisant ces antigènes pour la détection des IgM anti-ORF2

L'antigénicité des protéines ORF2-REF et ORF2-MUT a été comparée en immunoessai en utilisant l'automate d'immunoanalyse VIDAS® (bioMérieux). Le cône à usage unique sert à la fois de phase solide pour la réaction et de système de pipetage. La cartouche est composée de 10 puits (X0 à X9) recouverts d'une feuille d'aluminium scellée et étiquetée. Le premier puits (X0) comporte une partie prédécoupée pour faciliter l'introduction de l'échantillon. Le dernier puits (X9) est une cuvette optique dans laquelle la fluorescence du substrat est mesurée. Les différents réactifs nécessaires à l'analyse sont contenus dans les puits intermédiaires (X1 à X8). Toutes les étapes du test sont réalisées automatiquement par l'instrument. Elles sont constituées d'une succession de cycles d'aspiration/refoulement du milieu réactionnel.

a) Sensibilisation et passivation des cônes (coating)

Les cônes ont été sensibilisés avec 300 $\mu$L d'une solution d'ORF2-REF ou d'ORF2-MUT à 2 $\mu$g/mL dans un tampon carbonate 77 mM, pH 9,2. Après environ 20h d'incubation à +18/25°C avec la solution de sensibilisation, les cônes sont vidés. Ensuite, 300 $\mu$L d'une solution de Tris 200 mM contenant de 5 g/L d'albumine bovine sont ajoutés. La passivation se poursuit à +18/25°C sur la nuit. Les cônes sont vidés, séchés, puis conservés à +4°C jusqu'à utilisation, à l'abri de l'humidité.

b) Mode opératoire de l'immunoessai

L'automate VIDAS® mélange 600 μL de diluant échantillon contenant du Tris 20 mM pH 7,4, NaCl 300 mM et 5 g/L d'albumine sérique avec 38,3 μL de l'échantillon de sérum ou de plasma à tester. Dès que le cône VIDAS® est en contact avec l'échantillon, la première étape de la réaction immunologique commence. Cette étape permet la liaison spécifique des IgM anti-ORF2, présents ou non l'échantillon de sérum ou de plasma, à la protéine ORF2 adsorbée sur le cône. Après 4 minutes d'incubation à 37°C, les composants non liés sont éliminés par lavages avec un tampon Tris 200 mM pH 9, NaCl 300 mM, Triton X-100 0,275%. Lors de la seconde étape, le cône est incubé avec une solution de conjugué contenant environ 60 ng/mL d'une IgG de souris anti-IgM humain (bioMérieux), couplé à la phosphatase alcaline, dans un tampon phosphate 10 mM, contenant 300 mM de NaCl et 5 g/L d'albumine sérique bovine. Le puits X5 contient 400 μL de cette solution que le cône aspire/refoule pendant 5 minutes, toujours à 37°C. La seconde étape entraine la formation d'un complexe entre les IgM anti-ORF2 présents dans l'échantillon et le conjugué anti-IgM couplé à la phosphatase alcaline. Cette étape est suivie de 2 lavages successifs afin d'éliminer les composés non fixés.

Lors de l'étape finale de révélation, le substrat 4-méthylombelliferyl phosphate est aspiré puis refoulé dans le cône ; la phosphatase alcaline du conjugué catalyse la réaction d'hydrolyse de ce substrat en 4-méthylombelliferone dont la fluorescence émise est mesurée à 450 nm. La valeur du signal de fluorescence (RFV=relative fluorescence value) est proportionnelle à la concentration des IgM anti-ORF2 présents dans l'échantillon.

La procédure d'immunoessai pour la détection des IgM anti-ORF2 a été mise en œuvre dans 18 échantillons IgM HEV positifs et 21 échantillons IgM HEV négatifs. Ces échantillons, sérums ou plasmas, ont été principalement obtenus via les Etablissements Français du Sang (EFS) et ont été caractérisés au préalable par divers tests commerciaux : Wantai HEV-IgM ELISA (Ref. WE-7196), recomWell HEV IgM (Ref. 5005, Mikrogen Diagnostik) ou EIAgen HEV IgM kit (Ref. 071050 Adaltis). Le statut IgM HEV positif des échantillons a été ainsi défini si l'échantillon était positif au moins dans un des tests cités ci-dessus. Les échantillons dits HEV négatifs sont quant à eux négatifs dans l'ensemble des techniques commerciales mises en œuvre.

**Immunoréactivité.** A quantité égale, la protéine ORF2-MUT présente une réactivité antigénique largement supérieure à celle la protéine ORF2-REF. Cette supériorité est très significative statistiquement (*P*<0.0001, test de Wilcoxon apparié unilatéral) et est illustrée en Figure 6 qui représente les distributions des signaux RFV obtenus par les immunoessais IgM utilisant soit l'antigène ORF2-REF (appelé ensuite test IgM ORF2-REF), soit l'antigène ORF2-MUT (appelé ensuite test IgM ORF2-MUT), sur des échantillons HEV positifs (Tableau 1) et des échantillons HEV négatifs (Tableau 2). Pour tous les échantillons positifs, les signaux RFV obtenus avec l'antigène ORF2-MUT sont supérieurs à ceux obtenus avec l'antigène ORF2-REF. Pour les échantillons 155797, 154183, 154053 et 154050, le gain en RFV, très conséquent, atteint environ 1000 RFV. De plus, les signaux RFV obtenus sur les échantillons HEV négatifs par les tests IgM ORF2-REF et ORF2-MUT sont comparables et restent très bas (Figure 6).

**Sensibilité diagnostique.** Sur le panel d'échantillons positifs analysés présenté dans le Tableau 1, le test IgM ORF2-REF, selon l'art antérieur, présente deux faux-négatifs (échantillons 155118 et 136997), ce qui correspond à une sensibilité de 88,9% seulement alors que le test IgM ORF2-MUT n'a aucun faux-négatif ce qui se traduit par une sensibilité augmentée à 100%.

De plus, le panel analysé a été testé au préalable par le test Wantai afin de pouvoir identifier des échantillons pour lesquels ce dernier est négatif, mais qui ont été confirmés positifs par deux autres trousses IgM. Le but de cette sélection était de mettre en évidence les avantages des polypeptides 394-660 de l'invention. Le kit IgM de Wantai est la seule trousse commerciale IgM comprenant uniquement un antigène ORF2, appelé pE2, donc directement comparable à un immunoessai IgM utilisant ORF2-REF ou encore ORF2-MUT. Cependant, à la différence des polypeptides 394-660, la séquence de l'antigène pE2 ne comprend pas l'épitope C-terminal (aa 613-654). Sur le panel d'échantillons positifs testés, le test Wantai présente 6 faux-négatifs, soit une sensibilité de 66,6% uniquement. Parmi ces échantillons, 4/6 sont détectés positifs par le test IgM ORF2-REF, illustrant l'intérêt diagnostic de l'épitope C-terminal et surtout 6/6 sont détectés par le test IgM ORF2-MUT, illustrant une nouvelle fois la supériorité de ce polypeptide, ainsi que sa contribution à la sensibilité améliorée d'un immunoessai.

Tableau 1. Recherche des IgM anti-ORF2 dans les sérums des patients ayant une infection hépatite E aigue de manière certaine. Etude de sensibilité sur échantillons positifs confirmés.

| | Test Wantai (Pos si Indice >= 1) | | Immunoessai VIDAS IgM ORF2-REF (Pos si RFV >= 70) | | Immunoessai VIDAS IgM ORF2-MUT (Pos si RFV >= 70) | |
|---|---|---|---|---|---|---|
| Identifiant échantillo n | Indice | Interpréta tion | Signal (RFV) | Interpréta tion | Signal (RFV) | Interpréta tion |
| 155118 | 0,0 | **Neg** | 50 | **Neg** | 76 | Pos |
| 136997 | 0,5 | **Neg** | 64 | **Neg** | 122 | Pos |

(suite)

| Identifiant échantillo n | Test Wantai (Pos si Indice >= 1) | | Immunoessai VIDAS IgM ORF2-REF (Pos si RFV >= 70) | | Immunoessai VIDAS IgM ORF2-MUT (Pos si RFV >= 70) | |
|---|---|---|---|---|---|---|
| | Indice | Interpréta tion | Signal (RFV) | Interpréta tion | Signal (RFV) | Interpréta tion |
| 123971 | 0,5 | **Neg** | 98 | Pos | 145 | Pos |
| 143289 | 0,4 | **Neg** | 115 | Pos | 159 | Pos |
| 136360 | 1,5 | Pos | 156 | Pos | 225 | Pos |
| 130162 | 2,2 | Pos | 209 | Pos | 236 | Pos |
| 144054 | 0,0 | **Neg** | 217 | Pos | 252 | Pos |
| 39417 | 0,4 | **Neg** | 291 | Pos | 536 | Pos |
| 9264883 | 7,5 | Pos | 646 | Pos | 785 | Pos |
| 9264884 | 5,7 | Pos | 753 | Pos | 1014 | Pos |
| 155798 | 4,6 | Pos | 821 | Pos | 1025 | Pos |
| 155795 | 5,6 | Pos | 484 | Pos | 1072 | Pos |
| 155799 | 8,3 | Pos | 934 | Pos | 1191 | Pos |
| 155797 | 3,5 | Pos | 394 | Pos | 1737 | Pos |
| 154183 | 1,8 | Pos | 843 | Pos | 1820 | Pos |
| 154050 | 10,1 | Pos | 1596 | Pos | 2705 | Pos |
| 155796 | 8,5 | Pos | 2237 | Pos | 2916 | Pos |
| 154053 | 13,6 | Pos | 3200 | Pos | 4503 | Pos |
| Neg = Négatif et Pos = Positif | | | | | | |

**Spécificité diagnostique.** Sur le panel d'échantillons négatifs analysés (Tableau 2), le test IgM ORF2-REF présente deux faux-positifs (échantillons 129534 et 137163), ce qui correspond à une spécificité de 88,9% seulement alors que le test IgM ORF2-MUT n'a aucun faux-positif ce qui se traduit par une spécificité augmentée à 100%.

Il faut noter que la sensibilité améliorée du test IgM ORF2-MUT ne se fait pas au dépend de sa spécificité.

Tableau 2. Recherche des IgM anti-ORF2 dans les sérums des patients n'ayant pas d'infection hépatite E de manière certaine. Etude de spécificité sur échantillons négatifs confirmés.

| Identifiant échantillon | Immunoessai VIDAS IgM ORF2-REF (Neg si RFV < 70) | | Immunoessai VIDAS IgM ORF2-MUT (Neg si RFV < 70) | |
|---|---|---|---|---|
| | Signal (RFV) | Interprétatio n | Signal (RFV) | Interprétatio n |
| 34848 | 27 | Neg | 17 | Neg |
| 39418 | 39 | Neg | 39 | Neg |
| 40880 | 13 | Neg | 14 | Neg |
| 123933 | 33 | Neg | 24 | Neg |
| 129534 | 89 | **Pos** | 48 | Neg |
| 129624 | 30 | Neg | 44 | Neg |
| 134067 | 25 | Neg | 18 | Neg |
| 134700 | 48 | Neg | 50 | Neg |
| 134733 | 44 | Neg | 55 | Neg |
| 59091077237 | 67 | Neg | 54 | Neg |
| 134718 | 36 | Neg | 34 | Neg |
| 137163 | 82 | **Pos** | 54 | Neg |
| 141316 | 48 | Neg | 40 | Neg |
| 144810 | 50 | Neg | 49 | Neg |
| 144098 | 59 | Neg | 45 | Neg |
| 155122 | 41 | Neg | 61 | Neg |
| 34678 | 20 | Neg | 13 | Neg |

(suite)

| Identifiant échantillon | Immunoessai VIDAS IgM ORF2-REF (Neg si RFV < 70) | | Immunoessai VIDAS IgM ORF2-MUT (Neg si RFV < 70) | |
|---|---|---|---|---|
| | Signal (RFV) | Interprétatio n | Signal (RFV) | Interprétatio n |
| 34745 | 15 | Neg | 10 | Neg |
| 35049 | 17 | Neg | 14 | Neg |
| 39321 | 8 | Neg | 7 | Neg |
| 40879 | 15 | Neg | 10 | Neg |

En conclusion, l'antigène ORF2-MUT présente une meilleure immunoréactivité que l'antigène ORF2-REF, ce qui se traduit par des performances diagnostiques supérieures tant en sensibilité qu'en spécificité. Cette meilleure immunoréactivité de la protéine ORF2-MUT pourraient être expliquée par une meilleure présentation des épitopes conformationnels immunodominants du fait de sa structure plus homogène et plus oligomérique, comme montré dans l'Exemple 2 (plus de dimères non covalents) et dans l'Exemple 5 (formation de dodécamères), ce qui lui permettrait de présenter au global une structure antigénique beaucoup plus proche de celle de la particule virale.

**Exemple 7 : Reproductibilité des tests de détection des IgM anti-virus de l'hépatite E utilisant ORF2-REF ou ORF2-MUT**

Le même échantillon positif a été dosé en dupliquât, dans deux séries différentes, 3 jours de suite, par le test IgM ORF2-REF et le test IgM ORF2-MUT selon le mode opératoire décrit dans l'Exemple 6. Les résultats sont présentés dans le Tableau 3. Le coefficient de variation (CV) est le rapport de l'écart-type à la moyenne et permet la comparaison de distributions de valeurs dont les échelles de mesure ne sont pas comparables. Plus la valeur du coefficient de variation est faible, plus la dispersion autour de la moyenne est petite, donc plus la mesure est reproductible. Le coefficient de variation est de 5,4% pour le test IgM ORF2-REF et de 2,1% pour le test IgM ORF2-MUT. Les deux immunoessais sont bien reproductibles, le test IgM ORF2-MUT semble meilleur.

Tableau 3. Reproductibilité des tests de détection des IgM anti-ORF2 utilisant la protéine ORF2-REF ou ORF2-MUT.

| | Signal RFV | |
|---|---|---|
| | IgM ORF2-REF | IgM ORF2-MUT |
| Jour 1 - Série 1 | 274 | 566 |
| Jour 1 - Série 1 | 293 | 552 |
| Jour 1 - Série 2 | 289 | 577 |
| Jour 1 - Série 2 | 296 | 589 |
| Jour 2 - Série 1 | 307 | 554 |
| Jour 2 - Série 1 | 287 | 563 |
| Jour 2 - Série 2 | 269 | 547 |
| Jour 2 - Série 2 | 273 | 559 |
| Jour 3 - Série 1 | 297 | 573 |
| Jour 3 - Série 1 | 309 | 560 |
| Jour 3 - Série 2 | 265 | 553 |
| Jour 3 - Série 2 | 267 | 563 |
| Moyenne | 286 | 563 |
| CV% Total | 5,4% | 2,1% |

Afin de pouvoir déterminer si la différence observée entre les 2 CV est statistiquement significative, l'incertitude de chacun d'eux est estimée. En acceptant un risque a=0,05, (intervalle de confiance IC à 95%) et en supposant que

EP 3 383 887 B1

le risque est réparti de manière symétrique et bilatérale (i.e. autant de risque que le CV soit surestimé que sous-estimé), on en déduit le CV limite supérieure en appliquant la formule suivante :

$$\text{CV}_{\text{limite supérieure}} = \sqrt{Khi^2(0{,}025, ddl)} \div \sqrt{ddl} \times \text{CV}$$

Khi$^2$ (0,025, ddl) est la valeur de la loi Khi$^2$ pour un risque de 0,025 (la moitié de $\alpha$=0,05) et un degré de liberté (ddl) donné. Pour les séries présentées, le nombre de répétions est n=12 et ddl = n-1, soit 11. La valeur de la loi Khi$^2$ (0,025, 11) est de 21,92. La limite supérieure de l'IC 95% du CV est donnée par la formule. La limite inférieure de l'IC 95% est déduite en soustrayant au CV observé la différence entre la limite supérieure et le CV observé. On aboutit ainsi aux estimations suivantes :

|  | CV observé | CV Limite supérieure | CV Limite inférieure |
|---|---|---|---|
| ORF2-REF | 5,4% | 7,7% | 3,2% |
| ORF2-MUT | 2,1% | 3,0% | 1,3% |

Selon ces calculs, le CV du test IgM ORF2-REF peut être compris entre 3,2% et 7,7% et celui du test IgM ORF2-MUT entre 1,3% et 3,0%. Les deux intervalles ne se chevauchent pas, les deux CV observés, de 5,4% et de 2,1%, sont donc significativement différents.

En conséquence, le test IgM ORF2-MUT est plus reproductible que le test IgM ORF2-REF.

**Références Bibliographiques**

- Boersma YL, Plückthun A, 2011, Curr. Opin. Biotechnol, 22 : 849-857
- Ellington AD et Szostak JW., 1990, Nature, 346 : 818-822
- Emerson, S. U., & Purcell, R. H., 2007, Hepatitis E Virus. In D. M. Knipe, P. M. Howley, D. E. Griffin, R. A. Lamb, M. A. Martin & B. a. S. Roizman S.E. (Eds.), Fields Virology (5th ed., pp. 3047-3058). Philadelphia, USA: Lippincott Williams & Wilkins
- Fields et Noble, 1990, Int J Pept Protein Res., 35:161-214
- Meng J, et al., 2001, Virology, 288: 203-211
- Merrifield 1963, J Am Chem Soc. 85:2149-2154
- Riddell M.A., et al., 2000, Journal of Virology, 74(17) : 8011-8017

**Revendications**

1. Polypeptide apte à se lier à des anticorps anti-HEV, choisi parmi :

   - un polypeptide comprenant au moins la séquence 394-660 d'une protéine p-ORF2 du virus de l'hépatite E ayant une longueur de 660 acides aminés de long et dans lequel les trois acides aminés en positions 627, 630 et 638 sont différentes de la cystéine ; dans lequel, ladite protéine p-ORF2 de 660 acides aminés de long ayant une identité de séquence d'au moins 89 % avec SEQ ID N°11, et ladite séquence 394-660 ayant une identité de séquence d'au moins 90 % avec SEQ ID N°26 ; et

- un polypeptide comprenant une séquence analogue à SEQ ID N°26, ayant une identité de séquence d'au moins 90 % avec SEQ ID N°26 et provenant d'une protéine p-ORF2 du virus de l'hépatite E de longueur différente à 660 acides aminés et ayant une identité de séquence d'au moins 89 % avec SEQ ID N°11, et dans lequel les trois acides aminés correspondant aux trois cystéines des positions 627, 630 et 638 de SEQ ID N°11, sont différentes de la cystéine.

2. Polypeptide selon la revendication 1, dans lequel :

- ladite protéine p-ORF2 du virus de l'hépatite E ayant une longueur de 660 acides aminés et une identité de séquence d'au moins 89 % avec SEQ ID N°11, est choisie parmi les séquences SEQ ID N°11 à 23, et
- ladite protéine p-ORF2 du virus de l'hépatite E ayant une longueur différente à 660 acides aminés et une identité de séquence d'au moins 89 % avec SEQ ID N°11, est choisie parmi les séquences SEQ ID N°1 à 10 et 24.

3. Polypeptide selon l'une quelconque des revendications 1 à 2, dans lequel :

- ladite séquence 394-660 est choisie parmi les séquences SEQ ID N°26, et 38 à 49,
- ladite séquence analogue à SEQ ID N°26 est choisie parmi les séquences SEQ ID N°28 à 37 et 50.

4. Polypeptide selon l'une quelconque des revendications 1 à 3, **caractérisé en ce que** :

- les trois acides aminés en positions 627, 630 et 638 de ladite séquence 394-660, et
- les trois acides aminés de ladite séquence analogue, qui correspondent aux trois cystéines en positions 627, 630 et 638 de SEQ ID N°11,

sont différents de la cystéine et de la proline, des acides aminés dont les chaînes latérales sont chargées et des acides aminés dont les chaînes latérales comportent un cycle aromatique benzénique.

5. Polypeptide selon l'une quelconque des revendications 1 à 4, **caractérisé en ce que** :

- les trois acides aminés en positions 627, 630 et 638 de ladite séquence 394-660, et
- les trois acides aminés de ladite séquence analogue, qui correspondent aux trois cystéines en positions 627, 630 et 638 de SEQ ID N°11,

sont choisis parmi l'alanine, la glycine, la thréonine, la valine et la sérine.

6. Polypeptide selon l'une quelconque des revendications 1 à 5, **caractérisé en ce que** :

- les trois acides aminés en positions 627, 630 et 638 de ladite séquence 394-660, et
- les trois acides aminés de ladite séquence analogue, qui correspondent aux trois cystéines en positions 627, 630 et 638 de SEQ ID N°11,

sont de même acides aminés.

7. Polypeptide selon la revendication 6, **caractérisé en ce que** :

- les trois acides aminés en positions 627, 630 et 638 de ladite séquence 394-660, et
- les trois acides aminés de ladite séquence analogue, qui correspondent aux trois cystéines en positions 627, 630 et 638 de SEQ ID N°11,

sont des sérines.

8. Polypeptide selon l'une quelconque des revendications précédentes, **caractérisé en ce qu'**il est choisi parmi :

- un polypeptide provenant d'une protéine p-ORF2 du virus de l'hépatite E ayant une longueur de 660 acides aminés et une identité de séquence d'au moins 89 % avec SEQ ID N°11, ledit polypeptide consistant en la séquence 394-660 de ladite protéine p-ORF2 de 660 acides aminés de long, et dans laquelle les trois acides aminés en positions 627, 630 et 638 sont différentes de la cystéine, et
- un polypeptide provenant d'une protéine p-ORF2 du virus de l'hépatite E ayant une longueur différente à 660 acides aminés et une identité de séquence d'au moins 89 % avec SEQ ID N°11, ledit polypeptide consistant en une séquence analogue à SEQ ID N°26, et dans laquelle les trois acides aminés correspondant aux trois cystéines des positions 627, 630 et 638 de SEQ ID N°11, sont différentes de la cystéine.

9. Polypeptide selon l'une quelconque des revendications précédentes, **caractérisé en ce qu'**il est couplé à une molécule marqueur.

10. Acide nucléique isolé comprenant une séquence de nucléotides codant pour le polypeptide tel que défini dans l'une des revendications 1 à 9 ou une séquence complémentaire à ladite séquence codante.

11. Vecteur d'expression comprenant une séquence d'acide nucléique telle que définie dans la revendication 10.

12. Cellule hôte comprenant une séquence de nucléotides codant pour le polypeptide tel que défini dans l'une des revendications 1 à 9 ou une séquence complémentaire à ladite séquence codante ou un vecteur d'expression tel que défini dans la revendication 11.

13. Procédé de détermination par immunoessai de la présence d'une réponse anticorps dirigée contre la protéine p-ORF-2 du virus de l'hépatite E dans un échantillon biologique issu d'un sujet, susceptible de contenir les anticorps de ladite réponse, lequel comprend les étapes suivantes :

   - mettre en contact ledit échantillon biologique avec un polypeptide tel que défini dans l'une quelconque des revendications 1 à 9,
   - détecter un signal émis par la liaison entre ledit polypeptide et lesdits anticorps, s'ils sont présents, en utilisant un marqueur capable d'émettre un signal détectable,
   - comparer le signal ainsi obtenu avec un signal de référence S préalablement déterminé avec deux populations de témoins, l'une ayant développé lesdits anticorps et l'autre n'ayant pas développé lesdits anticorps,
   - un signal inférieur audit signal de référence S signifiant que l'échantillon ne contient pas lesdits anticorps, et
   - un signal supérieur audit signal de référence S signifiant que l'échantillon contient lesdits anticorps.

14. Procédé de détermination par immunoessai du taux d'anticorps dirigés contre la protéine p-ORF-2 du virus de l'hépatite E dans un échantillon biologique issu d'un sujet, susceptible de contenir lesdits anticorps, lequel comprend les étapes suivantes :

   - mettre en contact ledit échantillon biologique avec un polypeptide tel que défini dans l'une quelconque des revendications 1 à 9,
   - détecter un signal émis par la liaison entre ledit polypeptide et lesdits anticorps, s'ils sont présents, en utilisant un marqueur capable d'émettre un signal détectable,
   - transformer le signal détecté en un taux d'anticorps.

15. Procédé selon l'une des revendications 13 ou 14, **caractérisé en ce que** les anticorps recherchés sont des IgM ou des IgG.

16. Utilisation d'un procédé tel que défini dans l'une quelconque des revendications 13 à 15 pour l'aide au diagnostic *in vitro*, pour le diagnostic *in vitro* d'une infection au virus de l'hépatite E chez un sujet susceptible d'être infecté, pour le suivi thérapeutique d'un sujet infecté par le virus de l'hépatite E ou pour faire des études épidémiologiques de la séroprévalence des anticorps anti-HEV dans une population ou dans un territoire géographique donné.

17. Trousse pour la détermination par immunoessai de la présence de la réponse anticorps ou du taux d'anticorps dirigés contre la protéine p-ORF-2 du virus de l'hépatite E chez un sujet susceptible d'avoir produit ces anticorps, comprenant un polypeptide tel que défini dans l'une quelconque des revendications 1 à 9.

18. Trousse selon la revendication 17, comprenant également au moins un échantillon contrôle positif qui est un échantillon contenant un taux donné d'anticorps dirigés contre la protéine p-ORF-2 du virus de l'hépatite E.

**Patentansprüche**

1. Polypeptid, das dazu befähigt ist, an anti-HEV-Antikörper zu binden, ausgewählt aus:

- einem Polypeptid, das zumindest die Sequenz 394-660 eines Proteins p-ORF2 des Hepatitis-E-Virus mit einer Länge von 660 Aminosäuren umfasst, wobei darin die drei Aminosäuren an den Positionen 627, 630 und 638 sich von Cystein unterscheiden; wobei das Protein p-ORF2 mit einer Länge von 660 Aminosäuren eine Sequenzübereinstimmung von mindestens 89 % mit der SEQ ID Nr. 11 hat, und wobei die Sequenz 394-660 eine Sequenzüberstimmung von mindestens 90 % mit der SEQ ID Nr. 26 hat; und

- einem Polypeptid, das eine zur SEQ ID Nr. 26 analoge Sequenz umfasst, wobei es eine Sequenzübereinstimmung von mindestens 90 % mit der SEQ ID Nr. 26 aufweist und aus einem Protein p-ORF2 des Hepatitis-E-Virus mit einer Länge stammt, welche sich von 660 Aminosäuren unterscheidet, und wobei es eine Sequenzübereinstimmung von mindestens 89 % mit der SEQ ID Nr. 11 aufweist, und wobei die drei Aminosäuren, welche den drei Cysteinen der Positionen 627, 630 und 638 der SEQ ID Nr. 11 entsprechen, sich von Cystein unterscheiden.

2. Polypeptid nach Anspruch 1, wobei:

- das Protein p-ORF2 des Hepatitis-E-Virus, welches eine Länge von 660 Aminosäuren und eine Sequenzübereinstimmung von mindestens 89% mit der SEQ ID Nr. 11 hat, aus den Sequenzen SEQ ID Nr. 11 bis 23 ausgewählt ist, und

- das Protein p-ORF2 des Hepatitis-E-Virus, welches eine sich von 660 Aminosäuren unterscheidende Länge und eine Sequenzübereinstimmung von mindestens 89% mit der SEQ ID Nr. 11 hat, aus den Sequenzen SEQ ID Nr. 1 bis 10 und 24 ausgewählt ist.

3. Polypeptid nach einem beliebigen der Ansprüche 1 bis 2, wobei:

- die Sequenz 394-660 aus den Sequenzen SEQ ID Nr. 26 und 38 bis 49 ausgewählt ist,
- die zur SEQ ID Nr. 26 analoge Sequenz aus den Sequenzen SEQ ID Nr. 28 und 37 bis 50 ausgewählt ist.

4. Polypeptid nach einem beliebigen der Ansprüche 1 bis 3, **dadurch gekennzeichnet, dass**:

- die drei Aminosäuren an den Positionen 627, 630 und 638 der Sequenz 394-660 und
- die drei Aminosäuren der analogen Sequenz, welche den drei Cysteinen an den Positionen 627, 630 und 638 der SEQ ID Nr. 11 entsprechen,

sich von Cystein und von Prolin, von Aminosäuren, deren Seitenketten geladen sind, und von Aminosäuren unterscheiden, deren Seitenketten einen benzolartigen aromatischen Ring aufweisen.

5. Polypeptid nach einem beliebigen der Ansprüche 1 bis 4, **dadurch gekennzeichnet, dass**:

- die drei Aminosäuren an den Positionen 627, 630 und 638 der Sequenz 394-660 und
- die drei Aminosäuren der analogen Sequenz, welche den drei Cysteinen an den Positionen 627, 630 und 638 der SEQ ID Nr. 11 entsprechen,

aus Alanin, Glycin, Threonin, Valin und Serin ausgewählt sind.

6. Polypeptid nach einem beliebigen der Ansprüche 1 bis 5, **dadurch gekennzeichnet, dass**:

- es sich bei den drei Aminosäuren an den Positionen 627, 630 und 638 der Sequenz 394-660 und
- bei den drei Aminosäuren der analogen Sequenz, welche den drei Cysteinen an den Positionen 627, 630 und 638 der SEQ ID Nr. 11 entsprechen,

um gleichartige Aminosäuren handelt.

7. Polypeptid nach Anspruch 6, **dadurch gekennzeichnet, dass**:

- es sich bei den drei Aminosäuren an den Positionen 627, 630 und 638 der Sequenz 394-660 und
- bei den drei Aminosäuren der analogen Sequenz, welche den drei Cysteinen an den Positionen 627, 630 und 638 der SEQ ID Nr. 11 entsprechen,

jeweils um Serin handelt.

8. Polypeptid nach einem beliebigen der vorhergehenden Ansprüche, **dadurch gekennzeichnet, dass** es aus den folgenden ausgewählt ist:

- einem Polypeptid, das aus einem Protein p-ORF2 des Hepatitis-E-Virus mit einer Länge von 660 Aminosäuren und einer Sequenzübereinstimmung von mindestens 89 % mit der SEQ ID Nr. 11 stammt, wobei das Polypeptid aus der Sequenz 394-660 des Proteins p-ORF2 besteht, welches einer Länge von 660 Aminosäuren hat und bei welchem sich die drei Aminosäuren an den Positionen 627, 620 und 638 von Cystein unterscheiden; und
- einem Polypeptid, das aus einem Protein p-ORF2 des Hepatitis-E-Virus mit einer sich von 660 Aminosäuren unterscheidenden Länge und einer Sequenzübereinstimmung von mindestens 89 % mit der SEQ ID Nr. 11 stammt, wobei das Polypeptid aus einer Sequenz besteht, die zur SEQ ID Nr. 26 analog ist und bei welcher sich die drei Aminosäuren, welche den drei Cysteinen der Positionen 627, 630 und 638 der SEQ ID Nr. 11 entsprechen, von Cystein unterscheiden.

9. Polypeptid nach einem beliebigen der vorhergehenden Ansprüche, **dadurch gekennzeichnet, dass** es an ein Markermolekül gekoppelt ist.

10. Isolierte Nukleinsäure, die eine Nukleotidsequenz, welche für das Polypeptid gemäß der Begriffsbestimmung in einem der Ansprüche 1 bis 9 codiert, oder eine Sequenz umfasst, die zu der codierenden Sequenz komplementär ist.

11. Expressionsvektor, der eine Nukleinsäuresequenz gemäß der Begriffsbestimmung in Anspruch 10 umfasst.

12. Wirtszelle, die eine Nukleotidsequenz, welche für das Polypeptid gemäß der Begriffsbestimmung in einem der Ansprüche 1 bis 9 codiert, oder eine Sequenz, welche zu der codierenden Sequenz komplementär ist, oder einen Expressionsvektor gemäß der Begriffsbestimmung in Anspruch 11 umfasst.

13. Verfahren zum Nachweis, mittels eines Immunassays, des Vorliegens einer Antikörperantwort, die gegen das Protein p-ORF-2 des Hepatitis-E-Virus gerichtet ist, in einer biologischen Probe, welche von einem Individuum stammt, wobei sie möglicherweise die Antikörper dieser Antwort enthält, wobei das Verfahren die folgenden Schritte umfasst:

- Inkontaktbringen der biologischen Probe mit einem Polypeptid gemäß der Begriffsbestimmung in einem beliebigen der Ansprüche 1 bis 9,
- Nachweisen eines Signals, das von der Bindung zwischen dem Polypeptid und den Antikörpern ausgesendet wird, sofern solche vorliegen, wobei ein Marker verwendet wird, welcher dazu befähigt ist, ein nachweisbares Signal auszusenden,
- Vergleichen des auf diese Weise erhaltenen Signal mit einem Referenzsignal S, das im Vorfeld mit zwei Vergleichspopulationen bestimmt wurde, wobei eine davon die Antikörper entwickelt hatte und die andere diese Antikörper nicht entwickelt hatte,
- wobei ein Signal, das schwächer als das Referenzsignal S ist, bedeutet, dass die Probe diese Antikörper nicht enthält, und
- wobei ein Signal, das stärker als das Referenzsignal S ist, bedeutet, dass die Probe diese Antikörper enthält.

14. Verfahren zur Bestimmung, mittels eines Immunassays, des Gehalt an Antikörpern, die gegen das Protein p-ORF-2 des Hepatitis-E-Virus gerichtet sind, in einer biologischen Probe, die von einem Individuum stammt, wobei sie möglicherweise diese Antikörper enthält, wobei das Verfahren die folgenden Schritte umfasst:

- Inkontaktbringen der biologischen Probe mit einem Polypeptid gemäß der Begriffsbestimmung in einem beliebigen der Ansprüche 1 bis 9,
- Detektieren eines Signals, das von einer Bindung zwischen dem Polypeptid und den Antikörpern ausgesendet wird, sofern solche vorliegen, wobei ein Marker verwendet wird, welcher dazu befähigt ist, ein detektierbaren Signal auszusenden,
- Umwandeln des detektierten Signals in einen Antikörpergehalt.

15. Verfahren nach einem der Ansprüche 13 oder 14, **dadurch gekennzeichnet, dass** es sich bei den gesuchten Antikörpern um IgM oder IgG handelt.

16. Anwendung eines Verfahren gemäß der Begriffsbestimmung in einem beliebigen der Ansprüche 13 bis 15 zur Unterstützung bei der in-vitro-Diagnose, zur in-vitro-Diagnose einer Infektion mit dem Hepatitis-E-Virus bei einem Individuum, das möglicherweise infiziert ist, zur therapeutischen Nachsorge bei einem Individuum, das mit dem Hepatitis-E-Virus infiziert war, oder zur Durchführung epidemiologischer Studien zur Serumprävalenz von anti-HEV-Antikörpern in einer Population oder in einem bestimmten geografischen Gebiet.

17. Kit zur Bestimmung, mittels eines Immunassays, des Vorliegens der Antikörperantwort oder des Gehalts an Antikörpern, welche gegen das Protein p-ORF-2 des Hepatitis-E-Virus gerichtet sind, bei einem Individuum, welches möglicherweise diese Antikörper produziert hat, wobei es ein Polypeptid gemäß der Begriffsbestimmung in einem beliebigen der Ansprüche 1 bis 9 umfasst.

18. Kit nach Anspruch 17, wobei es weiterhin mindestens eine Positivkontrollprobe umfasst, bei welcher es sich um eine Probe handelt, welche einen bestimmten Gehalt an Antikörpern enthält, die gegen das Protein p-ORF-2 des Hepatitis-E-Virus gerichtet sind.

**Claims**

1. A polypeptide capable of binding to anti-HEV antibodies, chosen from:

   - a polypeptide comprising at least sequence 394-660 of a 660-amino acid p-ORF2 protein of the hepatitis E virus, and in which the three amino acids at positions 627, 630 and 638 are different from cysteine; wherein said 660-amino acid p-ORF2 protein having at least 89% identity with SEQ ID No. 11 and said sequence 394-660 having at least 90% identity with SEQ ID No. 26; and
   - a polypeptide comprising a sequence analog to SEQ ID No. 26 having at least 90% identity with SEQ ID No. 26 and derived from a p-ORF2 protein of the hepatitis E virus of a length different from 660 amino acids and having at least 89% identity with SEQ ID No. 11, and in which the three amino acids corresponding to the three cysteines at positions 627, 630 and 638 of SEQ ID No. 11 are different from cysteine.

2. The polypeptide as claimed in claim 1, wherein:

   - said 660-amino acid p-ORF2 protein having at least 89% identity with SEQ ID No. 11 is of a sequence chosen from sequences SEQ ID No. 11 to 23, and
   - said p-ORF2 protein of the hepatitis E virus of a length different from 660 amino acids and having at least 89% identity with SEQ ID No. 11 is chosen from SEQ ID No. 1 to 10, and 24.

3. The polypeptide as claimed in any one of claims 1 to 2, wherein:

   - said sequence 394-660 is chosen from sequences SEQ ID No. 26, and 38 to 49,
   - said sequence analog to SEQ ID No. 26 is chosen from sequences SEQ ID No. 28 to 37, and 50.

4. The polypeptide as claimed in any one of claims 1 to 3, wherein:

   - said three amino acids at positions 627, 630 and 638, and
   - said three amino acids of the analog sequence and that corresponds to the three cysteines at positions 627, 630 and 638 of SEQ ID No. 11,

   are different from cysteine, proline, amino acids the side chains are charged and amino acids the side chains comprise an aromatic benzene ring.

5. The polypeptide as claimed in any one of claims 1 to 4, wherein:

   - said three amino acids at positions 627, 630 and 638, and
   - said three amino acids of the analog sequence and that corresponds to the three cysteines at positions 627, 630 and 638 of SEQ ID No. 11,

   are chosen from alanine, glycine, threonine, valine and serine.

6. The polypeptide as claimed in any one of claims 1 to 5, wherein:

   - said three amino acids at positions 627, 630 and 638, and

- said three amino acids of the analog sequence and that corresponds to the three cysteines at positions 627, 630 and 638 of SEQ ID No. 11,

are the same amino acids.

7.  The polypeptide as claimed in any one of claims 1 to 6, wherein:

    - said three amino acids at positions 627, 630 and 638, and
    - said three amino acids of the analog sequence and that corresponds to the three cysteines at positions 627, 630 and 638 of SEQ ID No. 11,

    are serines.

8.  The polypeptide as claimed in any one of the preceding claims, wherein it chosen from:

    - a polypeptide consisting in sequence 394-660 of a 660-amino acid p-ORF2 protein of the hepatitis E virus, and in which the three amino acids at positions 627, 630 and 638 are different from cysteine; wherein said 660-amino acid p-ORF2 protein having at least 89% identity with SEQ ID No. 11 and said sequence 394-660 having at least 90% identity with SEQ ID No. 26; and
    - a polypeptide consisting in a sequence analog to SEQ ID No. 26 having at least 90% identity with SEQ ID No. 26 and derived from a p-ORF2 protein of the hepatitis E virus of a length different from 660 amino acids and having at least 89% identity with SEQ ID No. 11, and in which the three amino acids corresponding to the three cysteines at positions 627, 630 and 638 of SEQ ID No. 11 are different from cysteine.

9.  The polypeptide as claimed in any one of the preceding claims, wherein it is labeled.

10. An isolated nucleic acid comprising a nucleotide sequence encoding the polypeptide as defined in one of claims 1 to 9 or a sequence complementary to said coding sequence.

11. An expression vector comprising a nucleic acid sequence as defined in claim 10.

12. A host cell comprising a nucleotide sequence encoding the polypeptide as defined in one of claims 1 to 9 or a sequence complementary to said coding sequence or an expression vector as defined in claim 11.

13. A method for determining, by immunoassay, the presence of an antibody response directed against the p-ORF2 protein of the hepatitis E virus in a biological sample from a subject, which may contain the antibodies of said response, which method comprises the following steps:

    - bringing said biological sample into contact with a polypeptide as defined in any one of claims 1 to 9,
    - detecting a signal emitted by the binding between said polypeptide and said antibodies, if they are present, using a label capable of emitting a detectable signal,
    - comparing the signal thus obtained with a reference signal S predetermined with two populations for controls, one having developed said antibodies and the other not having developed said antibodies,
    - a signal lower than said reference signal S signifying that the sample does not contain said antibodies, and
    - a signal higher than said reference signal S signifying that the sample contains said antibodies.

14. A method for determining, by immunoassay, the titer of antibodies directed against the p-ORF2 protein of the hepatitis E virus in a biological sample from a subject, which may contain said antibodies, which method comprises the following steps:

    - bringing said biological sample into contact with a polypeptide as defined in any one of claims 1 to 9,
    - detecting a signal emitted by the binding between said polypeptide and said antibodies, if they are present, using a label capable of emitting a detectable signal,
    - converting the detected signal into an antibody titer.

15. The method as claimed in claim 13 or 14, wherein the antibodies which are sought are IgM or IgG.

16. The use of a method as defined in any one of claims 13 to 15 for assisting with the *in vitro* diagnosis, for the *in vitro* diagnosis of a hepatitis E virus infection in a subject who may be infected, for therapeutic monitoring of a subject infected with the hepatitis E virus or for carrying out epidemiological studies of the seroprevalence of anti-HEV antibodies in a population or in a given geographic territory.

17. A kit for determining, by immunoassay, the presence of the antibody response or the titer of antibodies directed against the p-ORF2 protein of the hepatitis E virus in a subject who may have produced these antibodies, comprising a polypeptide as defined in any one of claims 1 to 9.

18. The kit as claimed in claim 17, also comprising at least one positive control sample which is a sample containing a given titer of antibodies directed against the p-ORF2 protein of the hepatitis E virus.

EP 3 383 887 B1

```
Q8JJN2        Q8JJN2_HEV       1 MNNMFFCSVHGDATMRSRAFLFLFLVLLPMLPAPPAGQPSGRRRGRRSGGAGGGFWGDRV 60
Q80IR5        Q80IR5_HEV       1 MNNMFFCSVHGDATMRSRALLFLLFVLLPMLPAPPAGQPSGRRRGRRSGGAGGGFWGDRV 60
Q806D7        Q806D7_HEV       1 MNNMFFCSVHGDATMRSRALLFLLFVLLPMLPAPPAGQPSGRRRGRRSGGAGGGFWGDRV 60
Q6BD83        Q6BD83_HEV       1 MNNMFFCSVHGDATMRSRAFLFLFLVLLPMLPAPPAGQPSGRRRGRRSGGAGGGFWGDRV 60
Q6BD78        Q6BD78_HEV       1 MNNMFFCSVHGDATMRSRAFLFLFLVLLPMLPAPPAGQPSGRRRGRRSGGAGGGFWGDRV 60
B6VC89        B6VC89_HEV       1 MNNMFFCSLHGDATMRSRALLFLLLLLPMLPAPPAGQPSGRRRGRRSGGAGSGFWGDRV 60
Q6PMR3        Q6PMR3_HEV       1 MNNMFFCSAHGDATMRSRALLFLLLVFLPMLPAPPAGQPSGRRRGRRSGGAGSGFWGDRV 60
Q9IVZ8        CAPSD_HEVCT      1 MNNMFFCSVHGDATMRSRALLFLLFVLLPMLPAPPAGQPSGRRRGQ--AGCGGGFWGDRV 58
Q8JJM1        Q8JJM1_HEV       1 ---MFFCSVHGDATMRSRALLFLLFVLLPMLPAPPAGQPSGRRRGRRSGGAGGGFWGDRV 57
Q2PYP3        Q2PYP3_HEV       1 ---MFFCSVHGDATMRSRALLFLLLVFLPMLPALPAGQPSGRRRGRRSGSAGGGFWGDRV 57
Q81871        CAPSD_HEVCH      1 --------------MRPRPILLLLLMFLPMLPAPPPGQPSGRRRGRRSGGSGGGFWGDRA 46
P29326        CAPSD_HEVBU      1 --------------MRPRPILLLLLMFLPMLPAPPPGQPSGRRRGRRSGGSGGGFWGDRV 46
Q6J8F7        CAPSD_HEVMG      1 --------------MRPRAVLLLLFVLLPMLPAPPAGQPSGRRRGRRNGGAGGGFWGDRV 46
Q04611        CAPSD_HEVMY      1 --------------MRPRPILLLLLMFLPMLPAPPPGQPSGRRRGRRSGGSGGGFWGDRV 46
Q68985        CAPSD_HEVHY      1 --------------MGPRPILLLFLMFLPMLLAPPPGQPSGRRRGRRSGGSGGGFWGDRV 46
Q9YLQ9        CAPSD_HEVUS      1 --------------MRPRAVLLLLFVLLPMLPAPPAGQPSGRRRGRRSGGAGGGFWGDRV 46
P33426        CAPSD_HEVPA      1 --------------MRPRPILLLLLMFLPMLPAPPPGQPSGRRRGRRSGGSGGGFWGDRV 46
Q9YLR2        Q9YLR2_HEV       1 --------------MRPRAVLLFLMFLPMLPAPPAGQPSGRRRGRRSGGAGGGFWSDRV 46
Q0QC51        Q0QC51_HEV       1 --------------MRPRAVLLFFVLLPMLPAPPAGQPSGRRRGRRSGGTGGGFWGDRV 46
Q69411        Q69411_HEV       1 --------------MRPRPILLLLLMFLPMLPAPPPGQPSGRRRGRRSGGSGGGFWGDRV 46
A0A024D9U6    A0A024D9U6_HEV   1 --------------MRPRAVLLFFVLLPMLPAPPAGQPSGRRRGRRSGGAGGGFWGDRV 46
A0A024D9R2    A0A024D9R2_HEV   1 --------------MRPRAVLLLLFVLLPMLPAPPAGQPSGRRRGRRSGGAGGGFWGDRV 46
Q8V729        Q8V729_HEV       1 --------------MCPRAVLLLLFVLLPMLPAPPAGQPSGRRRGRRSGGAGGGFWGDRV 46
Q03500        CAPSD_HEVME      1 --------------MRPRPLLLLFLLFLPMLPAPPTGQPSGRRRGRRSGGTGGGFWGDRV 46
                                 *    *  .*:*::::****  *  * *********;   .. *.***.**.
```

Figure 1A

EP 3 383 887 B1

```
Q8JJN2       Q8JJN2_HEV         61  DSQPFALPYIHPTNPFASDIPAAAGAGARPRQPARPLGSAWRDQSQRPATSARRRSAPAG 120
Q80IR5       Q80IR5_HEV         61  DSQPFALPYIHPTNPFASDIPTAAGSGARPRQPARPLGSAWRDQSQRPAAPARRRSAPAG 120
Q806D7       Q806D7_HEV         61  DSQPFALPYIHPTNPFASDIPTAAGSGARPRQPARPLGSAWRDQSQRPAASARRRSAPAG 120
Q6BD83       Q6BD83_HEV         61  DSQPFALPYIHPTNPFASDIPAAAGAGARPRQPARPLGSAWRDQSQRPATSARRRSAPAG 120
Q6BD78       Q6BD78_HEV         61  DSQPFALPYIHPTNPFASDIPAAAGAGARPRQPARPLGSAWRDQSQRPATSARRRSAPAG 120
B6VC89       B6VC89_HEV         61  DSQPFALPYIHPTNPFASDIPAAAGAGARPRQPARPLGSAWRDQSQRPAAPARRRSAPAG 120
Q6PMR3       Q6PMR3_HEV         61  DSQPFALPYIHPTNPFASDIPAAAGAGARPRQPARPLGSAWRDQSQRPAASTRRRPAPAG 120
Q9IVZ8       CAPSD_HEVCT        59  DSQPFALPYIHPTNPFASDIPAAAGTGARPRQPIRPLGSAWRDQSQRPAASTRRRPAPAG 118
Q8JJM1       Q8JJM1_HEV         58  DSQPFALPYIHPTNPFASDIPTAAGSGARPRQPVRPLGSAWRDQSQRPAASARRRPAPAG 117
Q2PYP3       Q2PYP3_HEV         58  DSQPFALPYIHPTNPFASDIPTAAGAGARPRQPARPLGSAWRDQSQRPATSTRRRSAPVG 117
Q81871       CAPSD_HEVCH        47  DSQPFAIPYIHPTNPFAPDVTAAAGAGPRVRQPARPLGSAWRDQAQRPAAASRRRPTTAG 106
P29326       CAPSD_HEVBU        47  DSQPFAIPYIHPTNPFAPDVTAAAGAGPRVRQPARPLGSAWRDQAQRPAVASRRRPTTAG 106
Q6J8F7       CAPSD_HEVMG        47  DSQPFALPYIHPTNPFAADVVSQPGAGARPRQPPRPLGSAWRDQSQRPSTAPRRRSAPAG 106
Q04611       CAPSD_HEVMY        47  DSQPFAIPYIHPTNPFAPDVTAAAGAGPRVRQPARPLGSAWRDQAQRPAVASRRRPTTAG 106
Q68985       CAPSD_HEVHY        47  DSQPFAIPYIHPTNPFAPNVTAAAGAGPRVRQPVRPLGSAWRDQAQRPAAASRRRPTTAG 106
Q9YLQ9       CAPSD_HEVUS        47  DSQPFALPYIHPTNPFAADVVSQPGAGTRPRQPPRPLGSAWRDQSQRPSAAPRRRSAPAG 106
P33426       CAPSD_HEVPA        47  DSQPFAIPYIHPTNPFAPDVTAAAGAGPRVRQPARPLGSAWRDQAQRPAAASRRRPTTAG 106
Q9YLR2       Q9YLR2_HEV         47  DSQPFALPYIHPTNPFAADVVSQPGAGTRPRQPPRPLGSAWRDQSKRPSVAPRRRSTPAG 106
Q0QC51       Q0QC51_HEV         47  DSQPFALPYIHPTNPFASDIPTATGAGARPRQPARPLGSAWRDQSQRPAAPARRRSAPAG 106
Q69411       Q69411_HEV         47  DSQPFAIPHIHPTNPFAPDVTAAAGAGPRVRQPARPLGSAWRDQAQRPAATSRRRPTTAG 106
A0A024D9U6   A0A024D9U6_HEV     47  DSQPFALPYIHPTNPFAADVASQSGAGARPRQPPRPLGSAWRDQSQRPPAVPRRRSAPAG 106
A0A024D9R2   A0A024D9R2_HEV     47  DSQPFALPYIHPTNPFAADVVSQPGAGTRPRQPPRPLGSAWRDQSQRPSAAPRRRPAPAG 106
Q8V729       Q8V729_HEV         47  DSQPFALPYIHPTNPFAADVFSQSGAGARPRQPPRPLGSAWRDQSQRPSAAPRRRSTPAG 106
Q03500       CAPSD_HEVME        47  DSQPFAIPYIHPTNPFAPDVAAASGSGPRLRQPARPLGSTWRDQAQRPSAASRRRPATAG 106
                                    ******:*:*********  ::  :    *:*  *  ***  ****:****::**  .   ***  :  .*
```

Figure 1B

```
Q8JJN2        Q8JJN2_HEV       121 ASPLTAVAPAPDTAPVPDVDSRGAILRRQYNLSTSPLTSTIATGTNLVLYAAPLSPLLPL 180
Q80IR5        Q80IR5_HEV       121 ASPLTAVAPAPDTAPVPDVDSRGAILRRQYNLSTSPLTSTIATGTNLVLYAAPLSPLLPL 180
Q806D7        Q806D7_HEV       121 ASPLTAVAPAPDTAPVPDVDSRGAILRRQYNLSTSPLTSTIATGTNLVLYAAPLSPLLPL 180
Q6BD83        Q6BD83_HEV       121 ASPLTAVAPAPDTAPVPDVDSRGAILRRQYNLSTSPLTSTIATGTNLVLYAAPLSPLLPL 180
Q6BD78        Q6BD78_HEV       121 ASPLTAVAPAPDTAPVPDVDSRGAILRRQYNLSTSPLTSTIATGTNLVLYAAPLSPLLPL 180
B6VC89        B6VC89_HEV       121 ASPLTAVAPAPDTAPVPDVDSRGAILRRQYNLSTSPLTSTIATGTNLVLYAAPLSPLLPL 180
Q6PMR3        Q6PMR3_HEV       121 ASPLTAVAPAPDTAPVPDVDSRGAILRRQYNLSTSPLTSTIATGTNLVLYAAPLSPLLPL 180
Q9IVZ8        CAPSD_HEVCT      119 ASPLTAVAPAPDTAPVPDADSRGAILRRQYNLSTSPLTSTIATGTNFVLYAAPLSPLLPL 178
Q8JJM1        Q8JJM1_HEV       118 ASPLTAVAPAPDTAPVPDVDSRGAILRRQYNLSTSPLTSTIATGTNLVLYAAPLSPLLPL 177
Q2PYP3        Q2PYP3_HEV       118 ASPLTAVAPAPDTAPVPDVDSRGAILRRQYNLSTSPLTSTIATGTNLVLYAAPLSPLLPL 177
Q81871        CAPSD_HEVCH      107 AAPLTAVAPAHDTPPVPDVDSRGAILRRQYNLSTSPLTSSVATGTNLVLYAAPLSPLLPL 166
P29326        CAPSD_HEVBU      107 AAPLTAVAPAHDTPPVPDVDSRGAILRRQYNLSTSPLTSSVATGTNLVLYAAPLSPLLPL 166
Q6J8F7        CAPSD_HEVMG      107 AAPLTAVSPAPDTAPVPDVDSRGAILRRQYNLSTSPLTSSVAGTNLVLYAAPLNPLLPL 166
Q04611        CAPSD_HEVMY      107 AAPLTAVAPAHDTPPVPDVDSRAAILRRQYNLSTSPLTSSVATGTNLVLYAAPLSPLLPL 166
Q68985        CAPSD_HEVHY      107 AAPLTAVAPAHDTPPVPDVDSRGAILRRQYNLSTSPLTSSVATGTNLVLYAAPLSPLLPL 166
Q9YLQ9        CAPSD_HEVUS      107 AAPLTAVSPAPDTAPVPDVDSRGAILRRQYNLSTSPLTSSVASGTNLVLYAAPLNPLLPL 166
P33426        CAPSD_HEVPA      107 AAPLTAVAPAHDTPPVPDVDSRGAILRRQYNLSTSPLTSSVATGTNLVLYAAPLSPLLPL 166
Q9YLR2        Q9YLR2_HEV       107 AAPLTAISPAPDTAPVPDVDSRGAILRRQYNLSTSPLTSSVASGTNLVLYAAPLNPLLPL 166
Q0QC51        Q0QC51_HEV       107 ASPLTAVAPAPDTAPVPDVDSRGAILRRQYNLSTSPLTSTIATGTNLVLYAAPLSPLLPL 166
Q69411        Q69411_HEV       107 AAPLTAVAPAHDTPPVPDVDSRGAILRRQYNLSTSPLTSPVATGTNLVLYAAPLSPLLPL 166
A0A024D9U6    A0A024D9U6_HEV   107 AAPLTAISPAPDTAPVPDVDSRGAILRRQYNLSTSPLTSSVASGTNLVLYAAPLNPLLPL 166
A0A024D9R2    A0A024D9R2_HEV   107 ATPLTAVSPAPDAAPVPDVDSRGAILRRQYNLSTSPLTSSVASGTNLVLYAAPLNPLLPL 166
Q8V729        Q8V729_HEV       107 AAPLTATSPAPDTAPVPDVDSRGAILRRQYNLSTSPLTSSVASGTNLVLYAAPLNPLLPL 166
Q03500        CAPSD_HEVME      107 AAALTAVAPAHDTSPVPDVDSRGAILRRQYNLSTSPLTSSVASGTNLVLYAAPLNPPLPL 166
              *:  *** :** *: ****.***.****************** :*:***:*******.* ***
```

## Figure 1C

```
Q8JJN2         Q8JJN2_HEV       181 QDGTNTHIMATEASNYAQYRVVRATIRYRPLVPNAVGGYAISISFWPQTTTTPTSVDMNS 240
Q80IR5         Q80IR5_HEV       181 QDGTNTHIMATEASNYAQYRVVRATIRYRPLVPNAVGGYAISISFWPQTTTTPTSVDMNS 240
Q806D7         Q806D7_HEV       181 QDGTNTHIMATEASNYAQYRVVRATIRYRPLVPNAVGGYAISISFWPQTTTTPTSVDMNS 240
Q6BD83         Q6BD83_HEV       181 QDGTNTHIMATEASNYAQYRVVRATIRYRPLVPNAVGGYAISISFWPQTTTTPTSVDMNS 240
Q6BD78         Q6BD78_HEV       181 QDGTNTHIMATEASNYAQYRVVRATIRYRPLVPNAVGGYAISISFWPQTTTTPTSVDMNS 240
B6VC89         B6VC89_HEV       181 QDGTNTHIMATEASNYAQYRVVRATIRYRPLVPNAVGGYAISISFWPQTTTTPTSVDMNS 240
Q6PMR3         Q6PMR3_HEV       181 QDGTNTHIMATEASNYAQYRVVRATIRYRPLVPNAVGGYAISISFWPQTTTTPTSVDMNS 240
Q9IVZ8         CAPSD_HEVCT      179 QDGTNTHIMATEASNYAQYRVVRATIRYRPLVPNAVGGYAISISFWPQTTTTPTSVDMNS 238
Q8JJM1         Q8JJM1_HEV       178 QDGTNTHIMATEASNYAQYRVVRATIRYRPLVPNAVGGYAISISFWPQTTTTPTSVDMNS 237
Q2PYP3         Q2PYP3_HEV       178 QDGTNTHIMATEASNYAQYRVVRATIRYRPLVPNAVGGYAISISFWPQTTTTPTSVDMNS 237
Q8I871         CAPSD_HEVCH      167 QDGTNTHIMATEASNYAQYRVVRATIRYRPLVPNAVGGYAISISFWPQTTTTPTSVDMNS 226
P29326         CAPSD_HEVBU      167 QDGTNTHIMATEASNYAQYRVARATIRYRPLVPNAVGGYAISISFWPQTTTTPTSVDMNS 226
Q6J8F7         CAPSD_HEVMG      167 QDGTNTHIMATEASNYAQYRVVRATIRYRPLVPNAVGGYAISISFWPQTTTTPTSVDMNS 226
Q04611         CAPSD_HEVMY      167 QDGTNTHIMATEASNYAQYRVVRATIRYRPLVPNAVGGYAISISFWPQTTTTPTSVDMNS 226
Q68985         CAPSD_HEVHY      167 QDGTNTHIMATEASNYAQYRVARATIRYRPLVPNAVGGYAISISFWPQTTPTPTSVDMNS 226
Q9YLQ9         CAPSD_HEVUS      167 QDGTNTHIMATEASNYAQYRVVRATIRYRPLVPNAVGGYAISISFWPQTTTTPTSVDMNS 226
P33426         CAPSD_HEVPA      167 QDGTNTHIMATEASNYAQYRVARATIRYRPLVPNAVGGYAISISFWPQTTTTPTSVDMNS 226
Q9YLR2         Q9YLR2_HEV       167 QDGTNTHIMATEASNYAQYRVVRATIRYRPLVPNAVGGYAISISFWPQTTTTPTSVDMNS 226
Q0QC51         Q0QC51_HEV       167 QDGTNTHIIATEASNYAQYRVVRATIRYRPLVPNAVGGYAISISFWPQTTTTPTSVDMNS 226
Q69411         Q69411_HEV       167 QDGTNTHIMATEASNYAQYRVARATIRYRPLVPNAVGGYAISISFWPQTTTTPTSVDMNS 226
A0A024D9U6     A0A024D9U6_HEV   167 QDGTNTHIMATEASNYAQYRVVRATIRYRPLVPNAVGGYAISISFWPQTTTTPTSVDMNS 226
A0A024D9R2     A0A024D9R2_HEV   167 QDGTNTHIMATEASNYAQYRVVRATIRYRPLVPNAVGGYAVSISFWPQTTTTPTSVDMNS 226
Q8V729         Q8V729_HEV       167 QDGTNTHIMATEASNYAQYRVVRATIRYRPLVPNAVGGYAISISFWPQTTTTPTSVDMNS 226
Q03500         CAPSD_HEVME      167 QDGTNTHIMATEASNYAQYRVARATIRYRPLVPNAVGGYAISISFWPQTTTTPTSVDMNS 226
                                    *******:***********.******************:********* *********
```

## Figure 1D

```
Q8JJN2        Q8JJN2_HEV         241 ITSTDVRILVQPGIASELVIPSERLHYRNQGWRSVETSGVAEEEATSGLVMLCIHGSPVN 300
Q80IR5        Q80IR5_HEV         241 ITSTDVRILVQPGIASELVIPSERLHYRNQGWRSVETSGVAEEEATSGLVMLCIHGSPVN 300
Q806D7        Q806D7_HEV         241 ITSTDVRILVQPGIASELVIPSERLHYRNQGWRSVETSGVAEEEATSGLVMLCIHGSPVN 300
Q6BD83        Q6BD83_HEV         241 ITSTDVRILVQPGIASELVIPSERLHYRNQGWRSVETSGVAEEEATSGLVMLCIHGSPVN 300
Q6BD78        Q6BD78_HEV         241 ITSTDVRILVQPGVASELVIPSERLHYRNQGWRSVETSGVAEEEATSGLVMLCIHGSPVN 300
B6VC89        B6VC89_HEV         241 ITSTDVRILVQPGIASELVIPSERLHYRNQGWRSVETSGVAEEEATSGLVMLCIHGSPVN 300
Q6PMR3        Q6PMR3_HEV         241 ITSTDVRILVQPGIASELVIPSERLHYRNQGWRSVETSGVAEEEATSGLVMLCIHGSPVN 300
Q9IVZ8        CAPSD_HEVCT        239 ITSTDVRILVQPGIASELVTPSERLHYRNQGWRSVETSGVAEEEATSGLVMLCIHGSPVN 298
Q8JJM1        Q8JJM1_HEV         238 ITSTDVRILVQPGIASELVIPSERLHYRNQGWRSVETSGVAEEEATSGLVMLCIHGSPVN 297
Q2PYP3        Q2PYP3_HEV         238 ITSTDVRILVQSGIASELVIPSERLHYRNQGWRSVETSGVAEEEATSGLVMLCIHGSPVN 297
Q81871        CAPSD_HEVCH        227 ITSTDVRILVQPGIASEHVIPSERLHYRNQGWRSVETSGVAEEEATSGLVMLCIHGSLVN 286
P29326        CAPSD_HEVBU        227 ITSTDVRILVQPGIASELVIPSERLHYRNQGWRSVETSGVAEEEATSGLVMLCIHGSLVN 286
Q6J8F7        CAPSD_HEVMG        227 ITSTDVRILVQPGIASELVIPSERLHYRNQGWRSVETTGVAEEEATSGLVMLCIHGSPVN 286
Q04611        CAPSD_HEVMY        227 ITSTDVRILVQPGIASELVIPSERLHYRNQGWRSVETSGVAEEEATSGLVMLCIHGSPVN 286
Q68985        CAPSD_HEVHY        227 ITSTDVRILVQPGIASELVIPSERLHYRNQGWRSVETSGVAEEEATSGLVMLCIHGSPVN 286
Q9YLQ9        CAPSD_HEVUS        227 ITSTDVRILVQPGIASELVIPSERLHYRNQGWRSVETTGVAEEEATSGLVMLCIHGSPVN 286
P33426        CAPSD_HEVPA        227 ITSTDVRILVQPGIASELVIPSERLHYRNQGWRSVETSGVAEEEATSGLVMLCIHGSPVN 286
Q9YLR2        Q9YLR2_HEV         227 ITSTDVRILVQPGIASELVIPSERLHYRNQGWRSVETTGVAEEEATSGLVMLCIHGSPVN 286
Q0QC51        Q0QC51_HEV         227 ITSTDVRILVQPGIASELVIPSERLHYRNQGWRSVETSGVAEEEATSGLVMLCIHGSPVN 286
Q69411        Q69411_HEV         227 ITSTDVRILVQPGIASELVIPSERLHYRNQGWRSVETSGVAEEEATSGLVMLCIHGLPVN 286
A0A024D9U6    A0A024D9U6_HEV     227 ITSTDVRILVQPGIASELVIPSERLHYRNQGWRSVETTGVAEEEATSGLVMLCIHGSPVN 286
A0A024D9R2    A0A024D9R2_HEV     227 ITSTDVRILVQPGVASELVIPSERLHYRNQGWRSVETTGVAEEEATSGLVMLCIHGSPVN 286
Q8V729        Q8V729_HEV         227 ITSTDVRILVQPGIASELVIPSERLHYRNQGWRSVETTGVAEEEATSGLVMLCIHGSPVN 286
Q03500        CAPSD_HEVME        227 ITSTDVRILVQPGIASELVIPSERLHYRNQGWRSVETSGVAEEEATSGLVMLCIHGSPVN 286
                                     ********** *:*** *  *******************:***************  **
```

Figure 1E

```
Q8JN2        Q8JN2_HEV        301  SYTNTPYTGALGLLDFALELEFRNLTPGNTNTRVSRYSSSARHKLRRGPDGTAELTTTAA  360
Q8OIR5       Q8OIR5_HEV       301  SYTNTPYTGALGLLDFALELEFRNLTPGNTNTRVSRYSSSARHKLRRGPDGTAELTTTAA  360
Q8O6D7       Q8O6D7_HEV       301  SYTNTPYTGALGLLDFALELEFRNLTPGNTNTRVSRYSSSARHKLRRGPDGTAELTTTAA  360
Q6BD83       Q6BD83_HEV       301  SYTNTPYTGALGLLDFALELEFRNLTPGNTNTRVSRYSSSARHKLRRGPDGTAELTTTAA  360
Q6BD78       Q6BD78_HEV       301  SYTNTPYTGALGLLDFALELEFRNLTPGNTNTRVSRYSSSARHKLRRGPDGTAELTTTAA  360
B6VC89       B6VC89_HEV       301  SYTNTPYTGALGLLDFALELEFRNLTPGNTNTRVSRYSSSARHKLRRGPDGTAELTTTAA  360
Q6PMR3       Q6PMR3_HEV       301  SYTNTPYTGALGLLDFALELEFRNLTPGNTNTRVSRYSSSARHKLRRGPDGTVELTTTAA  360
Q9IVZ8       CAPSD_HEVCT      299  SYTNTPYTGALGLLDFALELEFRNLTPGNTNTRVSRYSSSARHKLRRGPDGTAELTTTAA  358
Q8JM1        Q8JM1_HEV        298  SYTNTPYTGALGLLDFALELEFRNLTPGNTNTRVSRYSSSARHKLRRGPDGTAELTTTAA  357
Q2PYP3       Q2PYP3_HEV       298  SYTNTPYTGALGLLDFALELEFRNLTPGNTNMRVSRHSSSARHKLRRGPDGTAELTTTAA  357
Q81871       CAPSD_HEVCH      287  SYTNTPYTGALGLLDFALELEFRNLTPGNTNTRVSRYSSTARHRLRRGADGTAELTTTAA  346
P29326       CAPSD_HEVBU      287  SYTNTPYTGALGLLDFALELEFRNLTPGNTNTRVSRYSSTARHRLRRGADGTAELTTTAA  346
Q6J8F7       CAPSD_HEVMG      287  SYTNTPYTGALGLLDFALELEFRNLTPGNTNTRVSRYTSTARHRLRRGADGTAELTTTAA  346
Q04611       CAPSD_HEVMY      287  SYTNTPYTGALGLLDFALELEFRNLTPGNTNTRVSRYSSTARHRLRRGADGTAELTTTAA  346
Q68985       CAPSD_HEVHY      287  SYTNTPYTGALGLLDFALELEFRNLTPGNTNTRVSRYSSTARHRLRRGADGTAELTTTAA  346
Q9YLQ9       CAPSD_HEVUS      287  SYTNTPYTGALGLLDFALELEFRNLTPGNTNTRVSRYTSTARHRLRRGADGTAELTTTAA  346
P33426       CAPSD_HEVPA      287  SYTNTPYTGALGLLDFALELEFRNLTPGNTNTRVSRYSSTARHRLRRGADGTAELTTTAA  346
Q9YLR2       Q9YLR2_HEV       287  SYTNTPYTGALGLLDFALELEFRNLTPGNTNTRVSRYSSTARHRLRRGADGTAELTTTAA  346
Q0QC51       Q0QC51_HEV       287  SYTNTPYTGALGLLDFALELEFRNLTPGNTNTRVSRYSSSARHKLCRGPDGTAELTTTAA  346
Q69411       Q69411_HEV       287  SYTNTPYTGALGLLDFALELEFRNLTPGNTNTRVSRYSSSARHRLRRGADGTAELTTTAA  346
A0A024D9U6   A0A024D9U6_HEV   287  SYTNTPYTGALGLLDFALELEFRNLTPGNTNTRVSRYSSTARHRLRRGADGTAELTTTAA  346
A0A024D9R2   A0A024D9R2_HEV   287  SYTNTPYTGALGLLDFALELEFRNLTPGNTNTRVSRYTSTARHRLRRGADGTAELTTTAA  346
Q8V729       Q8V729_HEV       287  SYTNTPYTGALGLLDFALELEFRNLTPGNTNTRVSRYTSTARHRLRRGADGTAELTTTAA  346
Q03500       CAPSD_HEVME      287  SYTNTPYTGALGLLDFALELEFRNLTTCNTNTRVSRYSSTARHS-ARGADGTAELTTTAA  345
                                   ************************** : :.:.***  **  ***.  .*.********
```

Figure 1F

```
Q8JJN2        Q8JJN2_HEV       361  TRFMKDLHFTGTNGVGEVGRGIALTLFNLADTLLGGLPTELISSAGGQLFYSRPVVSANG  420
Q80IR5        Q80IR5_HEV       361  TRFMKDLHFTGTNGVGEVGRGIALTLFNLADTLLGGLPTELISSAGGQLFYSRPVVSANG  420
Q806D7        Q806D7_HEV       361  TRFMKDLHFTGTNGVGEVGRGIALTLFNLADTLLGGLPTELISSAGGQLFYSRPVVSANG  420
Q6BD83        Q6BD83_HEV       361  TRFMKDLHFTGTNGVGEVGRGIALTLFNLADTLLGGLPTELISSAGGQLFYSRPVVSANG  420
Q6BD78        Q6BD78_HEV       361  TRFMKDLHFTGTNGVGEVGRGIALTLFNLADTLLGGLPTELISSAGGQLFYSRPVVSANG  420
B6VC89        B6VC89_HEV       361  TRFMKDLHFTGTNGVGEVGRGIALTLFNLADTLLGGLPTELISSAGGQLFYSRPVVSANG  420
Q6PMR3        Q6PMR3_HEV       361  TRFMKDLHFTGTNGVGEVGRGIALTLFNLADTLLGGLPTELISSAGGQLFYSRPVVSANG  420
Q9IVZ8        CAPSD_HEVCT      359  TRFMKDLHFTGTNGVGEVGRGIALTLFNLADTLLGGLPTELISSAGGQLFYSRPVVSANG  418
Q8JJM1        Q8JJM1_HEV       358  TRFMKDLHFTGTNGVGEVGRGIALTLFNLADTLLGGLPTELISSAGGQLFYSRPVVSANG  417
Q2PYP3        Q2PYP3_HEV       358  TRFMKDLHFTGTNGVGEVGRGIALTLFNLADTLLGGLPTELISSAGGQLFYSRPVVSANG  417
Q81871        CAPSD_HEVCH      347  TRFMKDLYFTSTNGVGEIGRGIALTLFNLADTLLGGLPTELISSAGGQLFYSRPVVSANG  406
P29326        CAPSD_HEVBU      347  TRFMKDLYFTSTNGVGEIGRGIALTLFNLADTLLGGLPTELISSAGGQLFYSRPVVSANG  406
Q6J8F7        CAPSD_HEVMG      347  TRFMKDLHFTGTNGVGEVGRGIALTLFNLADTLLGGLPTELISSAGGQLFYSRPVVSANG  406
Q04611        CAPSD_HEVMY      347  TRFMKDLYFTSTNGVGEIGRGIALTLFNLADTLLGGLPTELISSAGGQLFYSRPVVSAHG  406
Q68985        CAPSD_HEVHY      347  TRFMKDLYFTSTNGVGEIGRGIALTLFNLADTLLGGLPTELISSAGGQLFYSRPVVSANG  406
Q9YLQ9        CAPSD_HEVUS      347  TRFMKDLHFAGTNGVGEVGRGIALTLFNLADTLLGGLPTELISSAGGQLFYSRPVVSANG  406
P33426        CAPSD_HEVPA      347  TRFMKDLYFTSTNGVGEIGRGIALTLFNLADTLLGGLPTELISSAGGQLFYSRPVVSANG  406
Q9YLR2        Q9YLR2_HEV       347  TRFMKDLHFTGTNGVGEVGRGIALTLFNLADTLLGGLPTELISSAGGQLFYSRPVVSANG  406
Q0QC51        Q0QC51_HEV       347  TRFMKDLHFTGTNGVGEVGRGIALTLLNLADTLLGGLPTELISSAGGQLFYSRPVVSANG  406
Q69411        Q69411_HEV       347  TRFMKDLYFTSTNGVGEIGRGIALTLFNLADTLLGGLPTELISSAGGQLFYSRPVVSANG  406
A0A024D9U6    A0A024D9U6_HEV   347  TRFMKDLHFTGTNGVGEVGRGIALTLFNLADTLLGGLPTELISSAGGQLFYSRPVASANG  406
A0A024D9R2    A0A024D9R2_HEV   347  TRFMKDLHFTGTNGVGEVGRGIALTLFNLADTLLGGLPTELISSAGGQLFYSRPVVSANG  406
Q8V729        Q8V729_HEV       347  TRFMKDLHFTGTNGVGEVGRGIALTLFNLADTLLGGLPTELISSAGGQLFYSRPVVSANG  406
Q03500        CAPSD_HEVME      346  TRFMKDLHFTGLNGVGEVGRGIALTLLNLADTLLGGLPTELISSAGGQLFYSRPVVSANG  405
                                    *******:*:. ****:*******:****************************.**.*
```

**Figure 1G**

```
Q8JJN2      Q8JJN2_HEV       421 EPTVKLYTSVENAQQDKGIAIPHDIDLGESRVVIQDYDNQHEQDRPTPSPAPSRPFSVLR 480
Q8OIR5      Q8OIR5_HEV       421 EPTVKLYTSVENAQQDKGIAIPHDIDLGESRVVIQDYDNQHEQDRPTPSPAPSRPFSVLR 480
Q806D7      Q806D7_HEV       421 EPTVKLYTSVENAQQDKGIAIPHDIDLGESRVVIQDYDNQHEQDRPTPSPAPSRPFSVLR 480
Q6BD83      Q6BD83_HEV       421 EPTVKLYTSVENAQQDKGIAIPHDIDLGESRVVIQDYDNQHEQDRPTPSPAPSRPFSVLR 480
Q6BD78      Q6BD78_HEV       421 EPTVKLYTSVENAQQDKGIAIPHDIDLGESRVVIQDYDNQHEQDRPTPSPAPSRPFSVLR 480
B6VC89      B6VC89_HEV       421 EPTVKLYTSVENAQQDKGIAIPHDIDLGESRVVIQDYDNQHEQDRPTPSPAPSRPFSVLR 480
Q6PMR3      Q6PMR3_HEV       421 EPTVKLYTSVENAQQDKGIAIPHDIDLGESRVVIQDYDNQHEQDRPTPSPAPSRPFSVLR 480
Q9IVZ8      CAPSD_HEVCT      419 ELTVKLYTSVENAQQDKGVAIPHDIDLGESRVVIQDYDNQHEQDRPTPSPAPSRPFSVLR 478
Q8JJM1      Q8JJM1_HEV       418 EPTVKLYTSVENAQQDKGIAIPHDIDLGESRVVIQDYDNQHEQDRPTPSPAPSRPFSVLR 477
Q2PYP3      Q2PYP3_HEV       418 EPTVKLYTSVENAQQDKGIAIPHDIDLGESRVGIQDYDNQHEQDRPYPSPAPSRPFSVLR 477
Q81871      CAPSD_HEVCH      407 EPTVKLYTSVENAQQDKGIAIPHDIDLGESRVVIQDYDNQHEQDRPTPSPAPSRPFSVLR 466
P29326      CAPSD_HEVBU      407 EPTVKLYTSVENAQQDKGIAIPHDIDLGESRVVIQDYDNQHEQDRPTPSPAPSRPFSVLR 466
Q6J8F7      CAPSD_HEVMG      407 EPTVKLYTSVENAQQDKGITIPHDIDLGDSRVVIQDYDNQHEQDRPTPSPAPSRPFSVLR 466
Q04611      CAPSD_HEVMY      407 EPTVKLYTSVENAQQDKGIAIPHDIDLGESRVVIQDYDNQHEQDRPTPSPAPSRPFSVLR 466
Q68985      CAPSD_HEVHY      407 EPTVKLYTSVENAQQDKGIAIPNDIDLGESRVVIQDYDNQHEQDRPTPSPAPSRPFSVLR 466
Q9YLQ9      CAPSD_HEVUS      407 EPTVKLYTSVENAQQDKGITIPHDIDLGDSRVVIQDYDNQHEQDRPTPSPAPSRPFSVLR 466
P33426      CAPSD_HEVPA      407 EPTVKLYTSVENAQQDKGIAIPHDIDLGESRVVIQDYDNQHEQDRPTPSPAPSRPFSVLR 466
Q9YLR2      Q9YLR2_HEV       407 EPTVKLYTSVENAQQDKGITIPHDIDLGDSRVVIQDYDNQHEQDRPTPSPAPSRPFSVLR 466
Q0QC51      Q0QC51_HEV       407 EPTVKLYTSVENAQQDKGIAIPHDIDLGESRVVIQDYDNQHEQDRPTPSPAPSRPFSVLR 466
Q69411      Q69411_HEV       407 EPTVKLYTSVENAQQDKGIAIPHDIDLGESRVVIQDYDNQHEQDRPTPSPAPSRPFSVLR 466
A0A024D9U6  A0A024D9U6_HEV   407 EPTVKLYTSVENAQQDKGITIPHDIDLGDSRVVIQDYDNQHEQDRPTPSPAPSRPFSVLR 466
A0A024D9R2  A0A024D9R2_HEV   407 EPTVKLYTSVENAQQDKGITIPHDIDLGDSRVVIQDYDNQHEQDRPTPSPAPSRPFSVLR 466
Q8V729      Q8V729_HEV       407 EPTVKLYTSVENAQQDKGITIPHDIDLGDSRVVIQDYDNQHEQDRPTPSPAPSRPFSVLR 466
Q03500      CAPSD_HEVME      406 EPTVKLYTSVENAQQDKGVAIPHDIDLGDSRVVIQDYDNQHEQDRPTPSPAPSRPFSVLR 465
                             *  ***************::**.*****:*** ***************************
```

## Figure 1H

```
Q8JJN2      Q8JJN2_HEV       481 ANDVLWLSLTAAEYDQTTYGSSTNPMYVSDTVTFVNVATGAQGVSRSLDWSKVTLDGRPL 540
Q80IR5      Q80IR5_HEV       481 ANDVLWLSLTAAEYDQTTYGSSTNPMYVSDTVTFVNVATGAQGVSRSLDWSKVTLDGRPL 540
Q806D7      Q806D7_HEV       481 ANDVLWLSLTAAEYDQTTYGSSTNPMYVSDTVTFVNVATGAQGVSRSLDWSKVTLDGRPL 540
Q6BD83      Q6BD83_HEV       481 ANDVLWLSLTAAEYDQTTYGSSTNPMYVSDTVTFVNVATGAQGVSRSLDWSKVTLDGRPL 540
Q6BD78      Q6BD78_HEV       481 ANDVLWLSLTAAEYDQTTYGSSTNPMYVSDTVTFVNVATGAQGVSRSLDWSKVTLDGRPL 540
B6VC89      B6VC89_HEV       481 ANDVLWLSLTAAEYDQTTYGSSTNPMYVSDTVTFVNVATGAQGVSRSLDWSKVTLDGRPL 540
Q6PMR3      Q6PMR3_HEV       481 ANDVLWLSLTAAEYDQTTYGSSTNPMYVSDTVTFVNVATGAQGVSRSLDWSKVTLDGRPL 540
Q9IVZ8      CAPSD_HEVCT      479 ANDVLWLSLTAAEYDQTTYGSSTNPMYVSDTVTFVNVATGAQGVSRSLDWSKVTLDGRPL 538
Q8JJM1      Q8JJM1_HEV       478 ANDVLWLSLTAAEYDQTTYGSSTNPMYVSDTVTFVNVATGAQGVSRSLDWSKVTLDGRPL 537
Q2PYP3      Q2PYP3_HEV       478 ANDVLWLSLTAAEYDQTTYGSSTNPMYVSDTVTFVNVATGAQGVSRSLDWSKVTLDGRSL 537
Q8I871      CAPSD_HEVCH      467 ANDVLWLSLTAAEYDQSTYGSSTGPVYVSDSVTLVNVATGAQAVARSLDWTKVTLDGRPL 526
P29326      CAPSD_HEVBU      467 ANDVLWLSLTAAEYDQSTYGSSTGPVYVSDSVTLVNVATGAQAVARSLDWTKVTLDGRPL 526
Q6J8F7      CAPSD_HEVMG      467 ANDVLWLSLTAAEYDQTTYGSSTNPMYVSDTVTLVNVATGAQAVARSLDWSKVTLDGRPL 526
Q04611      CAPSD_HEVMY      467 ANDVLWLSLTAAEYDQSTYGSSTAPVYVSDSVTLVNVATGAQAVARSLDWTKVTLDGRPL 526
Q68985      CAPSD_HEVHY      467 ANDVLWLSLTAAEYDQSTYGSSTGPVYVSDSVTLVNVATGAQAVARSLDWTKVTLDGRPL 526
Q9YLQ9      CAPSD_HEVUS      467 ANDVLWLSLTAAEYDQTTYGSSTNPMYVSDTVTLVNVATGAQAVARSLDWSKVTLDGRPL 526
P33426      CAPSD_HEVPA      467 ANDVLWLSLTAAEYDQSTYGSSTGPVYVSDSVTLVNVATGAQAVARSLDWTKVTLDGRPL 526
Q9YLR2      Q9YLR2_HEV       467 ANDVLWLSLTAAEYXQTTYGSSTNPMYVSDTVTLVNVATGAQAVARSLDWSKVTLDGRPL 526
Q0QC51      Q0QC51_HEV       467 ANDVLWLSLTAAEYDQTTYGSSTNPMYVSDTVTFVNVATGTQGVSRSLDWSKVTLDGRPL 526
Q69411      Q69411_HEV       467 ANDVLWLSLTAAEYDQSTYGSSTGPVYVSDSVTLVNVATGAQAVARSLDWTKVTLDGRPL 526
A0A024D9U6  A0A024D9U6_HEV   467 ANDVLWLSLTVAEYDQTTYGSSTNPMYVSDTATFVNVATGAQAVARSLDWSKVTLDGRPL 526
A0A024D9R2  A0A024D9R2_HEV   467 ANDVLWLSLTAAEYDQTTYGSSTNPMYVSDTVTLVNVATGAQAVARSLDWSKVTLDGRPL 526
Q8V729      Q8V729_HEV       467 ANDVLWLSLTAAEYDQTTYGSSTNPMYVSDTVTFVNVATGAQAVARSLDWSKVTLDGRPL 526
Q03500      CAPSD_HEVME      466 ANDVLWLSLTAAEYDQSTYGSSTGPVYISDSVTLVNVATGAQAVARSLDWSKVTLDGRPL 525
                                 *********.*** *:****** *:*:**:.*:*****:*.*:****:*****  *
```

## Figure 1I

```
Q8JJN2      Q8JJN2_HEV        541 MTIQQYSKTFFVLPLRGKLSFWEAGTTKAGYPYNYNTTASDQILIENAAGHRVCISTYTT 600
Q80IR5      Q80IR5_HEV        541 TTIQQYSKTFFVLPLRGKLSFWEAGTTKAGYPYNYNTTASDQILIENAAGHRVCISTYTT 600
Q806D7      Q806D7_HEV        541 TTIQQYSKTFFVLPLRGKLSFWEAGTTKAGYPYNYNTTASDQILIENAAGHRVCISTYTT 600
Q6BD83      Q6BD83_HEV        541 MTIQQYSKTFFVLPLRGKLSFWEAGTTKAGYPYNYNTTASDQILIENAAGHRVCISTYTT 600
Q6BD78      Q6BD78_HEV        541 MTIQQYSKTFFVLPLRGKLSFWEAGTTKAGYPYNYNTTASDQILIENAAGHRVCISTYTT 600
B6VC89      B6VC89_HEV        541 TTIQQYSKTFYVLPLRGKLSFWEAGTTKAGYPYNYNTTASDQILIENAAGHRVCISTYTT 600
Q6PMR3      Q6PMR3_HEV        541 TTIQQYSKTFYVLPLRGKLSFWEAGTTKAGYPYNYNTTASDQILIENAAGHRVCISTYTT 600
Q9IVZ8      CAPSD_HEVCT       539 TTIQQYSKTFYVLPLRGKLSFWEAGTTKAGYPYNYNTTASDQILIENAAGHRVCISTYTT 598
Q8JJM1      Q8JJM1_HEV        538 TTIQQYSKTFFVLPLRGKLSFWEAGTTKAGYPYNYNTTASDQILIENAAGHRVCISTYTT 597
Q2PYP3      Q2PYP3_HEV        538 TTIQQYSKTFFVLPLRGKLSFWEAGTTKAGYPYNYNTTASDQILIENAAGHRVCISTYTT 597
Q81871      CAPSD_HEVCH       527 STTQQYSKTFFVLPLRGKLSFWEAGTTKAGYPYNYNTTASDQLLVENAAGHRVAISTYTT 586
P29326      CAPSD_HEVBU       527 STIQQYSKTFFVLPLRGKLSFWEAGTTKAGYPYNYNTTASDQLLVENAAGHRVAISTYTT 586
Q6J8F7      CAPSD_HEVMG       527 TTIQQYSKTFYVLPLRGKLSFWEAGTTKAGYPYNYNTTASDQLLVENAAGHRVAISTYTT 586
Q04611      CAPSD_HEVMY       527 STIQQYPKTFFVLPLRGKLSFWEAGTTKAGYPYNYNTTASDQLLVENAAGHRVAISTYTT 586
Q68985      CAPSD_HEVHY       527 STIQQYSKIFFVLPLRGKLSFWEAGTTRPGYPYNYNTTASDQLLVENAAGHRVAISTYTT 586
Q9YLQ9      CAPSD_HEVUS       527 TTIQQYSKTFYVLPLRGKLSFWEAGTTKAGYPYNYNTTASDQILIENAAGHRVAISTYTT 586
P33426      CAPSD_HEVPA       527 STIQQYSKTFFVLPLRGKLSFWEAGTTKAGYPYNYNTTASDQLLVENAAGHRVAISTYTT 586
Q9YLR2      Q9YLR2_HEV        527 TTIQQYSKKFYVLPLRGKLSFWEAGTTKAGYPYNYNTTASDQILIENAAGHRVAISTYTT 586
Q0QC51      Q0QC51_HEV        527 TTIQQYSKTFFVLPLRGKLSFWEAGTTKAGYPYNYNTTASDQILIENAPGHRVCISTYTT 586
Q69411      Q69411_HEV        527 STIQQYSKTFFVLPLRGKLSFWEAGTTKAGYPYNYNTTASDQLLIENAAGHRVAISTYTT 586
A0A024D9U6  A0A024D9U6_HEV    527 TTIQQYSKTFYVLPLRGKLSFWEAGTTKAGYPYNYNTAASDQILIENAAGHRVAISTYTT 586
A0A024D9R2  A0A024D9R2_HEV    527 TTIQQYSKTFYVLPLRGKLSFWEAGTTKAGYPYNYNTTASDQILIENASGHRVAISTYTT 586
Q8V729      Q8V729_HEV        527 TTIQQYSKTFYVLPLRGKLSFWEAGTTKAGYPYNYNTTASDQILIENAAGHRVAISTYTT 586
Q03500      CAPSD_HEVME       526 PTVEQYSKTFFVLPLRGKLSFWEAGTTKAGYPYNYNTTASDQILIENAAGHRVAISTYTT 585

             * :** * *:*************: *******:****:*:*** ****.******
```

## Figure 1J

EP 3 383 887 B1

```
Q8JJN2       Q8JJN2_HEV     601 NLGSGPVSISAVGVLAPHSALAALEDTVDYPARAHTFDDFCPECRALGLQGCAFQSTVAE 660
Q80IR5       Q80IR5_HEV     601 NLGSGPVSISSVGVLAPHSALAALEDTVDYPARAHTFDDFCPECRTLGLQGCAFQSTVAE 660
Q806D7       Q806D7_HEV     601 NLGSGPVSISSVGVLAPHSALAALEDTVDYPARAHTFDDFCPECRTLGLQGCAFQSTVAE 660
Q6BD83       Q6BD83_HEV     601 NLGSGPVSISAVGVLAPHSALAALEDTVDYPARAHTFDDFCPECRTLGLQGCAFQSTVAE 660
Q6BD78       Q6BD78_HEV     601 NLGSGPVSISAVGVLAPHSALAALEDTVDYPARAHTFDDFCPECRALGLQGCAFQSTVAE 660
B6VC89       B6VC89_HEV     601 NLGSGPVSISAVGVLAPHSALAVLEDTVDYPARAHTFDDFCPECRALGLQGCAFQSTVAE 660
Q6PMR3       Q6PMR3_HEV     601 NLGSGPVSISAVGVLAPHSALAILEDTADYPARAHTFDDFCPECRSLGLQGCAFQSTVAE 660
Q9IVZ8       CAPSD_HEVCT    599 NLGSGPVSVSAVGVLAPHSALAALEDTADYPARAHTFDDFCPECRALGLQGCAFQSTVGE 658
Q8JJM1       Q8JJM1_HEV     598 NLGSGPVSISAVGVLAPHSALAALEDTVDYPARAHTFDDFCPECRTLGLQGCAFQSTVAE 657
Q2PYP3       Q2PYP3_HEV     598 NLGSGPVSISAVGVLAPHSALAVLEDTVDYPARAHTFDDFCPECRALGLQGCAFQSTVAE 657
Q81871       CAPSD_HEVCH    587 SLGAGPVSISAVAVLAPHSALALLEDTMDYPARAHTFDDFCPECRPLGLQGCAFQSTVAE 646
P29326       CAPSD_HEVBU    587 SLGAGPVSISAVAVLAPHSALALLEDTLDYPARAHTFDDFCPECRPLGLQGCAFQSTVAE 646
Q6J8F7       CAPSD_HEVMG    587 SLGAGPTSISAVGVLAPHSALAVLEDTVDYPARAHTFDDFCPECRTLGLQGCAFQSTIAE 646
Q04611       CAPSD_HEVMY    587 SLGAGPVSISAVAVLAPHSALALLEDTLDYPACAHTFDDFCPECRPLGLQGCAFQSTVAE 646
Q68985       CAPSD_HEVHY    587 SLGAGPVSISAVAVLGPHSALALLEDTLDYPARAHTFDDFCPECRPLGLQGCAFQSTVAE 646
Q9YLQ9       CAPSD_HEVUS    587 SLGAGPTSISAVGVLAPHSALAVLEDTIDYPARAHTFDDFCPECRTLGLQGCAFQSTIAE 646
P33426       CAPSD_HEVPA    587 SLGAGPVSISAVAVLAPHSVLALLEDTMDYPARAHTFDDFCPECRPLGLQGCAFQSTVAE 646
Q9YLR2       Q9YLR2_HEV     587 SLGAGPTSISAVGVLAPHSALAVLEDTVDYPARAHTFDDFCPECRTLGLQGCAFQSTIAE 646
Q0QC51       Q0QC51_HEV     587 NLGSGPVSISAVGVLAPHSALAALEDTVDYPARAHTFDDFCPECRALGLQGCAFQSTVAE 646
Q69411       Q69411_HEV     587 SLGAGPVAISAVAVLAPHSALALLEDTMDYPARAHTFDDFCPECRPLGLQGCAFQSTVAE 646
A0A024D9U6   A0A024D9U6_HEV 587 SLGASPTSISAVGVLAPHSALAVLEDTVDYPARAHTFDDFCPECRTLGLQGCAFQSTIAE 646
A0A024D9R2   A0A024D9R2_HEV 587 SLGAGPTSISAVGVLAPHSALAVLEDTIDYPARAHTFDDFCPECRALGFQGCAFQSTIAE 646
Q8V729       Q8V729_HEV     587 SLGAGPTSISAVGVLAPHSALAVLEDTVDYPARAHTFDDFCPECRALGLQGCAFQSTVAE 646
Q03500       CAPSD_HEVME    586 RLGAGPVAISAAAVLAPRSALALLEDTFDYPGRAHTFDDFCPECRALGLQGCAFQSTVAE 645
                                 **:.*.::*:..**.*:*.** **** ***. *********** **:*******:.*
```

Figure 1K

| Q8JJN2 | Q8JJN2_HEV | 661 | LQRLKMKVGKTREY | 674 | SEQ ID N°1 |
| Q80IR5 | Q80IR5_HEV | 661 | LQRLKMKVGKTREY | 674 | SEQ ID N°2 |
| Q806D7 | Q806D7_HEV | 661 | LQRLKMKVGKTREY | 674 | SEQ ID N°3 |
| Q6BD83 | Q6BD83_HEV | 661 | LQRLKMKVGKTREY | 674 | SEQ ID N°4 |
| Q6BD78 | Q6BD78_HEV | 661 | LQRLKMKVGKTREY | 674 | SEQ ID N°5 |
| B6VC89 | B6VC89_HEV | 661 | LQRLKMKVGKTREY | 674 | SEQ ID N°6 |
| Q6PMR3 | Q6PMR3_HEV | 661 | LQRLKMKVGKTREY | 674 | SEQ ID N°7 |
| Q9IVZ8 | CAPSD_HEVCT | 659 | LQRLKMKVGKTREY | 672 | SEQ ID N°8 |
| Q8JJM1 | Q8JJM1_HEV | 658 | LQRLKMKVGKTREY | 671 | SEQ ID N°9 |
| Q2PYP3 | Q2PYP3_HEV | 658 | LQRLKMKVGNH--- | 668 | SEQ ID N°10 |
| Q81871 | CAPSD_HEVCH | 647 | LQRLKMKVGKTREL | 660 | SEQ ID N°11 |
| P29326 | CAPSD_HEVBU | 647 | LQRLKMKVGKTREL | 660 | SEQ ID N°12 |
| Q6J8F7 | CAPSD_HEVMG | 647 | LQRLKMKVGKTRES | 660 | SEQ ID N°13 |
| Q04611 | CAPSD_HEVMY | 647 | LQRLKMKVGKTREL | 660 | SEQ ID N°14 |
| Q68985 | CAPSD_HEVHY | 647 | LQRLKMKVGKTREL | 660 | SEQ ID N°15 |
| Q9YLQ9 | CAPSD_HEVUS | 647 | LQRLKMKVGKTRES | 660 | SEQ ID N°16 |
| P33426 | CAPSD_HEVPA | 647 | LQRLKMKVGKTREL | 660 | SEQ ID N°17 |
| Q9YLR2 | Q9YLR2_HEV | 647 | LQRLKMKVGKTRES | 660 | SEQ ID N°18 |
| Q0QC51 | Q0QC51_HEV | 647 | LQRLKMKVGKTQEY | 660 | SEQ ID N°19 |
| Q69411 | Q69411_HEV | 647 | LQRLKMKVGKTREL | 660 | SEQ ID N°20 |
| A0A024D9U6 | A0A024D9U6_HEV | 647 | LQRLKMKVGKTRES | 660 | SEQ ID N°21 |
| A0A024D9R2 | A0A024D9R2_HEV | 647 | LQRLKMKVGKTRES | 660 | SEQ ID N°22 |
| Q8V729 | Q8V729_HEV | 647 | LQRLKMKVGKTRES | 660 | SEQ ID N°23 |
| Q03500 | CAPSD_HEVME | 646 | LQRLKVKVGKTREL | 659 | SEQ ID N°24 |

*****:***:

**Figure 1L**

| Q8JJN2 | Q8JJN2_HEV | 408 | QLFYSRPVVSANG | 420 |
| Q80IR5 | Q80IR5_HEV | 408 | QLFYSRPVVSANG | 420 |
| Q806D7 | Q806D7_HEV | 408 | QLFYSRPVVSANG | 420 |
| Q6BD83 | Q6BD83_HEV | 408 | QLFYSRPVVSANG | 420 |
| Q6BD78 | Q6BD78_HEV | 408 | QLFYSRPVVSANG | 420 |
| B6VC89 | B6VC89_HEV | 408 | QLFYSRPVVSANG | 420 |
| Q6PMR3 | Q6PMR3_HEV | 408 | QLFYSRPVVSANG | 420 |
| Q9IVZ8 | CAPSD_HEVCT | 406 | QLFYSRPVVSANG | 418 |
| Q8JJM1 | Q8JJM1_HEV | 405 | QLFYSRPVVSANG | 417 |
| Q2PYP3 | Q2PYP3_HEV | 405 | QLFYSRPVVSANG | 417 |
| Q81871 | CAPSD_HEVCH | 394 | QLFYSRPVVSANG | 406 |
| P29326 | CAPSD_HEVBU | 394 | QLFYSRPVVSANG | 406 |
| Q6J8F7 | CAPSD_HEVMG | 394 | QLFYSRPVVSANG | 406 |
| Q04611 | CAPSD_HEVMY | 394 | QLFYSRPVVSAHG | 406 |
| Q68985 | CAPSD_HEVHY | 394 | QLFYSRPVVSANG | 406 |
| Q9YLQ9 | CAPSD_HEVUS | 394 | QLFYSRPVVSANG | 406 |
| P33426 | CAPSD_HEVPA | 394 | QLFYSRPVVSANG | 406 |
| Q9YLR2 | Q9YLR2_HEV | 394 | QLFYSRPVVSANG | 406 |
| Q0QC51 | Q0QC51_HEV | 394 | QLFYSRPVVSANG | 406 |
| Q69411 | Q69411_HEV | 394 | QLFYSRPVVSANG | 406 |
| A0A024D9U6 | A0A024D9U6_HEV | 394 | QLFYSRPVASANG | 406 |
| A0A024D9R2 | A0A024D9R2_HEV | 394 | QLFYSRPVVSANG | 406 |
| Q8V729 | Q8V729_HEV | 394 | QLFYSRPVVSANG | 406 |
| Q03500 | CAPSD_HEVME | 393 | QLFYSRPVVSANG | 405 |

```
                                  ********.**.*
```

## Figure 1M

EP 3 383 887 B1

| Q8JJN2 | Q8JJN2_HEV | 421 | EPTVKLYTSVENAQQDKGIAIPHDIDLGESRVVIQDYDNQHEQDRPTPSPAPSRPFSVLR | 480 |
| Q80IR5 | Q80IR5_HEV | 421 | EPTVKLYTSVENAQQDKGIAIPHDIDLGESRVVIQDYDNQHEQDRPTPSPAPSRPFSVLR | 480 |
| Q806D7 | Q806D7_HEV | 421 | EPTVKLYTSVENAQQDKGIAIPHDIDLGESRVVIQDYDNQHEQDRPTPSPAPSRPFSVLR | 480 |
| Q6BD83 | Q6BD83_HEV | 421 | EPTVKLYTSVENAQQDKGIAIPHDIDLGESRVVIQDYDNQHEQDRPTPSPAPSRPFSVLR | 480 |
| Q6BD78 | Q6BD78_HEV | 421 | EPTVKLYTSVENAQQDKGIAIPHDIDLGESRVVIQDYDNQHEQDRPTPSPAPSRPFSVLR | 480 |
| B6VC89 | B6VC89_HEV | 421 | EPTVKLYTSVENAQQDKGIAIPHDIDLGESRVVIQDYDNQHEQDRPTPSPAPSRPFSVLR | 480 |
| Q6PMR3 | Q6PMR3_HEV | 421 | EPTVKLYTSVENAQQDKGIAIPHDIDLGESRVVIQDYDNQHEQDRPTPSPAPSRPFSVLR | 480 |
| Q9IVZ8 | CAPSD_HEVCT | 419 | ELTVKLYTSVENAQQDKGVAIPHDIDLGESRVVIQDYDNQHEQDRPTPSPAPSRPFSVLR | 478 |
| Q8JJM1 | Q8JJM1_HEV | 418 | EPTVKLYTSVENAQQDKGIAIPHDIDLGESRVVIQDYDNQHEQDRPTPSPAPSRPFSVLR | 477 |
| Q2PYP3 | Q2PYP3_HEV | 418 | EPTVKLYTSVENAQQDKGIAIPHDIDLGESRVGIQDYDNQHEQDRPTPSPAPSRPFSVLR | 477 |
| Q81871 | CAPSD_HEVCH | 407 | EPTVKLYTSVENAQQDKGIAIPHDIDLGESRVVIQDYDNQHEQDRPTPSPAPSRPFSVLR | 466 |
| P29326 | CAPSD_HEVBU | 407 | EPTVKLYTSVENAQQDKGIAIPHDIDLGESRVVIQDYDNQHEQDRPTPSPAPSRPFSVLR | 466 |
| Q6J8F7 | CAPSD_HEVMG | 407 | EPTVKLYTSVENAQQDKGITIPHDIDLGDSRVVIQDYDNQHEQDRPTPSPAPSRPFSVLR | 466 |
| Q04611 | CAPSD_HEVMY | 407 | EPTVKLYTSVENAQQDKGIAIPHDIDLGESRVVIQDYDNQHEQDRPTPSPAPSRPFSVLR | 466 |
| Q68985 | CAPSD_HEVHY | 407 | EPTVKLYTSVENAQQDKGIAIPNDIDLGESRVVIQDYDNQHEQDRPTPSPAPSRPFSVLR | 466 |
| Q9YLQ9 | CAPSD_HEVUS | 407 | EPTVKLYTSVENAQQDKGITIPHDIDLGDSRVVIQDYDNQHEQDRPTPSPAPSRPFSVLR | 466 |
| P33426 | CAPSD_HEVPA | 407 | EPTVKLYTSVENAQQDKGIAIPHDIDLGESRVVIQDYDNQHEQDRPTPSPAPSRPFSVLR | 466 |
| Q9YLR2 | Q9YLR2_HEV | 407 | EPTVKLYTSVENAQQDKGITIPHDIDLGDSRVVIQDYDNQHEQDRPTPSPAPSRPFSVLR | 466 |
| Q0QC51 | Q0QC51_HEV | 407 | EPTVKLYTSVENAQQDKGIAIPHDIDLGESRVVIQDYDNQHEQDRPTPSPAPSRPFSVLR | 466 |
| Q69411 | Q69411_HEV | 407 | EPTVKLYTSVENAQQDKGIAIPHDIDLGESRVVIQDYDNQHEQDRPTPSPAPSRPFSVLR | 466 |
| A0A024D9U6 | A0A024D9U6_HEV | 407 | EPTVKLYTSVENAQQDKGITIPHDIDLGDSRVVIQDYDNQHEQDRPTPSPAPSRPFSVLR | 466 |
| A0A024D9R2 | A0A024D9R2_HEV | 407 | EPTVKLYTSVENAQQDKGITIPHDIDLGDSRVVIQDYDNQHEQDRPTPSPAPSRPFSVLR | 466 |
| Q8V729 | Q8V729_HEV | 407 | EPTVKLYTSVENAQQDKGITIPHDIDLGDSRVVIQDYDNQHEQDRPTPSPAPSRPFSVLR | 466 |
| Q03500 | CAPSD_HEVME | 406 | EPTVKLYTSVENAQQDKGVAIPHDIDLGDSRVVIQDYDNQHEQDRPTPSPAPSRPFSVLR | 465 |

```
* ***************::**.*****:*** ***************************
```

**Figure 1N**

```
Q8JJN2        Q8JJN2_HEV       481 ANDVLWLSLTAAEYDQTTYGSSTNPMYVSDTVTFVNVATGAQGVSRSLDWSKVTLDGRPL 540
Q80IR5        Q80IR5_HEV       481 ANDVLWLSLTAAEYDQTTYGSSTNPMYVSDTVTFVNVATGAQGVSRSLDWSKVTLDGRPL 540
Q806D7        Q806D7_HEV       481 ANDVLWLSLTAAEYDQTTYGSSTNPMYVSDTVTFVNVATGAQGVSRSLDWSKVTLDGRPL 540
Q6BD83        Q6BD83_HEV       481 ANDVLWLSLTAAEYDQTTYGSSTNPMYVSDTVTFVNVATGAQGVSRSLDWSKVTLDGRPL 540
Q6BD78        Q6BD78_HEV       481 ANDVLWLSLTAAEYDQTTYGSSTNPMYVSDTVTFVNVATGAQGVSRSLDWSKVTLDGRPL 540
B6VC89        B6VC89_HEV       481 ANDVLWLSLTAAEYDQTTYGSSTNPMYVSDTVTFVNVATGAQGVSRSLDWSKVTLDGRPL 540
Q6PMR3        Q6PMR3_HEV       481 ANDVLWLSLTAAEYDQTTYGSSTNPMYVSDTVTFVNVATGAQGVSRSLDWSKVTLDGRPL 540
Q9IVZ8        CAPSD_HEVCT      479 ANDVLWLSLTAAEYDQTTYGSSTNPMYVSDTVTFVNVATGAQGVSRSLDWSKVTLDGRPL 538
Q8JJM1        Q8JJM1_HEV       478 ANDVLWLSLTAAEYDQTTYGSSTNPMYVSDTVTFVNVATGAQGVSRSLDWSKVTLDGRPL 537
Q2PYP3        Q2PYP3_HEV       478 ANDVLWLSLTAAEYDQTTYGSSTNPMYVSDTVTFVNVATGAQGVSRSLDWSKVTLDGRSL 537
Q81871        CAPSD_HEVCH      467 ANDVLWLSLTAAEYDQSTYGSSTGPVYVSDSVTLVNVATGAQAVARSLDWTKVTLDGRPL 526
P29326        CAPSD_HEVBU      467 ANDVLWLSLTAAEYDQSTYGSSTGPVYVSDSVTLVNVATGAQAVARSLDWTKVTLDGRPL 526
Q6J8F7        CAPSD_HEVMG      467 ANDVLWLSLTAAEYDQTTYGSSTNPMYVSDTVTLVNVATGAQAVARSLDWSKVTLDGRPL 526
Q04611        CAPSD_HEVMY      467 ANDVLWLSLTAAEYDQSTYGSSTAPVYVSDSVTLVNVATGAQAVARSLDWTKVTLDGRPL 526
Q68985        CAPSD_HEVHY      467 ANDVLWLSLTAAEYDQSTYGSSTGPVYVSDSVTLVNVATGAQAVARSLDWTKVTLDGRPL 526
Q9YLQ9        CAPSD_HEVUS      467 ANDVLWLSLTAAEYDQTTYGSSTNPMYVSDTVTLVNVATGAQAVARSLDWSKVTLDGRPL 526
P33426        CAPSD_HEVPA      467 ANDVLWLSLTAAEYDQSTYGSSTGPVYVSDSVTLVNVATGAQAVARSLDWTKVTLDGRPL 526
Q9YLR2        Q9YLR2_HEV       467 ANDVLWLSLTAAEYXQTTYGSSTNPMYVSDTVTLVNVATGAQAVARSLDWSKVTLDGRPL 526
Q0QC51        Q0QC51_HEV       467 ANDVLWLSLTAAEYDQTTYGSSTNPMYVSDTVTFVNVATGTQGVSRSLDWSKVTLDGRPL 526
Q69411        Q69411_HEV       467 ANDVLWLSLTAAEYDQSTYGSSTGPVYVSDSVTLVNVATGAQAVARSLDWTKVTLDGRPL 526
A0A024D9U6    A0A024D9U6_HEV   467 ANDVLWLSLTVAEYDQTTYGSSTNPMYVSDTATFVNVATGAQAVARSLDWSKVTLDGRPL 526
A0A024D9R2    A0A024D9R2_HEV   467 ANDVLWLSLTAAEYDQTTYGSSTNPMYVSDTVTLVNVATGAQAVARSLDWSKVTLDGRPL 526
Q8V729        Q8V729_HEV       467 ANDVLWLSLTAAEYDQTTYGSSTNPMYVSDTVTFVNVATGAQAVARSLDWSKVTLDGRPL 526
Q03500        CAPSD_HEVME      466 ANDVLWLSLTAAEYDQSTYGSSTGPVYISDSVTLVNVATGAQAVARSLDWSKVTLDGRPL 525
                               *********.*** *:****** *:*:**:.*:*****:*.*:****:******* *
```

## Figure 10

EP 3 383 887 B1

```
Q8JJN2          Q8JJN2_HEV        541 MTIQQYSKTFFVLPLRGKLSFWEAGTTKAGYPYNYNTTASDQILIENAAGHRVCISTYTT 600
Q80IR5          Q80IR5_HEV        541 TTIQQYSKTFFVLPLRGKLSFWEAGTTKAGYPYNYNTTASDQILIENAAGHRVCISTYTT 600
Q806D7          Q806D7_HEV        541 TTIQQYSKTFFVLPLRGKLSFWEAGTTKAGYPYNYNTTASDQILIENAAGHRVCISTYTT 600
Q6BD83          Q6BD83_HEV        541 MTIQQYSKTFFVLPLRGKLSFWEAGTTKAGYPYNYNTTASDQILIENAAGHRVCISTYTT 600
Q6BD78          Q6BD78_HEV        541 MTIQQYSKTFFVLPLRGKLSFWEAGTTKAGYPYNYNTTASDQILIENAAGHRVCISTYTT 600
B6VC89          B6VC89_HEV        541 TTIQQYSKTFYVLPLRGKLSFWEAGTTKAGYPYNYNTTASDQILIENAAGHRVCISTYTT 600
Q6PMR3          Q6PMR3_HEV        541 TTIQQYSKTFYVLPLRGKLSFWEAGTTKAGYPYNYNTTASDQILIENAAGHRVCISTYTT 600
Q9IVZ8          CAPSD_HEVCT       539 TTIQQYSKTFYVLPLRGKLSFWEAGTTKAGYPYNYNTTASDQILIENAAGHRVCISTYTT 598
Q8JJM1          Q8JJM1_HEV        538 TTIQQYSKTFFVLPLRGKLSFWEAGTTKAGYPYNYNTTASDQILIENAAGHRVCISTYTT 597
Q2PYP3          Q2PYP3_HEV        538 TTIQQYSKTFFVLPLRGKLSFWEAGTTKAGYPYNYNTTASDQILIENAAGHRVCISTYTT 597
Q81871          CAPSD_HEVCH       527 STTQQYSKTFFVLPLRGKLSFWEAGTTKAGYPYNYNTTASDQLLVENAAGHRVAISTYTT 586
P29326          CAPSD_HEVBU       527 STIQQYSKTFFVLPLRGKLSFWEAGTTKAGYPYNYNTTASDQLLVENAAGHRVAISTYTT 586
Q6J8F7          CAPSD_HEVMG       527 TTIQQYSKTFYVLPLRGKLSFWEAGTTKAGYPYNYNTTASDQILIENAAGHRVAISTYTT 586
Q04611          CAPSD_HEVMY       527 STIQQYPKTFFVLPLRGKLSFWEAGTTKAGYPYNYNTTASDQLLVENAAGHRVAISTYTT 586
Q68985          CAPSD_HEVHY       527 STIQQYSKIFFVLPLRGKLSFWEAGTTRPGYPYNYNTTASDQLLVENAAGHRVAISTYTT 586
Q9YLQ9          CAPSD_HEVUS       527 TTIQQYSKTFYVLPLRGKLSFWEAGTTKAGYPYNYNTTASDQILIENAAGHRVAISTYTT 586
P33426          CAPSD_HEVPA       527 STIQQYSKTFFVLPLRGKLSFWEAGTTKAGYPYNYNTTASDQLLVENAAGHRVAISTYTT 586
Q9YLR2          Q9YLR2_HEV        527 TTIQQYSKKFYVLPLRGKLSFWEAGTTKAGYPYNYNTTASDQILIENAAGHRVAISTYTT 586
Q0QC51          Q0QC51_HEV        527 TTIQQYSKTFFVLPLRGKLSFWEAGTTKAGYPYNYNTTASDQILIENAPGHRVCISTYTT 586
Q69411          Q69411_HEV        527 STIQQYSKTFFVLPLRGKLSFWEAGTTKAGYPYNYNTTASDQLLIENAAGHRVAISTYTT 586
A0A024D9U6      A0A024D9U6_HEV    527 TTIQQYSKTFYVLPLRGKLSFWEAGTTKAGYPYNYNTAASDQILIENAAGHRVAISTYTT 586
A0A024D9R2      A0A024D9R2_HEV    527 TTIQQYSKTFYVLPLRGKLSFWEAGTTKAGYPYNYNTTASDQILIENASGHRVAISTYTT 586
Q8V729          Q8V729_HEV        527 TTIQQYSKTFYVLPLRGKLSFWEAGTTKAGYPYNYNTTASDQILIENAAGHRVAISTYTT 586
Q03500          CAPSD_HEVME       526 PTVEQYSKTFFVLPLRGKLSFWEAGTTKAGYPYNYNTTASDQILIENAAGHRVAISTYTT 585
                                      *  :** *  *:*************:  *******:****:*:*** ****.******
```

**Figure 1P**

EP 3 383 887 B1

```
Q8JJN2       Q8JJN2_HEV      601 NLGSGPVSISAVGVLAPHSALAALEDTVDYPARAHTFDDFCPECRALGLQGCAFQSTVAE 660
Q80IR5       Q80IR5_HEV      601 NLGSGPVSISSVGVLAPHSALAALEDTVDYPARAHTFDDFCPECRTLGLQGCAFQSTVAE 660
Q806D7       Q806D7_HEV      601 NLGSGPVSISSVGVLAPHSALAALEDTVDYPARAHTFDDFCPECRTLGLQGCAFQSTVAE 660
Q6BD83       Q6BD83_HEV      601 NLGSGPVSISAVGVLAPHSALAALEDTVDYPARAHTFDDFCPECRTLGLQGCAFQSTVAE 660
Q6BD78       Q6BD78·HEV      601 NLGSGPVSISAVGVLAPHSALAALEDTVDYPARAHTFDDFCPECRALGLQGCAFQSTVAE 660
B6VC89       B6VC89_HEV      601 NLGSGPVSISAVGVLAPHSALAVLEDTVDYPARAHTFDDFCPECRALGLQGCAFQSTVAE 660
Q6PMR3       Q6PMR3_HEV      601 NLGSGPVSISAVGVLAPHSALAILEDTADYPARAHTFDDFCPECRSLGLQGCAFQSTVAE 660
Q9IVZ8       CAPSD_HEVCT     599 NLGSGPVSVSAVGVLAPHSALAALEDTADYPARAHTFDDFCPECRALGLQGCAFQSTVGE 658
Q8JJM1       Q8JJM1_HEV      598 NLGSGPVSISAVGVLAPHSALAALEDTVDYPARAHTFDDFCPECRTLGLQGCAFQSTVAE 657
Q2PYP3       Q2PYP3_HEV      598 NLGSGPVSISAVGVLAPHSALAVLEDTVDYPARAHTFDDFCPECRALGLQGCAFQSTVAE 657
Q81871       CAPSD_HEVCH     587 SLGAGPVSISAVAVLAPHSALALLEDTMDYPARAHTFDDFCPECRPLGLQGCAFQSTVAE 646
P29326       CAPSD_HEVBU     587 SLGAGPVSISAVAVLAPHSALALLEDTLDYPARAHTFDDFCPECRPLGLQGCAFQSTVAE 646
Q6J8F7       CAPSD_HEVMG     587 SLGAGPTSISAVGVLAPHSALAVLEDTVDYPARAHTFDDFCPECRTLGLQGCAFQSTIAE 646
Q04611       CAPSD_HEVMY     587 SLGAGPVSISAVAVLAPHSALALLEDTLDYPACAHTFDDFCPECRPLGLQGCAFQSTVAE 646
Q68985       CAPSD_HEVHY     587 SLGAGPVSISAVAVLGPHSALALLEDTLDYPARAHTFDDFCPECRPLGLQGCAFQSTVAE 646
Q9YLQ9       CAPSD_HEVUS     587 SLGAGPTSISAVGVLAPHSALAVLEDTIDYPARAHTFDDFCPECRTLGLQGCAFQSTIAE 646
P33426       CAPSD_HEVPA     587 SLGAGPVSISAVAVLAPHSVLALLEDTMDYPARAHTFDDFCPECRPLGLQGCAFQSTVAE 646
Q9YLR2       Q9YLR2_HEV      587 SLGAGPTSISAVGVLAPHSALAVLEDTVDYPARAHTFDDFCPECRTLGLQGCAFQSTIAE 646
Q0QC51       Q0QC51_HEV      587 NLGSGPVSISAVGVLAPHSALAALEDTVDYPARAHTFDDFCPECRALGLQGCAFQSTVAE 646
Q69411       Q69411_HEV      587 SLGAGPVAISAVAVLAPHSALALLEDTMDYPARAHTFDDFCPECRPLGLQGCAFQSTVAE 646
A0A024D9U6   A0A024D9U6_HEV  587 SLGASPTSISAVGVLAPHSALAVLEDTVDYPARAHTFDDFCPECRTLGLQGCAFQSTIAE 646
A0A024D9R2   A0A024D9R2_HEV  587 SLGAGPTSISAVGVLAPHSALAVLEDTIDYPARAHTFDDFCPECRALGFQGCAFQSTIAE 646
Q8V729       Q8V729_HEV      587 SLGAGPTSISAVGVLAPHSALAVLEDTVDYPARAHTFDDFCPECRALGLQGCAFQSTVAE 646
Q03500       CAPSD_HEVME     586 RLGAGPVAISAAVLAPRSALALLEDTFDYPGRAHTFDDFCPECRALGLQGCAFQSTVAE 645

                                 **:.*.::*:..**.*:*.** **** ***. *********** **:********:.*
```

Figure 1Q

| | | | | |
|---|---|---|---|---|
| Q8JJN2 | Q8JJN2_HEV | 661 LQRLKMKVGKTREY 674 | | SEQ ID N°28 |
| Q80IR5 | Q80IR5_HEV | 661 LQRLKMKVGKTREY 674 | | SEQ ID N°29 |
| Q806D7 | Q806D7_HEV | 661 LQRLKMKVGKTREY 674 | | SEQ ID N°30 |
| Q6BD83 | Q6BD83_HEV | 661 LQRLKMKVGKTREY 674 | | SEQ ID N°31 |
| Q6BD78 | Q6BD78_HEV | 661 LQRLKMKVGKTREY 674 | | SEQ ID N°32 |
| B6VC89 | B6VC89_HEV | 661 LQRLKMKVGKTREY 674 | | SEQ ID N°33 |
| Q6PMR3 | Q6PMR3_HEV | 661 LQRLKMKVGKTREY 674 | | SEQ ID N°34 |
| Q9IVZ8 | CAPSD_HEVCT | 659 LQRLKMKVGKTREY 672 | | SEQ ID N°35 |
| Q8JJM1 | Q8JJM1_HEV | 658 LQRLKMKVGKTREY 671 | | SEQ ID N°36 |
| Q2PYP3 | Q2PYP3_HEV | 658 LQRLKMKVGNH--- 668 | | SEQ ID N°37 |
| Q81871 | CAPSD_HEVCH | 647 LQRLKMKVGKTREL 660 | | SEQ ID N°26 |
| P29326 | CAPSD_HEVBU | 647 LQRLKMKVGKTREL 660 | | SEQ ID N°38 |
| Q6J8F7 | CAPSD_HEVMG | 647 LQRLKMKVGKTRES 660 | | SEQ ID N°39 |
| Q04611 | CAPSD_HEVMY | 647 LQRLKMKVGKTREL 660 | | SEQ ID N°40 |
| Q68985 | CAPSD_HEVHY | 647 LQRLKMKVGKTREL 660 | | SEQ ID N°41 |
| Q9YLQ9 | CAPSD_HEVUS | 647 LQRLKMKVGKTRES 660 | | SEQ ID N°42 |
| P33426 | CAPSD_HEVPA | 647 LQRLKMKVGKTREL 660 | | SEQ ID N°43 |
| Q9YLR2 | Q9YLR2_HEV | 647 LQRLKMKVGKTRES 660 | | SEQ ID N°44 |
| Q0QC51 | Q0QC51_HEV | 647 LQRLKMKVGKTQEY 660 | | SEQ ID N°45 |
| Q69411 | Q69411_HEV | 647 LQRLKMKVGKTREL 660 | | SEQ ID N°46 |
| A0A024D9U6 | A0A024D9U6_HEV | 647 LQRLKMKVGKTRES 660 | | SEQ ID N°47 |
| A0A024D9R2 | A0A024D9R2_HEV | 647 LQRLKMKVGKTRES 660 | | SEQ ID N°48 |
| Q8V729 | Q8V729_HEV | 647 LQRLKMKVGKTRES 660 | | SEQ ID N°49 |
| Q03500 | CAPSD_HEVME | 646 LQRLKVKVGKTREL 659 | | SEQ ID N°50 |

*****:***:

**Figure 1R**

Figure 2

| | ORF2-REF | | | | M | ORF2-MUT | | | |
|---|---|---|---|---|---|---|---|---|---|
| CHAUFFE | + | + | – | – | | + | + | – | – |
| REDUIT | + | – | + | – | | + | – | + | – |

dimère covalent

dimère non-covalent

monomère

## Figure 3

Figure 4

Figure 5

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9314116 A
- WO 9508632 A
- WO 0122916 A
- FR 2781802
- WO 9508000 A
- FR 1561596

**Littérature non-brevet citée dans la description**

- **EMERSON, S. U. ; PURCELL, R. H.** *Hepeviridœ,* 2007
- **BOERSMA YL ; PLÜTCKTHUN A.** *Designed Ankyrin Repeat ProteINS,* 2011
- **BOERSMA YL ; PLÜCKTHUN A.** *Curr. Opin. Biotechnol,* 2011, vol. 22, 849-857
- **ELLINGTON AD ; SZOSTAK JW.** *Nature,* 1990, vol. 346, 818-822
- Hepatitis E Virus. **EMERSON, S. U. ; PURCELL, R. H.** Fields Virology. Lippincott Williams & Wilkins, 2007, 3047-3058
- **FIELDS ; NOBLE.** *Int J Pept Protein Res.,* 1990, vol. 35, 161-214
- **MENG J et al.** *Virology,* 2001, vol. 288, 203-211
- **MERRIFIELD.** *J Am Chem Soc.,* 1963, vol. 85, 2149-2154
- **RIDDELL M.A. et al.** *Journal of Virology,* 2000, vol. 74 (17), 8011-8017